# EUROPÄISCHE PATENTANMELDUNG

(11) **EP 0 702 004 A2**
(43) Veröffentlichungstag der Anmeldung: **20.03.1996**
(21) Anmeldenummer: 95113964.1
(22) Anmeldetag: 06.09.1995
(51) Int. Cl.: C07D 215/08, C07D 215/38, C07D 265/36, C07D 279/16, A61K 31/435, A61K 31/535, A61K 31/54

(54) **2,9-Diamino- und 2-amino-8-carbamoyl-4-hydroxy-alkansäureamid-derivative**

(30) Priorität: 15.09.1994 CH 2816/94
(71) Anmelder: CIBA-GEIGY AG, CH-4002 Basel (CH)
(72) Erfinder: Rasetti, Vittorio, Dr., CH-4125 Riehen (CH); Rüeger, Heinrich, Dr., CH-4112 Flüh (CH); Maibaum, Jürgen Klaus, Dr., D-79576 Weil-Haltingen (DE); Mah, Robert, Dr., CH-4056 Basel (CH); Grütter, Markus, Prof. Dr., CH-4146 Hochwald (CH); Cohen, Nissim Claude, Dr., F-68300 Village-Neuf (FR)

(57) **Zusammenfassung**

Verbindungen der Formel I 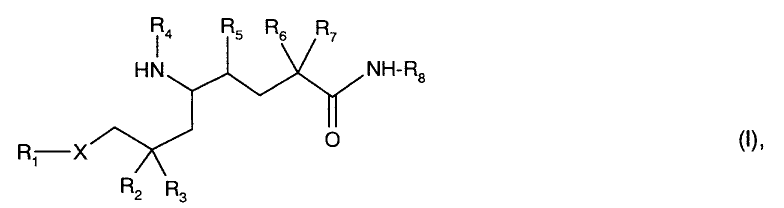
worin R₁ Arylamino, N-Aryl-N-(niederalkoxyniederalkyl)-amino, N-Aryl-N-arylniederalkylamino oder über ein Ringstickstoffatom gebundenes Heterocyclyl bedeutet, X für eine Carbonyl- oder Methylengruppe steht, R₂ und R₃ unabhängig voneinander Wasserstoff oder Niederalkyl bedeuten oder zusammen mit dem Kohlenstoffatom, mit dem sie verbunden sind, einen Cycloalkylidenrest darstellen, R₄ Wasserstoff, Niederalkyl, Niederalkanoyl oder Niederalkoxycarbonyl ist, R₅ Hydroxy, Niederalkanoyloxy oder Niederalkoxycarbonyloxy bedeutet, R₆ Wasserstoff, Niederalkyl, Niederalkenyl, Niederalkinyl, Cycloalkyl, Cycloalkylniederalkyl, Arylniederalkyl oder Heteroarylniederalkyl mit 5 bis 7 Ringatomen im Heteroarylring und R₇ Wasserstoff oder Niederalkyl bedeutet oder R₆ und R₇ zusammen mit dem Kohlenstoffatom, mit dem sie verbunden sind, einen Cycloalkylidenrest darstellen und R₈ einen aliphatischen, cycloaliphatisch-aliphatischen oder heteroarylaliphatischen Rest bedeutet,und ihre Salze können als Arzneimittelwirkstoffe für die Behandlung von Bluthochdruck verwendet werden.

## Beschreibung

Die Erfindung betrifft Verbindungen der Formel I 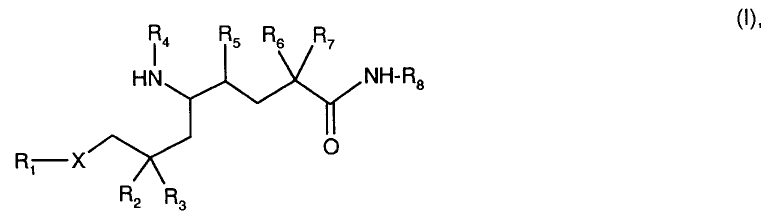
worin
R₁ Arylamino, N-Aryl-N-(niederalkoxyniederalkyl)-amino, N-Aryl-N-arylniederalkyl-amino oder über ein Ringstickstoffatom gebundenes Heterocyclyl bedeutet,
X für eine Carbonyl- oder Methylengruppe steht,
R₂ und R₃ unabhängig voneinander Wasserstoff oder Niederalkyl bedeuten oder zusammen mit dem Kohlenstoffatom, mit dem sie verbunden sind, einen Cycloalkylidenrest darstellen,
R₄ Wasserstoff, Niederalkyl, Niederalkanoyl oder Niederalkoxycarbonyl ist,
R₅ Hydroxy, Niederalkanoyloxy oder Niederalkoxycarbonyloxy bedeutet,
R₆ Wasserstoff, Niederalkyl, Niederalkenyl, Niederalkinyl, Cycloalkyl, Cycloalkylniederalkyl, Arylniederalkyl oder Heteroarylniederalkyl mit 5 bis 7 Ringatomen im Heteroarylring und R₇ Wasserstoff oder Niederalkyl bedeutet oder
R₆ und R₇ zusammen mit dem Kohlenstoffatom, mit dem sie verbunden sind, einen Cycloalkylidenrest darstellen und
R₈ einen aliphatischen, cycloaliphatisch-aliphatischen oder heteroarylaliphatischen Rest bedeutet,
und ihre Salze, Verfahren zur Herstellung der erfindungsgemäßen Verbindungen, diese enthaltende pharmazeutische Präparate und ihre Verwendung als Arzneimittelwirkstoffe.

Die durch R₂, R₃, R₄ NH-, R₅, R₆ und R₇ substituierten sowie weitere gegebenenfalls in Verbindungen der Formel I vorliegende asymmetrische Kohlenstoffatome können die R-, S- oder R,S-Konfiguration haben. Dementsprechend können die vorliegenden Verbindungen als Isomerengemische oder als reine Isomeren, insbesondere als Diastereomerengemische, Enantiomerenpaare oder reine Enantiomere auftreten. Analoges gilt für im Rest R₁ gegebenenfalls vorhandene asymmetrische C-Atome.

Aryl und Aryl in Arylamino, Arylniederalkyl, N-Aryl-N-(niederalkoxyniederalkyl)-amino N-Aryl-N-arylniederalkyl-amino enthält allgemein 1 - 14, vorzugsweise 6 - 10 Kohlenstoffatome und ist beispielsweise Phenyl, Indenyl, z.B. 2- oder 4-Indenyl, oder Naphthyl, z.B. 1- oder 2-Naphthyl. Bevorzugt ist Aryl mit 6 - 10 Kohlenstoffatomen, insbesondere Phenyl oder 1- oder 2-Naphthyl. Die genannten Reste können unsubstituiert oder beispielsweise durch Niederalkyl, Hydroxy, Niederalkoxy, Carbamoylniederalkoxy, Niederalkylcarbamoylniederalkoxy, Diniederalkylcarbamoylniederalkoxy, Amino, Niederalkyl- oder Diniederalkylamino, Carboxy, Niederalkoxycarbonyl, Carbamoyl, Sulfamoyl, Niederalkansulfonyl, Halogen, Nitro, Phenyl, 5- oder 6-gliedriges Heteroaryl, als Heteroatom enthaltend 1 Stickstoff-, Schwefel- oder Sauerstoffatom, 2 N-Atome, 1 N-Atom und 1 S-Atom oder 1 N-Atom und 1 O-Atom, wie Pyridyl, und/oder durch Cyano ein oder mehrfach, z.B. ein- oder zweifach, substituiert sein, wobei der Substituent in irgendeiner Stellung, z.B. in o-, m- oder p-Stellung des Phenylrestes oder in 3- oder 4-Stellung des 1- oder 2-Naphthylrestes stehen kann und auch mehrere gleiche oder verschiedene Substituenten vorhanden sein können.

Arylamino ist beispielsweise unsubstituiertes oder im Phenyl- oder Naphthylteil wie vorstehend angegeben substituiertes Anilino oder 1- oder 2-Naphthylamino.

Aliphatische Reste sind beispielsweise Niederalkyl, Niederalkoxyniederalkyl, Aminoniederalkyl, N-Niederalkylaminoniederalkyl, N,N-Diniederalkylaminoniederalkyl, N,N-Niederalkylenaminoniederalkyl, Carbamoylniederalkyl, N-Niederalkylcarbamoylniederalkyl, N,N-Diniederalkylcarbamoylniederalkyl, Cyanoniederalkyl

Heteroarylniederalkylreste mit 5 bis 7 Ringatomen im Heteroarylring, das ein Ringstickstoffatom enthält und ein weiteres Ringheteroatom, ausgewählt aus Sauerstoff, Schwefel oder Stickstoff, enthalten kann, bedeutet,

Arylniederalkyl ist beispielsweise unsubstituiertes oder im Phenylteil wie vorstehend angegeben substituiertes Phenylniederalkyl.

Cycloalkyl ist beispielsweise 3- bis 8-, insbesondere 3- bis 6-gliedriges Cycloalkyl, wie Cyclopropyl, Cyclobutyl, Cyclopentyl oder Cyclohexyl.

Cycloalkyliden ist beispielsweise 3- bis 8-, insbesondere 3- bis 6-gliedriges Cycloalkyliden, wie Cyclopropyliden, Cyclobutyliden, Cyclopentyliden oder Cyclohexyliden.

Cycloalkylniederalkyl ist beispielsweise 3- bis 8-, insbesondere 3- bis 6-gliedriges Cycloalkylniederalkyl, wie Cyclopropyl-, Cyclobutyl-, Cyclopentyl- oder Cyclohexylniederalkyl.

Heteroarylniederalkyl mit 5 bis 7 Ringatomen im Heteroarylring, sowie solches, das ein Ringstickstoffatom enthält und ein weiteres Ringheteroatom, ausgewählt aus Sauerstoff, Schwefel oder Stickstoff, enthalten kann, ist beispielsweise Pyridylniederalkyl oder Imidazolylniederalkyl.

N-Aryl-N-(niederalkoxyniederalkyl)-amino ist beispielsweise unsubstituiertes oder im Phenyl- oder Naphthylteil wie vorstehend angegeben substituiertes N-Phenyl- oder N-Naphthyl)-N-(niederalkoxyniederalkyl)-amino.

N-Aryl-N-arylniederalkyl-amino ist beispielsweise unsubstituiertes oder im Phenyl- oder Naphthylteil wie vorstehend angegeben substituiertes N-Phenyl- oder N-Naphthyl-N-(phenylniederalkyl)-amino.

Über ein Ringstickstoffatom gebundenes Heterocyclyl mit 4 bis 8 Ringatome weist insbesondere 5 bis 7 Ringatome auf und kann mit 1 oder 2 ankondensierten Phenyl- oder Cycloalkylresten kondensiert sein. Zu nennen sind beispielsweise Pyrrolidino, Piperidino, Piperazino, Morpholino, Thiomorpholino, Indolin-1-yl, Isoindolin-2-yl, 2,3-Dihydrobenzimidazol-1-yl, 1,2,3,4-Tetrahydrochinol-1-yl, 1,2,3,4-Tetrahydroisochinol-2-yl, 1,2,3,4-Tetrahydro-1,3-benzdiazin-1-yl oder -3-yl, 1,2,3,4-Tetrahydro-1,4-benzdiazin-1-yl, 3,4-Dihydro-2H-1,4-benzoxazin-4-yl, 3,4-Dihydro-2H-1,4-benzthiazin-4-yl, 3,4-Dihydro-2H-1,3-benzthiazin-1-yl, 3,4,5,6,7,8-Hexahydro-2H-1,4-benzoxazin-4-yl, 3,4,5,6,7,8-Hexahydro-2H-1,4-benzthiazin-4-yl, 2,3,4,5-Tetrahydro-1H-1-benz[6,7-b]azepin-1-yl und 5,6-Dihydrophenanthridin-5-yl. Bevorzugt sind benzokondensierte, über ein Stickstoffatom gebundene 5- bis 7-gliedrige Aza-, Diaza-, Azoxa- und Azathiacycloalkenylreste, insbesondere Indolin-1-yl, 1,2,3,4-Tetrahydrochinolin-1-yl, 1,2,3,4-Tetrahydroisochinolin-2-yl, 1,2,3,4-Tetrahydro-1,3-benzdiazin-1-yl, 1,2,3,4-Tetrahydro-1,4-benzdiazin-1-yl, 3,4-Dihydro-2H-1,4-benzoxazin-4-yl, 3,4-Dihydro-2H-1,4-benzthiazin-4-yl, 3,4-Dihydro-2H-1,3-benzthiazin-1-yl und 2,3,4,5-Tetrahydro-1H-1-benz[6,7-b]azepin-1-yl.

Die genannten Reste können unsubstituiert oder N-substituiert, S,S-substituiert und/oder C-substituiert sein, wobei insgesamt bis zu 3, insbesondere 1, 2 oder 3 Substituenten vorhanden sein können.

Als N-Substituenten kommen beispielsweise Niederalkyl, Niederalkanoyl, Niederalkoxycarbonyl oder Niederalkansulfonyl in Betracht. S-Substituenten sind beispielsweise 1 oder vorzugsweise 2 Oxygruppen. C-Substituenten sind beispielsweise Niederalkyl, Hydroxyniederalkyl, Niederalkoxyniederalkyl, Niederalkenyloxyniederalkyl, Naphthoxyniederalkyl, Phenyloxyniederalkyl, Phenylniederalkoxyniederalkyl, Niederalkanoyloxyniederalkyl, Benzoyloxyniederalkyl, Niederalkoxycarbonyloxyniederalkyl, Phenyloxycarbonyloxyniederalkyl, Phenylniederalkoxycarbonyloxyniederalkyl, Aminoniederalkyl, N-Niederalkylaminoniederalkyl, N,N-Diniederalkylaminoniederalkyl, Carbamoylniederalkyl, Niederalkanoylaminoniederalkyl, Benzoylaminoniederalkyl, Niederalkoxycarbonylaminoniederalkyl, Niederalkoxycarbonylniederalkyl, Niederalkoxyniederalkoxyniederalkyl, Niederalkylthioniederalkoxyniederalkyl, N-Niederalkoxyiminoniederalkyl, Cycloalkoxyniederalkyl, Cycloalkylniederalkoxyniederalkyl, Niederalkenyl, Niederalkenyloxy, Niederalkoxyniederalkenyl, Niederalkinyl, Niederalkinyloxy, Niederalkanoyl, Oxo, Hydroxy, Niederalkoxy, Carbamoylniederalkoxy, N-Niederalkylcarbamoylniederalkoxy, N-Niederalkylcarbamoyloxy, N,N-Diniederalkylcarbamoyloxy, Niederalkoxyniederalkoxy, Niederalkylthioniederalkoxy, Niederalkanoyloxy, Benzoyloxy, N-Niederalkylcarbamoyl, Amino, N-Niederalkylamino, N,N-Diniederalkylamino, Niederalkanoylamino, Benzoylamino, Cycloalkylcarbonylamino, Cycloalkylniederalkanoylamino, Niederalkoxycarbonylniederalkylamino, Niederalkenyloxycarbonylamino, Niederalkoxyniederalkoxycarbonylamino, Niederalkoxyniederalkanoylamino, N-Niederalkylcarbamoylamino, N,N-Diniederalkylcarbamoylamino, N-Niederalkanoyl-N-niederalkyl-amino, Niederalkoxycarbonylamino, N-Niederalkoxycarbonyl-N-niederalkylamino, N,N-Niederalkylenamino, N,N-(1-Oxoniederalkylen)amino, N,N-(1-Oxo2-oxa-niederalkylen)amino, Carboxy, Niederalkoxycarbonyl, Phenylniederalkoxycarbonyl, Phenyloxycarbonyl, Pyrrolidinylcarbonyl, Piperidinylcarbonyl, Piperazinylcarbonyl, Morpholinylcarbonyl, Thiomorpholinylcarbonyl, S,S-Dioxothiomorpholin-4-ylcarbonyl, Cyano, Carbamoyl, N,N-Diniederalkylcarbamoyl, N-Niederalkenylcarbamoyl, N-Cycloalkylcarbamoyl, N-Cycloalkylniederalkylcarbamoyl, N-Hydroxyniederalkylcarbamoyl, N-Niederalkoxyniederalkylcarbamoyl, N-Carboxyniederalkylcarbamoyl, Carbamoylniederalkylcarbamoyl, Niederalkoxycarbonylniederalkylcarbamoyl, Phenyl, Dioxolan-2-yl, Oxazol-2-yl, Oxazolin-2-yl, Oxazolidin-2-yl, Nitro, Sulfamoyl, Niederalkansulfonyl, Phosphono, Niederalkanphosphono, Diniederalkylphosphono und Halogen.

Besonders bevorzugte Reste R₁ sind bicyclische Reste der Formel Ia 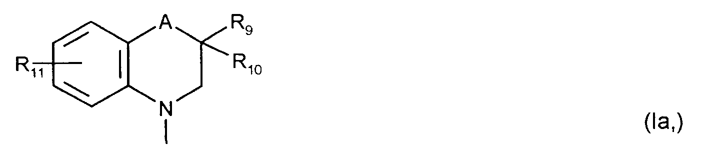
worin A eine direkte Bindung, Methylen, Ethylen, Imino, Oxy oder Thio darstellt, R₉ für Niederalkoxyniederalkyl, Niederalkenyloxyniederalkyl, Niederalkoxyniederalkoxyniederalkyl, Niederalkoxycarbonylaminoniederalkyl, N-Niederalkoxyiminoniederalkyl, Phenyl, Niederalkoxycarbonyl, Cyano, Carbamoyl, N-Niederalkylcarbamoyl, N-(Niederalkoxyniederalkyl)carbamoyl, Niederalkoxy, Niederalkoxyniederalkoxy, Niederalkanoyloxy, Benzoyloxy, Niederalkanoylamino, Niederalkoxycarbonylamino, 3- bis 6-gliedriges Cycloalkylcarbonylamino, N-(Niederalkoxyniederalkanoyl)amino, N-(Niederalkylcarbamoyl)amino, N,N-(1-Oxoniederalkylen)amino, oder N,N-(1-Oxo2-oxa-niederalkylen)amino steht, R₁₀ Wasserstoff oder Niederalkyl bedeutet und R₁₁ Wasserstoff oder Halogen ist.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

Aminoniederalkyl ist beispielsweise Amino-C₁-C₄-alkyl, wie Aminomethyl, 2-Aminoethyl, 3-Aminopropyl oder 4-Aminobutyl.

Benzoylaminoniederalkyl ist beispielsweise Benzoylamino-C₁-C₄-alkyl, wie Benzoylaminomethyl, 2-Benzoylaminoethyl, 3-Benzoylaminopropyl oder 4-Benzoylaminobutyl.

Benzoyloxyniederalkyl ist beispielsweise Benzoyloxy-C₁-C₄-alkyl, wie Benzoyloxymethyl, 2-Benzoyloxyethyl, 3-Benzoyloxypropyl oder 4-Benzoyloxybutyl, insbesondere Benzoyloxymethyl.

Carbamoylniederalkoxy ist beispielsweise Carbamoyl-C₁-C₄-alkoxy, wie Carbamoylmethoxy, 2-Carbamoylethoxy, 3-Carbamoylpropyloxy oder 4-Carbamoylbutyloxy.

Carbamoylniederalkyl ist beispielsweise Carbamoyl-C₁-C₄-alkyl, wie 2-(N-Methylcarbamoyl)ethyl, Carbamoylmethyl, 2-Carbamoylethyl, 3-Carbamoylpropyl oder 4-Carbamoylbutyl.

N-(Carbamoylniederalkyl)carbamoyl ist beispielsweise N-(Carbamoyl-C₁-C₄-alkyl)carbamoyl, wie N-(Carbamoylmethyl)carbamoyl, N-(2-Carbamoylethyl)carbamoyl, N-(3-Carbamoylpropyl)carbamoyl oder N-(4-Carbamoylbutyl)carbamoyl.

Cycloalkoxyniederalkyl ist beispielsweise C₃-C₇-Cycloalkoxy-C₁-C₄-alkyl, wie Cyclopropyl-C₁-C₄-alkyl, Cyclopentyl-C₁-C₄-alkyl oder Cyclohexyl-C₁-C₄-alkyl, insbesondere Cyclopropylmethyl.

Cycloalkylcarbonylamino ist beispielsweise N-C₃-C₇-Cycloalkylcarbonylamino, wie Cyclopropylcarbonylamino, Cyclopentylcarbonylamino oder Cyclohexylcarbonylamino, insbesondere N-Cyclopropylcarbonylamino.

Cycloalkylniederalkanoylamino ist beispielsweise N-(C₃-C₇-Cycloalkyl-C₁-C₄alkanoyl)amino, wie Cyclopropyl-C₁-C₄-alkanoyl)amino, Cyclopentyl-C₁-C₄-alkanoyl)amino oder Cyclohexyl-C₁-C₄-alkanoyl)amino, insbesondere Cyclopropylacetylamino.

Cycloalkylniederalkoxyniederalkyl ist beispielsweise C₃-C₇-Cycloalkyl-C₁-C₄-alkoxyC₁-C₄-alkyl, wie Cyclopropyl-C₁-C₄-alkoxy-C₁-C₄-alkyl, Cyclopentyl-C₁-C₄-alkoxyC₁-C₄-alkyl oder Cyclohexyl-C₁-C₄-alkoxy-C₁-C₄-alkyl, insbesondere Cyclopropylmethoxymethyl.

Cycloalkylniederalkyl ist beispielsweise C₃-C₇-Cycloalkyl-C₁-C₄-alkyl, wie Cyclopropyl-C₁-C₄-alkyl, Cyclopentyl-C₁-C₄-alkyl oder Cyclohexyl-C₁-C₄-alkyl, insbesondere Cyclopropylmethyl.

Hydroxyniederalkyl ist beispielsweise Hydroxy-C₁-C₄-alkyl, wie Hydroxymethyl, 2-Hydroxyethyl, 3-Hydroxypropyl oder 4-Hydroxybutyl, insbesondere 2-Hydroxyethyl.

Imidazolylniederalkyl ist beispielsweise 4-Imidazolylmethyl.

Naphthyloxyniederalkyl ist beispielsweise Naphthyloxy-C₁-C₄-alkyl, insbesondere Naphth-1-yl- oder Naphth-2-yl-C₁-C₄-alkoxy, wobei C₁-C₄-Alkyl beispielsweise Methyl, Ethyl, Propyl oder Butyl bedeutet, wie Naphth-1-yl- oder Naphth-2-yloxymethyl.

Naphthylniederalkanoyl ist beispielsweise Naphthyl-C₁-C₄-alkanoyl, wie Naphth-1-yl-oder Naphth-2-ylacetyl.

Naphthylniederalkoxycarbonyl ist beispielsweise Naphthyl-C₁-C₄-alkoxycarbonyl, insbesondere Naphth-1-yl- oder Naphth-2-yl-C₁-C₄-alkoxycarbonyl, wobei C₁-C₄-Alkyl beispielsweise Methyl, Ethyl, Propyl oder Butyl bedeutet, wie Naphth-1-yl- oder Naphth-2-yloxycarbonyl.

Naphthylniederalkyl ist beispielsweise Naphthyl-C₁-C₄-alkyl, wie Naphth-1-yl- oder Naphth-2-ylmethyl oder 1- oder 2-Naphthylethyl.

Niederalkanoyl ist beispielsweise C₁-C₇-Alkanoyl, insbesondere C₁-C₄-Alkanoyl, wie Formyl, Acetyl, Propionyl, Butyryl oder Pivaloyl. Niederalkanoyl R₄ ist insbesondere Formyl, Acetyl, Propionyl, Butyryl, Isobutyryl oder Pivaloyl.

Niederalkanoylamino ist beispielsweise C₁-C₇-Alkanoylamino, insbesondere C₁-C₄-Alkanoylamino, wie Acetylamino, Propionylamino, Butyrylamino oder Pivaloylamino.

Niederalkanoylaminoniederalkyl ist beispielsweise C₁-C₇-Alkanoylamino-C₁-C₄-alkyl, insbesondere C₁-C₄-Alkanoylamino-C₁-C₄-alkyl, wie Formylaminomethyl, Acetylaminomethyl, Propionylaminomethyl, Butyrylaminomethyl, Pivaloylaminomethyl, 2-Formylaminoethyl, 2-Acetylaminoethyl, 2-Propionylaminomethyl, 2-Butyrylaminoethyl oder 2-Pivaloylaminoethyl.

Niederalkanoyloxy ist beispielsweise C₁-C₇-Alkanoyloxy, insbesondere C₁-C₄-Alkanoyloxy, wie Acetyloxy, Propionyloxy, Butyryloxy oder Pivaloyloxy.

Niederalkanoyloxyniederalkyl ist beispielsweise C₁-C₇-Alkanoyloxy-C₁-C₄-alkyl, insbesondere C₁-C₄-Alkanoyloxy-C₁-C₄-alkyl, wie Acetyloxy-C₁-C₄-alkyl, wobei C₁-C₄-Alkyl beispielsweise Methyl, Ethyl, Propyl oder Butyl bedeutet, insbesondere Propionyloxymethyl.

N-Niederalkanoyl-N-niederalkyl-amino ist beispielsweise N-(C₁-C₇-Alkanoyl)-N(C₁-C₄-alkyl)-amino, insbesondere N-(C₁-C₄-Alkanoyl)-N-(C₁-C₄-alkyl)-amino, wie N-Formyl-N-methylamino, N-Acetyl-N-methyl-amino, N-Propionyl-N-methyl-amino oder N-Butyryl-N-methylamino.

Niederalkansulfonyl ist beispielsweise C₁-C₇-Alkansulfonyl, insbesondere C₁-C₄-Alkansulfonyl, wie Methansulfonyl, Ethansulfonyl, Propansulfonyl, Propan-2-sulfonyl, Butansulfonyl, 2-Methylpropansulfony, Butan-2-sulfonyl oder 2,2-Dimethylethansulfonyl. Niederalkansulfonyl R₄ ist insbesondere Methan-, Ethan-, Propan-, 2-Methanpropan-, Butan-2-, 2,2-Dimethylethan-, n-Butan-, n-Pentan-, Isopentan-, Neopentan-, Tertiärpentan-, n-Hexan-, Isohexan- oder n-Heptansulfonyl.

Niederalkenyl ist beispielsweise C₂-C₇-Alkenyl, insbesondere C₃-C₅-Alkenyl, wie Allyl. N-Niederalkenylcarbamoyl ist beispielsweise N-C₂-C₇-Alkenylcarbamoyl, insbesondere N-C₃-C₅-Alkenylcarbamoyl, wie Allylcarbamoyl.

Niederalkenyloxy ist beispielsweise C₂-C₇-Alkenyloxy, insbesondere C₃-C₅-Alkenyloxy, wie Allyloxy oder Methallyloxy.

N-Niederalkenylcarbamoyl ist beispielsweise N-C₂-C₇-Alkenylcarbamoyl, insbesondere N-C₃-C₅-Alkenylcarbamoyl, wie N-Allylcarbamoyl oder N-Methallylcarbamoyl.

Niederalkenyloxycarbonylamino ist beispielsweise C₂-C₇-Alkenyloxycarbonylamino, insbesondere C₃-C₅-Alkenyloxycarbonylamino, wie Allyloxycarbonylamino oder Methallyloxycarbonylamino.

Niederalkenyloxyniederalkyl ist beispielsweise C₂-C₇-Alkenyloxy-C₁-C₄-alkyl, insbesondere C₃-C₅-Alkenyloxy-C₁-C₄-alkyl, wie Allyloxy-C₁-C₄-alkyl, insbesondere Allyloxymethyl.

Niederalkinyl ist beispielsweise C₃-C₇-Alkinyl, insbesondere C₃-C₅-Alkinyl, wie Propargyl.

Niederalkinyloxy ist beispielsweise C₃-C₇-Alkinyloxy, insbesondere C₃-C₅-Alkinyloxy, wie Propargyloxy.

Niederalkoxy ist beispielsweise C₁-C₇-Alkoxy, insbesondere C₁-C₄-Alkoxy, wie Methoxy, Ethoxy, Propyloxy, Isopropyloxy, Butyloxy, Isobutyloxy, Sekundärbutyloxy oder Tertiärbutyloxy, kann aber auch eine C₅-C₇-Alkoxy-, wie Pentyloxy-, Hexyloxy-oder Heptyloxygruppe sein.

Niederalkoxycarbonyl ist beispielsweise C₁-C₇-Alkoxycarbonyl, insbesondere C₁-C₄-Alkoxycarbonyl, wie Methoxycarbonyl, Ethoxycarbonyl, Propyloxycarbonyl, Isopropyloxycarbonyl, Butyloxycarbonyl, Isobutyloxycarbonyl, Sekundärbutyloxycarbonyl oder Tertiärbutyloxycarbonyl.

Niederalkoxycarbonylamino ist beispielsweise C₁-C₇-Alkoxycarbonylamino, insbesondere C₁-C₄-Alkoxycarbonylamino, wie Methoxycarbonylamino, Ethoxycarbonylamino, Propyloxycarbonylamino, Isopropyloxycarbonylamino oder Butyloxycarbonylamino.

Niederalkoxycarbonylaminoniederalkyl ist beispielsweise C₁-C₇-Alkoxycarbonylamino-C₁-C₄-alkyl, insbesondere C₁-C₄-Alkoxycarbonylamino-C₁-C₄-alkyl, wie Methoxycarbonylamino-C₁-C₄-alkyl, Ethoxycarbonylamino-C₁-C₄-alkyl, Propyloxycarbonylamino-C₁-C₄-alkyl, Isopropyloxycarbonylamino-C₁-C₄-alkyl, Butyloxycarbonylamino-C₁-C₄-alkyl, Isobutyloxycarbonylamino-C₁-C₄-alkyl, Sekundärbutyloxycarbonylamino-C₁-C₄-alkyl oder Tertiärbutyloxycarbonylamino-C₁-C₄-alkyl, wobei C₁-C₄-Alkyl beispielsweise Methyl, Ethyl, Propyl oder Butyl bedeutet, wie Ethoxycarbonylaminomethyl.

Niederalkoxycarbonylniederalkyl ist beispielsweise C₁-C₇-Alkoxycarbonyl-C₁-C₄-alkyl, insbesondere C₁-C₄-Alkoxycarbonyl-C₁-C₄-alkyl, wie Methoxycarbonyl-C₁-C₄-alkyl, Ethoxycarbonyl-C₁-C₄-alkyl, Propyloxycarbonyl-C₁-C₄-alkyl, Isopropyloxycarbonyl-C₁-C₄-alkyl, Butyloxycarbonyl-C₁-C₄-alkyl, Isobutyloxycarbonyl-C₁-C₄-alkyl, Sekundärbutyloxycarbonyl-C₁-C₄-alkyl oder Tertiärbutyloxycarbonyl-C₁-C₄-alkyl, wobei C₁-C₄-Alkyl beispielsweise Methyl, Ethyl, Propyl oder Butyl bedeutet, z.B. Methoxycarbonylmethyl oder Ethoxycarbonylmethyl.

Niederalkoxycarbonylniederalkylamino ist beispielsweise C₁-C₇-Alkoxycarbonyl-C₁-C₄-alkylamino, insbesondere C₁-C₄-Alkoxycarbonyl-C₁-C₄-alkylamino, wie Methoxycarbonyl-C₁-C₄-alkylamino, Ethoxycarbonyl-C₁-C₄-alkylamino, Propyloxycarbonyl-C₁-C₄-alkylamino, Isopropyloxycarbonyl-C₁-C₄-alkylamino, Butyloxycarbonyl-C₁-C₄-alkylamino, Isobutyloxycarbonyl-C₁-C₄-alkylamino, Sekundärbutyloxycarbonyl-C₁-C₄-alkylamino oder Tertiärbutyloxycarbonyl-C₁-C₄-alkylamino, wobei C₁-C₄-Alkyl beispielsweise Methyl, Ethyl, Propyl oder Butyl bedeutet.

N-(Niederalkoxycarbonylniederalkyl)carbamoyl ist beispielsweise N-(C₁-C₇-Alkoxycarbonyl-C₁-C₄-alkyl)carbamoyl, insbesondere N-(C₁-C₄-Alkoxycarbonyl-C₁-C₄-alkyl)carbamoyl, wie N-(Methoxycarbonyl-C₁-C₄-alkyl)carbamoyl, N-(Ethoxycarbonyl-C₁-C₄-alkyl)carbamoyl, N-(Propyloxycarbonyl-C₁-C₄-alkyl)carbamoyl, N-(Isopropyloxycarbonyl-C₁-C₄-alkyl)carbamoyl, N-(Butyloxycarbonyl-C₁-C₄-alkyl)carbamoyl oder N-(Tertiärbutyloxycarbonyl-C₁-C₄-alkyl)carbamoyl, wobei C₁-C₄-Alkyl beispielsweise Methyl, Ethyl, Propyl oder Butyl bedeutet, insbesondere N-(Methoxy- oder Ethoxycarbonylmethyl)carbamoyl.

Niederalkoxycarbonyloxyniederalkyl ist beispielsweise C₁-C₇-Alkoxycarbonyloxy-C₁-C₄-alkyl, insbesondere C₁-C₄-Alkoxycarbonyloxy-C₁-C₄-alkyl, wie Methoxycarbonyloxy-C₁-C₄-alkyl, Ethoxycarbonyloxy-C₁-C₄-alkyl, Propyloxycarbonyloxy-C₁-C₄-alkyl, Isopropyloxycarbonyloxy-C₁-C₄-alkyl, Butyloxycarbonyloxy-C₁-C₄-alkyl, oder Tertiärbutyloxycarbonyloxy-C₁-C₄-alkyl, wobei C₁-C₄-Alkyl beispielsweise Methyl, Ethyl, Propyl oder Butyl bedeutet, insbesondere Propyloxycarbonyloxymethyl.

N-Niederalkoxycarbonyl-N-niederalkyl-amino ist beispielsweise C₁-C₇-Alkoxycarbonyl-N-C₁-C₄-alkyl-amino, insbesondere C₁-C₄-Alkoxycarbonyl-N-C₁-C₄-alkyl-amino, wie Methoxycarbonyl-N-C₁-C₄-alkyl-amino, Ethoxycarbonyl-N-C₁-C₄-alkyl-amino, Propyloxycarbonyl-N-C₁-C₄-alkyl-amino, Isopropyloxycarbonyl-N-C₁-C₄-alkyl-amino, Butyloxycarbonyl-N-C₁-C₄-alkyl-amino, oder Tertiärbutyloxycarbonyl-N-C₁-C₄-alkylamino, wobei C₁-C₄-Alkyl beispielsweise Methyl, Ethyl, Propyl oder Butyl bedeutet, insbesondere N-(Methoxy- oder Ethoxycarbonyl)-N-amino.

Niederalkoxyiminoniederalkyl ist beispielsweise C₁-C₄-Alkoxyimino-C₁-C₄-alkyl, wie Methoxyimino-C₁-C₄-alkyl, Ethoxyimino-C₁-C₄-alkyl, Propoxyimino-C₁-C₄-alkyl oder Butoxyimino-C₁-C₄-alkyl, wobei C₁-C₄-Alkyl beispielsweise Methyl oder Ethyl bedeutet, insbesondere Methoxyiminomethyl, Ethoxyiminomethyl oder Propoxyiminomethyl.

Niederalkoxyniederalkanoylamino ist beispielsweise C₁-C₄-Alkoxy-C₁-C₄-alkanoylamino, wie Methoxy-C₁-C₄-alkanoylamino, Ethoxy-C₁-C₄-alkanoylamino, Propyloxy-C₁-C₄-alkanoylamino, Isopropyloxy-C₁-C₄-alkanoylamino oder Butyloxy-C₁-C₄-alkanoylamino, wobei C₁-C₄-Alkanoyl beispielsweise Acetyl, Propionyl oder Butyryl bedeutet.

Niederalkoxyniederalkenyl ist beispielsweise C₁-C₄-Alkoxy-C₃-C₅-alkenyl, wie Methoxy-C₃-C₅-alkenyl, Ethoxy-C₃-C₅-alkenyl, Propyloxy-C₃-C₅-alkenyl, Isopropyloxy-C₃-C₅-alkenyl oder Butyloxy-C₃-C₅-alkenyl, wobei C₃-C₅-Alkenyl beispielsweise Allyl oder Methallyl bedeutet, insbesondere 3-Ethoxyallyl.

Niederalkoxyniederalkoxy ist beispielsweise C₁-C₇-Alkoxy-C₁-C₄-alkoxy, insbesondere C₁-C₄-Alkoxy-C₁-C₄-alkoxy, wie Methoxy-C₁-C₄-alkoxy, Ethoxy-C₁-C₄-alkoxy, Propyloxy-C₁-C₄-alkoxy, Isopropyloxy-C₁-C₄-alkoxy, Butyloxy-C₁-C₄-alkoxy, Isobutyloxy-C₁-C₄-alkoxy, Sekundärbutyloxy-C₁-C₄-alkoxy oder Tertiärbutyloxy-C₁-C₄-alkoxy, wobei C₁-C₄-Alkoxy beispielsweise Methoxy, Ethoxy, Propyloxy oder Butyloxy bedeutet, insbesondere Methoxy- oder Ethoxymethoxy, 2-(Methoxy)- oder 2-(Ethoxy)ethoxy.

Niederalkoxyniederalkoxycarbonylamino ist beispielsweise C₁-C₇-Alkoxy-C₁-C₄-alkoxycarbonylamino, insbesondere C₁-C₄-Alkoxy-C₁-C₄-alkoxycarbonylamino, wie Methoxy-C₁-C₄-alkoxycarbonylamino, Ethoxy-C₁-C₄-alkoxycarbonylamino, Propyloxy-C₁-C₄alkoxycarbonylamino, Isopropyloxy-C₁-C₄-alkoxycarbonylamino, Butyloxy-C₁-C₄alkoxycarbonylamino, Isobutyloxy-C₁-C₄-alkoxycarbonylamino, Sekundärbutyloxy-C₁-C₄-alkoxycarbonylamino oder Tertiärbutyloxy-C₁-C₄-alkoxycarbonylamino, wobei C₁-C₄-Alkoxy beispielsweise Methoxy, Ethoxy, Propyloxy oder Butyloxy bedeutet, insbesondere N-(2-Ethoxyethoxycarbonyl)amino.

Niederalkoxyniederalkoxyniederalkyl ist beispielsweise C₁-C₇-Alkoxy-C₁-C₄-alkoxy-C₁-C₄-alkyl, insbesondere C₁-C₄-Alkoxy-C₁-C₄-alkoxy-C₁-C₄-alkyl, wie Methoxy-C₁-C₄-alkoxy-C₁-C₄-alkyl, Ethoxy-C₁-C₄-alkoxy-C₁-C₄-alkyl, Propyloxy-C₁-C₄-alkoxy-C₁-C₄-alkyl, Isopropyloxy-C₁-C₄-alkoxy-C₁-C₄-alkyl oder Butyloxy-C₁-C₄-alkoxy-C₁-C₄-alkyl, wobei C₁-C₄-Alkoxy beispielsweise Methoxy, Ethoxy, Propyloxy oder Butyloxy und -C₁-C₄-Alkyl beispielsweise Methyl, Ethyl, Propyl oder Butyl bedeutet, insbesondere 2-Methoxyethoxymethyl.

Niederalkoxyniederalkyl ist beispielsweise C₁-C₇-Alkoxy-C₁-C₄-alkyl, insbesondere C₁-C₄-Alkoxy-C₁-C₄-alkyl, wie Methoxy-C₁-C₄-alkyl, Ethoxy-C₁-C₄-alkyl, Propyloxy-C₁-C₄-alkyl, Isopropyloxy-C₁-C₄-alkyl, Butyloxy-C₁-C₄-alkyl, Isobutyloxy-C₁-C₄-alkyl, Sekundärbutyloxy-C₁-C₄-alkyl oder Tertiärbutyloxy-C₁-C₄-alkyl, wobei C₁-C₄-Alkyl beispielsweise Methyl, Ethyl, Propyl oder Butyl bedeutet, insbesondere Ethoxymethyl.

Niederalkoxyniederalkyl ist beispielsweise C₁-C₇-Alkoxy-C₁-C₄-alkyl, insbesondere C₁-C₄-Alkoxy-C₁-C₄-alkyl, wie Methoxy-C₁-C₄-alkyl, Ethoxy-C₁-C₄-alkyl, Propyloxy-C₁-C₄-alkyl, Isopropyloxy-C₁-C₄-alkyl, Butyloxy-C₁-C₄-alkyl, Isobutyloxy-C₁-C₄-alkyl, Sekundärbutyloxy-C₁-C₄-alkyl oder Tertiärbutyloxy-C₁-C₄-alkyl, wobei C₁-C₄-Alkyl beispielsweise Methyl, Ethyl, Propyl oder Butyl bedeutet.

N-Niederalkoxyniederalkylcarbamoyl ist beispielsweise N-C₁-C₇-Alkoxy-C₁-C₄-alkylcarbamoyl, insbesondere C₁-C₄-Alkoxy-C₁-C₄-alkylcarbamoyl, wie Methoxy-C₁-C₄-alkylcarbamoyl, Ethoxy-C₁-C₄-alkylcarbamoyl, Propyloxy-C₁-C₄-alkylcarbamoyl, Isopropyloxy-C₁-C₄-alkylcarbamoyl, Butyloxy-C₁-C₄-alkylcarbamoyl, Isobutyloxy-C₁-C₄-alkylcarbamoyl, Sekundärbutyloxy-C₁-C₄-alkyl oder Tertiärbutyloxy-C₁-C₄-alkylcarbamoyl, wobei C₁-C₄-Alkyl beispielsweise Methyl, Ethyl, Propyl oder Butyl bedeutet, insbesondere N-(2-Propyloxyethoyl)carbamoyl.

Niederalkyl ist verzweigt oder unverzweigt und bedeutet beispielsweise C₁-C₇-Alkyl, insbesondere C₁-C₄-Alkyl, wie Methyl, Ethyl, n-Propyl, Isopropyl, n-Butyl, Isobutyl, Sekundärbutyl, Tertiärbutyl, n-Pentyl, Isopentyl, Neopentyl, Tertiärpentyl, n-Hexyl, Isohexyl oder n- Heptyl. Niederalkyl R₂ bzw. R₃ ist vorzugsweise Methyl, Ethyl oder Propyl.

N-Niederalkylamino ist beispielsweise N-C₁-C₇-Alkylamino, insbesondere C₁-C₄-Alkylamino, wie Methylamino, Ethylamino, Propylamino, Butylamino, Isobutylamino, Sekundärbutylamino oder Tertiärbutylamino.

N,N-Diniederalkylamino ist beispielsweise N,N-Di-C₁-C₄-alkylamino, wie N,N-Dimethylamino, N,N-Diethylamino, N,N-Dipropylamino, N-Methyl-N-ethyl-amino oder N-Methyl-N-propylamino.

N-Niederalkylaminoniederalkyl ist beispielsweise N-C₁-C₄-Alkylamino-C₁-C₄-alkyl, wie N-Methylamino-C₁-C₄-alkyl, N-Ethylamino-C₁-C₄-alkyl, N-Propylamino-C₁-C₄-alkyl oder N-Butylamino-C₁-C₄-alkyl, wobei C₁-C₄-Alkyl beispielsweise Methyl oder Ethyl bedeutet, insbesondere N-Methylaminomethyl, N-Ethylaminomethyl oder N-Propylaminomethyl.

N-Niederalkylcarbamoyl ist beispielsweise N-C₁-C₄-Alkylcarbamoyl, wie N-Methylcarbamoyl, N-Ethylcarbamoyl, N-Propylcarbamoyl oder N-Butylcarbamoyl.

N-Niederalkylcarbamoylamino ist beispielsweise N-C₁-C₄-Alkylcarbamoylamino, wie N-Methylcarbamoylamino, N-Ethylcarbamoylamino, N-Propylcarbamoylamino oder N-Butylcarbamoylamino.

N-Niederalkylcarbamoylniederalkoxy ist beispielsweise N-C₁-C₄-Alkylcarbamoyl-C₁-C₄alkoxy, wie N-Methylcarbamoyl-C₁-C₄-alkoxy, N-Ethylcarbamoyl-C₁-C₄-alkoxy, N-Propylcarbamoyl-C₁-C₄-alkoxy oder N-Butylcarbamoyl-C₁-C₄-alkoxy, wobei C₁-C₄-Alkoxy beispielsweise Methoxy oder Ethoxy bedeutet, insbesondere N-Methylcarbamoylmethoxy, N-Ethylcarbamoylmethoxy, 2-(N-Methylcarbamoyl)ethoxy oder N-Propylcarbamoylmethoxy.

N-Niederalkylcarbamoyloxy ist beispielsweise N-C₁-C₄-Alkylcarbamoyloxy, wie N-Methylcarbamoyloxy, N-Ethylcarbamoyloxy, N-Propylcarbamoyloxy oder N-Butylcarbamoyloxy.

N-Phenyl-N-(niederalkoxyniederalkyl)-amino ist beispielsweise N-Phenyl-N(C₁-C₄-alkoxy-C₁-C₄-alkyl)-amino, wie N-Phenyl-N-(methoxy-C₁-C₄-alkyl)-amino, N-Phenyl-N-(ethoxy-C₁-C₄-alkyl)-amino, N-Phenyl-N-(propyloxy-C₁-C₄-alkyl)-amino, N-Phenyl-N-(isopropyloxy-C₁-C₄-alkyl)-amino oder N-Phenyl-N-(butyloxy-C₁-C₄-alkyl)amino, wobei C₁-C₄-Alkyl beispielsweise Methyl, Ethyl, Propyl oder Butyl bedeutet, insbesondere N-Phenyl-N-(ethoxymethyl)amino.

N-Phenyl-N-niederalkyl-amino ist beispielsweise N-Phenyl-N-C₁-C₄-alkyl-amino, wie N-Phenyl-N-methyl-amino, N-Phenyl-N-ethyl-amino, N-Phenyl-N-propyl-amino, N-Phenyl-N-isopropyl-amino oder N-Phenyl-N-butyl-amino, insbesondere N-Phenyl-N-methyl-amino.

N-Phenyl-N-(phenylniederalkyl)-amino ist beispielsweise N-Phenyl-N-(phenyl-C₁-C₄-alkyl)-amino, wie N-Phenyl-N-benzyl-amino, N-Phenyl-N-(2-phenylethyl)-amino, N-Phenyl-N-(3-phenylpropyl)-amino oder N-Phenyl-N-(4-phenylbutyl)-amino, insbesondere N-Phenyl-N-(2-phenylethyl)-amino.

Niederalkanphosphono ist beispielsweise C₁-C₇-Alkanphosphono, insbesondere C₁-C₄-Alkanphosphono, wie Methanphosphono, Ethanphosphono, Propanphosphono, Propan-2-phosphono, Butanphosphono, 2-Methylpropanphosphono,
Butan-2-phosphono oder 2,2-Dimethylethanphosphono.

Niederalkylthioniederalkoxy ist beispielsweise C₁-C₇-Alkylthio-C₁-C₄-alkoxy, insbesondere C₁-C₄-Alkylthio-C₁-C₄-alkoxy, wie Methylthio-C₁-C₄-alkoxy, Ethylthio-C₁-C₄-alkoxy, Propylylthio-C₁-C₄-alkoxy, Isopropylylthio-C₁-C₄-alkoxy, Butylylthio-C₁-C₄-alkoxy, Isobutylylthio-C₁-C₄-alkoxy, Sekundärbutylylthio-C₁-C₄-alkoxy oder Tertiärbutylylthio-C₁-C₄-alkoxy, wobei C₁-C₄-Alkoxy beispielsweise Methoxy, Ethoxy, Propyloxy oder Butyloxy bedeutet, insbesondere Methylthio- oder Ethylthiomethoxy, 2-(Methylthio)- oder 2-(Methylthio)ethoxy.

Niederalkylthioniederalkoxyniederalkyl ist beispielsweise C₁-C₇-Alkylthio-C₁-C₄-alkoxy-C₁-C₄-alkyl, insbesondere C₁-C₄-Alkylthio-C₁-C₄-alkoxy-C₁-C₄-alkyl, wie Methylthio-C₁-C₄-alkoxy-C₁-C₄-alkyl, Ethylthio-C₁-C₄-alkoxy-C₁-C₄-alkyl, Propylylthio-C₁-C₄-alkoxy-C₁-C₄-alkyl, Isopropylylthio-C₁-C₄-alkoxy-C₁-C₄-alkyl oder Butylylthio-C₁-C₄-alkoxy-C₁-C₄-alkyl, wobei C₁-C₄-Alkoxy beispielsweise Methoxy, Ethoxy, Propyloxy oder Butyloxy und -C₁-C₄-Alkyl beispielsweise Methyl, Ethyl, Propyl oder Butyl bedeutet, insbesondere 2-Methylthioethoxymethyl.

N,N-Diniederalkylamino ist beispielsweise N,N-Di-C₁-C₄-alkylamino, wie N,N-Dimethylamino, N,N-Diethylamino, N,N-Dipropylamino, N-Methyl-N-ethyl-amino oder N-Methyl-N-propyl-amino.

N,N-Diniederalkylaminoniederalkyl ist beispielsweise N,N-Di-C₁-C₄-alkylamino-C₁-C₄-alkyl, wie N,N-Dimethylamino-C₁-C₄-alkyl, N,N-Diethylamino-C₁-C₄-alkyl, N,N-Dipropylamino-C₁-C₄-alkyl, N-Methyl-N-ethyl-amino oder N-Methyl-N-propylamino.

N,N-Diniederalkylcarbamoyl ist beispielsweise N,N-Di-C₁-C₄-alkylcarbamoyl, wie N,N-Dimethylcarbamoyl, N,N-Diethylcarbamoyl, N,N-Dipropylcarbamoyl, N-Methyl-N-ethylcarbamoyl oder N-Methyl-N-propyl-carbamoyl.

N,N-Diniederalkylcarbamoylamino ist beispielsweise N,N-Di-C₁-C₄-alkylcarbamoylamino, wie N,N-Dimethylcarbamoylamino, N,N-Diethylcarbamoylamino, N,N-Dipropylcarbamoylamino, N-Methyl-N-ethyl-carbamoylamino oder N-Methyl-N-propyl-carbamoylamino.

N,N-Diniederalkylcarbamoyloxy ist beispielsweise N,N-Di-C₁-C₄-alkylcarbamoyloxy, wie N,N-Dimethylcarbamoyloxy, N,N-Diethylcarbamoyloxy, N,N-Dipropylcarbamoyloxy, N-Methyl-N-ethyl-carbamoyloxy oder N-Methyl-N-propyl-carbamoyloxy.

N,N-Diniederalkylcarbamoylniederalkoxy ist beispielsweise N,N-Dimethylcarbamoyl-C₁-C₄-alkoxy, N,N-Diethylcarbamoyl-C₁-C₄-alkoxy, N,N-Dipropylcarbamoyl-C₁-C₄alkoxy, N-Methyl-N-ethyl-carbamoyl-C₁-C₄-alkoxy oder N-Methyl-N-propylcarbamoyl-C₁-C₄-alkoxy, wobei C₁-C₄-Alkoxy beispielsweise Methoxy, Ethoxy, Propyloxy oder Butyloxy bedeutet, insbesondere N,N-Dimethylcarbamoylmethoxy.

N,N-Niederalkylenamino weist beispielsweise 3 bis 8, insbesondere 5 bis 7, Ringglieder auf und ist beispielsweise 5- oder 6-gliedriges N,N-Niederalkylenamino, wie Pyrrolidino. Piperidino oder Hexahydroazepino.

N,N-Niederalkylenaminoniederalkyl weist beispielsweise 3 bis 8, insbesondere 5 bis 7, Ringglieder auf und ist beispielsweise Pyrrolidino-C₁-C₄-alkyl oder Piperidino-C₁-C₄-alkyl, -, wobei C₁-C₄-Alkyl beispielsweise Methyl, Ethyl, Propyl oder Butyl bedeutet, insbesondere Piperidinomethylmethyl.

N,N-(1-Oxoniederalkylen)amino weist beispielsweise 3 bis 8, insbesondere 5 bis 7, Ringglieder auf und ist beispielsweise 5- oder 6-gliedriges N,N-(1-Oxoniederalkylen)amino, wie 2-Oxopyrrolidin-1-yl.

N,N-(1-Oxo2-oxa-niederalkylen)amino weist beispielsweise 3 bis 8, insbesondere 5 bis 7, Ringglieder auf und ist beispielsweise 5- oder 6-gliedriges N,N-(1-Oxo2-oxa-niederalkylen)amino, wie 2-Oxo-oxazolidin-3-yl.

N,N-(Azaniederalkylen)amino weist beispielsweise 3 bis 8, insbesondere 5 bis 7, Ringglieder auf und ist beispielsweise 5- oder 6-gliedriges N,N-(Azaniederalkylen)amino, wie Piperazino, N'-(C₁-C₄-Alkyl)piperazino, wie N'-Methylpiperazino, oder N'-(C₁-C₄-Alkanoyl)piperazino, wie N'-Acetylpiperazino.

N,N-(Azaniederalkylenamino)niederalkyl weist beispielsweise 3 bis 8, insbesondere 5 bis 7, Ringglieder auf und ist beispielsweise 5- oder 6-gliedriges N,N-(Azaniederalkylenamino)niederalkyl, wie Piperazino-C₁-C₄-alkyl, N'-(C₁-C₄-Alkyl)piperazino-C₁-C₄-alkyl, wie N'-Methylpiperazino-C₁-C₄-alkyl, oder N'-(C₁-C₄-Alkanoyl)piperazino-C₁-C₄-alkyl, wie N'-Acetylpiperazino-C₁-C₄-alkyl, wobei C₁-C₄-Alkyl beispielsweise Methyl, Ethyl, Propyl oder Butyl bedeutet, insbesondere N'-Methylpiperazinomethyl.

N,N-(Oxaniederalkylen)amino weist beispielsweise 3 bis 8, insbesondere 5 bis 7, Ringglieder auf und ist beispielsweise 5- oder 6-gliedriges N,N-(Oxaniederalkylen)amino, wie Morpholino.

N,N-(Oxaniederalkylenamino)niederalkyl weist beispielsweise 3 bis 8, insbesondere 5 bis 7, Ringglieder auf und ist 5- oder 6-gliedriges N,N-(Oxaniederalkylenamino)niederalkyl, wie Morpholino-C₁-C₄-alkyl, wobei C₁-C₄-Alkyl beispielsweise Methyl, Ethyl, Propyl oder Butyl bedeutet, insbesondere Morpholinomethyl.

N,N-(Thianiederalkylen)amino weist beispielsweise 3 bis 8, insbesondere 5 bis 7, Ringglieder auf und ist beispielsweise 5- oder 6-gliedriges N,N-(Thianiederalkylen)amino, wie Thiomorpholino oder S,S-Dioxythiomorpholino.

N,N-(Thianiederalkylenamino)niederalkyl weist beispielsweise 3 bis 8, insbesondere 5 bis 7, Ringglieder auf und ist beispielsweise 5- oder 6-gliedriges N,N-(Thianiederalkylenamino)niederalkyl, wie Thiomorpholino-C₁-C₄-alkyl oder S,S-Dioxythiomorpholino-C₁-C₄-alkyl, wobei C₁-C₄-Alkyl beispielsweise Methyl, Ethyl, Propyl oder Butyl bedeutet, insbesondere Thiomorpholinomethyl.

N,N-(2-Oxoniederalkylen)amino weist beispielsweise 3 bis 8, insbesondere 5 bis 7, Ringglieder auf und ist beispielsweise 2-Oxopyrrolidino.

N-Carboxyniederalkylcarbamoyl ist beispielsweise N-Carboxy-C₁-C₄-alkylcarbamoyl, wie N-Carboxymethylcarbamoyl, N-(2-Carboxyethyl)carbamoyl oder N-(3-Carboxypropyl)carbamoyl.

N-Cycloalkylcarbamoyl ist beispielsweise N-C₃-C₇-Cycloalkylcarbamoyl wie Cyclopropyl-, Cyclopentyl- oder Cyclohexylcarbamoyl, insbesondere N-Cyclopropylcarbamoyl.

N-Cycloalkylniederalkylcarbamoyl ist beispielsweise N-(C₃-C₇-Cycloalkyl-C₁-C₄-alkyl)carbamoyl wie N-(Cyclopropyl-C₁-C₄-alkyl)carbamoyl, N-(Cyclopentyl-C₁-C₄-alkyl)carbamoyl oder N-(Cyclohexyl-C₁-C₄-alkyl)carbamoyl, insbesondere N-(Cyclopropylmethyl)carbamoyl.

N-Hydroxyniederalkylcarbamoyl ist beispielsweise N-Hydroxy-C₁-C₄-alkylcarbamoyl, wie N-Hydroxymethylcarbamoyl, N-(2-Hydroxyethyl)carbamoyl, N-(3-Hydroxypropyl)carbamoyl oder N-(4-Hydroxybutyl)carbamoyl, insbesondere N-(2-Hydroxyethyl)carbamoyl.

Phenylniederalkoxycarbonyl ist beispielsweise Phenyl-C₁-C₇-alkoxycarbonyl, insbesondere Phenyl-C₁-C₄-alkoxycarbonyl, wobei C₁-C₄-Alkyl beispielsweise Methyl, Ethyl, Propyl oder Butyl bedeutet, wie Benzyloxycarbonyl.

Phenylniederalkoxycarbonyl R₄ ist insbesondere Benzyloxycarbonyl, 1- oder 2-Naphthylmethoxycarbonyl oder 9-Fluorenylmethoxycarbonyl.

Phenylniederalkoxycarbonyloxyniederalkyl ist beispielsweise Phenyl-C₁-C₇-alkoxycarbonyloxy-C₁-C₄-alkyl, insbesondere Phenyl-C₁-C₄-alkoxycarbonyloxy-C₁-C₄-alkyl, wobei C₁-C₄-Alkyl jeweils beispielsweise Methyl, Ethyl, Propyl oder Butyl bedeutet, wie Benzyloxycarbonyloxymethyl.

Phenylniederalkanoyl ist beispielsweise Phenyl-C₁-C₄-alkanoyl, wobei C₁-C₄-Alkanoyl beispielsweise Acetyl bedeutet, insbesondere Phenylacetyl.

Phenylniederalkyl ist beispielsweise Phenyl-C₁-C₇-alkyl, insbesondere Phenyl-C₁-C₄-alkyl, wobei C₁-C₄-Alkyl beispielsweise Methyl bedeutet, insbesondere Benzyl oder 2-Phenethyl.

Phenyloxyniederalkyl ist beispielsweise Phenyloxy-C₁-C₇-alkyl, insbesondere Phenyloxy-C₁-C₄-alkyl, wobei C₁-C₄-Alkyl beispielsweise Methyl bedeutet, insbesondere Benzyloxy oder 2-Phenethoxy.

Pyridylniederalkyl ist beispielsweise Pyridyl-C₁-C₄-alkyl, insbesondere Pyrid-2-yl-C₁-C₄-alkyl, wobei C₁-C₄-Alkyl beispielsweise Methyl bedeutet, insbesondere Pyrid-2-ylmethyl oder 2-(Pyrid-2-yl)ethyl.

Salze von Verbindungen mit salzbildenden Gruppen sind insbesondere Säureadditionssalze, Salze mit Basen oder bei Vorliegen mehrerer salzbildender Gruppen gegebenenfalls auch Mischsalze oder innere Salze.

Salze sind in erster Linie die pharmazeutisch verwendbaren oder nicht-toxischen Salze von Verbindungen der Formel I.

Solche Salze werden beispielsweise von Verbindungen der Formel I mit einer sauren Gruppe, z.B. einer Carboxy- oder Sulfogruppe, gebildet und sind beispielsweise deren Salze mit geeigneten Basen, wie nicht-toxische, von Metallen der Gruppe Ia, Ib, IIa und IIb des Periodensystems der Elemente abgeleitete Metallsalze, z.B. Alkalimetall-, insbesondere Lithium-, Natrium- oder Kaliumsalze, Erdalkalimetallsalze, beispielsweise Magnesium- oder Calciumsalze, ferner Zinksalze oder Ammoniumsalze, auch solche Salze, welche mit organischen Aminen, wie gegebenenfalls durch Hydroxy substituierten Mono-, Di oder Trialkylaminen, insbesondere Mono-, Di- oder Triniederalkylaminen, oder mit quaternären Ammoniumbasen gebildet werden, z.B. Methyl-, Ethyl-, Diethyl- oder Triethylamin, Mono-, Bis- oder Tris-(2-hydroxyniederalkyl)-aminen, wie Ethanol-, Diethanol- oder Triethanolamin, Tris-(hydroxymethyl)-methylamin oder 2-Hydroxytert.-butylamin, N,N-Diniederalkyl-N-(hydroxyniederalkyl)-aminen, wie N,N-Dimethyl-N-(2-hydroxyethyl)-amin, oder N-Methyl-D-glucamin, oder quaternären Ammoniumhydroxiden, wie Tetrabutylammoniumhydroxid. Die Verbindungen der Formel I mit einer basischen Gruppe, z.B. einer Aminogruppe, können Säureadditionssalze bilden, z.B. mit geeigneten anorganischen Säuren, z.B. Halogenwasserstoffsäure, wie Salzsäure, Bromwasserstoffsäure, Schwefelsäure unter Ersatz eines oder beider Protonen, Phosphorsäure unter Ersatz eines oder mehrerer Protonen, z.B. ortho-Phosphorsäure oder meta-Phosphorsäure, oder Pyrophosporsäure unter Ersatz eines oder mehrerer Protonen, oder mit organischen Carbon-, Sulfon-, Sulfo- oder Phosphonsäuren oder N-substituierter Sulfaminsäuren, z.B. Essigsäure, Propionsäure, Glykolsäure, Bernsteinsäure, Maleinsäure, Hydroxymaleinsäure, Methylmaleinsäure, Fumarsäure, Äpfelsäure, Weinsäure, Gluconsäure, Glucarsäure, Glucuronsäure, Zitronensäure, Benzoesäure, Zimtsäure, Mandelsäure, Salicylsäure, 4-Aminosalicylsäure, 2-Phenoxybenzoesäure, 2-Acetoxybenzoesäure, Embonsäure, Nicotinsäure, Isonicotinsäure, ferner Aminosäuren, wie z.B. den weiter vorn genannten α-Aminosäuren, sowie Methansulfonsäure, Ethansulfonsäure, 2-Hydroxyethansulfonsäure, Ethan-1,2-disulfonsäure, Benzolsulfonsäure, 4-Methylbenzolsulfonsäure, Naphthalin-2-sulfonsäure, 2- oder 3- Phosphoglycerat, Glucose-6-Phosphat, N-Cyclohexylsulfaminsäure (unter Bildung der Cyclamate) oder mit anderen sauren organischen Verbindungen, wie Ascorbinsäure. Verbindungen der Formel I mit sauren und basischen Gruppen können auch innere Salze bilden.

Zur Isolierung und Reinigung können auch pharmazeutisch ungeeignete Salze Verwendung finden.

Die Verbindungen der vorliegenden Erfindung weisen Enzym-hemmende Eigenschaften auf. Insbesondere hemmen sie die Wirkung des natürlichen Enzyms Renin. Letzteres gelangt aus den Nieren ins Blut und bewirkt dort die Spaltung von Angiotensinogen unter Freisetzung des Dekapeptides Angiotensin I, das dann in der Lunge, den Nieren und anderen Organen zum Octapeptid Angiotensinogen II gespalten wird. Das Octapeptid erhöht den Blutdruck sowohl direkt durch arterielle Vasokonstriktion als auch indirekt durch die Freisetzung des Natriumionen zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II zurückzuführen. Hemmer der enzymatischen Aktivität von Renin bewirken eine Verringerung der Bildung von Angiotensin I. Als Folge davon entsteht eine geringere Menge Angiotensin II. Die verminderte Konzentration dieses aktiven Peptidhormons ist die unmittelbare Ursache für die blutdrucksenkende Wirkung von Renin-Hemmern.

Die Wirkung von Reninhemmern wird unter anderem experimentell mittels *in vitro*-Tests nachgewiesen, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende *in vitro*-Test verwendet: Ein Extrakt von menschlichem Renin aus der Niere (0,5 mGU [Milli-Goldblattinheiten]/ml) wird eine Stunde lang bei 37° C und pH 7,2 in 1-molarer wäßriger 2-N-(Trishydroxymethylmethyl)amino-ethansulfonsäure-Pufferlösung mit 23 µg/ml synthetischem Renin-Substrat, dem Tetradecapeptid H-Asp-Arg-Val-Tyr-Ile-His-ProPhe-His-Leu-Leu-Val-Tyr-Ser-OH, inkubiert. Die Menge an gebildetem Angiotensin I wird in einem Radioimmunoassay ermittelt. Die erfindungsgemäßen Hemmstoffe werden dem Inkubationgemisch jeweils in verschiedenen Konzentrationen zugefügt. AIs IC₅₀ wird diejenige Konzentration des jeweiligen Hemmstoffes bezeichnet, die die Bildung von Angiotensin I um 50 % reduziert. Die Verbindungen der vorliegenden Erfindung zeigen in den *in vitro*-Systemen Hemmwirkungen bei minimalen Konzentrationen von etwa 10⁻⁶ bis etwa 10⁻¹⁰ Mol/l.

An salzverarmten Tieren bewirken Reninhemmer einen Blutdruckabfall. Das menschliche Renin unterscheidet sich von Renin anderer Spezies. Zur Prüfung von Hemmstoffen des humanen Renins werden Primaten (Marmosets, Callithrix jacchus) verwendet, weil humanes Renin und Primaten-Renin im enzymatisch aktiven Bereich weitgehende homolog sind. Unter anderem wird der folgende *in vivo*-Test eingesetzt: Die Testverbindungen werden an normotensiven Marmosets beider Geschlechter mit einem Körpergewicht von etwa 300 g, die bei Bewußtsein sind, geprüft. Blutdruck und Herzfrequenz werden mit einem Katheter in der Oberschenkelarterie gemessen. Die endogene Freisetzung von Renin wird durch die intravenöse Injektion von Furosemid (5-(Aminosulfonyl)-4-chloro-2-[(2-furanylmethyl)amino]benzoesäure) (5 mg/kg) angeregt. 30 Minuten nach der Injektion von Furosemid werden die Testsubstanzen entweder über einen Katheter in der lateralen Schwanzvene oder durch eine kontinuierliche Infusion verabreicht oder als Suspension oder Lösung über einen Tubus in den Magen gepumpt und ihre Wirkung auf Blutdruck und Herzfrequenz ausgewertet. Die Verbindungen der vorliegenden Erfindung sind in dem beschriebenen *in vivo*-Test bei Dosen von etwa 0,1 bis etwa 1,0 mg/kg i.v. und bei Dosen von etwa 3 bis etwa 30 mg/kg p.o. wirksam.

Die Verbindungen der vorliegenden Erfindung besitzen auch die Eigenschaft, den Augeninnendruck regulieren, insbesondere senken zu können.

Die Verbindungen der vorliegenden Erfindungen können als Antihypertensiva, ferner zur Behandlung von Herzinsuffizienz, kongestiven Herzerkrankungen oder auch zur Behandlung von erhöhtem Augeninnendruck und Glaukom Verwendung finden.

Die Erfindung betrifft beispielsweise Verbindungen der Formel I, worin
R₁ Arylamino, N-Aryl-N-(niederalkoxyniederalkyl)-amino, N-Aryl-N-arylniederalkyl-amino oder über ein Ringstickstoffatom gebundenes Heterocyclyl bedeutet, wobei das genannte Heterocyclyl außer dem Ringstickstoffatom, über das es gebunden ist, weitere Ringheteroatome, ausgewählt aus Sauerstoff, Stickstoff, durch Niederalkyl, Niederalkanoyl, Niederalkansulfonyl oder Niederalkoxycarbonyl substituiertem Stickstoff, Schwefel und mit 1 oder 2 Sauerstoffatomen verknüpftem Schwefel enthalten kann,
X für eine Carbonyl- oder Methylengruppe steht,
R₂ und R₃ unabhängig voneinander Wasserstoff oder Niederalkyl bedeuten oder zusammen mit dem Kohlenstoffatom, mit dem sie verbunden sind, einen Cycloalkylidenrest darstellen,
R₄ Wasserstoff, Niederalkyl, Niederalkanoyl oder Niederalkoxycarbonyl ist,
R₅ Hydroxy, Niederalkanoyloxy oder Niederalkoxycarbonyloxy bedeutet, R₆ Wasserstoff, Niederalkyl, Niederalkenyl, Niederalkinyl, Cycloalkyl, Cycloalkylniederalkyl, Arylniederalkyl oder Heteroarylniederalkyl mit 5 bis 7 Ringatomen im Heteroarylring und R₇ Wasserstoff oder Niederalkyl bedeutet oder
R₆ und R₇ zusammen mit dem Kohlenstoffatom, mit dem sie verbunden sind, einen Cycloalkylidenrest darstellen und
R₈ Niederalkyl, Niederalkoxyniederalkyl, Aminoniederalkyl, N-Niederalkylaminoniederalkyl, N,N-Diniederalkylaminoniederalkyl, N,N-Niederalkylenaminoniederalkyl, N,N-(Azaniederalkylenamino)niederalkyl, N,N-(Oxaniederalkylenamino)niederalkyl, N,N-(Thianiederalkylenamino)niederalkyl, Carbamoylniederalkyl, N-Niederalkylcarbamoylniederalkyl, N,N-Diniederalkylcarbamoylniederalkyl, Cyanoniederalkyl oder Heteroarylniederalkyl mit 5 bis 7 Ringatomen im Heteroarylring, das ein Ringstickstoffatom enthält und ein weiteres Ringheteroatom, ausgewählt aus Sauerstoff, Schwefel oder Stickstoff, enthalten kann, bedeutet,
und ihre Salze.

Die Erfindung betrifft in erster Linie Verbindungen der Formel I, worin
R₁ unsubstituiertes oder im Phenyl- oder Naphthylteil wie nachstehend angegeben substituiertes Anilino oder Naphthylamino, unsubstituiertes oder im Phenyl- oder Naphthylteil wie nachstehend angegeben substituiertes N-Phenyl- oder N-Naphthyl-N(niederalkoxyniederalkyl)-amino, unsubstituiertes oder im Phenyl- oder Naphthylteil wie nachstehend angegeben substituiertes N-Phenyl- oder N-Naphthyl-N-(niederalkyl)-amino oder über ein Ringstickstoffatom gebundenes, gegebenenfalls mit 1 oder 2 ankondensierten Phenyl- oder Cycloalkylresten kondensiertes 5- bis 8-gliedriges Heterocyclyl bedeutet, welches 1 oder 2 weitere Ringheteroatome, ausgewählt aus Sauerstoff, Stickstoff und gegebenenfalls oxydiertem Schwefel enthalten kann,
X für eine Carbonyl- oder Methylengruppe steht,
R₂ und R₃ unabhängig voneinander Wasserstoff oder Niederalkyl bedeuten oder zusammen mit dem Kohlenstoffatom, mit dem sie verbunden sind, 3- bis 8-gliedriges Cycloalkyliden darstellen,
R₄ Wasserstoff, Niederalkyl, Niederalkanoyl oder Niederalkoxycarbonyl bedeutet,
R₅ Hydroxy, Niederalkanoyloxy oder Niederalkoxycarbonyloxy bedeutet,
R₆ Wasserstoff, Niederalkyl, 3- bis 8-gliedriges Cycloalkyl, 3- bis 8-gliedriges Cycloalkylniederalkyl, Niederalkenyl, Niederalkinyl, unsubstituiertes oder wie nachstehend angegeben substituiertes Phenyl, Indenyl, Naphthyl, unsubstituiertes oder im Phenyl- oder Naphthylteil wie nachstehend angegeben substituiertes Phenyl- oder Naphthylniederalkyl, Pyridylniederalkyl oder Imidazolylniederalkyl darstellt,
R₇ Wasserstoff oder Niederalkyl bedeutet oder
R₆ und R₇ zusammen mit dem Kohlenstoffatom, mit dem sie verbunden sind, 3- bis 8-gliedriges Cycloalkyliden darstellen und
R₈ Niederalkyl, Niederalkoxyniederalkyl, Aminoniederalkyl, N-Niederalkylaminoniederalkyl, N,N-Diniederalkylaminoniederalkyl, N,N-Niederalkylenaminoniederalkyl N,N-(Azaniederalkylenamino)niederalkyl, N,N-(Oxaniederalkylenamino)niederalkyl, N,N-(Thianiederalkylenamino)niederalkyl, Heteroarylniederalkyl mit 5 bis 7 Ringatomen im Heteroarylring, welches ein Ringstickstoffatom enthält und ein weiteres Ringheteroatom, ausgewählt aus Sauerstoff, Schwefel oder Stickstoff, enthalten kann,
wobei Phenyl, Naphthyl, und Phenyl- bzw. Naphthylreste als Bestandteil von Naphthylamino, N-Phenyl- oder N-Naphthyl)-N-(niederalkoxyniederalkyl)-amino, N-Phenyl- oder N-Naphthyl)N-niederalkyl-amino, Indenyl, Phenyl- oder Naphthylniederalkyl und N-Phenyl-N-(phenylniederalkyl)-amino durch Niederalkyl, Hydroxy, Niederalkoxy, Carbamoylniederalkoxy, N-Niederalkylcarbamoylniederalkoxy, N-Niederalkylcarbamoyl, N,N-Diniederalkylcarbamoylniederalkoxy, Amino, N-Niederalkyl- oder N,N-Diniederalkylamino, Carboxy, Niederalkoxycarbonyl, Carbamoyl, Sulfamoyl, Niederalkansulfonyl, Halogen, Nitro, Phenyl, 5- oder 6-gliedriges Heteroaryl, als Heteroatom enthaltend 1 Stickstoff-, Schwefel- oder Sauerstoffatom, 2 N-Atome, 1 N-Atom und 1 S-Atom oder 1 N-Atom und 1 O-Atom, wie Pyridyl, und/oder durch Cyano ein oder mehrfach, z.B. ein- oder zweifach substituiert und Reste R₁ durch Niederalkyl, Niederalkanoyl, Niederalkoxycarbonyl oder Niederalkansulfonyl N-substituiert, durch Oxy S-mono- oder S,S-disubstituiert und/oder durch Niederalkyl, Hydroxyniederalkyl, Niederalkoxyniederalkyl, Niederalkenyloxyniederalkyl, Naphthoxyniederalkyl, Phenyloxyniederalkyl, Phenylniederalkoxyniederalkyl, Niederalkanoyloxyniederalkyl, Benzoyloxyniederalkyl, Niederalkoxycarbonyloxyniederalkyl, Phenyloxycarbonyloxyniederalkyl, Phenylniederalkoxycarbonyloxyniederalkyl, Aminoniederalkyl, N-Niederalkylaminoniederalkyl, N,N-Diniederalkylaminoniederalkyl, Carbamoylniederalkyl, Niederalkanoylaminoniederalkyl, Benzoylaminoniederalkyl, Niederalkoxycarbonylaminoniederalkyl, Niederalkoxycarbonylniederalkyl, Niederalkoxyniederalkoxyniederalkyl, Niederalkylthioniederalkoxyniederalkyl, N-Niederalkoxyiminoniederalkyl, Cycloalkoxyniederalkyl, Cycloalkylniederalkoxyniederalkyl, Niederalkenyl, Niederalkenyloxy, Niederalkoxyniederalkenyl, Niederalkinyl, Niederalkinyloxy, Niederalkanoyl, Oxo, Hydroxy, Niederalkoxy, Carbamoylniederalkoxy, N-Niederalkylcarbamoylniederalkoxy, N-Niederalkylcarbamoyloxy, N,N-Diniederalkylcarbamoyloxy, Niederalkoxyniederalkoxy, Niederalkylthioniederalkoxy, Niederalkanoyloxy, Benzoyloxy, N-Niederalkylcarbamoyl, Amino, N-Niederalkylamino, N,N-Diniederalkylamino, Niederalkanoylamino, Benzoylamino, Cycloalkylcarbonylamino, Cycloalkylniederalkanoylamino, Niederalkoxycarbonylniederalkylamino, Niederalkenyloxycarbonylamino, Niederalkoxyniederalkoxycarbonylamino, Niederalkoxyniederalkanoylamino, N-Niederalkylcarbamoylamino, N,N-Diniederalkylcarbamoylamino, N-Niederalkanoyl-N-niederalkyl-amino, Niederalkoxycarbonylamino, N-Niederalkoxycarbonyl-N-niederalkylamino, N,N-Niederalkylenamino, N,N-(1-Oxoniederalkylen)amino, N,N-(1-Oxo2-oxa-niederalkylen)amino, Carboxy, Niederalkoxycarbonyl, Phenylniederalkoxycarbonyl, Phenyloxycarbonyl, Pyrrolidinylcarbonyl, Piperidinylcarbonyl, Piperazinylcarbonyl, Morpholinylcarbonyl, Thiomorpholinylcarbonyl, S,S-Dioxothiomorpholin-4-ylcarbonyl, Cyano, Carbamoyl, N,N-Diniederalkylcarbamoyl, N-Niederalkenylcarbamoyl, N-Cycloalkylcarbamoyl, N-Cycloalkylniederalkylcarbamoyl, N-Hydroxyniederalkylcarbamoyl, N-Niederalkoxyniederalkylcarbamoyl, N-Carboxyniederalkylcarbamoyl, Carbamoylniederalkylcarbamoyl, Niederalkoxycarbonylniederalkylcarbamoyl, Phenyl, Dioxolan-2-yl, Oxazol-2-yl, Oxazolin-2-yl, Oxazolidin-2-yl, Nitro, Sulfamoyl, Niederalkansulfonyl, Phosphono, Niederalkanphosphono, Diniederalkylphosphono und/oder Halogen ein- oder zweifach C-substituiert sein können, und ihre Salze, insbesondere ihre pharmazeutisch verwendbaren Salze.

Die Erfindung betrifft vor allem Verbindungen der Formel I, worin
R₁ unsubstituiertes oder im Phenyl- oder Naphthylteil durch C₁-C₄-Alkoxy, wie Methoxy, C₁-C₄-Alkoxycarbonyl, wie Methoxy-, Ethoxy-, Isopropyloxy oder Tertiärbutyloxycarbonyl, Carbamoyl-C₁-C₄-alkoxy, wie Carbamoylmethoxy, C₁-C₄-Alkanoylamino-C₁-C₄alkyl, wie Formylaminomethyl, C₁-C₄-Alkoxycarbonylamino-C₁-C₄-alkyl, wie Methoxycarbonylaminomethyl, Halogen und/oder gegebenenfalls N-oxidiertes Pyridyl, wie Pyrid-3-yl oder 1-Oxidopyrid-3-yl, mono- oder disubstituiertes Anilino, Naphthylamino, N-Phenyl-N-(C₁-C₄-alkoxy-C₁-C₄-alkyl)-amino, wie N-Phenyl-N(ethoxymethyl)amino, oder N-Phenyl-N-(phenyl-C₁-C₄-alkyl)-amino, wie N-Phenyl-N-(2-phenylethyl)-amino, oder jeweils unsubstituiertes oder durch C₁-C₄-Alkyl, wie Methyl, Hydroxy-C₁-C₄-alkyl, wie Hydroxymethyl, C₁-C₄-Alkoxy-C₁-C₄-alkyl, wie Methoxymethyl oder Propyloxymethyl, C₃-C₅-Alkenyloxy-C₁-C₄-alkyl, wie Allyloxymethyl, C₁-C₄-Alkoxy-C₁-C₄-alkoxy-C₁-C₄-alkyl, wie Methoxymethoxymethyl oder 2-Methoxyethoxymethyl, C₁-C₄-Alkoxycarbonylamino-C₁-C₄-alkyl, wie Methoxy- oder Ethoxycarbonylaminomethyl, C₁-C₄-Alkoxyimino-C₁-C₄-alkyl, wie Methoxyiminomethyl, Ethoxyiminomethyl oder Propoxyiminomethyl, Carboxy, C₁-C₇-Alkoxycarbonyl, wie Methoxy-, Ethoxy-, Isopropyloxy- oder Tertiärbutyloxycarbonyl, Cyano, Carbamoyl, N-C₁-C₇-Alkylcarbamoyl, wie N-Methyl- oder N-Butylcarbamoyl, N,N-Di-C₁-C₄-alkylcarbamoyl, wie N,N-Dimethylcarbamoyl, C₁-C₄-Alkoxy-C₁-C₄-alkylcarbamoyl, wie N-(2-Propyloxyethyl)carbamoyl, N-Carboxy-C₁-C₄-alkylcarbamoyl, wie N-Carboxymethylcarbamoyl, Morpholinocarbonyl, C₁-C₄-Alkoxy, wie Propyloxy, C₁-C₄-Alkoxy-C₁-C₄-alkoxy, wie Methoxymethoxy oder 2-Methoxyethoxy, C₁-C₇-Alkanoyloxy, wie Acetoxy, Benzoyloxy, C₁-C₄-Alkanoylamino, wie Acetylamino, C₁-C₇-Alkoxycarbonylamino, wie Methoxycarbonylamino, 3- bis 6-gliedriges Cycloalkylcarbonylamino, wie Cyclopropylcarbonylamino, N-C₁-C₄-Alkoxy-C₁-C₄-alkanoylamino, wie Methoxyacetylamino, N-C₁-C₄-Alkylcarbamoylamino, wie Methylcarbamoylamino, oder 5- oder 6-gliedriges N,N-(1-Oxoniederalkylen)amino oder N,N-(1-Oxo2-oxa-niederalkylen)amino, wie 2-Oxopyrrolidin-1-yl oder 2-Oxo-oxazolidin-3-yl, C₁-C₇-Alkanoyl, wie Acetyl, Oxo, Nitro, C₁-C₄-Alkansulfonyl, wie Methan- oder Ethansulfonyl, und/oder Halogen mono-, di- oder trisubstituiertes Pyrrolidino, Piperidino, Piperazino, Morpholino oder Thiomorpholino, Indolin-1-yl, Isoindolin-2-yl, 2,3-Dihydrobenzimidazol-1-yl, 1,2,3,4-Tetrahydrochinol-1-yl, 1,2,3,4-Tetrahydroisochinol-2-yl, 1,2,3,4-Tetrahydro-1,3-benzdiazin-1-yl, 1,2,3,4-Tetrahydro-1,4-benzdiazin-1-yl, 3,4-Dihydro-2H-1,4-benzoxazin-4-yl, gegebenenfalls S,S-dioxydiertes 3,4-Dihydro-2H-1,3-benzthiazin-1-yl 3,4,5,6,7,8-Hexahydro-2H-1,4-benzoxazin-4-yl, 3,4,5,6,7,8-Hexahydro-2H-1,4-benzthiazin-4-yl, 2,3,4,5-Tetrahydro-1H-1-benz[6,7-b]azepin-1-yl oder 5,6-Dihydrophenanthridin-5-yl bedeutet, X für eine Carbonylgruppe steht,
R₂ und R₃ unabhängig voneinander Wasserstoff oder C₁-C₄-Alkyl, wie Methyl, bedeuten oder zusammen mit dem Kohlenstoffatom, mit dem sie verbunden sind, 3- bis 8-gliedriges Cycloalkyliden, wie Cyclopropyliden, darstellen,
R₄ Wasserstoff oder C₁-C₄-Alkanoyl, wie Formyl, bedeutet,
R₅ Hydroxy bedeutet,
R₆ Wasserstoff, C₁-C₄-Alkyl, wie Methyl, Ethyl, Propyl, Isopropyl, Butyl oder Isobutyl, 3- bis 8-gliedriges Cycloalkyl, wie Cyclopropyl, oder Phenyl-C₁-C₄-alkyl, wie Benzyl oder 2-Phenethyl, darstellt und
R₇ Wasserstoff bedeutet oder R₆ und R₇ zusammen mit dem Kohlenstoffatom, mit dem sie verbunden sind, 3- bis 8-gliedriges Cycloalkyliden, wie Cyclopropyliden, darstellen und R₈ C₁-C₇-Alkyl, wie Butyl, C₁-C₄-Alkoxy-C₁-C₄-alkyl, wie 2-Methoxyethyl, Amino-C₁-C₄-alkyl, wie 3-Aminopropyl, N-C₁-C₄-Alkylamino-C₁-C₄-alkyl, wie 2-(N-Methylamino)ethyl, N,N-Di-C₁-C₄-alkylamino-C₁-C₄-alkyl, wie 2-(N-Methylamino)ethyl, 2-(N,N-Dimethylamino)ethyl oder 3-(N,N-Dimethyl)propyl, 5- oder 6-gliedriges N,N-Niederalkylenamino-C₁-C₄-alkyl bzw. N,N-(Aza)-, N,N-(Oxa)-oder N,N-(Thia)niederalkylenamino-C₁-C₄-alkyl, wie 2-Morpholinoethyl oder 3-Morpholinopropyl, Carbamoyl-C₁-C₄-alkyl, wie 2-(N-Methylcarbamoyl)ethyl, Carbamoylmethyl, 2-Carbamoylethyl, 3-Carbamoylpropyl oder 4-Carbamoylbutyl, N-C₁-C₄-Alkylcarbamoyl, wie N-Methylcarbamoyl, N-Ethylcarbamoyl, N-Propylcarbamoyl oder N-Butylcarbamoyl, N,N-Di-C₁-C₄-alkylcarbamoyl, wie N,N-Dimethylcarbamoyl, N,N-Diethylcarbamoyl, N,N-Dipropylcarbamoyl, N-Methyl-N-ethyl-carbamoyl oder N-Methyl-N-propylcarbamoyl, Cyano-C₁-C₄-alkyl, wie Cyanomethyl, 2-Cyanoethyl, 3-Cyanopropyl oder 4-Cyanobutyl, oder Pyridyl-C₁-C₄-alkyl, wie 2-Pyrid-2-ylethyl, bedeutet,
und ihre Salze, insbesondere ihre pharmazeutisch verwendbaren Salze.

Die Erfindung betrifft insbesondere Verbindungen der Formel I, worin R₁ ein Gruppe der Formel Ia oder Ib 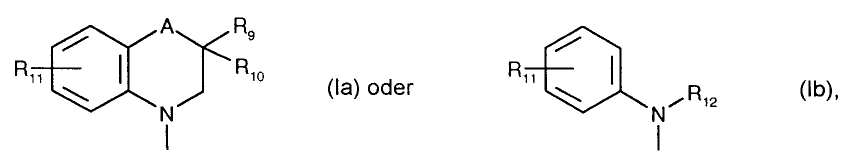
darstellt, in denen
A eine direkte Bindung, Methylen, Ethylen, Imino, Oxy oder Thio darstellt,
R₉ für C₁-C₄-Alkoxy-C₁-C₄-alkyl, wie Methoxy- oder Propyloxymethyl, C₃-C₅-Alkenyloxy-C₁-C₄-alkyl, wie Allyloxymethyl, C₁-C₄-Alkoxy-C₁-C₄-alkoxy-C₁-C₄-alkyl, wie Methoxymethoxymethyl oder 2-Methoxyethoxymethyl, C₁-C₄-Alkoxycarbonylamino-C₁-C₄-alkyl, wie Methoxy- oder Ethoxycarbonylaminomethyl, C₁-C₄-Alkoxyimino-C₁-C₄-alkyl, wie Methoxyiminomethyl, Phenyl, C₁-C₄-Alkoxycarbonyl, wie Methoxycarbonyl, Ethoxycarbonyl oder Isopropyloxycarbonyl, Cyano, Carbamoyl, N-C₁-C₄-Alkylcarbamoyl, wie N-Methylcarbamoyl, N-Ethylcarbamoyl oder N-Butylcarbamoyl, C₁-C₄-Alkoxy-C₁-C₄-alkylcarbamoyl, wie N-(2-Methoxyethyl)carbamoyl, C₁-C₄-Alkoxy, wie Propyloxy, C₁-C₄-Alkoxy-C₁-C₄-alkoxy, wie Methoxymethoxy oder 2-Methoxyethoxy, C₁-C₇-Alkanoyloxy, wie Acetoxy, Benzoyloxy, N-C₁-C₄-Alkylcarbamoylamino, wie N-Methylcarbamoylamino, C₁-C₄-Alkanoylamino, wie Acetylamino, C₁-C₇-Alkoxycarbonylamino, wie Methoxycarbonylamino, 3- bis 3- bis 6-gliedriges Cycloalkylcarbonylamino, wie Cyclopropylcarbonylamino, C₁-C₄-Alkoxy-C₁-C₄-alkanoylamino, wie Methoxyacetylamino, oder 5- oder 6-gliedriges N,N-(1-Oxoniederalkylen)amino oder N,N-(1-Oxo2-oxa-niederalkylen)amino, wie 2-Oxopyrrolidin-1-yl oder 2-Oxo-oxazolidin-3-yl, N-C₁-C₄-Alkylcarbamoylamino, wie Methylcarbamoylamino, steht,
R₁₀ Wasserstoff ist, aber auch C₁-C₄-Alkyl, wie Methyl, bedeuten kann,
R₁₁ Wasserstoff oder Halogen ist und
R₁₂ C₁-C₄-Alkoxy-C₁-C₄-alkyl, wie Methoxy-C₁-C₄-alkyl, Ethoxy-C₁-C₄-alkyl, Propyloxy-C₁-C₄-alkyl, Isopropyloxy-C₁-C₄-alkyl, Butyloxy-C₁-C₄-alkyl, Isobutyloxy-C₁-C₄-alkyl, Sekundärbutyloxy-C₁-C₄-alkyl oder Tertiärbutyloxy-C₁-C₄-alkyl darstellt, wobei C₁-C₄-Alkyl beispielsweise Ethyl, Propyl oder Butyl bedeutet, und insbesondere für 3-Methoxypropyl steht,
X für eine Carbonylgruppe steht,
R₂ und R₃ unabhängig voneinander Wasserstoff oder C₁-C₄-Alkyl, wie Methyl, bedeuten oder zusammen mit dem Kohlenstoffatom, mit dem sie verbunden sind, 3- bis 8-gliedriges Cycloalkyliden, wie Cyclopropyliden, darstellen,
R₄ Wasserstoff oder C₁-C₄-Alkanoyl, wie Formyl, bedeutet,
R₅ Hydroxy bedeutet,
R₆ Wasserstoff, C₁-C₄-Alkyl, wie Methyl, Ethyl, Propyl, Isopropyl, Butyl oder Isobutyl, 3- bis 8-gliedriges Cycloalkyl, wie Cyclopropyl, oder Phenyl-C₁-C₄-alkyl, wie Benzyl oder 2-Phenethyl, darstellt,
R₇ Wasserstoff bedeutet und
R₈ C₁-C₇-Alkyl, wie Butyl, C₁-C₄-Alkoxy-C₁-C₄-alkyl, wie 2-Methoxyethyl, Amino-C₁-C₄-alkyl, wie 3-Aminopropyl, N-C₁-C₄Alkylamino-C₁-C₄-alkyl, wie 2-(N-Methylamino)ethyl, N,N-Di-C₁-C₄-alkylamino-C₁-C₄-alkyl, wie 2-(N-Methylamino)ethyl, 2-(N,N-Dimethylamino)ethyl oder 3-(N,N-Dimethyl)propyl, 5- oder 6-gliedriges N,N-Niederalkylenamino-C₁-C₄-alkyl bzw. N,N-(Aza)-, N,N-(Oxa)- oder N,N-(Thia)niederalkylenamino-C₁-C₄-alkyl, wie 2-Morpholinoethyl oder 3-Morpholinopropyl, oder Pyridyl-C₁-C₄-alkyl, wie 2-Pyrid-2-ylethyl, bedeutet, und ihre Salze, insbesondere ihre pharmazeutisch verwendbaren Salze.

Besonders wirksam sind jeweils diejenigen Verbindungen der Formel I mit der in der Formel 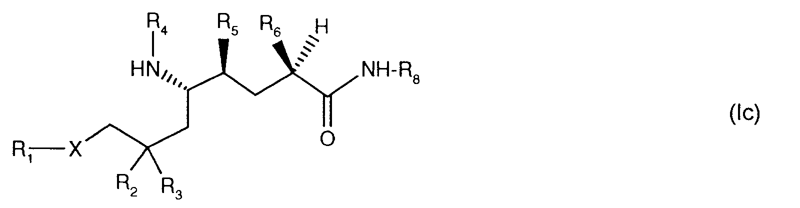
gezeigten Stereotaxie der Hauptkette. Die Erfindung betrifft jeweils vorzugsweise die Stereoisomeren von Verbindungen der Formel I mit der in der Formel Ia gezeigten Stereotaxie der Hauptkette, wobei die Variablen die vorstehend angegebenen Bedeutungen haben, und ihre Salze, insbesondere ihre pharmazeutisch verwendbaren Salze.

Bevorzugter Gegenstand der Erfindung sind Verbindungen der Formel Ib, worin R₁, R₂, R₃, R₄, R₅, R₆, R₇, R₈, R₉, R₁₀ und R₁₁ die vorstehend angegebenen Bedeutungen haben, und ihre Salze, insbesondere ihre pharmazeutisch verwendbaren Salze.

Die Erfindung betrifft namentlich die in den Beispielen genannten Verbindungen der Formel I und ihre Salze, insbesondere ihre pharmazeutisch verwendbaren Salze.

Die Erfindung betrifft in allererster Linie Verbindungen der Formel Id 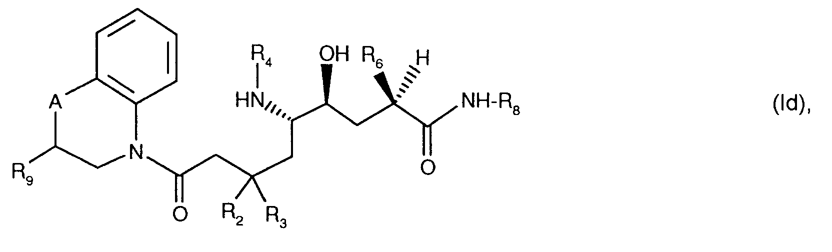
worin
A eine Methylen, Oxy oder Thio darstellt,
R₂ und R₃ C₁-C₄-Alkyl, wie Methyl, bedeuten,
R₄ Wasserstoff oder C₁-C₄-Alkanoyl, wie Formyl, bedeutet,
R₆ C₁-C₄-Alkyl, wie Methyl, Ethyl, Isopropyl, Butyl oder Isobutyl, oder Phenyl-C₁-C₄-alkyl, wie Benzyl, bedeutet,
R₈ C₁-C₇-Alkyl, wie Butyl, bedeutet,
R₉ für C₁-C₄-Alkoxycarbonylamino, wie Methoxycarbonylamino, Ethoxycarbonylamino, Propyloxycarbonylamino, Isopropyloxycarbonylamino oder Butyloxycarbonylamino, C₁-C₄-Alkoxy-C₁-C₄-alkoxy-C₁-C₄-alkyl, wie Methoxy-C₁-C₄alkoxy-C₁-C₄-alkyl, Ethoxy-C₁-C₄-alkoxy-C₁-C₄-alkyl, Propyloxy-C₁-C₄-alkoxy-C₁-C₄-alkyl, Isopropyloxy-C₁-C₄-alkoxy-C₁-C₄-alkyl oder Butyloxy-C₁-C₄-alkoxy-C₁-C₄-alkyl, wobei C₁-C₄-Alkoxy beispielsweise Methoxy, Ethoxy, Propyloxy oder Butyloxy und -C₁-C₄-Alkyl beispielsweise Methyl, Ethyl, Propyl oder Butyl bedeutet, insbesondere Methoxymethoxymethyl, 2-Methoxyethoxymethyl oder 3-Methoxypropyl, C₁-C₄-Alkoxy-C₁-C₄-alkyl, wie Methoxy-C₁-C₄-alkyl, Ethoxy-C₁-C₄-alkyl, Propyloxy-C₁-C₄-alkyl, Isopropyloxy-C₁-C₄-alkyl, Butyloxy-C₁-C₄-alkyl, Isobutyloxy-C₁-C₄-alkyl, Sekundärbutyloxy-C₁-C₄-alkyl oder Tertiärbutyloxy-C₁-C₄-alkyl, wobei C₁-C₄-Alkyl beispielsweise Methyl, Ethyl, Propyl oder Butyl bedeutet, insbesondere Ethoxymethyl oder 2-Methoxyethyl, oder N-C₁-C₄-Alkylcarbamoyl, wie N-Methylcarbamoyl, N-Ethylcarbamoyl, N-Propylcarbamoyl oder N-Butylcarbamoyl, darstellt,
und ihre Salze, insbesondere ihre pharmazeutisch verwendbaren Salze.

Die erfindungsgemäßen Verbindungen der Formel I und Salze von solchen Verbindungen mit mindestens einer salzbildenden Gruppe werden nach an sich bekannten Verfahren erhalten, z.B. indem man
a) eine Verbindung der Formel II 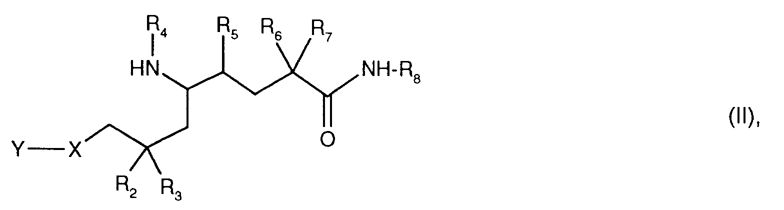 worin Y freies oder reaktionsfähiges funktionell abgewandeltes Hydroxy bedeutet und X, R₂, R₃, R₄, R₅, R₆, R₇ und R₈ die oben genannten Bedeutungen haben, oder ein Salz davon mit einer Verbindung der Formel R₁-H (III), worin R₁ die genannten Bedeutungen hat, unter Bildung einer Amin- bzw. Amidbindung kondensiert, wobei in den Reaktionskomponenten vorhandene freie funktionelle Gruppen mit Ausnahme der an der Reaktion teilnehmenden Gruppen in geschützter Form vorliegen, und vorhandene Schutzgruppen abspaltet oder
b) eine Carbonsäure der Formel IV 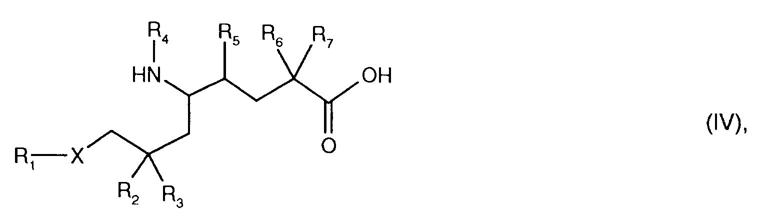 worin R₁, X, R₂, R₃, R₄, R₅, R₆ und R₇ die oben genannten Bedeutungen haben, oder ein Salz oder ein reaktionsfähiges Säurederivat davon mit einer Verbindung der Formel H-N(H)-R₈ (V) oder einem reaktionsfähigen Derivat davon mit einer reaktionsfähigen Aminogruppe, worin R₈ die genannten Bedeutungen hat unter Bildung einer Amidbindung kondensiert, wobei in den Reaktionskomponenten vorhandene freie funktionelle Gruppen mit Ausnahme der an der Reaktion teilnehmenden Gruppen in geschützter Form vorliegen, und vorhandene Schutzgruppen abspaltet oder
c) zur Herstellung einer Verbindung der Formel I, worin R₅ Hydroxy bedeutet und die übrigen Substituenten die genannten Bedeutungen haben, in einer Verbindung der Formel VI 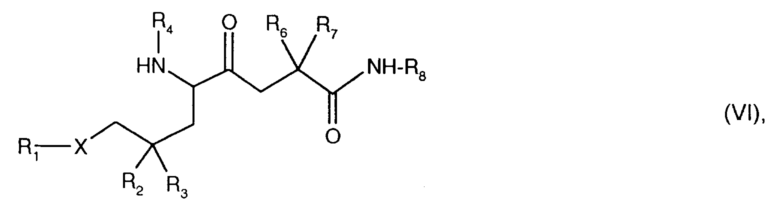 in der R₁, X, R₂, R₃, R₄, R₆, R₇ und R₈ die oben genannten Bedeutungen haben und freie funktionelle Gruppen mit Ausnahme der an der Reaktion teilnehmenden Ketogruppe in geschützter Form vorliegen, oder in einem Salz davon die Ketogruppe zu Hydroxymethylen reduziert und gegebenenfalls vorhandene Schutzgruppen abspaltet oder
d) zur Herstellung einer Verbindung der Formel I, worin R₆ Alkyl, Arylalkyl oder Heteroaralkyl, insbesondere Methyl, und R₇ Wasserstoff bedeutet und die übrigen Substituenten die genannten Bedeutungen haben, in einer Verbindung der Formel VII 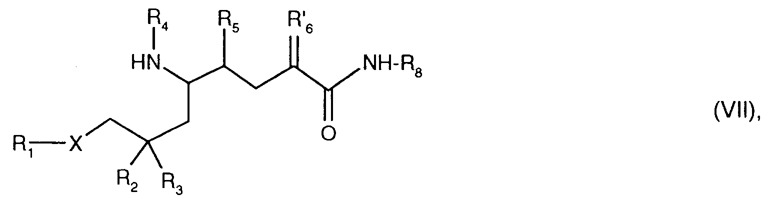 worin R 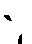 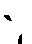 ₆ Alkyliden, Arylalkyliden oder Heteroaralkyliden, insbesondere Methylen, bedeutet und R₁, X, R₂, R₃, R₄, R₅ und R₈ die genannten Bedeutungen haben, wobei freie funktionelle Gruppen gegebenenfalls in geschützter Form vorliegen, oder einem Salz davon die Gruppe R 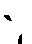 ₆ zur gewünschten Gruppe R₆ reduziert oder
e) zur Herstellung einer Verbindung der Formel I, in der R₄ Wasserstoff bedeutet und die übrigen Substituenten die genannten Bedeutungen haben, in einer Verbindung der Formel VIII 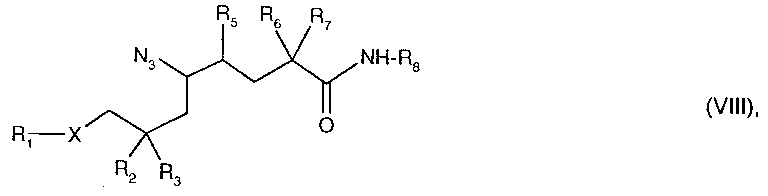 worin die Substituenten R₁, X, R₂, R₃, R₅, R₆, R₇ und R₈ die genannten Bedeutungen haben und freie funktionelle Gruppen gegebenenfalls in geschützter Form vorliegen, oder einem Salz davon die Azidogruppe zu Amino reduziert und vorhandene Schutzgruppen abspaltet und jeweils gewünschtenfalls eine nach einem der vorstehend genannten Verfahren a) bis e) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemäße Verbindung der Formel I in eine andere erfindungsgemäße Verbindung der Formel I umwandelt.

Die Durchführung der verfahrensgemäßen Reaktionen sowie die Herstellung neuer Ausgangsstoffe bzw. Zwischenprodukte erfolgt in Analogie zur Reaktions- und Bildungsweise bekannter Ausgangsstoffe bzw. Zwischenprodukte. Dabei werden, auch wenn nachstehend nicht ausdrücklich erwähnt, die jeweils üblichen Hilfsmittel, wie Katalysatoren, Kondensations- sowie Solvolysemittel und/oder Lösungs- bzw. Verdünnungsmittel, und Reaktions-, wie Temperatur- und Druckbedingungen, sowie gegebenenfalls Schutzgase verwendet.

Die Ausgangsstoffe der Formel II für die Verfahrensvariante a) enthalten eine endständige Carboxy- oder Hydroxymethylgruppe oder sind reaktionsfähige funktionelle Derivate davon, z.B. von Säuren der Formel II (Y= Hydroxy; X= Carbonyl) abgeleitete aktivierte Ester oder Anhydride, ferner reaktionsfähige cyclische Amide, oder von Alkoholen der Formel II (Y= Hydroxy; X= Methylen) abgeleitete reaktionsfähige Ester, worin die reaktionsfähige funktionell abgewandelte Hydroxygruppe insbesondere mit einer starken anorganischen Säure, Mineralsäure, z.B. Halogenwasserstoffsäure, wie Chlor-, Brom- oder Jodwasserstoffsäure, mit einer starken organischen Sulfonsäure oder mit Stickstoffwasserstoffsäure verestertes Hydroxy bedeutet. Die reaktionsfähigen Säure- bzw. Alkoholderivate können auch in situ gebildet werden.

Aktivierte Ester der Formel II sind insbesondere am Verknüpfungskohlenstoffatom des veresternden Restes ungesättigte Ester, z.B. vom Vinylester-Typ, wie Vinylester (erhältlich z.B. durch Umesterung eines entsprechenden Esters mit Vinylacetat; Methode des aktivierten Vinylesters), Carbamoylester (erhältlich z.B. durch Behandeln der entsprechenden Säure mit einem Isoxazoliumreagens; 1,2-Oxazolinium- oder Woodward-Methode), oder 1-Niederalkoxyvinylester (erhältlich z.B. durch Behandeln der entsprechenden Säure mit einem Niederalkoxyacetylen; Ethoxyacetylen-Methode), oder Ester vom Amidinotyp, wie N,N'-disubstituierte Amidinoester (erhältlich z.B. durch Behandeln der entsprechenden Säure mit einem geeigneten N,N'-disubstituierten Carbodiimid, z.B. N,N'-Dicyclohexylcarbodiimid; Carbodiimid-Methode), oder N,N-disubstituierte Amidinoester (erhältlich z.B. durch Behandeln der entsprechenden Säure mit einem N,N-disubstituierten Cyanamid; Cyanamid-Methode), geeignete Arylester, insbesondere durch elektronenanziehende Substituenten geeignet substituierte Phenylester (erhältlich z.B. durch Behandeln der entsprechenden Säure mit einem geeignet substituierten Phenol, z.B. 4-Nitrophenol, 4-Methylsulfonylphenol, 2,4,5-Trichlorphenol 2,3,4,5,6-Pentachlorphenol oder 4-Phenyldiazophenol, in Gegenwart eines Kondensationsmittels, wie N,N'-Dicyclohexylcarbodiimid; Methode der aktivierten Arylester), Cyanmethylester (erhältlich z.B. durch Behandeln der entsprechenden Säure mit Chloracetonitril in Gegenwart einer Base; Cyanmethylester-Methode), Thioester, insbesondere gegebenenfalls, z.B. durch Nitro, substituierte Phenylthioester (erhältlich z.B. durch Behandeln der entsprechenden Säure mit gegebenenfalls, z.B. durch Nitro, substituierten Thiophenolen, u. a. mit Hilfe der Anhydrid- oder Carbodiimid-Methode; Methode der aktivierten Thiolester), oder insbesondere Amino- oder Amidoester (erhältlich z.B. durch Behandeln der entsprechenden Säure mit einer N-Hydroxyamino- bzw. N-Hydroxyamidoverbindung, z.B. N-Hydroxysuccinimid, N-Hydroxypiperidin, N-Hydroxyphthalimid, N-Hydroxy-5-norbornen-2,3-dicarbonsäureimid, 1-Hydroxybenztriazol oder 3-Hydroxy-3,4-dihydro-1,2,3-benztriazin-4-on, z.B. nach der Anhydrid- oder Carbodiimid-Methode; Methode der aktivierten N-Hydroxyester). Es sind auch innere Ester, z.B. γ-Lactone, einsetzbar.

Anhydride der Formel II von Säuren können symmetrische oder vorzugsweise gemischte Anhydride dieser Säuren sein, z.B. Anhydride mit anorganischen Säuren, wie Säurehalogenide, insbesondere Säurechloride (erhältlich z.B. durch Behandeln der entsprechenden Säure mit Thionylchlorid, Phosphorpentachlorid, Oxalylchlorid oder 1-Chlor-N,N,2-trimethyl-propenylamin; Säurechloridmethode), Azide (erhältlich z.B. aus einem entsprechenden Säureester über das entsprechende Hydrazid und dessen Behandlung mit salpetriger Säure; Azidmethode), Anhydride mit Kohlensäurehalbestern, z.B. Kohlensäureniederalkylhalbestern (erhältlich z.B. durch Behandeln der entsprechenden Säure mit Chlorameisensäureniederalkylestern oder mit einem 1-Niederalkoxycarbonyl-2-niederalkoxy-1,2-dihydrochinolin; Methode der gemischten O-Alkylkohlensäureanhydride), oder Anhydride mit dihalogenierter, insbesondere dichlorierter Phosphorsäure (erhältlich z.B. durch Behandeln der entsprechenden Säure mit Phosphoroxychlorid; Phosphoroxychloridmethode), Anhydride mit anderen Phosphorsäurederivaten (z.B. solchen, die man mit Phenyl-N-phenyl-phosphoroamidochloridat oder Bis(2-oxo-3-oxazolidinyl)-phosphinsäurechlorid erhalten kann) oder mit Phosphorigsäurederivaten, oder Anhydride mit organischen Säuren, wie gemischte Anhydride mit organischen Carbonsäuren (erhältlich z.B. durch Behandeln der entsprechenden Säure mit einem gegebenenfalls substituierten Niederalkan- oder Phenylniederalkancarbonsäurehalogenid, z.B. Phenylessigsäure-, Pivalinsäure- oder Trifluoressigsäurechlorid; Methode der gemischten Carbonsäureanhydride) oder mit organischen Sulfonsäuren (erhältlich z.B. durch Behandeln eines Salzes, wie eines Alkalimetallsalzes, der entsprechenden Säure mit einem geeigneten organischen Sulfonsäurehalogenid, wie Niederalkan- oder Aryl-, z.B. Methan- oder p-Toluolsulfonsäurechlorid; Methode der gemischten Sulfonsäureanhydride), sowie symmetrische Anhydride (erhältlich z.B. durch Kondensation der entsprechenden Säure in Gegenwart eines Carbodiimides oder von 1-Diethylaminopropin (Methode der symmetrischen Anhydride).

Geeignete reaktionsfähige cyclische Amide sind insbesondere Amide mit fünfgliedrigen Diazacyclen aromatischen Charakters, wie Amide mit Imidazolen, z.B. Imidazol (erhältlich z.B. durch Behandeln der entsprechenden Säure mit N,N'-Carbonyldiimidazol; Imidazol-Methode), oder Pyrazol, z.B. 3,5-Dimethylpyrazol (erhältlich z.B. über das Säurehydrazid durch Behandeln mit Acetylaceton; Pyrazolid-Methode).

Von Alkoholen der Formel II (Y= Hydroxy; X= Methylen) abgeleitete reaktionsfähige Ester sind beispielsweise Ester derselben mit einer starken anorganischen Säure, wie mit einer Mineralsäure, z.B. Halogenwasserstoffsäure, wie Chlor-, Brom- oder Jodwasserstoffsäure, ferner Schwefelsäure oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z. B. einer gegebenenfalls durch Niederalkyl, wie Methyl, Halogen, wie Brom und/oder Nitro substituierten Benzolsulfonsäure, z.B. einer Methansulfon-, Trifluormethansulfon- oder p-Toluolsulfonsäure, oder mit Stickstoffwasserstoffsäure.

Wie erwähnt, können Derivate von Carbonsäuren und Alkoholen der Formel II auch in situ gebildet werden. So kann man z.B. N,N'-disubstituierte Amidinoester in situ bilden, indem man das Gemisch einer Carbonsäure der Formel II und der Aminkomponente der Formel III in Gegenwart eines geeigneten N,N'-disubstituierten Carbodiimides, z.B. von N,N'-Cyclohexylcarbodiimid, zur Reaktion bringt. Ferner kann man Amino- oder Amidoester von Säuren der Formel II in Gegenwart des zu acylierenden Ausgangsmaterials der Formel III bilden, indem man das Gemisch der entsprechenden Säure- und Amino-Ausgangsstoffe in Gegenwart eines N,N'-disubstituierten Carbodiimides, z.B. N,N'-Dicyclohexylcarbodiimid, und eines N-Hydroxyamins oder N-Hydroxyamides, z.B. N-Hydroxysuccinimid, gegebenenfalls in Anwesenheit einer geeigneten Base, z.B. 4-Dimethylaminopyridin, umsetzt.

Ebenso können reaktionsfähige funktionelle Derivate von Alkoholen der Formel II durch Umsetzung desselben mit einem Halogenierungsmittel, wie einer Halogenwasserstoffsäure oder Thionylchlorid bzw. -bromid, einem Diniederalkylsulfat oder einem Sulfonylierungsmittel, wie Fluorsulfonylchlorid oder einem von einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom und/oder Nitro substituierten Benzolsulfonsäure, z.B. einer Methansulfon-, Trifluormethansulfon- oder p-Toluolsulfonsäure, abgeleiteten Halogenid in situ erhalten werden.

Die Kondensation von Verbindungen der Formeln II und III kann in an sich bekannter Weise durchgeführt werden. Verfahrensweisen zur Herstellung einer Amidbindung sind beispielsweise in Standardwerken, wie "Houben-Weyl, Methoden der organischen Chemie", 4. Auflage, Band 15/II (1974), Band IX (1955) Band E 11 (1985), Georg Thieme Verlag, Stuttgart, "The Peptides" (E. Gross und J. Meienhofer, Hg.), Band 1 und 2, Academic Press, London und New York, 1979/1980, oder M. Bodansky, "Principles of Peptide Synthesis", Springer-Verlag, Berlin 1984, beschrieben.

Die Kondensation von freien Carbonsäuren bzw. Alkoholen der Formeln II (Y= Hydroxy) mit dem Amin der Formel III kann vorzugsweise in Gegenwart eines der üblichen Kondensationsmittel durchgeführt werden. Übliche Kondensationsmittel sind z.B. Carbodiimide, beispielsweise Diethyl-, Dipropyl-, N-Ethyl-N'-(3-dimethylaminopropyl)-carbodiimid oder insbesondere Dicyclohexylcarbodiimid, ferner geeignete Carbonylverbindungen, beispielsweise Carbonyldiimidazol, 1,2-Oxazoliumverbindungen, z.B. 2-Ethyl-5-phenyl-1,2-oxazolium-3'-sulfonat und 2-Tertiärbutyl-5-methyl-isoxazoliumperchlorat, oder eine geeignete Acylaminoverbindung, z.B. 2-Ethoxy-1-ethoxycarbonyl-1,2-dihydrochinolin, ferner aktivierte Phosphorsäurederivate, z.B. Diphenylphosphorylazid, Diethylphosphorylcyanid, Phenyl-N-phenylphosphoroamidochloridat, Bis-(2-oxo-3-oxazolidinyl)-phosphinsäurechlorid oder 1-Benztriazolyloxy-tris-(dimethylamino)-phosphonium-hexafluorophosphat.

Gewünschtenfalls wird eine organische Base zugegeben, z.B. ein Triniederalkylamin mit voluminösen Resten, z.B. N-Ethyl-N,N-diisopropyl-amin, und/oder eine heterocyclische Base, z.B. Pyridin, N-Methylmorpholin oder bevorzugt 4-Dimethylaminopyridin.

Die Kondensation aktivierter Ester, reaktionsfähiger Anhydride oder reaktionsfähiger cyclischer Amide von Säuren der Formel II bzw. reaktionsfähigen Estern von Alkoholen der Formel II mit den entsprechenden Aminen wird üblicherweise in Gegenwart einer organischen Base, z.B. einfachen Triniederalkylaminen, z.B. Triethylamin oder Tributylamin, oder einer der vorstehend genannten organischen Basen durchgeführt. Gewünschtenfalls wird zusätzlich noch ein Kondensationsmittel verwendet, wie für freie Carbonsäuren beschrieben ist.

Die Kondensation von Säureanhydriden mit Aminen kann z.B. in Gegenwart von anorganischen Carbonaten, z.B. Ammonium- oder Alkalimetallcarbonaten oder -hydrogencarbonaten, wie Natrium- oder Kaliumcarbonat oder -hydrogencarbonat (üblicherweise zusammen mit einem Sulfat), erfolgen.

Carbonsäurechloride, beispielsweise die von der Säure der Formel II abgeleiteten Chlorkohlensäurederivate, werden mit den entsprechenden Aminen vorzugsweise in Gegenwart eines organischen Amins, z.B. der vorstehend genannten Triniederalkylamine oder heterocyclischen Basen, insbesondere von 4-Dimethylaminopyridin, gegebenenfalls in Gegenwart eines Hydrogensulfates, kondensiert. Diese Kondensation wird vorzugsweise in einem inerten, aprotischen, vorzugsweise wasserfreien, Lösungsmittel oder Lösungsmittelgemisch durchgeführt, beispielsweise in einem Carbonsäureamid, z.B. Dimethylformamid, einem halogenierten Kohlenwasserstoff, z.B. Methylenchlorid, Tetrachlorkohlenstoff oder Chlorbenzol, einem Keton, z.B. Aceton, einem cyclischen Ether, z.B. Tetrahydrofuran, einem Ester, z.B. Essigsäureethylester, oder einem Nitril, z.B. Acetonitril, oder in einer Mischung davon, gegebenenfalls bei erniedrigter oder erhöhter Temperatur, z.B. in einem Temperaturbereich von etwa -40° C bis etwa +100° C, bevorzugt von etwa -10° C bis etwa +50° C, im Falle der Verwendung von Arylsulfonylestern auch bei etwa +100° C bis +200° C, und gegebenenfalls unter Inertgas-, z.B. Stickstoff- oder Argonatmosphäre. Auch wäßrige, beispielsweise alkoholische, z.B. Ethanol, oder aromatische Lösungsmittel, z.B. Benzol oder Toluol, sind möglich. Bei Gegenwart von Alkalihydroxiden als Basen kann gegebenenfalls auch Aceton zugesetzt werden. Die Kondensation kann auch gemäß der als Festphasen-Synthese bekannten Technik erfolgen, die auf R. Merrifield zurückgeht und beispielsweise in Angew. Chem. 97, 801 - 812 (1985), Naturwissenschaften 71, 252 - 258 (1984) oder in R. A. Houghten, Proc. Natl. Acad. Sci USA 82, 5131-5135 (1985) beschrieben ist.

Funktionelle Gruppen in Ausgangsmaterialien, deren Umsetzung vermieden werden soll, insbesondere Carboxy-, Amino-, Hydroxy- und Mercaptogruppen, können durch geeignete Schutzgruppen (conventional protecting groups) geschützt sein, die üblicherweise bei der Synthese von Peptidverbindungen, aber auch von Cephalosporinen und Penicillinen sowie Nucleinsäurederivaten und Zuckern verwendet werden. Diese Schutzgruppen können bereits in den Vorstufen vorhanden sein und sollen die betreffenden funktionellen Gruppen gegen unerwünschte Nebenreaktionen, wie Acylierungen, Veretherungen, Veresterungen, Oxidationen, Solvolyse etc. schützen. In bestimmten Fällen können die Schutzgruppen darüber hinaus einen selektiven, beispielsweise stereoselektiven Vertauf von Umsetzungen bewirken. Charakteristisch für Schutzgruppen ist, daß sie leicht, d. h. ohne unerwünschte Nebenreaktionen abspaltbar sind, beispielsweise solvolytisch, reduktiv, photolytisch oder auch enzymatisch, z.B. unter physiologischen Bedingungen. Schutzgruppen können aber auch in den Endstoffen vorhanden sein. Verbindungen der Formel I mit geschützten funktionellen Gruppen können eine höhere metabolische Stabilität oder anderweitig verbesserte pharmakodynamische Eigenschaften aufweisen als die entsprechenden Verbindungen mit freien funktionellen Gruppen.

Der Schutz von funktionellen Gruppen durch solche Schutzgruppen, die Schutzgruppen selbst, sowie ihre Abspaltungsreaktionen sind beispielsweise in Standardwerken wie J. F. W. McOmie, "Protective Groups in Organic Chemistry", Plenum Press, London und New York 1973, in Th. W. Greene, "Protective Groups in Organic Synthesis". Wiley, New York 1981, in "The Peptides"; Band 3 (E. Gross und J. Meienhofer, Herausg.), Academic Press, London und New York 1981, in "Methoden der organischen Chemie", HoubenWeyl, 4. Auflage, Band 15/I, Georg Thieme Verlag, Stuttgart 1974, in H.-D. Jakubke und H. Jescheit, "Aminosäuren, Peptide, Proteine", Verlag Chemie, Weinheim, Deerfield Beach und Basel 1982, und in Jochen Lehmann, "Chemie der Kohlenhydrate: Monosaccharide und Derivate", Georg Thieme Verlag, Stuttgart 1974, beschrieben.

Eine Carboxygruppe ist z.B. als eine Estergruppe geschützt, die unter schonenden Bedingungen selektiv spaltbar ist. Eine in veresterter Form geschützte Carboxygruppe ist in erster Linie durch eine Niederalkylgruppe verestert, welche bevorzugt in 1-Stellung der Niederalkylgruppe verzweigt oder in 1- oder 2-Stellung der Niederalkylgruppe durch geeignete Substituenten substituiert ist, beispielsweise Methoxycarbonyl oder Ethoxycarbonyl, tert.-Niederalkoxycarbonyl, z.B. Tertiärbutyloxycarbonyl, Arylmethoxycarbonyl mit einem oder zwei Arylresten, worin Aryl unsubstituiertes oder z.B. durch Niederalkyl, z.B. tert.-Niederalkyl, wie Tertiärbutyl, Niederalkoxy, z.B. Methoxy, Hydroxy, Halogen, z.B. Chlor, und/oder Nitro mono-, di- oder trisubstituiertes Phenyl bedeutet, beispielsweise Benzyloxycarbonyl, durch die genannten Substituenten substituiertes Benzyloxycarbonyl, z.B. 4-Nitrobenzyloxycarbonyl oder 4-Methoxybenzyloxycarbonyl, Diphenylmethoxycarbonyl oder durch die genannten Substituenten substituiertes Diphenylmethoxycarbonyl, z.B. Di-(4-methoxyphenyl)-methoxycarbonyl, 1-Niederalkoxyniederalkoxycarbonyl, z.B. Methoxymethoxycarbonyl, 1-Methoxyethoxycarbonyl oder 1-Ethoxyethoxycarbonyl, 1-Niederalkylthioniederalkoxycarbonyl, z.B. 1-Methylthiomethoxycarbonyl oder 1-Ethylthioethoxycarbonyl, Aroylmethoxycarbonyl, worin die Aroylgruppe gegebenenfalls beispielsweise durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Jodethoxycarbonyl, 2-Triniederalkylsilylniederalkoxycarbonyl, wie 2-Triniederalkylsilylethoxycarbonyl, z.B. 2-Trimethylsilylethoxycarbonyl oder 2-(Di-n-butyl-methyl-silyl)-ethoxycarbonyl, oder 2-Triarylsilylethoxycarbonyl, wie Triphenylsilylethoxycarbonyl.

Eine Carboxygruppe kann auch als Triniederalkylsilyloxycarbonylgruppe, z.B. Trimethylsilyloxycarbonyl, geschützt sein.

Eine geschützte Carboxygruppe ist bevorzugt Tertiärniederalkoxycarbonyl, z.B. Tertiärbutoxycarbonyl, Benzyloxycarbonyl, 4-Nitrobenzyloxycarbonyl, 9-Fluorenylmethoxycarbonyl oder Diphenylmethoxycarbonyl.

Eine geschützte Aminogruppe kann z.B. in Form einer Acylamino-, Arylmethylamino-, veretherten Mercaptoamino-, 2-Acyl-niederalk-1-enylamino oder Silylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Niederalkancarbonsäure oder gegebenenfalls, z.B. durch Halogen, Niederalkoxy oder Nitro, substituierten Benzoesäure, oder bevorzugt eines Kohlensäurehalbesters. Solche Acylgruppen sind beispielsweise Niederalkanoyl, wie Formyl, Acetyl, Propionyl oder Pivaloyl, Halogenniederalkanoyl, z.B. 2-Halogenacetyl, wie 2-Chlor-, 2-Brom-, 2-Jod-, 2,2,2-Trifluor- oder 2,2,2-Trichloracetyl, gegebenenfalls z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, beispielsweise Tertiärniederalkoxycarbonyl, wie Tertiärbutoxycarbonyl, Arylmethoxycarbonyl mit einem oder zwei Arylresten, die gegebenenfalls, z.B. durch Niederalkyl, z.B. tert.-Niederalkyl, wie Tertiärbutyl, Niederalkoxy, wie Methoxy, Hydroxy, Halogen, wie Chlor, und/oder Nitro mono- oder polysubstituiertes Phenyl darstellen, z.B. Benzyloxycarbonyl, gegebenenfalls substituiertes Benzyloxycarbonyl, wie 4-Nitrobenzyloxycarbonyl, Diphenylmethoxycarbonyl, Fluorenylmethoxycarbonyl oder substituiertes Diphenylmethoxycarbonyl, wie Di-(4-methoxyphenyl)methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Jodethoxycarbonyl, 2-(trisubstituiertes Silyl)-niederalkoxycarbonyl, z.B.2-Triniederalkylsilylniederalkoxycarbonyl, z.B. 2-Trimethylsilylethoxycarbonyl oder 2-(Di-n-butyl-methyl-silyl)-ethoxycarbonyl, oder Triarylsilylniederalkoxycarbonyl, z.B. 2-Triphenylsilylethoxycarbonyl.

In einer Arylmethylaminogruppe, die z.B. eine Mono-, Di- oder insbesondere Triarylmethylaminogruppe darstellt, sind die Arylreste insbesondere gegebenenfalls substituierte Phenylreste. Solche Gruppen sind z.B. Benzyl-, Diphenylmethyl- oder insbesondere Tritylamino.

In einem als Aminoschutzgruppe verwendbaren 2-Acyl-niederalk-1-enylrest ist Acyl z.B. der entsprechende Rest einer Niederalkancarbonsäure, einer gegebenenfalls z.B. durch Niederalkyl, wie Methyl oder Tertiärbutyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder Niederalkoxycarbonyl-prop-1-en-2-yl, z.B. 1-Ethoxycarbonyl-prop-1-en-2-yl.

Eine Silylaminogruppe ist beispielsweise eine Triniederalkylsilylaminogruppe, z.B. Trimethylsilylamino. Das Siliziumatom der Silylaminogruppe kann auch nur durch zwei Niederalkylgruppen, z.B. Methylgruppen, und die Aminogruppe oder Carboxylgruppe eines zweiten Moleküls der Formel I substituiert sein. Verbindungen mit solchen Schutzgruppen lassen sich z.B. mit Dimethylchlorsilan als Silylierungsmittel herstellen.

Eine Aminogruppe kann auch durch Überführung in die protonierte Form geschützt werden; als entsprechende Anionen kommen in erster Linie diejenigen von starken anorganischen Säuren, wie von Schwefelsäure, Phosphorsäure oder Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere Tertiärbutoxycarbonyl oder Fluorenylmethoxycarbonyl, gegebenenfalls substituiertes Benzyloxycarbonyl, z.B. 4-Nitrobenzyloxycarbonyl, Diphenylmethoxycarbonyl, 2-Halogenniederalkoxycarbonyl, z.B. 2,2,2-Trichlorethoxycarbonyl, ferner Trityl oder Formyl.

Eine Hydroxygruppe kann beispielsweise durch eine Acylgruppe, z.B. durch Halogen, wie Chlor, substituiertes Niederalkanoyl, z.B. 2,2-Dichloracetyl, oder insbesondere durch einen für geschützte Aminogruppen genannten Acylrest eines Kohlensäurehalbesters geschützt sein. Eine bevorzugte Hydroxyschutzgruppe ist beispielsweise 2,2,2-Trichlorethoxycarbonyl, 4-Nitrobenzyloxycarbonyl, Diphenylmethoxycarbonyl oder Trityl. Eine Hydroxygruppe kann ferner durch Triniederalkylsilyl, z.B. Trimethylsilyl, Triisopropylsilyl oder Dimethyl-tert.-butylsilyl, eine leicht abspaltbare verethernde Gruppe, z.B. eine Alkylgruppe, wie tert.-Niederalkyl, z.B. Tertiärbutyl, einen oxa- oder einen thia-aliphatischen oder -cycloaliphatischen, insbesondere 2-oxa- oder 2-thia-aliphatischen oder -cycloaliphatischen, Kohlenwasserstoffrest, beispielsweise 1-Niederalkoxyniederalkyl oder 1-Niederalkylthioniederalkyl, z.B. Methoxymethyl, 1-Methoxyethyl, 1-Ethoxyethyl, Methylthiomethyl, 1-Methylthioethyl oder 1-Ethylthioethyl, oder 2-Oxa- oder 2-Thiacycloalkyl mit 5-7 Ringatomen, z.B. 2-Tetrahydrofuryl oder 2-Tetrahydropyranyl, oder ein entsprechendes Thia-analoges, sowie durch 1-Phenylniederalkyl, z.B. Benzyl, Diphenylmethyl oder Trityl, wobei die Phenylreste beispielsweise durch Halogen, z.B. Chlor, Niederalkoxy, z.B. Methoxy, und/oder Nitro substituiert sein können, geschützt sein.

Zwei in einem Molekül vorkommende, insbesondere benachbarte Hydroxygruppen oder eine benachbarte Hydroxy- und Aminogruppe können beispielsweise durch bivalente Schutzgruppen, wie eine vorzugsweise, etwa durch ein oder zwei Alkylreste, substituierte Methylengruppe, geschützt sein. An der benachbarten Hydroxy- und Aminogruppe durch eine bivalente Schutzgruppe intermediär geschützte Verbindungen der Formel II sind vorzugsweise solche der Formel II' 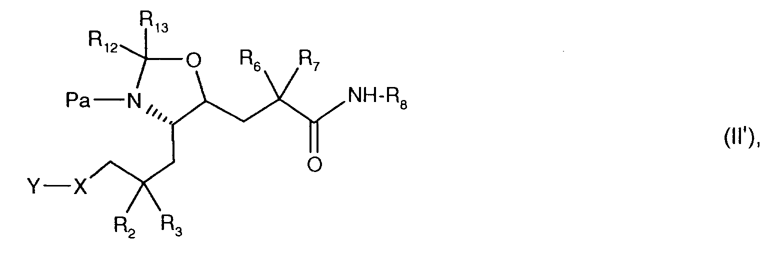
worin Y, X, R₂, R₃, R₄, R₅, R₆, R₇ und R₈ die oben genannten Bedeutungen haben, Pa eine der vorstehend genannten Aminoschutzgruppen ist und die Gruppe >C(R₁₂)(R₁₃) Carbonyl oder insbesondere unsubstituiertes oder substituiertes Alkyliden, wie Niederalkyliden, z.B. Isopropyliden, Cycloalkyliden, z.B. Cyclohexyliden, eine Carbonylgruppe oder Benzyliden, bedeutet.

Die Ausgangsmaterialien zur Durchführung des Verfahrens a) können nach an sich bekannten Verfahren hergestellt werden.

So kann man Verbindungen der Formel II herstellen, indem man in einer Verbindung der Formel IIa 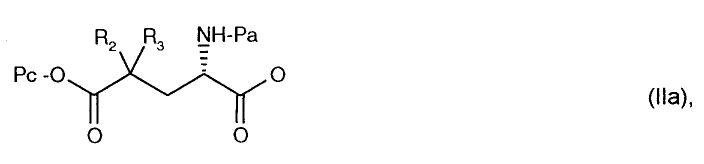
worin Pc eine Carboxyschutzgruppe und Pa eine univalente Aminoschutzgruppe bedeutet und R₂ und R₃ die genannten Bedeutungen haben, die freie Carboxygruppe zu Hydroxymethyl reduziert, die erhaltene Verbindung der Formel IIb 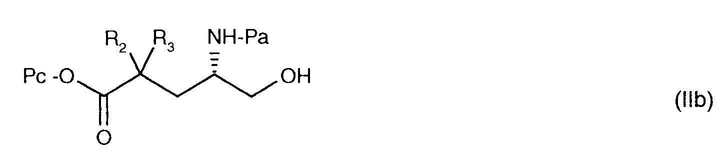
mit einem vorstehend genannten Reagenz umsetzt, welches eine bivalente Schutzgruppe unter Erhalt einer Verbindung der Formel IIc 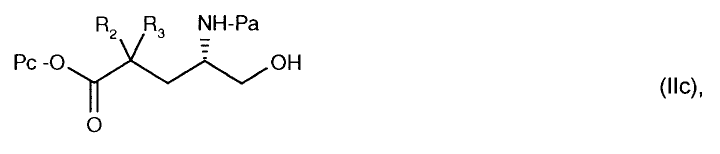
worin R₁₂ und R₁₃ unabhängig voneinander Wasserstoff oder Alkyl, insbesondere Niederalkyl, z.B. Methyl, oder gemeinsam Oxo bedeuten, einführt, in der so erhältlichen Verbindung, sofern R₂ und R₃ Wasserstoff bedeuten, gewünschtenfalls Wasserstoff durch die oben genannten Reste R₂ und/oder R₃ außer Wasserstoff ersetzt, die geschützte Carboxygruppe unter Abspaltung der Schutzgruppe Po zu Hydroxymethyl reduziert, die so erhältliche Verbindung der Formel IId 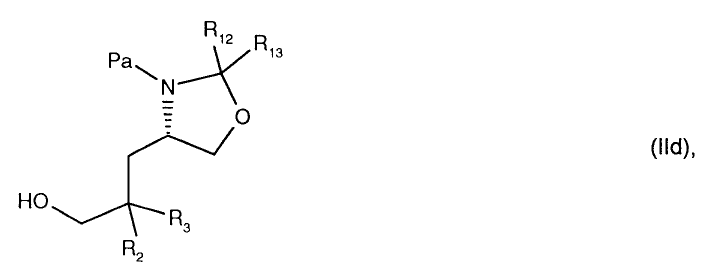
worin die Reste die genannten Bedeutungen haben, zum entsprechenden Aldehyd oxidiert diesen mit einem Reagenz umsetzt, das den Sauerstoff der Aldehydgruppe durch eine Methylengruppe ersetzt, die so erhältliche Verbindung der Formel IIe 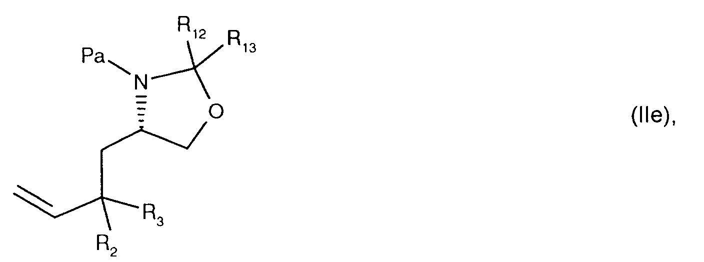
worin die Reste die genannten Bedeutungen haben, entgegen der Regel von Markovnikov hydratisiert, die so erhältliche Verbindung der Formel IIf 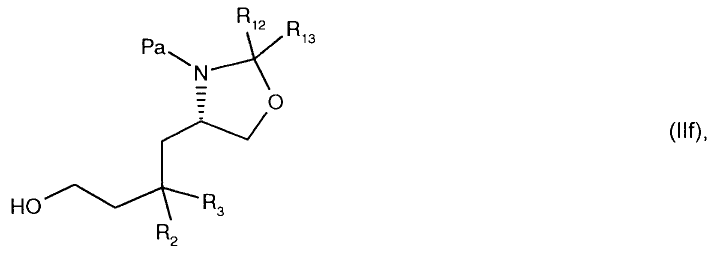
worin die Reste die genannten Bedeutungen haben, mit einem Reagenz umsetzt, das eine Hydroxyschutzgruppe Po einführt, die bivalente Schutzgruppe, welche die vicinalen Hydroxy- und Aminofunktionen überbrückt, abspaltet, die so erhältliche Verbindung der Formel IIg 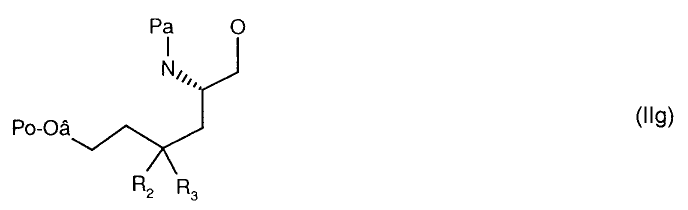
worin die Reste die genannten Bedeutungen haben, zum entsprechenden Aldehyd oxidiert, den Aldehyd der Formel IIh 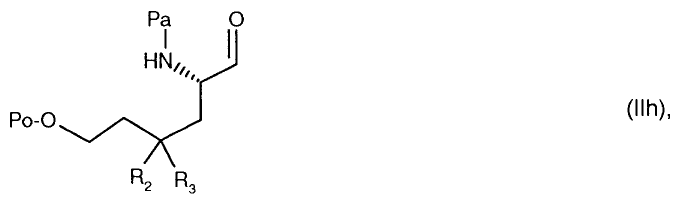
worin die Substituenten die angegebenen Bedeutungen haben, mit einer Verbindung der Formel IIi 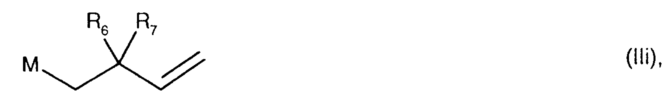
worin M einen metallischen Rest, beispielsweise eine Halogenmagnesiumgruppe, bedeutet, zur entsprechenden Verbindung der Formel IIj 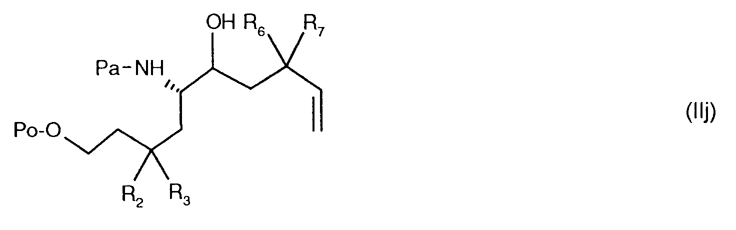
umsetzt, diese durch eine bivalente Schutzgruppe der Formel >C(R₁₂)(R₁₃) schützt, die terminale Vinylgruppe, beispielsweise mittels Ru-III-chlorid/Kaliummetaperjodat oder Kaliumpermanganat, zu Carboxy oxidiert, wobei man eine Säure der Formel IIk 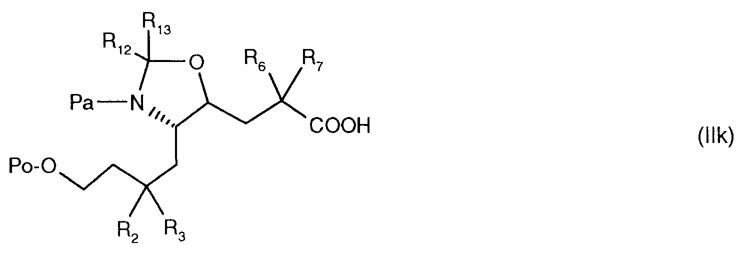
erhält, die dann durch Umsetzung mit einem Amin V in das entsprechende Zwischenprodukt der Formel IIm 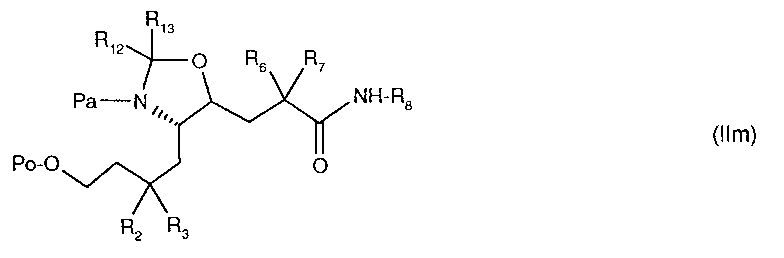
überführt.

Diese Stufe bietet auch eine gute Möglichkeit der Konfigurationsumkehr an dem die Oxyfunktion tragenden C-Atom der Hauptkette. Dazu wird die bivalente Schutzgruppe der Formel >C(R₁₂)(R₁₃) und die Aminoschutzgruppe Pa entfernt, die erhaltene Verbindung der Formel II'm 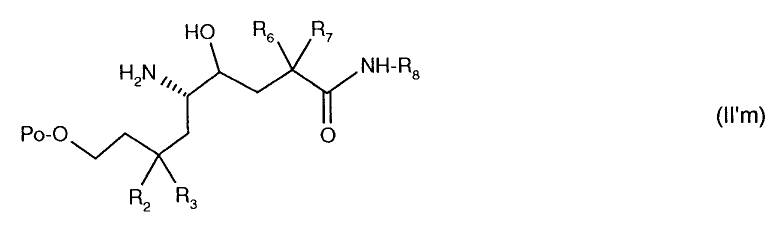
zunächst an der Aminogruppe durch eine von einem Halbester der Kohlensäure abgeleitete Acylgruppe, beispielsweise Benzyloxycarbonyl, N-substituiert, dann unter Bedingungen, welche die Hydroxygruppe intermediär in eine nukleofuge Abgangsgruppe, wie Chlor, überführen, z.B. in Gegenwart von Thionylchlorid, unter Konfigurationsumkehr an dem vorher die Hydroxygruppe tragenden C-Atom zur entsprechenden Oxazolidinonverbindung der Formel II''m 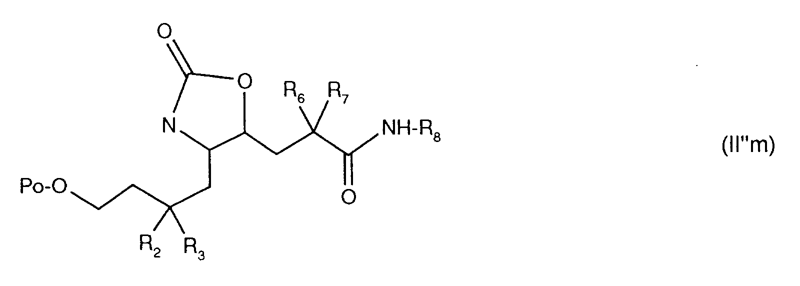
cyclisiert und anschließend unter Bildung des Zwischenproduktes IIm mit der gewünschten Stereotaxie erneut eine Aminoschutzgruppe Pa eingeführt.

Um Ausgangsstoffe der Formel II 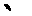 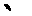 zu erhalten, worin Y freies oder reaktionsfähiges funktionell abgewandeltes Hydroxy und X Methylen ist, wird im Zwischenprodukt IIm die Schutzgruppen Po abgespalten und die freigesetzte terminale Hydroxygruppe gewünschtenfalls reaktionsfähig funktionell abgewandelt. Soweit nach der Natur von Y möglich, kann man durch anschließende Abspaltung der bivalenten Schutzgruppe >C(R₁₂)-(R₁₃) sowie der Aminoschutzgruppe Pa die entsprechende Verbindung der Formel II erhalten werden kann.

Um Ausgangsstoffe der Formel II zu erhalten, worin das Fragment Y-X- freies oder funktionell abgewandeltes Carboxy ist, wird im Zwischenprodukte IIm zunächst die Schutzgruppe Po abgespalten, die freigesetzte Hydroxyverbindung zu Carboxy oxidiert, die Carboxygruppe gewünschtenfalls funktionell abgewandelt. Soweit nach der Natur von Y möglich, kann man durch anschließende Abspaltung der bivalenten Schutzgruppe >C(R₁₂)-(R₁₃) sowie der Aminoschutzgruppe Pa die entsprechende Verbindung der Formel II erhalten.

Gewünschtenfalls kann anschließend jeweils unter Reaktion mit einer entsprechenden Verbindung der Formel R₄-S, worin S eine Abgangsgruppe oder eine Hydroxyfunktion ist, jeweils einen von Wasserstoff verschiedenen Rest R₄ einführen und/oder mit einem acylierenden Reagenz Hydroxy R₅ acylieren.

Für Schlüsselverbindungen der vorstehend beschriebenen Reaktionsfolge stehen einige vorteilhafte Bildungsweisen zur Verfügung.

So kann man zu Herstellung von Verbindungen der Formel IIm, worin R₆ Alkyl oder Cycloalkyl und R₇ Wasserstoff bedeutet, wobei die übrigen Substituenten die genannten Bedeutungen haben, den Aldehyd der Formel IIh mit einem Amid der Formel IIn 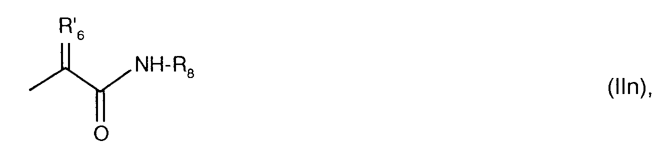
worin R 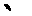 ₆ Alkyliden oder Cycloalkyliden bedeutet und R₈ die genannte Bedeutung hat, umsetzen und die erhaltene Verbindung der Formel IIo 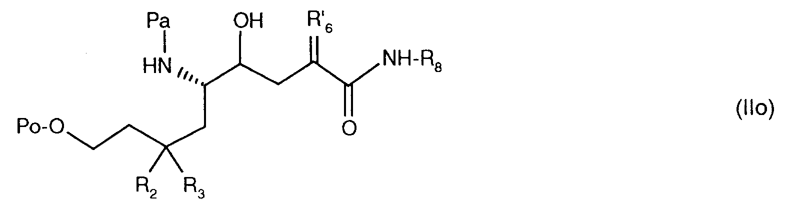
gewünschtenfalls in die Stereoisomeren bezüglich des die Hydroxygruppe tragenden C-Atomes auftrennen, anschließend die Gruppe der Formel >C=R 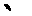 ₆ zur gewünschten Gruppe der Formel >CH-R₆ reduziert, wobei die Reduktion vorteilhaft stereoselektiv bezüglich des die Gruppe der Formel >CH-R₆ tragenden C-Atomes durchgeführt wird, dann die Hydroxy- und Aminogruppe durch eine bivalente Schutzgruppe der Formel >C(R₁₂)(R₁₃) schützen, wobei man die entsprechende Verbindung der Formel II'm, worin die Reste die genannten Bedeutungen haben, erhält.

Ebenso kann man Zwischenprodukte der Formel IIh, worin mindestens einer der Reste R₂ und R₃ von Wasserstoff verschieden ist, vorteilhaft auch ausgehend von entsprechenden Verbindungen der Formel IIp 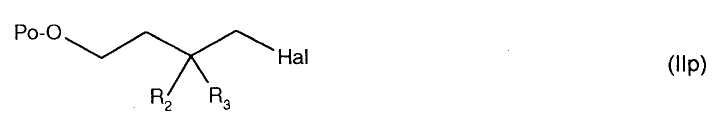
durch Umsetzung mit einem üblicherweise zum Aufbau von geschützten Aminosäurederivaten verwendeten chiralen oder achiralen nukleophilen Synthesebaustein, beispielsweise mit einer Verbindung der Formel IIq 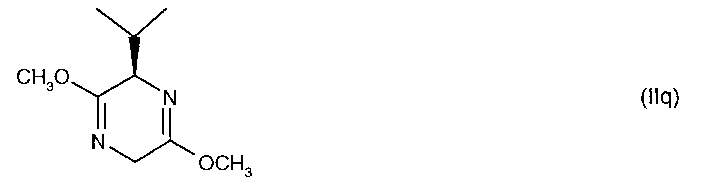
in Gegenwart einer Metallbase, z.B. von Butyllithium, vorteilhaft in Tetrahydrofuran, Hydrolyse der erhaltenen Verbindung der Formel IIr 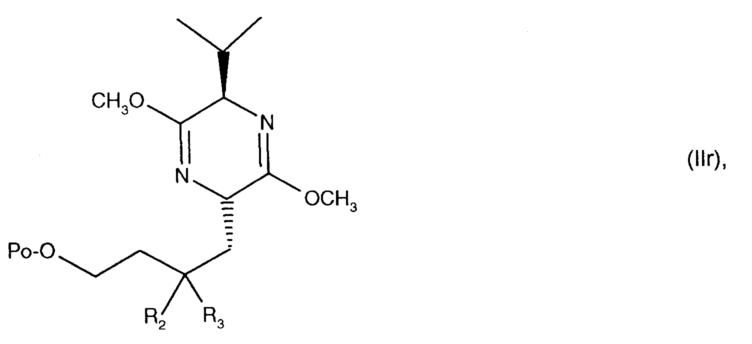
gefolgt von der Einführung der univalenten Aminoschutzgruppe Pa, und Reduktion der gebildeten Methoxycarbonylgruppe in einem so erhältlichen Zwischenprodukt der Formel IIs 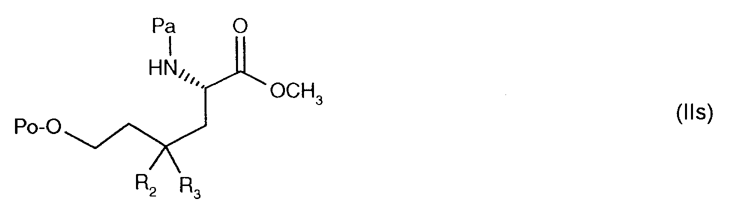
zu Formyl herstellen.

Als Carboxyschutzgruppe Pc in Verbindungen der Formel IIa dient insbesondere eine der oben genannten Niederalkoxygruppen, z.B. Methoxy, Ethoxy oder Tertiärbutyloxy. Die Einführung einer Aminoschutzgruppe Pa erfolgt wie oben beschrieben, insbesondere durch Umsetzung mit einer Niederalkoxycarbonylverbindung, wie Diniederalkoxydicarbonat, z.B. Di-tert.-butyldicarbonat, bevorzugt in wäßriger Lösung, der gegebenenfalls wasserlösliche organische Lösungsmittel, wie cyclische Ether, z.B. Dioxan, und eine Base, beispielsweise eine schwache Base wie Alkalimetallhydrogencarbonat, z.B. Natriumbicarbonat, beigefügt sind, bei Temperaturen von 0° bis 100° C, bevorzugt etwa bei Raumtemperatur. Die Reduktion zu Verbindungen der Formel IIc erfolgt in inerten organischen Lösungsmitteln, z.B. Ethern, wie Tetrahydrofuran, gegebenenfalls nach Aktivierung der zu reduzierenden Carboxygruppe, wie unter Verfahren a) beschrieben, z.B. als Carbonsäurehydrazid, wie Carbonsäurechlorid, mit einem geeigneten Reduktionsmittel, z.B. komplexen Hydriden, wie Lithiumborhydrid, bei Temperaturen von -50° bis 50° C, bevorzugt -30° bis 20° C.

Die Umsetzung von Verbindungen der Formel IIb bzw. IIj zur Einführung bivalenter Schutzgruppen unter Erhalt von Verbindungen der Formel IIc bzw. IIk erfolgt, wie vorstehend zur Einführung der entsprechenden Schutzgruppen beschrieben, bevorzugt in inerten Lösungsmitteln, wie Halogenalkanen, z.B. Dichlormethan, unter Verwendung von geeigneten Reagenzien, insbesondere Enolethern, wie Niederalkyloxyniederalkenen, z.B. Isopropenylmethylether, bei Temperaturen von -50° bis 50° C, insbesondere von -10° bis 40°C.

Die Umsetzung von Verbindungen der Formel IIc zur Einführung von Resten R₂ und/oder R₃ außer Wasserstoff erfolgt in Gegenwart von Reagenzien, die die Wasserstoffatome R₂ und R₃ als Protonen unter Bildung eines Carbanions abspalten können, wie starken Basen, z.B. Hexaniederalkyl-, wie Hexamethyldisilazan und/oder Niederalkyl-, wie Butyllithium, in organischen Lösungsmitteln, wie Ethern, z.B. Tetrahydrofuran, und/oder Kohlenwasserstoffen, wie Hexan, unter Einführung der Reste R₂ und R₃ als Verbindungen mit guten Abgangsgruppen, insbesondere Halogenide, z.B. Niederalkylhalogenide, wie Methyljodid, bei Temperaturen zwischen -100° und 50° C, bevorzugt zwischen -80° und 20° C.

Die Reduktion der durch Pc geschützten Carboxygruppe unter Schutzgruppenabspaltung zu Verbindungen der Formel IId erfolgt mit geeigneten Reduktionsmitteln, insbesondere mit Hydrierungsmitteln, wie komplexen Hydriden, z.B. Lithiumaluminiumhydrid, in inerten Lösungsmitteln, wie Ethern, z.B. Tetrahydrofuran, bei Temperaturen von -50° bis 50° C, insbesondere von -20° bis 30° C.

Die Oxidation der Hydroxymethylgruppe in Verbindungen der Formel IId zu Formyl erfolgt beispielsweise durch Umsetzung mit milden Oxidationsmitteln, die eine Umsetzung der Hydroxy- in eine Aldehydfunktion ermöglichen, z.B. nach Überführung der Hydroxyfunktion in ein Halogenid, z.B. Chlorid, beispielsweise mittels eines Säurehalogenides, wie Oxalylchlorid, mit Diniederalkylsulfoxiden, z.B. Dimethylsulfoxid, in inerten Lösungsmitteln, z.B. Methylenchlorid, bei Temperaturen von - 100° bis 50° C, bevorzugt -80° bis 20° C.

Zur Überführung der Formylgruppe in Vinyl zur Herstellung des Zwischenproduktes IIe werden Reagenzien eingesetzt, die aus den entsprechenden Verbindungen, die die Methylengruppe einführen können, Protonen unter Bildung eines entsprechenden Ylides, z.B. Methyltriarylphosphoniumsalzes, wie Methyltriphenylphosphoniumsalzes, abspalten können, beispielsweise starke Basen, z.B. Hexaniederalkyl-, wie Hexamethyldisilazan und/oder Niederalkyl-, wie Butyllithium, in organischen Lösungsmitteln, wie Ethern, z.B. Tetrahydrofuran, und/oder Kohlenwasserstoffen, wie Hexan, bei Temperaturen von -80° bis 100° C, insbesondere von -50° bis 50° C.

Die Hydratisierung von Verbindungen der Formel IIe entgegen der Regel von Markovnikov erfolgt insbesondere durch Hydroborierung, beispielsweise mit Diboran, das in situ erzeugt wird, oder bevorzugt mit Boran-Dimethylsulfid-Komplex, in organischen Lösungsmitteln, wie Ethern, z.B. Tetrahydrofuran, bei Temperaturen zwischen -20° bis 30° C, und anschließende Hydrolyse der entsprechenden Borverbindung durch Zugabe einer wäßrigen Lösung eines organischen Lösungsmittels, wie Ethers, z.B. Tetrahydrofuran in Wasser, gegebenenfalls unter Zusatz einer Base, wie einer Lauge, z.B. eines Alkalimetallhydroxides, wie Natriumhydroxid, und von Wasserstoffperoxid bei Temperaturen von -20° bis 50° C, bevorzugt -10° bis 30° C.

Die Einführung einer Hydroxyschutzgruppe in Verbindungen der Formel IIf erfolgt wie vorstehend beschrieben, insbesondere durch Benzylierung, z.B. mit Benzylbromid, oder unter Verwendung eines Triniederalkylchlorsilans, wie Triisopropylchlorsilan, in inerten Lösungsmitteln, z.B. Methylenchlorid, in Gegenwart von Basen, z.B. Imidazol, und/oder Katalysatoren, wie Dimethylaminopyridin, bei Temperaturen von etwa -20° bis 50° C.

Die Abspaltung der bivalenten Schutzgruppe aus Verbindungen der Formel IIf erfolgt beispielsweise nach einer der nachstehend für die Schutzgruppenabspaltung aufgeführten Methoden, insbesondere in Gegenwart von Salzen organischer Basen und Säuren, wie Pyridinium-p-toluolsulfonat, in polaren organischen Lösungsmitteln, wie Alkoholen, z.B. Methanol, bei Temperaturen von 0° bis 60° C.

Die Oxidation der freien Hydroxygruppe in Verbindungen der Formel IIg erfolgt beispielsweise wie oben für Verbindungen der Formel IId angegeben.

Die Herstellung von Verbindungen der Formel IIo durch Umsetzung von Verbindungen der Formeln IIh und IIn erfolgt insbesondere in Gegenwart von starken Basen, wie Metallalkylen, beispielsweise Niederalkylalkalimetallverbindungen, z.B. Butyllithium, in Gegenwart geeigneter Lewissäuren, z.B. von Chlortitantriisopropyloxid, und in organischen Lösungsmitteln, wie Kohlenwasserstoffen, z.B. Hexan, bei Temperaturen von -100° bis 50° C, wobei die Verbindung der Formel IIn deprotoniert wird.

Anschließend erfolgt die Reduktion des Zwischenproduktes IIo, insbesondere durch Hydrogenolyse, bevorzugt mit katalytisch angeregtem Wasserstoff in Gegenwart eines Metall-, beispielsweise Edelmetall-, wie Palladiumkatalysators, z.B. Palladiumkohle, in inerten Lösungsmitteln, z.B. Ethern, wie Tetrahydrofuran, bei Temperaturen von -50° bis 50° C, gegebenenfalls unter erhöhtem Druck. Vorteilhaft kann man die Hydrogenolyse in Gegenwart eines geeigneten optisch aktiven Edelmetall-Ligand-Komplexes stereoselektiv durchführen. Solche Katalysatoren sind insbesondere Komplexe von Ruthenium oder Rutheniumsalzen, wie Ru-II-halogeniden, wie RuCl₂. Ru₂Cl₄ oder RuHCl, gegebenenfalls halogenierten Ru-II-niederalkanoylaten, wie Ru(OAc)₂ oder Ru(OOC-CF₃)₂, mit (S)-Bis(2,2'-diphenylphosphino)-1,1'-binaphthyl (S-BINAP) oder Derivaten davon, die anstelle von Phenyl substituierte Phenylreste, wie p-Toluyl oder p-Methoxyphenyl, enthalten, ebenso Rutheniumkomplexe mit (S)-Bis(2,2'-diphenylphosphino)5,5'-dimethyl-diphenyl und dergl. Bei der Hydrierung mit Komplexen dieser Art arbeitet man vorzugsweise in Alkoholen, wie Niederalkanolen, oder Alkylhaliden, wie Methylenchlorid, im Druckbereich von etwa 1 bis 100, vorzugsweise 20 bis 30. bar und im Temperaturbereich von etwa 10° bis 80° C, vorzugsweise 15 bis 25° C. Insbesondere zu nennen ist z.B. Bis[(S)-(-)-2,2 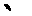 -bis(diphenylphosphino)-1,1 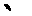 -binaphthyl](triethylamino)diruthenium-tetrachlorid.

Die Umsetzung von Verbindungen der Formel IIo mit einem Reagenz zur Einführung einer bivalenten Schutzgruppe erfolgt analog der Umsetzung der Verbindungen der Formel IIc oder bevorzugt unter Verwendung von Diniederalkylketalen von Diniederalkylketonen, wie Aceton, z.B. Dimethoxypropan, in inerten Lösungsmitteln, wie Methylenchlorid, in Gegenwart von Säuren, wie p-Toluolsulfonsäure, bei Temperaturen von 0° bis 50° C. Die Abspaltung der Hydroxyschutzgruppe Po erfolgt beispielsweise unter den vorstehend genannten Bedingungen, bevorzugt unter Verwendung von Ammoniumfluoridderivaten, wie Tetraniederalkylammoniumfluoriden, z.B. Tetrabutylammoniumfluorid, in inerten Lösungsmitteln, z.B. Methylenchlorid, bei Temperaturen von 0° bis 50° C, oder durch Hydrierung.

Verbindungen der Formel III sind bekannt oder können nach an sich bekannten Verfahren hergestellt werden.

Beispielsweise können die Verbindungen der Formel III, die Heterocyclyl R₁ enthalten, hergestellt werden, indem ungesättigte unkondensierte oder kondensierte, unsubstituierte oder wie oben beschrieben substituierte Heterocyclylreste durch Umsetzung mit Reduktionsmitteln, wie Wasserstoff in Gegenwart von Hydrierungskatalysatoren, teilweise oder völlig gesättigt werden, wobei die teilweise oder völlig gesättigten Heterocyclen an dem Stickstoffatom, das bei der Kondensation mit Resten der Formel II zu Verbindungen der Formel I reagieren soll, und gegebenenfalls an weiteren funktionellen Gruppen durch Umsetzung mit Reagenzien, die Schutzgruppen einführen, geschützt werden oder geschützt vorliegen, und die erhältlichen Verbindungen, sofern sie Carboxygruppen als Substituenten enthalten, mit Reagenzien umgesetzt werden, die die Carboxygruppen a) verestern zu den entsprechenden Carbonsäureestern; und/oder b) amidieren zu den entsprechenden unsubstituierten oder substituierten Carbamoylgruppen; und/oder c) reduzieren zu Formylgruppen oder zu Hydroxymethylgruppen, welche gegebenenfalls durch oxidierende Reagenzien zu Formylgruppen umgesetzt werden, wonach die erhältlichen Verbindungen mit Formylgruppen durch Metallalkyle alkyliert und die entstehenden Hydroxyalkylverbindungen reduziert und dann zu den entsprechenden Carboxyalkylverbindungen oder direkt zu den entsprechenden Carboxyalkylverbindungen oxidiert und dann gegebenenfalls an den Doppelbindungen reduziert werden.

Die Kondensation von Verbindungen mit der Formel IV mit Verbindungen der Formel V gemäß der Verfahrensvariante b) sowie die Vor- und Nachbehandlung erfolgen in der unter a) angegebenen Weise mit zu den dort beschriebenen, als Acylierungsmittel verwendbaren reaktionsfähigen Carbonsäurederivaten analogen Verbindungen, der dort beschriebenen Kondensation zur Herstellung der Amidbildung und den dort beschriebenen Schutzgruppen und Verfahren zur Abspaltung der Schutzgruppen, sofern diese nicht Bestandteil des gewünschten Endproduktes der Formel I sind.

Eine besondere Variante dieses Verfahrens dient der Herstellung solcher Verbindungen der Formel I, in denen R₅ Hydroxy bedeutet und die übrigen Reste die oben angegebenen Bedeutungen haben. Als aktivierte Ester werden hier die inneren Ester (γ-Lactone) der Verbindungen der Formel IV mit der Formel IVa 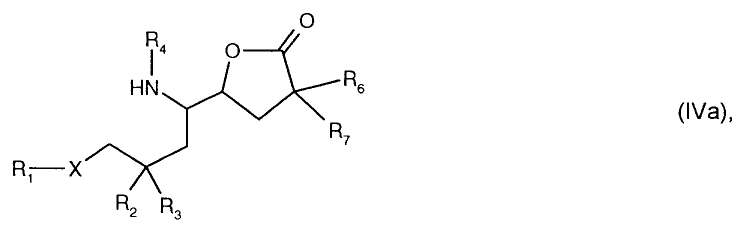
mit den Verbindungen der Formel V umgesetzt, wobei in den Reaktionskomponenten vorhandene freie funktionelle Gruppen mit Ausnahme der an der Reaktion teilnehmenden Gruppen gegebenenfalls, wie oben dargelegt, in geschützter Form vorliegen und die vorhandenen Schutzgruppen wie oben beschrieben abgespalten werden. Die Kondensation unter Bildung der Amidbindung erfolgt unter den geeigneten Bedingungen nach Verfahrensvariante a). Insbesondere kann ein γ-Lacton der Formel IVa mit einem primären Amin der Formel V ohne Lösungsmittel oder in Gegenwart eines polaren Lösungsmittels, z.B. einem niederen Alkohol, wie Methanol oder Ethanol, einem polaren Ether, wie Tetrahydrofuran oder Dioxan, einem Nitril, wie Acetonitril, einem Amid, wie Dimethylformamid, N,N-Dimethylacetamid, N-Methylpyrrolidon oder Hexamethylphosphorsäuretriamid, einem Harnstoff, z.B. N,N'-Dimethyl-N,N'-propylenylharnstoff, einem Niederalkoxyniederalkanol, z.B. Diethylenglykolmonomethylether, in Dimethylsulfoxid oder einem Gemisch der genannten Lösungsmittel oder einem Gemisch eines oder mehrerer der genannten Lösungsmittel mit Wasser bei Temperaturen von -30° bis 100° C, bevorzugt von 20° bis 80° C, umgesetzt werden, wobei für die Schutzgruppen das oben Gesagte gilt.

Die Ausgangsmaterialien für die Verfahrenvariante b) können nach an sich bekannten Methoden, insbesondere in Analogie zu den vorstehend beschriebenen Bildungswesen von Verbindungen der Formel II beschrieben, hergestellt werden, beispielsweise indem man einen Aldehyd der Formel IIh 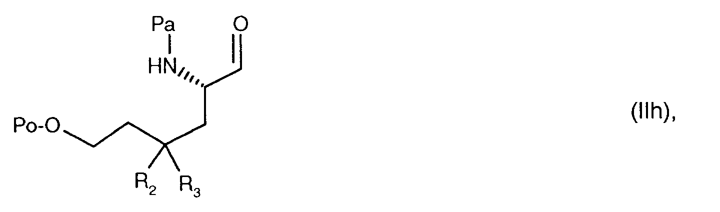
worin die Substituenten die angegebenen Bedeutungen haben, mit einer metallorganischen Verbindung der Formel IVb 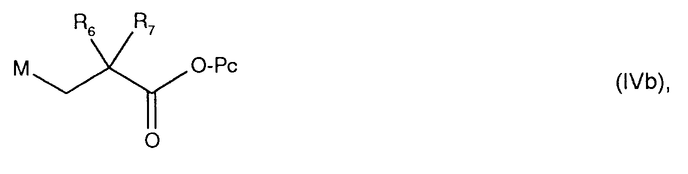
worin M einen metallischen Rest, beispielsweise eine Halogenzinkgruppe, und Pc eine der unter Verfahrensvariante a) erwähnten Carboxyschutzgruppen bedeutet, in Gegenwart einer geeigneten Lewissäure zur entsprechenden Verbindung der Formel IVc 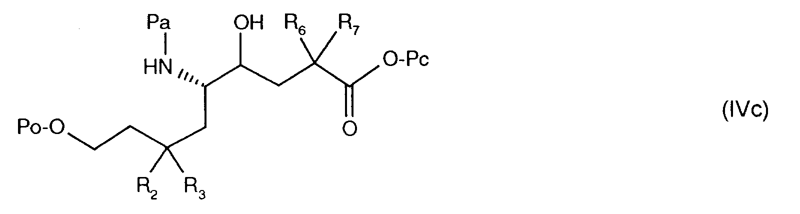
umsetzt, diese wie unter der Verfahrensvariante a) beschrieben durch eine bivalente Schutzgruppe schützt, die Gruppe Po-O-CH₂-in einen Rest der Formel R₁-X- überführt und aus der erhaltenen Verbindung der Formel IVd 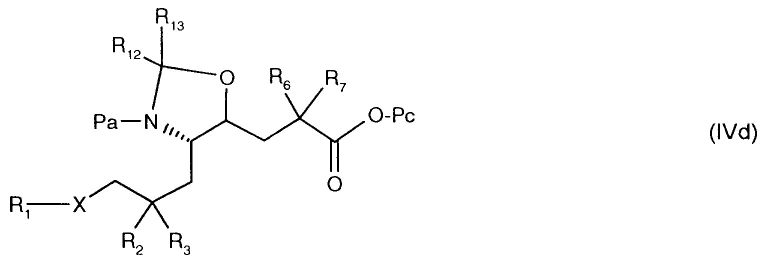
die Schutzgruppen >C(R₁₂)(R₁₃) und Pc abspaltet.

Die bevorzugten Ausgangsstoffe der Formel IVa können aus Zwischenprodukten der Formel IVc durch intramolekulare Kondensation unter Abspaltung der Schutzgruppe Pc, beispielsweise durch Säurebehandlung, z.B. mit Eisessig in Toluol, hergestellt werden.

Bevorzugt ist jedoch die Umsetzung des Aldehydes der Formel IIh mit einer metallorganischen Verbindung der Formel IVe 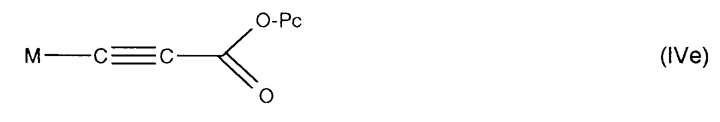
worin M einen metallischen Rest, z.B. Lithium, und Pc eine der unter Verfahrensvariante a) erwähnten Carboxyschutzgruppen bedeutet, erforderlichenfalls in Gegenwart einer Lewissäure, wie von Zinkchlorid, zur entsprechenden Verbindung der Formel IVf 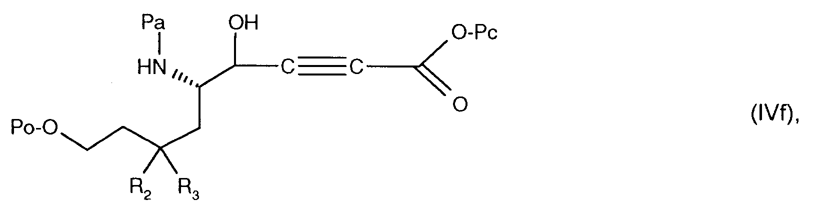
die durch vollständige Hydrierung der Dreifachbindung in Gegenwart eines geeigneten Katalysators, z.B. von Platin auf Kohle, beispielsweise bei Normaldruck und Raumtemperatur, und anschließende Abspaltung der Pc-Schutzgruppe, insbesondere wie vorstehend beschrieben, unter intramolekulare Kondensation zur entsprechenden Verbindung der Formel IVa (R₆=R₇=H) cyclisiert wird. In diese kann dann durch Umsetzung mit einer Verbindung der Formel R''₆-Y₁ (IVg) und gewünschtenfalls mit einer Verbindung der Formel R''₇-Y₁(IVh), worin R''₆ einen von Wasserstoff verschiedenen Rest R₆ und gegebenenfalls R''₇ einen von Wasserstoff verschiedenen Rest R₇ und Y₁ reaktionsfähiges funktionell abgewandeltes Hydroxy, wie unter der Verfahrensvariante a) beschrieben, beispielsweise Halogen oder Sulfonyloxy, wie Niederalkansulfonyloxy oder gegebenenfalls substituiertes Benzolsulfonyloxy, bedeutet, vorteilhaft in Gegenwart einer Base, wie eines Alkalimetalldisilazides, z.B. von Natriumhexamethyldisilazid, ein von Wasserstoff verschiedener Rest R₆ und gegebenenfalls R₇ eingeführt werden.

In einem Ausgangsmaterial der Formel VI für die Verfahrensvariante c) sind funktionelle Gruppen mit Ausnahme der zu reduzierenden Ketogruppe gegebenenfalls durch eine der unter Verfahren a) genannten Schutzgruppen geschützt.

Zur Reduktion der Ketogruppe in einer derartigen Verbindung der Formel VI eignen sich solche Reduktionsmittel, die unter den Reaktionsbedingungen des Verfahrens eine isolierte Ketogruppe selektiv oder schneller als die in Verbindungen der Formel VI vorhandenen Amidgruppen reduzieren.

In erster Linie zu nennen sind geeignete Borhydride, wie Alkalimetallborhydride, insbesondere Natrium-, Lithium- oder Natriumcyanoborhydrid, oder geeignete Aluminiumhydride, wie Alkalimetallniederalkylaluminiumhydride mit voluminösen Resten, z.B. Lithiumtris-tertiärbutylaluminiumhydrid.

Die Reduktion kann auch mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, oder nach Meerwein-Ponndorf-Verley mit Hilfe von Aluminiumalkanolaten, bevorzugt Aluminium-2-propanolat oder -ethanolat, durchgeführt werden.

Die Reduktion kann vorzugsweise mit stöchiometrischen Mengen oder einem sinnvoll bemessenen Überschuß des Reduktionsmittels in einem inerten Lösungsmittel bei Temperaturen zwischen -80° C und dem Siedepunkt des Lösungsmittels, vorzugsweise zwischen -20° und 100° C, wenn nötig unter Schutzgas, z.B. Stickstoff oder Argon, durchgeführt werden. Ein Überschuß des Reduktionsmittels ist insbesondere dann nötig, wenn dieses auch mit dem Lösungsmittel, z.B. den Protonen eines protischen Lösungsmittels, reagiert.

Bei Verwendung von Natriumborhydrid eignen sich polare, protische Lösungsmittel, z.B. Methanol, Ethanol oder Isopropanol; bei Verwendung der anderen Reduktionsmittel die unter Verfahren a) genannten polaren, aprotischen Lösungsmittel, z.B. Tetrahydrofuran.

Bevorzugt sind solche Reduktionsmittel oder Reduktionsverfahren, welche die Ketogruppe unter Ausbildung der bevorzugten (S)-Konfiguration an dem mit der gebildeten Hydroxygruppe verbundenen C-Atom reduzieren.

Die Reduktion der Ketogruppe kann auch enzymatisch durch Behandlung mit geeigneten Mikroorganismen erfolgen, beispielsweise mit Bakterien, Hefen oder Pilzen, vorzugsweise im Temperaturbereich von etwa 15° bis etwa 50° C, beispielsweise bei etwa 15° bis etwa 25° C, in wäßriger oder wäßrig/organischer Lösung, z.B. in wäßrigem Ethanol, erfolgen.

Die als Ausgangsmaterialien für Verfahren c) zu verwendenden 4-Ketoamide der Formel VI können beispielsweise durch milde Oxidation entsprechender Hydroxyamidverbindungen der Formel I erhalten werden. Im Hinblick auf die erwähnte stereoselektive Reduktionsmöglichkeit der Ketoamide ist diese Verfahrensvariante insbesondere für die Herstellung von Verbindungen der Formel I mit spezifischer Stereotaxie an dem die gebildete Hydroxygruppe tragenden C-Atoms geeignet.

In einem Ausgangsmaterial der Formel VII für die Verfahrensvariante d) sind funktionelle Gruppen, die nicht an der Reaktion teilnehmen sollen, durch geeignete unter a) genannte Schutzgruppen geschützt.

Zur Hydrierung der olefinischen Doppelbindung in einer Verbindung der Formel VII eignen sich solche Hydrierungsmittel, die unter den Reaktionsbedingungen des Verfahrens die Doppelbindung selektiv oder schneller als die in Verbindungen der Formel VII vorhandenen Amidbindungen reduzieren.

Besonders geeignet sind Hydrierungsmittel wie Wasserstoff in Gegenwart geeigneter Katalysatoren.

Zur Hydrierung geeignete Katalysatoren sind Metalle, wie z.B. Nickel, Eisen, Kobalt oder Ruthenium, oder Edelmetalle bzw. deren Oxide, wie Palladium oder Rhodium bzw. deren Oxide, gegebenenfalls auf geeignetem Trägermaterial, wie Bariumsulfat, Aluminiumoxid oder Aktivkohle aufgezogen, oder als Skelettkatalysatoren, wie z.B. Raney-Nickel, insbesondere aber homogene oder heterogene optisch aktive Metall- oder Edelmetall-Ligand-Komplexe, vor allem solche die die jeweils gewünschte Konfiguration an dem die Gruppe R₆ tragendem C-Atom herbeiführen. Dafür besonders geeignete Katalysatoren sind optisch aktive Edelmetall-Ligand-Komplexe, z.B. die vorstehend genannten, insbesondere Bis[(S)-(-)-2,2 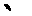 -bis(diphenylphosphino)-1,1 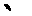 -binaphthyl](triethylamino)dirutheniumtetrachlorid.

Gebräuchliche Lösungsmittel für die katalytische Hydrierung sind polare organische oder anorganische Lösungsmittel, z.B. Wasser, Alkohole, Ester, Dioxan, Eisessig oder Gemische dieser Lösungsmittel. Die Hydrierung erfolgt bei Temperaturen von 0° C bis 250° C, bevorzugt von Raumtemperatur bis etwa 100° C und bei Wasserstoffdrucken von 1 bis 200 bar.

Die Ausgangsmaterialien für Verfahren d) können nach an sich bekannten Verfahren hergestellt werden, beispielsweise indem man eine Verbindung der Formel IIo 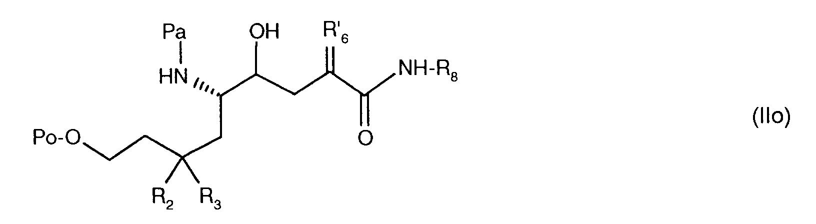
deren Herstellung unter der Verfahrensvariante a) beschrieben ist, gewünschtenfalls in die Stereoisomeren bezüglich des die Hydroxygruppe tragenden C-Atomes auftrennt, die Hydroxy- und Aminogruppe durch eine bivalente Schutzgruppe der Formel >C(R₁₂)(R₁₃) schützt, die erhältliche Verbindung der Formel VIIa 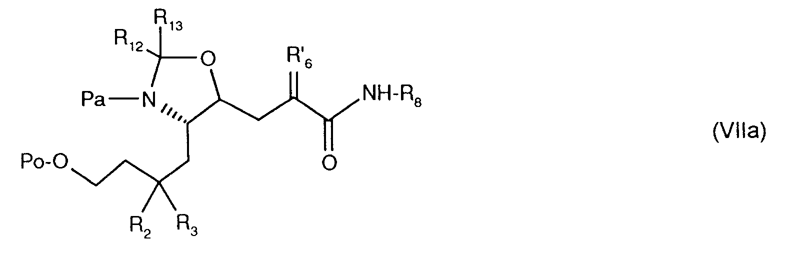
mit einem Reagenz umsetzt, das die Hydroxyschutzgruppe Po abspaltet, die freigesetzte Hydroxyverbindung an der freien Hydroxygruppe zur entsprechenden Carbonsäure der Formel VIIb 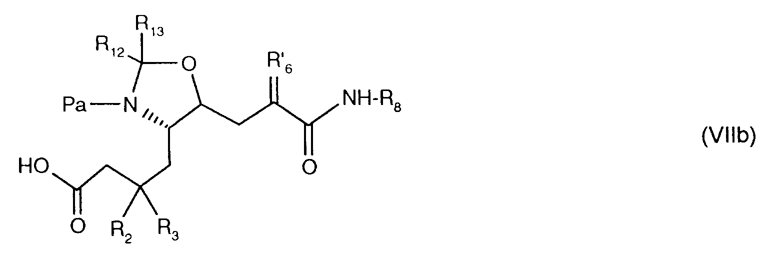
oxidiert, worin R₂, R₃, R 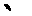 ₆ und R₈ die genannten Bedeutungen haben, diese wie unter der Verfahrensvariante a) angegeben zur entsprechenden Diamidverbindung der Formel VIIc 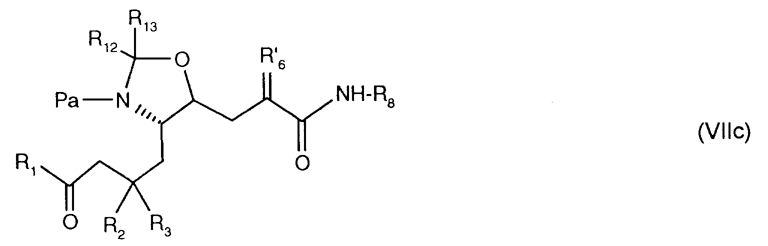
amidiert, die bivalente Schutzgruppe der Formel >C(R₁₂)(R₁₃) und die Aminoschutzgruppe Pa abspaltet und gewünschtenfalls unter Reaktion mit einer Verbindung der Formel R₄-S, worin S eine Abgangsgruppe oder eine Hydroxyfunktion ist, jeweils einen von Wasserstoff verschiedenen Rest R₄ einführt und/oder mit einem acylierenden Reagenz Hydroxy R₅ acyliert.

In Ausgangsmaterialien der Formel VIII für die Verfahrensvariante e) sind funktionelle Gruppen, die nicht an der Reaktion teilnehmen sollen, durch eine der unter Verfahren a) genannten Schutzgruppen geschützt.

Zur Reduktion der Azidogruppe in einer derartigen Verbindung der Formel VIII eignen sich solche Reduktionsmittel, die unter den Reaktionsbedingungen des Verfahrens eine Keto- oder Azidogruppe selektiv oder schneller als die in Verbindungen der Formel VIII vorhandenen Amidgruppen reduzieren.

In erster Linie zu nennen sind geeignete Borhydride, wie Alkalimetallborhydride, insbesondere Natriumborhydrid, Lithiumborhydrid oder Natriumcyanoborhydrid, oder geeignete Aluminiumhydride, wie Alkalimetallaluminiumhydride, z.B. Lithiumaluminiumhydrid, oder Alkalimetallniederalkoxyaluminiumhydride mit voluminösen Resten, z.B. Lithium-tris-tertiärbutylaluminiumhydrid.

Die Reduktion kann auch mit Wasserstoff in Gegenwart geeigneter Schwermetallkatalysatoren, z.B. Raney-Nickel oder Platin- oder Palladiumkatalysatoren, z.B. Platin- oder Palladium-Aktivkohle, durchgeführt werden.

Die Reduktion kann vorzugsweise mit stöchiometrischen Mengen oder einem sinnvoll bemessenen Überschuß des Reduktionsmittels in einem inerten Lösungsmittel bei Temperaturen zwischen -80° C und dem Siedepunkt des Lösungsmittels, vorzugsweise zwischen -20° und 100° C, wenn nötig unter Schutzgas, z.B. Stickstoff oder Argon, durchgeführt werden. Ein Überschuß des Reduktionsmittels ist insbesondere dann nötig, wenn dieses auch mit dem Lösungsmittel, z.B. den Protonen eines protischen Lösungsmittels, reagiert.

Bei Verwendung von Natriumborhydrid eignen sich polare, protische Lösungsmittel, z.B. Methanol, Ethanol oder Isopropanol; bei Verwendung der anderen Reduktionsmittel die unter Verfahren a) genannten polaren, aprotischen Lösungsmittel, z.B. Tetrahydrofuran oder Diethylether.

Zwischenprodukte der Formel VIII können beispielsweise hergestellt werden, indem man eine Verbindung der Formel VIIIa 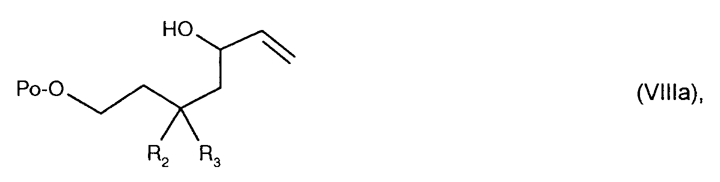
worin Po eine Hydroxyschutzgruppe bedeutet und R₂ sowie R₃ die angegebenen Bedeutungen haben, mit einem Thionylhalogenid, wie Thionylbromid, zur entsprechenden Verbindung der Formel VIIIb 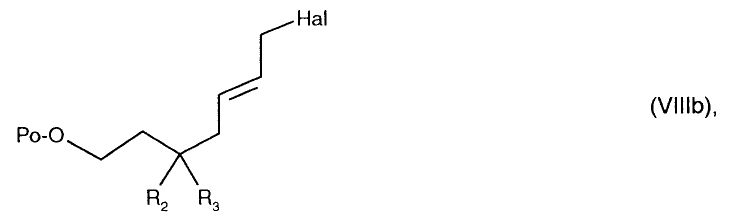
worin Hal Halogen, wie Brom, ist, umsetzt, diese mit einem substituierten Malonester der Formel VIIIc 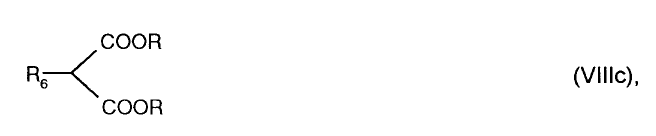
worin R z.B. Niederalkyl ist, kondensiert, die nachfolgend durch Hydrolyse und Decarboxylierung erhaltene Alkensäureverbindung der Formel VIIId 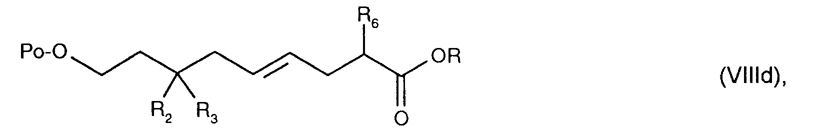
gegebenenfalls nach Einführung eines von Wasserstoff verschiedenen Restes R₇ und/oder Spaltung in die Enantiomeren bezüglich des zur ROOC-Gruppe α -ständigen Ketten-C-atomes durch intermediäre Überführung in ein chirales Amid und Auftrennung in die Diastereomeren, in üblicher Weise zur entsprechenden Verbindung der Formel VIIIe 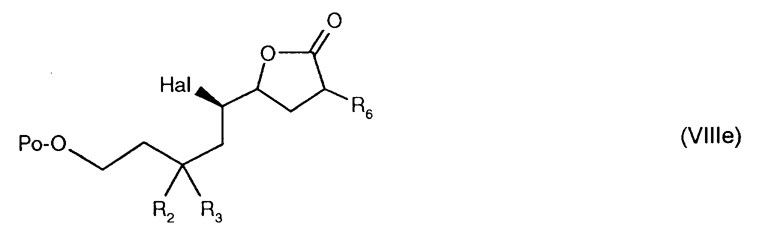
halolaktonisiert, diese mit einem Alkalimetallazid, wie Natriumazid, zum entsprechenden Azid der Formel VIIIf 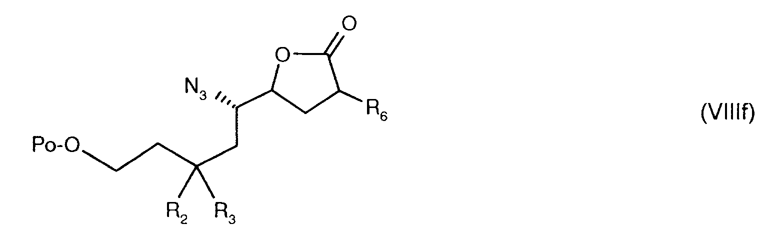
umsetzt, in üblicher Weise, beispielsweise wie unter der Verfahrensvariante a) für Verbindungen der Formel Im beschrieben, die Schutzgruppe Po abspaltet, die gebildete terminale Hydroxymethylgruppe zu Carboxy oxidiert und die erhaltene Säure mit einem Amin der Formel H-N(H)-R₁ (III) und anschließend mit einem Amin der Formel H-N(H)-R₈(V) umsetzt.

Die Abspaltung der Schutzgruppen, die nicht Bestandteil des gewünschten Endproduktes der Formel I sind, z.B. der Carboxy-, Amino- und/oder Hydroxyschutzgruppen, erfolgt in an sich bekannter Weise, z.B. mittels Solvolyse, insbesondere Hydrolyse, Alkoholyse oder Acidolyse, oder mittels Reduktion, insbesondere Hydrogenolyse oder chemischer Reduktion, sowie Photolyse, gegebenenfalls stufenweise oder gleichzeitig, wobei auch enzymatische Methoden verwendet werden können. Die Abspaltung der Schutzgruppen ist beispielsweise in den weiter vorn im Abschnitt über "Schutzgruppen" genannten Standardwerken beschrieben.

So kann beispielsweise geschütztes Carboxy, z.B. Tertiärniederalkoxycarbonyl, in 2-Stellung durch eine trisubstituierte Silylgruppe oder in 1-Stellung durch Niederalkoxy oder Niederalkylthio substituiertes Niederalkoxycarbonyl oder gegebenenfalls substituiertes Diphenylmethoxycarbonyl durch Behandeln mit einer geeigneten Säure, wie Ameisensäure oder Trifluoressigsäure, gegebenenfalls unter Zugabe einer nukleophilen Verbindung, wie Phenol oder Anisol, in freies Carboxy überführt werden. Gegebenenfalls substituiertes Benzyloxycarbonyl kann z.B. mittels Hydrogenolyse, d.h. durch Behandeln mit Wasserstoff in Gegenwart eines metallischen Hydrierungskatalysators, wie eines Palladiumkatalysators, freigesetzt werden. Ferner kann geeignet substituiertes Benzyloxycarbonyl, wie 4-Nitrobenzyloxycarbonyl, auch durch Reduktion, z.B. durch Behandeln mit einem Alkalimetalldithionit, wie Natriumdithionit, oder mit einem reduzierenden Metall, z.B. Zink, oder reduzierenden Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff erzeugen kann, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glykolsäure, Diphenylglykolsäure, Milchsäure, Mandelsäure, 4-Chlormandelsäure oder Weinsäure, oder eines Alkohols oder Thiols, wobei man vorzugsweise Wasser zugibt, in freies Carboxy überführt werden. Durch Behandeln mit einem reduzierenden Metall oder Metallsalz, wie oben beschrieben, kann auch 2-Halogenniederalkoxycarbonyl (gegebenenfalls nach Umwandlung einer 2-Bromniederalkoxycarbonylgruppe in eine entsprechende 2-Iodniederalkoxycarbonylgruppe) oder Aroylmethoxycarbonyl in freies Carboxy umgewandelt werden. Aroylmethoxycarbonyl kann ebenfalls durch Behandeln mit einem nukleophilen, vorzugsweise salzbildenden Reagenz, wie Natriumthiophenolat oder Natriumjodid, gespalten werden. 2-(trisubstituiertes Silyl)niederalkoxycarbonyl, wie 2-Triniederalkylsilylniederalkoxycarbonyl, kann auch durch Behandeln mit einem das Fluoridanion liefernden Salz der Fluorwasserstoffsäure, wie einem Alkalimetallfluorid, z.B. Natrium- oder Kaliumfluorid, gegebenenfalls in Anwesenheit eines macrocyclischen Polyethers ("Kronenether"), oder mit einem Fluorid einer organischen quaternären Base, wie Tetraniederalkylammoniumfluorid oder N,N,N-Triniederalkyl-N-aryl-ammoniumfluorid, z.B. Tetraethylammoniumfluorid oder Tetrabutylammoniumfluorid, in Gegenwart eines aprotischen, polaren Lösungsmittels, wie Dimethylsulfoxid oder N,N-Dimethylacetamid, in freies Carboxy überführt werden. Als organisches Silyloxycarbonyl, wie Triniederalkylsilyloxycarbonyl, z.B. Trimethylsilyloxycarbonyl, geschütztes Carboxy kann in üblicher Weise solvolytisch, z.B. durch Behandeln mit Wasser, einem Alkohol oder Säure, oder außerdem Fluorid, wie oben beschrieben, freigesetzt werden. Verestertes Carboxy kann auch enzymatisch freigesetzt werden, beispielsweise durch Esterasen oder geeignete Peptidasen.

Eine geschützte Aminogruppe setzt man in an sich bekannter und je nach Art der Schutzgruppen in verschiedenartiger Weise, vorzugsweise mittels Solvolyse oder Reduktion, frei. 2-Halogenniederalkoxycarbonylamino (gegebenenfalls nach Umwandlung einer 2-Bromniederalkoxycarbonylaminogruppe in eine 2-Iodniederalkoxycarbonylaminogruppe), Aroylmethoxycarbonylamino oder 4-Nitrobenzyloxycarbonylamino kann z.B. durch Behandeln mit einem geeigneten Reduktionsmittel, wie Zink in Gegenwart einer geeigneten Carbonsäure, wie wäßriger Essigsäure, gespalten werden. Aroylmethoxycarbonylamino kann auch durch Behandeln mit einem nukleophilen, vorzugsweise salzbildenden Reagenz, wie Natriumthiophenolat, und 4-Nitrobenzyloxycarbonylamino auch durch Behandeln mit einem Alkalimetall, z.B. Natriumdithionit, gespalten werden. Gegebenenfalls substituiertes Diphenylmethoxycarbonylamino, tert.-Niederalkoxycarbonylamino oder 2-(trisubstituiertes Silyl)-niederalkoxycarbonylamino, wie 2-Triniederalkylsilylniederalkoxycarbonylamino, kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierungskatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, und eine als Silylamino geschützten Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat des Thioharnstoffs, und anschließende Solvolyse, wie Alkoholyse oder Hydrolyse, des entstandenen Kondensationsproduktes freigesetzt werden. Eine durch 2-(trisubstituiertes Silyl)-niederalkoxycarbonyl, wie 2-Triniederalkylsilylniederalkoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechenden geschützten Carboxygruppe angegeben, in die freie Aminogruppe überführt werden. Ebenso kann man direkt an ein Heteroatom, wie Stickstoff, gebundenes Silyl, wie Trimethylsilyl, mittels Fluoridionen abspalten.

In Form einer Azidogruppe geschütztes Amino wird z.B. durch Reduktion in freies Amino überführt, beispielsweise durch katalytische Hydrierung mit Wasserstoff in Gegenwart eines Hydrierungskatalysators, wie Platinoxid, Palladium oder Raney-Nickel, durch Reduktion mittels Mercaptoverbindungen, wie Dithiothreitol oder Mercaptoethanol, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20° C bis 25° C, oder auch unter Kühlen oder Erwärmen, durchgeführt.

Eine durch eine geeignete Acylgruppe, eine Triniederalkylsilylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte Hydroxy- bzw. Mercaptogruppe wird z.B. durch basische Hydrolyse, eine durch tert.-Niederalkyl oder durch einen 2-oxa- oder 2-thia-aliphatischen oder -cycloaliphatischen Kohlenwasserstoffrest geschützte Hydroxy bzw. Mercaptogruppe durch Acidolyse, z.B. durch Behandeln mit einer Mineralsäure oder einer starken Carbonsäure, z.B. Trifluoressigsäure, freigesetzt. Durch Pyridyldiphenylmethyl geschütztes Mercapto kann z.B. durch Quecksilber-II-salze bei pH 2-6 oder durch Zink/Essigsäure oder elektrolytische Reduktion, Acetamidomethyl und iso-Butyrylamidomethyl z.B. durch Reaktion mit Quecksilber-II-salzen bei pH 2-6, 2-Chloracetamidomethyl z.B. durch 1-Piperidinothiocarboxamid, S-Ethylthio, S-Tertiärbutylthio und S-Sulfo z.B. durch Thiolyse mit Thiophenol, Thioglykolsäure, Natriumthiophenolat oder 1,4-Dithiothreitol freigesetzt werden. Zwei Hydroxygruppen oder eine benachbarte Amino- und Hydroxygruppe, die zusammen mittels einer bivalenten Schutzgruppe, vorzugsweise z.B. einer durch Alkyl ein- oder zweifach substituierten Methylengruppe, wie durch Niederalkyliden, z.B. Isopropyliden, Cycloyalkyliden, z.B. Cyclohexyliden, oder Benzyliden, geschützt sind, können durch saure Solvolyse, besonders in Gegenwart einer Mineralsäure oder einer starken organischen Säure, freigesetzt werden. 2-Halogenniederalkoxycarbonyl wird auch durch die oben genannten Reduktionsmittel, z.B. reduzierendes Metall, wie Zink, reduzierende Metallsalze, wie Chrom-II-salze, oder durch Schwefelverbindungen, beispielsweise Natriumdithionit oder bevorzugt Natriumsulfid und Schwefelkohlenstoff, entfernt.

Beim Vorhandensein von mehreren geschützten funktionellen Gruppen können, wenn erwünscht, die Schutzgruppen so gewählt werden, daß gleichzeitig mehr als eine solche Gruppe abgespalten werden kann, beispielsweise acidolytisch, wie durch Behandeln mit Trifluoressigsäure, oder mit Wasserstoff und einem Hydrierungskatalysator, wie einem Palladium-Kohle-Katalysator. Umgekehrt können die Gruppen auch so gewählt werden, daß sie nicht alle gleichzeitig, sondern in gewünschter Reihenfolge oder nur teilweise abgespalten werden.

Bei jedem der oben genannten Verfahren können die Ausgangsverbindungen auch als Salze verwendet werden, sofern die Reaktionsbedingungen dies zulassen.

Als fakultative Folgemaßnahmen sind insbesondere die Umwandlung verfahrensgemäß erhältlicher Verbindungen durch Abwandlung der Variablen R₁, R₂, R₃, R₄, R₅, R₆, R₇ und/oder NH-R₈ in andere Verbindungen der Formel I, die Auftrennung verfahrensgemäß erhältlicher Gemische von Stereoisomeren in die individuellen Stereoisomeren und die Überführung verfahrensgemäß erhältlicher freier salzbildender Verbindungen in ihre Salze bzw. die Überführung verfahrensgemäß erhältlicher Salze in die freien Verbindungen zu nennen.

So kann man beispielsweise in einer erhältlichen Verbindung der Formel I eine in freier oder in reaktionsfähiger Form vorliegende Carboxygruppe verestern oder amidieren bzw. eine veresterte oder amidierte Carboxygruppe in eine freie Carboxygruppe überführen.

Zur Veresterung oder Amidierung einer Carboxygruppe in einer Verbindung der Formel I kann man, wenn erwünscht, die freie Säure verwenden oder die freie Säure in eines der oben genannten reaktionsfähigen Derivate überführen und mit einem Alkohol, Ammoniak, einem primären oder einem sekundären Amin umsetzen, oder man kann zur Veresterung die freie Säure oder ein reaktionsfähiges Salz, z. B. das Cäsiumsalz, mit einem reaktionsfähigen Derivat eines Alkohols umsetzen. Beispielsweise kann man das Cäsiumsalz einer Carbonsäure mit einem dem Alkohol entsprechenden Halogenid oder Sulfonsäureester umsetzen. Die Veresterung der Carboxygruppe kann auch mit anderen üblichen Alkylierungsmitteln erfolgen, z.B. mit Diazomethan, Meerweinsalzen oder 1-substituierten 3-Aryltriazenen.

Zur Überführung einer veresterten oder amidierten Carboxygruppe in die freie Carboxygruppe kann eine der oben bei der Abspaltung der Carboxyschutzgruppen beschriebenen Methoden oder gewünschtenfalls eine alkalische Verseifung nach den im Organikum, 17. Auflage, VEB Deutscher Verlag der Wissenschaften, Berlin 1988, genannten Reaktionsbedingungen angewendet werden.

In einer Verbindung der Formel I kann man eine veresterte Carboxygruppe durch Aminolyse mit Ammoniak oder einem primären oder sekundären Amin in eine gegebenenfalls substituierte Carboxamidgruppe überführen. Die Aminolyse kann nach den im Organikum, 15. Auflage, VEB Deutscher Verlag der Wissenschaften, Berlin (Ost) 1976, für derartige Umsetzungen genannten Reaktionsbedingungen erfolgen.

In einer Verbindung der Formel I kann man eine vorhandene freie Aminogruppe zu Alkanoylamino bzw. Alkoxycarbonylamino R₄ acylieren oder zu Mono- oder Dialkylamino R₄ alkylieren. Die Acylierung und die Alkylierung können erfolgen nach einer der für Schutzgruppen genannten Methoden oder nach einem der im Organikum, 17. Auflage, VEB Deutscher Verlag der Wissenschaften, Berlin (Ost) 1988, genannten Verfahren.

In einer Verbindung der Formel I kann man eine vorhandene freie Hydroxygruppe acylieren, beispielsweise zur Einführung eines Restes R₅ außer Hydroxy. Die Acylierung kann erfolgen mit acylierenden Reagenzien nach einer der für Schutzgruppen genannten Methoden oder nach einem der im Organikum, 17. Auflage, VEB Deutscher Verlag der Wissenschaften, Berlin (Ost) 1988, genannten Verfahren.

In einer erhältlichen Verbindung der Formel I kann man aus einem Sulfid das entsprechende Sulfoxid oder Sulfon hergestellt werden.

Die Oxidation zur Sulfonylgruppe kann mit den meisten der üblichen Oxidationsmittel durchgeführt werden. Besonders bevorzugt verwendet man solche Oxidationsmittel, die die Thiogruppe oder den Sulfidschwefel selektiv in Gegenwart anderer funktioneller Gruppen der jeweiligen Verbindung der Formel I, z.B. von Amino oder Hydroxygruppen, oxidieren, beispielsweise aromatische oder aliphatische Peroxycarbonsäuren, z.B. Peroxybenzoesäure, Monoperphthalsäure, m-Chlorperbenzoesäure, Peressigsäure, Perameisensäure oder Trifluorperessigsäure. Die Oxidation mit Peroxycarbonsäuren erfolgt in den üblichen dafür geeigneten Lösungsmitteln, beispielsweise Chlorkohlenwasserstoffen, z.B. Methylenchlorid oder Chloroform, Ethern, wie Diethylether, Estern, wie Essigester oder dergleichen, bei Temperaturen zwischen -78° C und Raumtemperatur, z.B. zwischen -20° C und +10° C, bevorzugt um 0° C. Die Peroxycarbonsäure kann auch in situ gebildet werden, z.B. mit Wasserstoffperoxid in Essigsäure oder Ameisensäure, die gegebenenfalls Essigsäureanhydrid enthält, z.B. mit 30 % oder 90 % Wasserstoffperoxid in Essigsäure/Essigsäureanhydrid. Geeignet sind auch andere Peroxoverbindungen, beispielsweise Kaliumperoxymonosulfat in Niederalkanol/Wasser-Mischungen, z.B. Methanol/Wasser oder Ethanol/Wasser, oder in wäßriger Essigsäure bei Temperaturen zwischen -70° C und +30° C, z.B. zwischen -20° C und Raumtemperatur, ferner Natriummetaperjodat in Methanol oder Methanol/Wasser-Gemischen bei Temperaturen zwischen 0° C und 50° C, z.B. um Raumtemperatur. Bei Einsatz stöchiometrischer Mengen der genannten Oxidationsmittel können auch die entsprechenden Sulfoxide erhalten werden.

Stereoisomerengemische, also Gemische von Diastereomeren und/oder Enantiomeren, wie beispielsweise racemische Gemische, können in an sich bekannter Weise durch geeignete Trennverfahren in die entsprechenden Isomeren aufgetrennt werden. So können Diastereomerengemische durch fraktionierte Kristallisation, Chromatographie, Lösungsmittelverteilung etc. in die einzelnen Diastereomeren aufgetrennt werden. Racemate können nach Überführung der optischen Antipoden in Diastereomere, beispielsweise durch Umsetzung mit optisch aktiven Verbindungen, z.B. optisch aktiven Säuren oder Basen, durch Chromatographie an mit optisch aktiven Verbindungen belegten Säulenmaterialien oder durch enzymatische Methoden, z.B. durch selektive Umsetzung nur eines der beiden Enantiomeren, voneinander getrennt werden. Diese Trennung kann sowohl auf der Stufe eines der Ausgangsprodukte als auch bei den Verbindungen der Formel I selbst erfolgen.

An einzelnen Chiralitätszentren in einer Verbindung der Formel I kann die Konfiguration gezielt umgekehrt werden. Beispielsweise kann man die Konfiguration asymmetrischer Kohlenstoffatome, welche nukleophile Substituenten, wie Amino oder Hydroxy, tragen, durch nukleophile Substitution zweiter Ordnung, gegebenenfalls nach Überführung des gebundenen nukleophilen Substituenten in eine geeignete nukleofuge Abgangsgruppe und Reaktion mit einem den ursprünglichen Substituenten einführenden Reagenz, umkehren, oder man kann die Konfiguration an Kohlenstoffatomen mit Hydroxygruppen, wie das R₅ tragende Kohlenstoffatom der Formel I, durch Oxidation und Reduktion, analog dem Verfahren in der Europäischen Patentanmeldung EP-A-0 236 734, umkehren.

Vorteilhaft ist auch die reaktionsfähig funktionelle Abwandlung der Hydroxygruppe R₅ und anschließender Ersatz derselben durch Hydroxy oder Acyloxy R₅ unter Konfigurationsumkehr an dem die Hydroxygruppe tragenden C-Atom . So kann man eine Verbindung der Formel I, worin R₄ Amino und R₅ Hydroxy ist, zunächst an der Aminogruppe durch eine Aminoschutzgruppe Pa, beispielsweise Benzyloxycarbonyl, schützen und dann unter Bedingungen, welche die Hydroxygruppe R₅ intermediär in eine Abgangsgruppe X₁, wie Chlor, überführen, z.B. in Gegenwart von Thionylchlorid, unter Konfigurationsumkehr an dem ursprünglich die Hydroxygruppe R₅ tragenden C-Atom zur entsprechenden Oxazolidinonverbindung der Formel IX 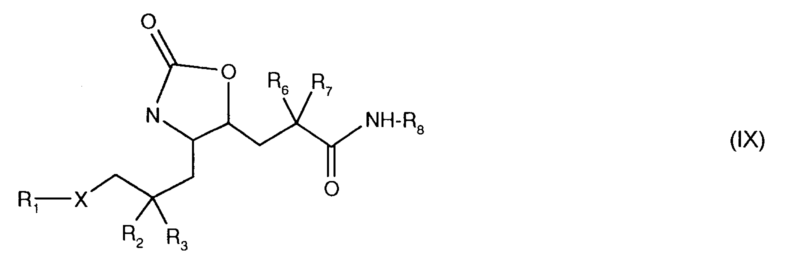
cyclisieren. Aus dem reaktiven Zwischenprodukt der Formel IX kann dann in üblicher Weise, insbesondere hydrolytisch, die entsprechende Verbindung der Formel I freigesetzt werden.

Salze von Verbindungen der Formel I mit mindestens einer salzbildenden Gruppe können in an sich bekannter Weise hergestellt werden. So kann man Salze von Verbindungen der Formel I mit sauren Gruppen z.B. durch Behandeln mit Metallverbindungen, wie Alkalimetallsalzen von geeigneten organischen Carbonsäuren, z.B. dem Natriumsalz der 2-Ethylhexansäure, oder mit organischen Alkali- oder Erdalkalimetallverbindungen, wie den entsprechenden Hydroxiden, Carbonaten oder Hydrogencarbonaten, wie Natrium- und Kaliumhydroxid, -carbonat oder -hydrogencarbonat, mit entsprechenden Calciumverbindungen oder mit Ammoniak oder einem geeigneten organischen Amin bilden, wobei man vorzugsweise stöchiometrische Mengen oder nur einen kleinen Überschuß des salzbildenden Mittels verwendet. Säureadditionssalze von Verbindungen der Formel I erhält man in üblicher Weise, z.B. durch Behandeln mit einer Säure oder einem geeigneten Anionenaustauscherreagenz. Innere Salze von Verbindungen der Formel I, welche saure und basische salzbildende Gruppen enthalten, beispielsweise eine freie Carboxygruppe und eine freie Aminogruppe, können z.B. durch Neutralisieren von Salzen, wie Säureadditionssalzen, auf den isoelektrischen Punkt, z.B. mit schwachen Basen, oder durch Behandeln mit Ionenaustauschern gebildet werden.

Salze können in üblicher Weise in die freien Verbindungen überführt werden, Metall- und Ammoniumsalze beispielsweise durch Behandeln mit geeigneten Säuren, und Säureadditionssalze beispielsweise durch Behandeln mit einem geeigneten basischen Mittel.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder das Verfahren auf irgendeiner Stufe abbricht oder einen Ausgangsstoff unter den Reaktionsbedingungen bildet oder in Form eines reaktionsfähigen Derivates oder Salzes verwendet oder eine nach dem erfindungsgemäßen Verfahren erhältliche Verbindung unter den Verfahrensbedingungen erzeugt und in situ weiterverarbeitet. Dabei geht man vorzugsweise von solchen Ausgangsstoffen aus, die zu den oben als sehr bevorzugt oder ganz besonders bevorzugt beschriebenen Verbindungen führen.

Neue Ausgangsmaterialien und/oder Zwischenprodukte, sowie Verfahren zu ihrer Herstellung, sind ebenfalls Gegenstand der vorliegenden Erfindung. Vorzugsweise werden solche Ausgangsstoffe verwendet und die Reaktionsbedingungen so gewählt, daß man zu den als in allererster Linie bevorzugt aufgeführten Verbindungen gelangt. Insbesondere können alle genannten Ausgangsverbindungen beim Vorhandensein funktioneller Gruppen in geeigneter Weise durch Schutzgruppen geschützt sein und Schutzgruppen auf den verschiedenen Reaktionsstufen gegebenenfalls entfernt oder eingeführt werden.

Die Erfindung betrifft auch pharmazeutische Präparate enthaltend Verbindungen der Formel I.

Die pharmakologisch verwendbaren Verbindungen der vorliegenden Erfindung können z.B. zur Herstellung von pharmazeutischen Präparaten verwendet werden, welche eine wirksame Menge des Wirkstoffes zusammen oder im Gemisch mit einer signifikanten Menge von anorganischen oder organischen, festen oder flüssigen, pharmazeutisch verwendbaren Trägerstoffen enthalten.

Bei den erfindungsgemäßen pharmazeutischen Präparaten handelt es sich um solche zur enteralen, wie nasalen, rektalen oder oralen, oder parenteralen, wie intramuskulären oder intravenösen, Verabreichung an Warmblüter (Menschen und Tiere), welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffs hängt von der Warmblüter-Spezies, dem Körpergewicht, dem Alter und dem individuellen Zustand, individuellen pharmakokinetischen Gegebenheiten, der zu behandelnden Krankheit sowie der Applikationsweise ab.

Die pharmazeutischen Präparate enthalten von etwa 1 % bis etwa 95 %, vorzugsweise von etwa 20 % bis etwa 90 % des Wirkstoffes. Erfindungsgemäße pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen, und zwar insbesondere isotonische wäßrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natriumcarboxymethylcellulose, Carboxymethylcellulose, Dextran, Polyvinylpyrrolidon oder Gelatine, enthalten.

Suspensionen in Öl enthalten als ölige Komponente die für Injektionszwecke üblichen vegetabilen, synthetischen oder halbsynthetischen Öle. Als solche sind insbesondere flüssige Fettsäureester zu nennen, die als Säurekomponente eine langkettige Fettsäure mit 8-22, insbesondere 12-22, Kohlenstoffatomen, wie z.B. Laurinsäure, Tridecylsäure, Myristinsäure, Pentadecylsäure, Palmitinsäure, Margarinsäure, Stearinsäure, Arachidinsäure, Behensäure oder entsprechende ungesättigte Säuren, wie z.B. Ölsäure, Elaidinsäure, Erucasäure, Brasidinsäure oder Linolsäure, enthalten, gegebenenfalls unter Zusatz von Antioxidantien, wie z.B. Vitamin E, β-Carotin oder 3,5-Di-tertiärbutyl-4-hydroxytoluol. Die Alkoholkomponente dieser Fettsäureester hat maximal 6 Kohlenstoffatome und ist ein ein- oder mehrwertiger, z.B. ein-, zwei- oder dreiwertiger Alkohol, z.B. Methanol, Ethanol, Propanol, Butanol oder Pentanol oder deren Isomere, vor allem aber Glykol und Glycerin. Als Fettsäureester sind daher beispielsweise zu nennen: Ethyloleat, Isopropylmyristat, Isopropylpalmitat, "Labrafil® M 2375" (Polyoxyethylenglycerintrioleat der Firma Gattefossé, Paris), "Myglyol® 812" (Triglycerid gesättigter Fettsäuren der Kettenlänge C₈ bis C₁₂ der Firma Chemische Werke Witten/Ruhr, Deutschland), besonders aber vegetabile Öle wie Baumwollsaatöl, Mandelöl, Olivenöl, Rizinusöl, Sesamöl, Sojabohnenöl und vor allem Erdnußöl.

Die Herstellung der Injektionspräparate erfolgt in üblicher Weise unter sterilen Bedingungen, ebenso das Abfüllen in Ampullen oder Vialen sowie das Verschließen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch, wenn erwünscht oder notwendig, nach Zugabe von geeigneten Hilfsstoffen, zu Tabletten, Dragée-Kernen oder Kapseln verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate, und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropylmethylcellulose, Natriumcarboxymethylcellulose und/oder Polyvinylpyrrolidon, und/oder, wenn erwünscht, Sprengmittel, wie die oben genannten Stärken, ferner Carboxymethylstärke, quervernetztes Polyvinylpyrrolidon, Agar, Alginsäure oder ein Salz davon, wie Natriumalginat. Hilfsmittel sind in erster Linie Fließregulier- und Schmiermittel, z.B. Kieselsäure, Talk, Stearinsäure oder Salze davon, wie Magnesium- oder Calciumstearat, und/oder Polyethylenglykol. Dragée-Kerne werden mit geeigneten, gegebenenfalls magensaftresistenten Überzügen versehen, wobei man u.a. konzentrierte Zuckerlösungen, welche gegebenenfalls arabischen Gummi, Talk, Polyvinylpyrrolidon, Polyethylenglykol und/oder Titandioxid enthalten, Lacklösungen in geeigneten organischen Lösungsmitteln oder, zur Herstellung von magensaftresistenten Überzügen, Lösungen von geeigneten Cellulosepräparaten, wie Ethylcellulosephthalat oder Hydroxypropylmethylcellulosephthalat, verwendet. Kapseln sind Steckkapseln aus Gelatine sowie weiche, geschlossene Kapseln aus Gelatine und einem Weichmacher, wie Glycerin oder Sorbit. Die Steckkapseln können den Wirkstoff in Form eines Granates, z.B. mit Füllstoffen, wie Lactose, Bindemitteln, wie Stärken, und/oder Gleitmitteln, wie Talk oder Magnesiumstearat, und gegebenenfalls mit Stabilisatoren, enthalten. In weichen Kapseln ist der Wirkstoff vorzugsweise in geeigneten öligen Hilfsstoffen, wie fetten Ölen, Paraffinöl oder flüssigen Polyethylenglykolen, gelöst oder suspendiert, wobei ebenfalls Stabilisatoren und/oder antibakterielle Mittel zugefügt sein können. Den Tabletten oder Dragée-Überzügen und den Kapselhüllen können Farbstoffe oder Pigmente, z.B. zur Identifizierung oder zur Kennzeichnung verschiedener Wirkstoffdosen, beigefügt werden.

Die Erfindung betrifft auch die Verwendung von Verbindungen der Formel I zur Behandlung von Bluthochdruck und/oder Glaukom, insbesondere bei Warmblütern, die einer derartigen Behandlung bedürfen, weil sie an Bluthochdruck oder Glaukom erkrankt sind.

Die am Warmblüter, z.B. Menschen, von beispielsweise etwa 70 kg Körpergewicht, zu verabreichenden Dosismengen, insbesondere die zur Hemmung des Enzyms Renin, zur Blutdrucksenkung und/oder zur Besserung der Glaukomsymptomatik wirksamen Dosen, liegen zwischen etwa 3 mg und etwa 3 g, vorzugsweise zwischen etwa 10 mg und etwa 1 g, z.B. bei ungefähr 300 mg pro Person und Tag, verteilt auf vorzugsweise 1 bis 4 Einzeldosen, die z.B. gleich groß sein können. Üblicherweise erhalten Kinder etwa die halbe Dosis von Erwachsenen. Die individuell notwendige Dosierung kann z.B. durch Messung der Serumkonzentration des Wirkstoffes überwacht und optimal eingestellt werden.

Die nachfolgen Beispiele dienen zur Illustration der Erfindung; Temperaturen sind in Celsiusgraden, Drucke in mbar angegeben. Ferner gelten folgende Abkürzungen:

In der Dünnschicht- und Flash-Säulenchromatographie verwendete Fließmittelsysteme:
- A: Essigester - n-Hexan 1:1
- B: Essigester - n-Hexan 1:2
- C: Essigester - n-Hexan 1:3
- D: Essigester - n-Hexan 1:4
- E: Essigester - n-Hexan 1:5
- F: Essigester - n-Hexan 1:6
- G: Essigester - n-Hexan 1:10
- H: Essigester - n-Hexan 3:1
- I: Methylenchlorid - Methanol 98.5:1.5
- J: Methylenchlorid - Methanol 98:2
- K: Methylenchlorid - Methanol 97:3
- L: Methylenchlorid - Methanol 95:5
- M: Methylenchlorid - Methanol 92.5:7.5
- N: Methylenchlorid - Methanol 20:1
- O: Methylenchlorid - Methanol 10:1
- P: Methylenchlorid - Methanol 9:1
- Q: Methylenchlorid - Methanol 4:1
- R: Methylenchlorid - Methanol - Ammoniak konz. 200:10:1
- S: Methylenchlorid - Methanol - Ammoniak konz. 90:10:1
- T: Methylenchlorid - Methanol - Ammoniak konz. 65:10:1
- U: Methylenchlorid - Methanol - Ammoniak konz. 40:10:1
- V: Methylenchlorid - Methanol - Wasser -Eisessig 170:26:3:1
- W: Methylenchlorid - Methanol - Wasser - Eisessig 150:54:10:1
- X: Essigester - Eisessig 100:1
- Y: Methylenchlorid - Diethylether 1:1
- Z: Methylenchlorid - Diethylether 9:1

HPLC - Gradienten auf C₁₈- Nucleosil 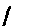 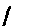 (5µm); Säule: 4.6 x 250 mm

- I: 90% Wasser/10% Acetonitril/0.1% Trifluoressigsäure zu 0% Wasser/100% Acetonitril/0.1% Trifluoressigsäure in 30 min;
- II: 50% Wasser/50% Acetonitril/0.1% Trifluoressigsäure zu 0% Wasser/100% Acetonitril/0.1% Trifluoressigsäure in 30 min;
- III: 100% Wasser/0% Acetonitril/0.1% Trifluoressigsäure zu 0% Wasser/100% Acetonitril/0.1% Trifluoressigsäure in 60 min;
- IV: 100% Wasser/0% Acetonitril/0.1% Trifluoressigsäure zu 10% Wasser/90% Acetonitril/0.1% Trifluoressigsäure in 60 min;
- V: 70% Wasser/30% Acetonitril/0.1% Trifluoressigsäure zu 10% Wasser/90% Acetonitril/0.1% Trifluoressigsäure in 60 min.

Die Abkürzung "R_{f}(A)" bedeutet beispielsweise, daß der R_{f}-Wert im Lösungsmittelsystem A ermittelt wird. Das Mengenverhältnis von Lösungsmitteln zueinander ist stets in Volumenanteilen angegeben.

Für die Bezeichnung der Fließmittelsysteme werden bei der Flash-Chromatographie und der Mitteldruckchromatographie die gleichen Abkürzungen verwendet.

Die verwendeten Kurzbezeichnungen und Abkürzungen haben die folgenden Bedeutungen:
- atm: Druck in atmosphärischen Einheiten
- bar: Druck in Bar
- c: Konzentration bei Messung der optischen Drehung
- C₁₈-Nucleosil®: Handelsname für mit Octadecylresten belegtes "Reversed Phase"-Säulenmaterial für HPLC (Nucleosil 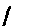 5C₁₈, Macherey & Nagel, BRD)
- Chiralcel®: CA-1 Handelsname für Triacetylcellulose (Daicel, Japan)
- DC: Dünnschichtchromatographie
- DMAP: 4-Dimethylaminopyridin
- DMF: Dimethylformamid
- Essigester: Essigsäureethylester
- FAB-MS: "Fast-Atom-Bombardment Mass-Spectroscopy"
- FC: "Flash-Chromatographie"
- h: Stunde(n)
- HPLC: Hochleistungs-Flüssigchromatographie
- Hyflo®: Handelsname für Filterhilfsmittel (Fluka, Buchs, Schweiz)
- IR: Infrarotspektroskopie
- Kp: Siedepunkt beim in Torr angegebenen Druck
- ml: Milliliter
- min: Minute(n)
- MS: Massenspektroskopie
- R_{f}: Verhältnis von Laufstrecke einer Substanz zu Entfernung der Laufmittelfront vom Startpunkt bei DC
- Rₜ: Retentionszeit einer Substanz bei HPLC (in min)
- Smp.: Schmelzpunkt (Temperatur)
Die Werte für IR-Spektren werden in cm⁻¹ angegeben, in runden Klammern findet sich das jeweilige Lösungsmittel. Zusätzlich bedeutet w eine starke, m eine mittlere und w eine schwache Intensität der jeweiligen Bande.

Massenspektroskopische Messwerte werden entweder durch konventionelle MS oder nach der "Fast-Atom-Bombardment" (FAB-MS)-Methode erhalten. Die Massenangaben beziehen sich im ersten Fall auf das unprotonierte Molekülion (M)⁺ oder das protonierte Molekülion (M+H)⁺.

Der Messwert für die optische Drehung ist der spezifische Drehwinkel [α]^{D}, in Klammern finden sich die Konzentration des zu untersuchenden Stoffes in g/ml und das betreffende Lösungsmittel.

Bei der Elementaranalyse werden zunächst jeweils nach der Summenformel die berechneten prozentualen Gewichtsanteile der betreffenden Atome angegeben (Anal. kalk.) und dann die tatsächlich gefundenen Messwerte (gef.).

### Beispiel 1: 5(S)-Amino-4(S)-hydroxy-2(R),7,7-trimethyl-8-[3(R,S)-methoxycarbonyl-1,2,3,4-tetrahydrochinolin-1-ylcarbonyl]-octansäure(Nbutyl)amid

95 mg (4O,5N-Isopropyliden)-5(S)-tertiärbutoxycarbonylamino-4(S)-hydroxy-2(R),7,7-trimethyl-8-[3(R,S)-methoxycarbonyl-1,2,3,4-tetrahydrochinolin-1-ylcarbonyl]-octansäure(N-butyl)amid werden bei Raumtemperatur in 95%-iger Trifluoressigsäure gelöst. Die Lösung wird nach 20 min am Wasserstrahlvakuum eingeengt. Das Rohprodukt wird durch FC an 10 g Kieselgel (Laufmittel O) gereinigt. Die Titelverbindung wird als Diastereomerengemisch erhalten: R_{f}(V)= 0.31; Rₜ(I)= 19.0 und 19.8 min; FAB-MS (M+H)⁺= 490.

Die Ausgangsmaterialien werden folgendermassen hergestellt:
a) (4O,5NIsopropyliden)-5(S)-tertiärbutoxycarbonylamino-4(S)-hydroxy-2(R),7,7-trimethyl-8-[3(R,S)-methoxycarbonyl-1,2,3,4-tetrahydrochinolin-1-ylcarbonyl]-octansäure(Nbutyl)amid
   100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid werden in 5 ml Methylenchlorid gelöst und bei 05 °C mit 0.07 ml 1Chlor-N,N,2trimethylpropenylamin versetzt. Nach 1 h werden 96 mg 3(R,S)-methoxycarbonyl-1,2,3,4-tetrahydrochinolin (Beispiel 1r)), 0.09 ml Diisopropylethylamin und 3mg 4-DMAP gelöst in 1 ml Methylenchlorid dazugetropft und 1 h bei 25 °C gerührt. Das Reaktionsgemisch wird eingeengt und das Rohprodukt mittels FC (10 g Kieselgel, Laufmittel B) gereinigt. Die Titelverbindung wird als Diastereomerengemisch erhalten: R_{f}(A)= 0.55; Rₜ(II)= 21.0 min.
b) 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid
   2.9 g 3-[3-Tertiärbutoxycarbonyl-4(S)-(4-hydroxy-2,2-dimethylbutyl)-2,2-dimethyloxazolidin-5(S)-yl]-2(R)-methyl-propionsäure(Nbutyl)amid werden in 45 ml Acetonitril und 45 ml Tetrachlorkohlenstoff gelöst und zu einer Lösung von 8.56 g Natriumperjodat und 150 mg Ruthenium(III)chloridHydrat in 85 ml Wasser addiert. Nach 5 h wird das Rohprodukt durch Extraktion mit Methylenchlorid isoliert. Anschliessend wird das Rohprodukt in 200ml Diethylether gelöst, mit 10 g Hyflo® verrührt und nach 30 min über Hyflo® abfiltriert. Das Filtrat wird eingeengt und der Rückstand durch FC (100 g Kieselgel, Laufmittel S) gereinigt. Man erhält die Titelverbindung: R_{f}(S)= 0.35; Rₜ(II)= 11.4 min; FABMS: (M+H)⁺= 457.
c) 3-[3-Tertiärbutoxycarbonyl-4(S)-(4-hydroxy-2,2-dimethylbutyl)-2,2-dimethyl-1,3-oxazolidin-5(S)-yl]-2(R)-methyl-propionsäure(Nbutyl)amid
   7.2 g 3-[3-Tertiärbutoxycarbonyl-4(S)(2,2-dimethyl-4-triisopropylsilyloxybutyloxy-2,2-dimethyl-1,3-oxazolidin-5(S)-yl]-2-(R,S)-methyl-propionsäure(N-butyl)amid werden in 70ml Tetrahydrofuran gelöst und bei Raumtemperatur mit 25 ml einer 1 M Tetrabutylammoniumfluoridlösung in Tetrahydrofuran versetzt. Nach 2 h wird das Reaktionsgemisch eingedampft. Das Diastereomerengemisch wird mittels FC aufgetrennt (500 g Kieselgel, Laufmittel A/Essigester). Man erhält die Titelverbindung (Diastereomer I): R_{f}(Essigester)= 0.39; FABMS: (M+H)⁺= 443; und Diastereomer II: R_{f}(Essigester)= 0.33; FABMS: (M+H)⁺= 443.
d) 3-[3-Tertiärbutoxycarbonyl-4(S)-(2,2-dimethyl-4-triisopropylsilyloxybutyl)-2,2-dimethyl-1,3-oxazolidin-5(S)-yl]-2-(R,S)-methyl-propionsäure(N-butyl)amid
   13 g 5(S)-Tertiärbutoxycarbonylamino-4(S)-hydroxy-9-triisopropylsilyloxy-2(R,S),7,7-trimethyl-nonansäurebutylamid und 20 ml 2,2-Dimethoxypropan werden in 40 ml Methylenchlorid gelöst und bei Raumtemperatur mit 0.3 g p-Toluolsulfonsäure-Hydrat versetzt. Nach 2 h wird 1 ml Triethylamin zugegeben. Das Reaktionsgemisch wird eingeengt und das Rohprodukt mittels FC (500 g Kieselgel, Laufmittel D) gereinigt. Die Titelverbindung wird als Diastereomerengemisch isoliert: R_{f}(D)= 0.27/0.22.
e) 5(S)-Tertiärbutoxycarbonylamino-4(S)-hydroxy-9-triisopropylsilyloxy-2(R,S),7,7-trimethylnonansäure(N-butyl)amid
   wird durch Hydrierung von 13.3 g 5(S)-Tertiärbutoxycarbonylamino-4(S)-hydroxy-9-triisopropylsilyloxy-7,7-dimethyl-2-methylen-nonansäure(N-butyl)amid in Gegenwart von 10 g PalladiumKohle (10 % Pd) in 200ml Tetrahydrofuran bei Raumtemperatur und Normaldruck über 4 h und anschliessende Filtration des Reaktionsgemisches über Hyflo® und Einengen des Filtrats erhalten. Die Titelverbindung ist ein Gemisch aus den beiden 2(R)- und 2(S)-Diastereomeren im Verhältnis circa (5:4): R_{f}(A)= 0.26; Rₜ(II)= 34.0 und 34.4 min; FAB-MS: (M+H)⁺= 559.
f) 5(S)-Tertiärbutoxycarbonylamino-4(S)-hydroxy-9-triisopropylsilyloxy-7,7-dimethyl-2-methylen-nonansäure(N-butyl)amid
   14.6 g Methacrylsäurebutylamid werden in 400 ml Tetrahydrofuran gelöst und bei -75 °C mit 130 ml einer 1.6 M n-Butyllithiumlösung in Hexan deprotoniert. Anschliessend wird das Reaktionsgemisch für 30 min bei 0 °C gerührt. Zu dieser Lösung werden bei -75 °C 160 ml einer 1.0 M Chlortriisopropyloxytitanlösung in Hexan während 30 min zugegeben. Nach 15 min wird bei -70 °C eine Lösung von 18.7 g 2(S)-Tertiärbutoxycarbonylamino-6-triisopropylsilyloxy-4,4-dimethyl-hexanal in 100 ml absolutem Tetrahydrofuran zugetropft. Das Reaktionsgemisch wird für 15 min bei -70°C gerührt, dann langsam auf 0 °C erwärmen lassen und nach 40 min bei -15 °C mit 200ml einer gesättigten Ammoniumchloridlösung versetzt. Die Suspension wird über Hyflo® filtriert und das Produkt wird mit Diethylether extrahiert. Das Diastereomerengemisch wird mittels FC (600 g Kieselgel, Laufmittel B) getrennt. Man erhält die Titelverbindung (Diastereomer I): R_{f}(A)= 0.44; Rₜ(II)= 36.0 min; sowie Diastereomer II: R_{f}(A)= 0.36; Rₜ(II)= 35.3 min.
g) 2(S)-Tertiärbutoxycarbonylamino-6-triisopropylsilyloxy-4,4-dimethyl-hexanal
   Zu einer Lösung von 13.4 ml Oxalylchlorid in 200 ml Methylenchlorid werden bei -60 °C 14.7 ml Dimethylsulfoxid in 200 ml Methylenchlorid zugetropft. Nach 15 min wird bei -60 °C eine Lösung von 43.2 g 2(S)-Tertiärbutoxycarbonylamino-6triisopropylsilyloxy-4,4dimethylhexan-1-ol in 320 ml Methylenchlorid unter kräftigem Rühren innerhalb von 40 min zugetropft. Anschliessend werden 58 ml Triethylamin zugegeben und die Reaktionsmischung bei - 60 °C über 90 min gerührt. Man läßt auf - 10 °C aufwärmen, addiert 200 ml einer 20 %igen Kaliumhydrogensulfatlösung, rührt kurz nach und extrahiert das Produkt mit Methylenchlorid. Man erhält 43 g der Titelverbindung, die ohne weitere Reinigung weiter umgesetzt wird: R_{f}(A)= 0.62, R_{f}(D) 0.3; Rₜ(II)= 34.8 min.
h) 2(S)-Tertiärbutoxycarbonylamino-6-triisopropylsilyloxy-4,4-dimethyl-hexan-1-ol
   5.7 g 3-Tertiärbutoxycarbonyl-4(S)-(4-triisopropylsilyloxy-2,2-dimethylbutyl)-2,2-dimethyl-1,3-oxazolidin werden in 100 ml Methanol gelöst und mit 180 mg Pyridiniump-toluolsulfonat versetzt. Nach 72 h Rühren bei Raumtemperatur wird eingeengt und das Rohprodukt mittels FC gereinigt (300 g Kieselgel, Laufmittel G und B). Man erhält die Titelverbindung: R_{f}(B)= 0.42; IR(CH₂Cl₂): 3600 w, 3440 m, 1705 s (cm⁻¹).
i) 3-Tertiärbutoxycarbonyl-4(S)-(4-triisopropylsilyloxy-2,2-dimethylbutyloxy-2,2-dimethyl-1,3-oxazolidin
   5.6 g 3-Tertiärbutoxycarbonyl-4(S)-(4-hydroxy-2,2-dimethylbutyloxy-2,2-dimethyl-1,3-oxazolidin werden in 50 ml Methylenchlorid gelöst und bei Raumtemperatur nacheinander mit 1.6 g Imidazol, 4.3 g Triisopropylchlorsilan und 20 mg 4-DMAP versetzt. Man läßt 16 h bei 20 °C rühren, filtriert das ausgefallene Salz ab und reinigt den eingeengten Rückstand mittels FC an 500 g Kieselgel mit einem 1:20-Gemisch aus Essigester und Hexan als Laufmittel. Man erhält die reine Titelverbindung: R_{f} (G)= 0.45; IR (CH₂Cl₂): 1690 s (cm⁻¹).
j) 3-Tertiärbutoxycarbonyl-4(S)-(4-hydroxy-2,2-dimethylbutyloxy-2,2-dimethyl-1,3-oxazolidin
   68.6 g 3-Tertiärbutoxycarbonyl-2,2-dimethyl-4(S)-(2,2-dimethylbut-3-enyl)-1,3-oxazolidin werden in 500 ml Tetrahydrofuran gelöst und bei 0 °C 10 ml Boran/Dimethylsulfid-Komplex zugetropft. Das Reaktionsgemisch wird 1 h bei 0 °C und 2 h bei Raumtemperatur gerührt. Anschliessend werden bei Raumtemperatur 60 ml eines 3:1-Gemisches aus Tetrahydrofuran und Wasser (v/v), dann 61 ml einer 2N Natriumhydroxidlösung und schliesslich noch bei 0°C 35 ml einer 30 % Wasserstoffperoxidlösung langsam zugetropft. Das Reaktionsgemisch wird 1 h bei Raumtemperatur gerührt und dann mit 200 ml gesättigter Kaliumcarbonat-Lösung verdünnt. Das Produkt wird mit Essigester extrahiert und die Titelverbindung durch FC (600 g Kieselgel, Laufmittel B) in reiner Form erhalten: R_{f} (A)= 0.47; FAB-MS: (M+H)⁺= 302.
k) 3-Tertiärbutoxycarbonyl-2,2-dimethyl-4(S)-(2,2-dimethylbut-3-enyl)-1,3-oxazolidin
   Zu einer Lösung von 85 ml Hexamethyldisilazan in 800 ml Tetrahydrofuran werden bei -40°C 255 ml einer 1.6 M Butyllithiumlösung in Hexan zugegeben. Nach 15 min werden bei 0°C 140 g Methyltriphenylphosphoniumbromid addiert, und das Reaktionsgemisch während 30 min bei 30 °C gerührt. Anschliessend werden 77 g 3-Tertiärbutoxycarbonyl-2,2-dimethyl-4(S)-(2-formyl-2-methylpropyl)-1,3-oxazolidin, gelöst in 200 ml Tetrahydrofuran, bei -30 °C zur Reaktionslösung gegeben. Das Reaktionsgemisch wird für 1 h bei Raumtemperatur gerührt und anschliessend bei 0 °C 600 ml Hexan zugegeben. Das ausgefallene Triphenylphosphinoxid wird abfiltriert und das Filtrat nach Verdünnung mit 400 ml gesättigter Ammoniumchloridlösung mit Diethylether extrahiert. Das Rohprodukt wird mittels FC (400 g Kieselgel, Laufmittel G) gereinigt: R_{f} (D)= 0.55, R_{f}(Essigester)= 0.44; [α]^{D} = + 4.2 (c=1.25 in CHCl₃); MS: (M)⁺= 283.
l) 3-Tertiärbutoxycarbonyl-2,2-dimethyl-4(S)-(2-formyl-2-methylpropyl)-1,3-oxazolidin
   Zu einer Lösung von 37.4 ml Oxalylchlorid in 750 ml Methylenchlorid werden bei -60 °C 58ml Dimethylsulfoxid zugetropft. Nach 5 min wird bei -60 °C eine Lösung von 77.8 g 3-TertiärbutoxycarbonyI-2,2-dimethyl-4(S)-(3-hydroxy-2,2-dimethylpropyl)-1,3-oxazolidin in 250 ml Methylenchlorid zugetropft. Das Reaktionsgemisch wird für 30 min bei -30°C gerührt, dann bei -60°C mit 133 ml Triethylamin versetzt und während 30 min auf 0°C erwärmt. Anschliessend wird das Reaktionsgemisch mehrmals mit Wasser gewaschen und dann eingedampft. Das Produkt wird in 200 ml Toluol gelöst, übe Natriumsulfat getrocknet, filtriert und erneut eingeengt. Man erhält die Titelverbindung: R_{f}(A)= 0.70, R_{f}(D)= 0.35; [α]^{D} = + 20.7 (c=1.25 in CHCl₃); FAB-MS: (M+H)⁺= 286.
m) 3-Tertiärbutoxycarbonyl-2,2-dimethyl-4(S)-(3-hydroxy-2,2-dimethylpropyl)-1,3-oxazolidin
   Zu einer Suspension von 22 g Lithiumaluminiumhydrid in 700 ml Tetrahydrofuran wird bei -10°C eine Lösung von 85.5 g 3-[3-Tertiärbutoxycarbonyl-2,2-dimethyl-1,3-oxazolidin-4(S)-yl]-2,2-dimethyl-propionsäuremethylester in 300 ml Tetrahydrofuran zugegeben und anschliessend 30 min bei 0 °C gerührt. Danach werden bei 0 °C nacheinander 22 ml Wasser, 22 ml einer 5N-Natriumhydroxidlösung und 66 ml Wasser zugetropft, und es wird 30 min nachgerührt. Die Suspension wird über Hyflo® abfiltriert und eingedampft. Die Titelverbindung wird nach Trocknung mit Natriumsulfat in Methylenchlorid in reiner Form erhalten: weisse Kristalle; Smp. 45-6 °C; R_{f}(A)= 0.53, R_{f}(D)= 0.18; (α)^{D} = + 52.8 (c=0.91 in CHCl₃); FAB-MS: (M+H)⁺= 288; Anal. kalk. für C₁₅H₂₉NO₄: C 62.69 %, H 10.17 %, N 4.87 %; gef. C 62.88 %. H 10.11 %, N 4.86 %.
n) 3-[3-Tertiärbutoxycarbonyl-2,2-dimethyl-1,3-oxazolidin-4(S)-yl]-2,2-dimethylpropionsäuremethylester
   Zu einer Lösung von 168 ml Hexamethyldisilazan in 800 ml Tetrahydrofuran werden bei -40 °C 500 ml einer 1.6 M n-Butyllithiumlösung in Hexan zugegeben. Nach 20 min wird bei -70 °C bis -75 °C 50 ml Methyljodid und anschliessend eine Lösung von 86.2 g 3-[3-Tertiärbutoxycarbonyl-2,2-dimethyl-1,3-oxazolidin-4(S)-yl]-propionsäuremethylester in 400ml Tetrahydrofuran zugetropft. Das Reaktionsgemisch wird für 15min bei -70 °C gerührt, dann im Laufe von 1 h auf 0 °C erwärmt und nach 30 min mit 300 ml gesättigter Ammoniumchloridlösung und 400 ml Wasser versetzt. Die Titelverbindung wird durch Extraktion mit Diethylether und anschliessende Vakuumdestillation erhalten: Kp_{0.003}= 95-8 °C; [α]^{D} = + 23.7 (c=1.0 in CHCl₃); R_{f}(D)=0.42; FAB-MS: (M+H)⁺= 316; Anal. kalk. für C₁₆H₂₉NO₅: C 60.93 %, H 9.27 %, N 4.44 %; gef. C 61.10 %. H 9.14 %, N 4.66 %.
o) 3-[3-Tertiärbutoxycarbonyl-2,2-dimethyl-1,3-oxazolidin-4(S)-yl]-propionsäuremethylester
   Zu einer Lösung von 159.5 g 4(S)-Tertiärbutoxycarbonylamino5hydroxyvaleriansäuremethylester und 3 g p-Toluolsulfonsäure-Hydrat in 500 ml Methylenchlorid werden bei 0°C 120 ml Isopropenylmethylether zugegeben. Das Reaktionsgemisch wird für 2 h bei 0°C und 3 h bei Raumtemperatur gerührt. Die Reaktionslösung wird mit 100 ml gesättigter NatriumbicarbonatLösung gewaschen und eingedampft. Die Titelverbindung wird durch FC (800 g Kieselgel, Laufmittel E) erhalten: [α]^{D} = + 20.5 (c=0.8 in CHCl₃); R_{f}(D)= 0.24; MS: (M)⁺= 287.
p) 4(S)-Tertiärbutoxycarbonylamino5-hydroxyvaleriansäuremethylester
   130.5 g 2(S)-Tertiärbutoxycarbonylglutaminsäure-5-methylester und 76.7 ml Triethylamin werden in 500 ml Tetrahydrofuran gelöst, und bei -20 °C werden 76 ml Chlorameisensäuremethylester unter Argon zugetropft. Nach 20 min Rühren bei -15 ⁰C werden während 30 min portionsweise 10 g Lithiumborhydrid zugegeben. Das Reaktionsgemisch wird für 15 min bei - 20 °C gerührt, dann mit 2N Schwefelsäure auf pH 3 angesäuert und anschliessend mit Essigester extrahiert. Das Rohprodukt wird mittels Kristallisation aus Essigester/Hexan gereinigt. Man erhält die Titelverbindung: Smp. 48-9 °C; R_{f}(O)= 0.58, R_{f}(V)= 0.46; [α]^{D} = -11.5 (c=0.96 in CHCl₃); FAB-MS: (M+H)⁺= 248; Anal. kalk. für C₁₁H₂₁NO₅: C 53.43 %, H 8.56 %, N 5.66 %; gef. C 53.22 %, H 8.64 %, N 5.67 %.
q) 2(S)-Tertiärbutoxycarbonylglutaminsäure-5-methylester
   100 g (S)-Glutaminsäure5methylester und 52.1 g Natriumbicarbonat werden in 300 ml Wasser und 300 ml Dioxan gelöst. Zu dieser Lösung wird bei Raumtemperatur 150 g Ditertiärbutyldicarbonat zugegeben. Nach 3 h wird das Reaktionsgemisch mit 370 ml einer 2 M KaliumhydrogensulfatLösung verdünnt und das Produkt mit Essigester extrahiert. Die Titelverbindung wird durch Kristallisation aus Diethylether/Hexan erhalten: Smp. 75-6 °C; [α]^{D} = - 10.8 (c=1.0 in Essigsäure); R_{f}(D) = 0.43.
r) 3(R,S)-Methoxycarbonyl-1,2,3,4-tetrahydrochinolin
   wird durch Hydrierung von 495 mg N-Benzyloxycarbonyl-3(R,S)-methoxycarbonyl-1,2,3,4-tetrahydrochinolin in Gegenwart von 100 mg PalladiumKohle (10 % Pd) in 20 ml Methanol bei Raumtemperatur und Normaldruck für 4 h gewonnen. Das Reaktionsgemisch wird über Hyflo® filtriert und eingeengt. Die Titelverbindung wird als reines Öl erhalten: R_{f}(D)= 0.28; Rₜ(II)= 3.5 min; FAB-MS: (M)⁺= 191.
s) N-Benzyloxycarbonyl-3(R,S)-methoxycarbonyl-1,2,3,4-tetrahydrochinolin
   1.87 g N-Benzyloxycarbonyl-1,2,3,4-tetrahydrochinolin3(R,S)-carbonsäure und 5 ml N,N-Dimethylformamiddimethylacetal werden in 20 ml Toluol für 4 h auf 80 °C erhitzt. Das Reaktionsgemisch wird eingeengt und der Rückstand wird mittels FC (50 g Kieselgel, Laufmittel D) gereinigt: R_{f}(D)= 0.29; MS: (M)⁺= 325.
t) N-Benzyloxycarbonyl-1,2,3,4-tetrahydrochinolin-3(R,S)-carbonsäure
   5 g Chinolin3-carbonsäure werden in Gegenwart von 5 g RaneyNickel bei Raumtemperatur in 100 ml 1N-Natronlauge für 16 h bei 5.1 bar (5 atm) hydriert. Das Reaktionsgemisch wird über Hyflo® filtriert und bei 0 - 5 °C mit 100 ml Tetrahydrofuran und 6 g Chlorameisensäurebenzylester versetzt. Nach 1 h wird das Reaktionsgemisch zur Hälfte eingeengt und mit Diethylether extrahiert. Die wäßrige Phase wird anschliessend auf pH 2.5 angesäuert und mit Methylenchlorid extrahiert. Die Titelverbindung wird durch Umkristallisation aus Diethylether/Hexan in reiner Form erhalten: Smp. 113 °C; R_{f}(X)= 0.43; MS: (M)⁺= 311; Anal. kalk. für C₁₁H₁₇NO₄: C 69.44 %, H 5.50 %, N 4.50 %; gef. C 69.27 %, H 5.68 %, N 4.49 %.

### Beispiel 2: 4(S)-Amino-3(S)-hydroxy-1(R),6,6-trimethyl-heptandicarbonsäure-1-(N-butyl)amid-7-(N-naphth-1-yl)amid

Analog wie in Beispiel 1) beschrieben wird die Titelverbindung ausgehend von 195mg (3O,4N-Isopropyliden)4(S)-tertiärbutoxycarbonylamino-3(S)-hydroxy-1(R),6,6trimethylheptandicarbonsäure-1-(N-butyl)amid-7-(N-naphth-1-yl)amid hergestellt und durch FC an 50 g Kieselgel (LaufmittelS) gereinigt. Dies ergibt die reine Titelverbindung: R_{f}(S)=0.22; R_{f}(Y)=0.43; FAB-MS: (M+H)⁺=442.

Das Ausgangsmaterial wird beispielsweise folgendermassen hergestellt:
a) (3O,4N-Isopropyliden)4(S)-tertiärbutoxycarbonylamino-3(S)-hydroxy-1(R),6,6trimethylheptandicarbonsäure-1-(N-butyl)amid-7-(N-naphth-1-yl)amid
   Die Titelverbindung wird durch Rühren von 370mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl propionsäure(N-butyl)amid, 0.56ml Ethyldiisopropylamin, 413mg Bis-(2-oxo-3-oxazolidinyl)-phosphinsäurechlorid, 350mg 1-Naphthylamin und 10mg 4-DMAP in 5 ml Methylenchlorid während 24h bei Raumtemperatur erhalten. Das Rohprodukt wird mittels FC an 70 g Kieselgel (LaufmittelZ und Y) gereinigt. Dies ergibt die reine Titelverbindung: R_{f}(Y)=0.50.

### Beispiel 3: 5(S)-Amino-4(S)-hydroxy-2(R),7,7-trimethyl-8-(1,2,3,4-tetrahydrochinolin-1-yl-carbonyl)-octansäure(N-butyl)amid

In analoger Weise wie in den Beispielen 2 und 2a) für die entsprechenden Reaktionsstufen beschrieben erhält man die Titelverbindung ausgehend von 150mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 0.25ml 1,2,3,4-Tetrahydrochinolin über das (4O,5N-Isopropyliden)-5(S)-tertiärbutoxycarbonylamino-4(S)-hydroxy-2(R),7,7-trimethyl-8-(1,2,3,4-tetrahydrochinolin-1-ylcarbonyl]-octansäure(N-butyl)amid (R_{f}(Y)=0.36): R_{f}(S)=0.32; FAB-MS: (M+H)⁺=432.

### Beispiel 4: 5(S)-Amino-4(S)-hydroxy-2(R),7,7-trimethyl-8-(2,3-dihydroindol1-ylcarbonyl)-octansäure(N-butyl)amid

In analoger Weise wie in den Beispielen 2 und 2a) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 0. 120ml Indolin über das (4O,5N-Isopropyliden)-5(S)-tertiärbutoxycarbonylamino-4(S)-hydroxy-2(R),7,7-trimethyl-8-(2,3-dihydroindol1-ylcarbonyl)-octansäure(N-butyl)amid (R_{f}(Y)=0.71) : R_{f}(S)=0.33; R_{f}(T)=0.38; FAB-MS: (M+H)⁺=418.

### Beispiel 5: 4(S)-Amino-3(S)-hydroxy-1(R),6,6-trimethyl-heptandicarbonsäure-1-(N-butyl)amid-7-(N-phenyl)amid

In analoger Weise wie in den Beispielen 2 und 2a) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 0.1 ml Anilin über das (3O,4N-Isopropyliden)4(S)-N-tertiärbutoxycarbonylamino-3(S)-hydroxy-1(R),6,6-trimethyl heptandicarbonsäure-1-(N-butyl)amid-7-(N-phenyl)amid (R_{f}(Y)=0.63) und Reinigung durch FC an 30 g Kieselgel (LaufmittelN): R_{f}(S)=0.36; FAB-MS: (M+H)⁺=392.

### Beispiel 6: 4(S)-Amino-3(S)-hydroxy-1(R),6,6-trimethyl-heptandicarbonsäure-1-(N-butyl)amid-7-[N-[2-(methoxycarbonylaminomethyl)phenyl]]amid

In analoger Weise wie in den Beispielen 2 und 2a) für die entsprechenden Reaktionsstufen beschrieben erhält man die Titelverbindung ausgehend von 300 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 473 mg 2-(Methoxycarbonylaminomethyl)anilin über das (3O,4N-Isopropyliden)4(S)-tertiärbutoxycarbonylamino-3(S)-hydroxy-1(R),6,6-trimethyl-heptandicarbonsäure-1-(N-butyl)amid-7-[N-[2 (methoxycarbonylaminomethyl)phenyl]]amid (R_{f}(B)= 0.35) und Reinigung an Kieselgel (Laufmittel S): R_{f}(S)= 0.24; FAB-MS: (M+H)⁺= 479.

Die eingesetzte Amin-Komponente erhält man ausgehend von 2.0 g 2-(Aminomethyl)anilin, gelöst in 30 ml Methylenchlorid, durch Reaktion mit 1.3 ml Chlorameisensäuremethylester in Gegenwart von 3.1 ml Diisopropylethylamin bei 5 °C. Nach Reinigung an 80 g Kieselgel (Laufmittel A) erhält man das 2-(Methoxycarbonylaminomethyl)anilin: R_{f}(A)= 0.23; R_{f}(Z)= 0.31.

### Beispiel 7: 4(S)-Amino-3(S)-hydroxy-1(R),6,6-trimethyl-heptandicarbonsäure-1-(N-butyl)amid-7-[N-(4-bromnaphth-1-yl)]amid

In analoger Weise wie in den Beispielen 2 und 2a) für die entsprechenden Reaktionsstufen beschrieben erhält man die Titelverbindung ausgehend von 80mg 3-[N-Tertiärbutoxycarbonyl-4(S)-3-carboxy-2,2-dimethylpropyloxy-2,2-dimethyl-1,3-oxazolidin-5(S)-yl]-2(R)-methyl-propionsäure(N-butyl)amid und 233mg 4-Bromnaphthylamin über das (3O,4N-Isopropyliden)4(S)-tertiärbutoxycarbonylamino-3(S)-hydroxy-1(R),6,6-trimethylheptandicarbonsäure-1-(N-butyl)amid-7-[N-(4-bromnaphth-1-yl)]amid (R_{f}(Y)=0.61): R_{f}(S)=0.20; R_{f}(T)=0.28; FAB-MS: (M+H)⁺=520.

### Beispiel 8: 5(S)-Amino-4(S)-hydroxy-2(R),7,7-trimethyl-8-(5,6-dihydrophenanthridinocarbonyl)-octansäure(N-butyl)amid

In analoger Weise wie in den Beispielen 2 und 2a) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 190mg Dihydrophenanthridin über das (4O,5N-Isopropyliden)-5(S)-tertiärbutoxycarbonylamino-4(S)-hydroxy-2(R),7,7-trimethyl-8-(5,6-dihydrophenanthridinocarbonyl)-octansäure(N-butyl)amid (R_{f}(Y)=0.61) : R_{f}(S)=0.36; FAB-MS: (M+H)⁺=480.

Das als Ausgangsmaterial eingesetzte 5,6-Dihydrophenanthridin wird beispielsweise folgendermassen hergestellt:
5.0 g 6(5H)-Phenanthridinon werden in 50ml Tetrahydrofuran unter Zusatz von 1.46 g Lithiumaluminiumhydrid während 26h bei Raumtemperatur reduziert. Die Reinigung des Rohproduktes erfolgt durch FC an 250 g Kieselgel mit Dichlormethan: R_{f}(1:1-Gemisch aus Methylenchlorid und Hexan) = 0.25.

### Beispiel 9: 5(S)-Amino-4(S)-hydroxy-2(R),7,7-trimethyl-8-(1,2,3,4-tetrahydroisochinolin-2-ylcarbonyl)-octansäure(N-butyl)amid

In analoger Weise wie in den Beispielen 2 und 2a) für die entsprechenden Reaktionsstufen beschrieben erhält man die Titelverbindung ausgehend von 80mg 3-[N-Tertiärbutoxycarbonyl-4(S)-3-carboxy-2,2-dimethylpropyloxy-2,2-dimethyl-1,3-oxazolidin-5(S)-yl]-2(R)-methyl-propionsäure(N-butyl)amid und 0.14ml 1,2,3,4-Tetrahydroisochinolin über das (4O,5N-Isopropyliden)-5(S)-tertiärbutoxycarbonylamino-4(S)-hydroxy-2(R),7,7-trimethyl-8-(1,2,3,4-tetrahydroisochinolin2-ylcarbonyl)-octansäure(N-butyl)amid (R_{f}(Y)=0.49) und Reinigung an 30 g Kieselgel (LaufmittelN): R_{f}(S)=0.28; R_{f}(T)=0.44; FAB-MS: (M+H)⁺=432.

### Beispiel 10: 4(S)-Amino-3(S)-hydroxy-1(R),6,6-trimethyl-heptandicarbonsäure-1-(N-butyl)amid-7-[N-[2-(formylaminomethyl)phenyl]]amid

In analoger Weise wie in den Beispielen 2 und 2a) für die entsprechenden Reaktionsstufen beschrieben erhält man die Titelverbindung ausgehend von 300 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 394 mg 2-(Formylaminomethyl)anilin über das (3O,4N-Isopropyliden)4(S)-tertiärbutoxycarbonylamino-3(S)-hydroxy-1(R),6,6-trimethyl-heptandicarbonsäure-1-(N-butyl)amid-7-[N-[2-(formylaminomethyl)phenyl]]amid (R_{f}(O)= 0.46): R_{f}(T)= 0.38; FAB-MS: (M+H)⁺= 449.

Die eingesetzte Amin-Komponente wird durch Umsetzung von 2.6 g 2-(Aminomethyl)anilin, gelöst in 50 ml Methylenchlorid, mit 3.91 g Ameisensäure-4nitrophenylester über 4 h bei Raumtemperatur hergestellt. Reinigung an 250 g Kieselgel (Laufmittel N) ergibt das 2-(Formylaminomethyl)anilin: R_{f}(N)= 0.22.

### Beispiel 11: 4(S)-Formylamino-3(S)-hydroxy-1(R),6,6-trimethyl-heptandicarbonsäure-1-(N-butyl)amid-7-(N-phenyl)amid

28mg 4(S)-Amino-3(S)-hydroxy-1(R),6,6-trimethyl-heptandicarbonsäure-1-(N-butyl)amid-7-(N-phenyl)amid (Beispiel5) und 16mg Ameisensäure4nitrophenylester werden während 2h in 2 ml Dichlormethan gerührt. Das eingeengte Rohprodukt wird anschliessend an 10 g Kieselgel (LaufmittelO) gereinigt: R_{f}(T)=0.42; FAB-MS: (M+H)⁺=420.

### Beispiel 12: 5(S)-Amino-4(S)-hydroxy-2(R),7,7-trimethyl-8-[3(R)- oder 3(S)-ethoxycarbonylpiperidin-1ylcarbonyl]-octansäure(N-butyl)amid

In analoger Weise wie in den Beispielen 1 und 1a) für die entsprechenden Reaktionsstufen beschrieben erhält man die Titelverbindung ausgehend von 30 mg (4O,5N-Isopropyliden)-5(S)-tertiärbutoxycarbonylamino-4(S)-hydroxy-2(R),7,7-trimethyl-8-[3(R)- oder 3(S)-ethoxycarbonylpiperidin-1-ylcarbonyl)-octansäure(N-butyl)amid (Diastereomer I; Beispiel 13) und Reinigung mittels FC (5 g Kieselgel, Laufmittel O und U) als reines Stereoisomer: R_{f}(U)= 0.71 ; Rₜ(II)= 3.8 min; FAB-MS: (M+H)⁺= 456.

### Beispiel 13: 5(S)-Amino-4(S)-hydroxy-2(R),7,7-trimethyl-8-[3(R)- oder 3(S)-ethoxycarbonylpiperidin-1-ylcarbonyl]-octansäure(N-butyl)amid

In analoger Weise wie in den Beispielen 1 und 1a) für die entsprechenden Reaktionsstufen beschrieben erhält man die Titelverbindung ausgehend von 30mg (4O,5N-Isopropyliden)-5(S)-tertiärbutoxycarbonylamino-4(S)-hydroxy-2(R),7,7-trimethyl-8-[3(R)- oder 3(S)-ethoxycarbonylpiperidin-1-ylcarbonyl)-octansäure(N-butyl)amid (Diastereomer II) und Reinigung mittels FC (5 g Kieselgel, Laufmittel O und U): R_{f}(U)= 0.75; Rₜ(II)= 4.2 min; FAB-MS: (M+H)⁺= 456.

Das in den vorangehenden Beispielen 12 und 13) als Ausgangsmaterial in Form des reinen 3(R)- bzw. 3(S)-Stereoisomeren eingesetzte (4O,5N-Isopropyliden)-5(S)-tertiärbutoxycarbonylamino-4(S)-hydroxy-2(R),7,7-trimethyl-8-[3(R)- oder 3(S)-ethoxycarbonylpiperidin-1-ylcarbonyl)-octansäure(N-butyl)amid wird hergestellt ausgehend von 60 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 0.05 ml Piperidin3(R,S)-carbonsäureethylester. Das erhaltene DiastereomerenGemisch wird durch FC an 10 g Kieselgel (Laufmittel A und Essigester) aufgetrennt. Man erhält das Diastereomere I: R_{f}(A)= 0.20; und das Diastereomere II: R_{f}(A)= 0.13.

### Beispiel 14: 5(S)-Amino-4(S)-hydroxy-2(R),7,7-trimethyl-9-(1,2,3,4-tetrahydrochinolin-1yl)nonansäure(N-butyl)amid

In analoger Weise wie in den Beispielen 1 und 1a) für die entsprechenden Reaktionsstufen beschrieben erhält man die Titelverbindung ausgehend von 90 mg (4O,5N-Isopropyliden)-5(S)-tertiärbutoxycarbonylamino-4(S)-hydroxy-2(R),7,7-trimethyl-9-(1,2,3,4-tetrahydrochinolin-1-yl)nonansäure(N-butyl)amid: R_{f}(S)=0.44; FAB-MS: (M+H)⁺= 418.

Die Ausgangsmaterialien werden beispielsweise wie folgt hergestellt:
a) (4O,5N-Isopropyliden)-5(S)-tertiärbutoxycarbonylamino-4(S)-hydroxy-2(R),7,7-trimethyl-9-(1,2,3,4-tetrahydrochinolin-1-yl)nonansäure(N-butyl)amid
   174 mg 3-[3-Tertiärbutoxycarbonyl-4(S)-(4-jod-2,2-dimethylbutyl)-2,2-dimethyl-1,3-oxazolidin-5(S)-yl]-2(R)-methyl-propionsäure(N-butyl)amid werden in 2 ml 1,2,3,4-Tetrahydrochinolin bei 100 °C während 6 h gerührt. Das Reaktionsgemisch wird am Hochvakuum eingeengt und das erhaltene Rohprodukt an 65 g Kieselgel mit einem 20:1 - Gemisch aus Methylenchlorid und Diethylether gereinigt. Man erhält die Titelverbindung als gelbliches Öl: R_{f}(20:1-Gemisch aus Methylenchlorid und Diethylether)= 0.21.
b) 3-[3-Tertiärbutoxycarbonyl-4(S)-(4-jod-2,2-dimethylbutyl)-2,2-dimethyl-1,3-oxazolidin-5(S)-yl]-2(R)-methyl-propionsäure(N-butyl)amid
   Zu einer Lösung von 300 mg 3-[3-Tertiärbutoxycarbonyl-4(S)-(4-hydroxy-2,2-dimethylbutyl)-2,2-dimethyl-1,3-oxazolidin-5(S)-yl]-2(R)-methyl-propionsäure(N-butyl)amid (Beispiel 1c)) in 5 ml Methylenchlorid werden bei 0 °C 168 mg N-Jodsuccinimid und 196 mg Triphenylphosphin addiert und der Reaktionsansatz über 7 h gerührt. Die Reinigung des Rohprodukts nach üblicher Aufarbeitung erfolgt an 65 g Kieselgel (Laufmittel B) zur Titelverbindung: R_{f}(B)=0.55.

### Beispiel 15: 5(S)-Amino-4(S)-hydroxy-2(R),7,7-trimethyl-8-[3(R,S)-carboxy-1,2,3,4-tetrahydrochinolin-1-ylcarbonyl]-octansäure(N-butyl)amid

49 mg 5(S)-Amino-4(S)-hydroxy-2(R),7,7-trimethyl-8-[3(R,S)-carboxy-1,2,3,4-tetrahydmchinolin-1-ylcarbonyl]-octansäure(N-butyl)amid (aus Beispiel 1) werden in 1 ml 1N-Natronlauge und 2 ml Dioxan für 1 h bei 25 °C gerührt. Das Reaktionsgemisch wird mit 1.05 ml einer 2N Salzsäure-Lösung angesäuert, eingeengt, und das Rohprodukt mittels FC (10 g Kieselgel, Laufmittel V und W) gereinigt. Man erhält die Titelverbindung als Diastereomerengemisch: R_{f}(V)= 0.09; Rₜ(I)= 17.0/18.0 min; FAB-MS: (M+H)⁺= 476.

### Beispiel 16: 5(S)-Amino-4(S)-hydroxy-2(R),7,7-trimethyl-8-[2(R,S)-ethoxycarbonvl-3,4-dihydro-2H-1,4-benzoxazin-4-ylcarbonyl]-octansäure(N-butyl)amid:

In analoger Weise wie in den Beispielen 2 und 2a) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 213mg2(R,S)-Ethoxycarbonyl-3,4-dihydro-2H-1,4-benzoxazin (hergestellt nach dem von H. Bartsch und O.Schwarz in Arch. Pharm. 315, 538 (1982) beschriebenen Verfahren) über das (4O,5N-Isopropyliden)-5(S)-tertiärbutoxycarbonylamino-4(S)-hydroxy-2(R),7,7-trimethyl-8-[2(R,S)-ethoxycarbonyl-3,4-dihydro-2H-1,4-benzoxazin-4-ylcarbonyl]-octansäure(N-butyl)amid (R_{f}(Y)=0.51) als Diastereomerengemisch: R_{f}(S)=0.34; R_{f}(T)=0.39; FAB-MS: (M+H)⁺=506.

### Beispiel 17: 5(S)-Formylamino-4(S)-hydroxy-2(R),7,7-trimethyl-8-[2(R,S)-ethoxycarbonyl-4-dihydro-2H-1,4-benzoxazin-4-ylcarbonyl]-octansäure(N-butyl)amid

25mg 5(S)-Amino-4(S)-hydroxy-2(R),7,7-trimethyl-8-[2(R,S)-ethoxycarbonyl-3,4-dihydro-2H-1,4-benzoxazin-4-ylcarbonyl]-octansäure(N-butyl)amid werden mit 10mg Ameisensäure4-nitrophenylester in 2mlDichlormethan während 26h bei Raumtemperatur gerührt. Das Rohprodukt wird anschliessend mittels FC (LaufmittelS) an 20 gKieselgel gereinigt. Man erhält die reine Titelverbindung: R_{f}(S)=0.49; FAB-MS: (M+H)⁺=534.

### Beispiel 18: 5(S)-Amino-4(S)-hydroxy-2(R),7,7-trimethyl-8-[2(R,S)-methoxycarbonyl-3,4-dihydro-2H-1,4-benzthiazin-4-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 267mg2(R,S)-methoxycarbonyl-3,4-dihydro-2H-1,4-benzthiazin (hergestellt nach dem von R.C.M.Butleretal. in J.Heterocyclic Chem 22, 177(1985) beschriebenen Verfahren) über das (4O,5N-Isopropyliden)-5(S)-tertiärbutoxycarbonylamino-4(S)-hydroxy-2(R),7,7-trimethyl-8-[2(R,S)-methoxycarbonyl-3,4-dihydro-2H-1,4-benzthiazin-4-ylcarbonyl]-octansäure(N-butyl)amid (R_{f}(Y) = 0.44) und Reinigung durch FC an 50 gKieselgel (LaufmittelS) als Diastereomerengemisch: R_{f}(S)=0.36; R_{f}(T)=0.40; FAB-MS: (M+H)⁺=508.

### Beispiel 19: 5(S)-Amino-4(S)-hydroxy-2(R),7,7-trimethyl-8-[3(R,S)-ethoxycarbonyl-3,4-dihydro-2H-1,4-benzoxazin-4-ylcarbonyl[-octansäure(N-butyl)amid

In analoger Weise wie in den Beispielen 2 und 2a) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 218mg 3(R,S)Ethoxycarbonyl-3,4-dihydro-2H-1,4-benzoxazin (hergestellt nach dem von H. Bartsch und O.Schwarz in Arch. Pharm. 315, 538 (1982) beschriebenen Verfahren) über das (4O,5N-Isopropyliden)-5(S)-tertiärbutoxycarbonylamino-4(S)-hydroxy-2(R),7,7-trimethyl-8-[3(R,S)-ethoxycarbonyl-3,4-dihydro-2H-1,4-benzoxazin-4-ylcarbonyl]-octansäure(N-butyl)amid (R_{f}(Y)=0.80) als Gemisch von Diastereomeren: R_{f}(S)=0.38; R_{f}(T)=0.47; FAB-MS: (M+H)⁺=506.

### Beispiel 20: 5(S)-Amino-4(S)-hydroxy-2(R),7,7-trimethyl-8-[3(R,S)-tertiärbutoxycarbonyl-1,2,3,4-tetrahydrochinolin-1-ylcarbonyl]-octansäure(N-butyl)amid

28 mg 5(S)-Tertiärbutoxycarbonylamino-4(S)-hydroxy-2(R),7,7-trimethyl-8-[3(R,S)-tertiärbutoxycarbonyl-1,2,3,4-tetrahydrochinolin-1-ylcarbonyl]-octansäure(N-butyl)amid werden in 2 ml 4N-Salzsäure in Dioxan gelöst und für 30 min bei 20 °C gerührt. Das Reaktionsgemisch wird mit Dioxan verdünnt und lyophilisiert. Das Rohprodukt wird mittels FC (5 g Kieselgel, Laufmittel N und O) gereinigt. Man erhält die Titelverbindung als DiastereomerenGemisch: R_{f}(O)= 0.32; Rₜ(I)= 23.8 min; FAB-MS: (M+H)⁺= 532.

Die Ausgangsmaterialien werden beispielsweise folgendermassen hergestellt:
a) 5(S)-Tertiärbutoxycarbonylamino-4(S)-hydroxy-2(R),7,7-trimethyl-8-[3(R,S)-tertiärbutoxycarbonyl-1,2,3,4-tetrahydrochinolin-1-ylcarbonyl]-octansäure(N-butyl)amid
   Die Mischung aus 41 mg (4O,5N-Isopropyliden)-5(S)-tertiärbutoxycarbonylamino-4(S)-hydroxy-2(R),7,7-trimethyl-8-[3(R,S)-tertiärbutoxycarbonyl-1,2,3,4-tetrahydrochinolin-1-yl-carbonyl]-octansäure(N-butyl)amid, 4 mg p-Toluolsulfonsäure-Hydrat und 3 ml Methanol wird bei Raumtemperatur gerührt. Nach beendeter Reaktion (DC-Kontrolle) wird mit 1 ml Toluol versetzt und anschliessend das Reaktionsgemisch eingeengt. Das so erhaltene Rohprodukt wird mittels FC (10 g Kieselgel, Essigester als Laufmittel) gereinigt. Man erhält die Titelverbindung: R_{f}(A)= 0.10.
b) (4O,5N-Isopropyliden)-5(S)-tertiärbutoxycarbonylamino-4(S)-hydroxy-2(R),7,7-trimethyl-8-[3(R,S)-tertiärbutoxycarbonyl-1,2,3,4-tetrahydrochinolin-1-ylcarbonyl]-octansäure(N-butyl)amid
   Die Titelverbindung wird ausgehend von 57 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid, 66 mg Bis-(2-oxo-3-oxazolidinyl)phosphinsäurechlorid, 58 mg 3(R,S)-Tertiärbutoxycarbonyl-1,2,3,4-tetrahydrochinolin und 0.08 ml Triethylamin analog Beispiel 2a) erhalten und durch FC (20 g Kieselgel, Laufmittel B) gereinigt. Die Titelverbindung wird als Diastereomerengemisch erhalten: R_{f}(A)= 0.31/0.34; Rₜ(II)= 26.3/26.6 min.
c) 3(R,S)-Tertiärbutoxycarbonyl-1,2,3,4-tetrahydrochinolin
   Die Titelverbindung wird analog Beispiel 1r) ausgehend von 367 mg N-Benzyloxycarbonyl-3(R,S)-tertiärbutoxycarbonyl-1,2,3,4-tetrahydrochinolin hergestellt: R_{f}(A)= 0.62; MS: (M)⁺= 233.
d) N-Benzyloxycarbonyl-3(R,S)-tertiärbutoxycarbonyl-1,2,3,4-tetrahydrochinolin
   Die Titelverbindung wird analog Beispiel 1s) ausgehend von 622 mg N-Benzyloxycarbonyl-1,2,3,4-tetrahydrochinolin3(R,S)-carbonsäure und 2.2 ml N,N-Dimethylformamidditertiärbutylacetal hergestellt und mittels FC (50 g Kieselgel, Laufmittel G und E) gereinigt: R_{f}(D)= 0.48; MS: (M)⁺= 367.

### Beispiel 21: 5(S)-Amino-4(S)-hydroxy-2(R),7,7-trimethyl-8-[3(R)-oder 3(S)-methoxycarbonyl-1,2,3,4-tetrahydrochinolin-1-ylcarbonyl]-octansäure(N-butyl)amid

Analog Beispiel 20) wird die Titelverbindung ausgehend von 88mg 5(S)-Tertiärbutoxycarbonylamino-4(S)-hydroxy-2(R),7,7-trimethyl-8-[3(R)- oder 3(S)-methoxycarbonyl-1,2,3,4-tetrahydrochinolin-1-ylcarbonyl]-octansäure(N-butyl)amid und 1 ml 4N-Salzsäure in Dioxan hergestellt und mittels FC (10 g Kieselgel, Laufmittel N und O) gereinigt. Man erhält die Titelverbindung als reines Stereoisomeres: R_{f}(O)= 0.25; Rₜ(I)= 19.7 min.

Die Ausgangsmaterialien werden beispielsweise folgendermassen hergestellt:
a) 5(S)-Tertiärbutoxycarbonylamino-4(S)-hydroxy-2(R),7,7-trimethyl-8-[3(R)- oder-3(S)-methoxycarbonyl-1,2,3,4-tetrahydrochinolin-1-ylcarbonyl]-octansäure(N-butyl)amid
   Die Titelverbindung wird analog Beispiel 20a) ausgehend von 150 mg (4O,5N-Isopropyliden)-5(S)-tertiärbutoxycarbonylamino-4(S)-hydroxy-2(R),7,7-trimethyl-8-[3(R)- oder 3(S)-methoxycarbonyl-1,2,3,4-tetrahydrochinolin-1-ylcarbonyl]-octansäure(N-butyl)amid und 5 mg p-Toluolsulfonsäure-Hydrat erhalten und mittels FC (10 g Kieselgel, Laufmittel A) gereinigt: R_{f}(X)= 0.45; R_{f}(O)= 0.74; Rₜ(II)= 26.4 min.
b) (4O,5N-Isopropyliden)-5(S)-tertiärbutoxycarbonylamino-4(S)-hydroxy-2(R),7,7-trimethyl-8-[3(R)- oder 3(S)-methoxycarbonyl-1,2,3,4-tetrahydrochinolin-1-ylcarbonyl]-octansäure(N-butyl)amid
   Die Titelverbindung wird ausgehend von 183 mg 3-[N-Tertiärbutoxycarbonyl-4(S)-(3-carboxy-2,2-dimethylpropyloxy-2,2-dimethyl-1,3-oxazolidin-5(S)-yl]-2(R)-methylpropionsäure(N-butyl)amid und 115 mg 3(R)- oder 3(S)-Methoxycarbonyl-1,2,3,4-tetrahydrochinolin analog wie in Beispiel 1a) beschrieben erhalten und mittels FC (10 g Kieselgel, Laufmittel A) gereinigt: R_{f}(A)= 0.32.
c) 3(R)- oder 3(S)-Methoxycarbonyl-1,2,3,4-tetrahydrochinolin
   Die Titelverbindung wird analog Beispiel 1r) ausgehend von 495 mg N-Benzyloxycarbonyl-3(R)- oder 3(S)-methoxycarbonyl-1,2,3,4-tetrahydrochinolin hergestellt: Rₜ(II)= 3.5 min; [α]^{D} = -25.6 (c=0.7 in Ethanol).
d) N-Benzyloxycarbonyl-3(R)- oder 3(S)-methoxycarbonyl-1,2,3,4-tetrahydrochinolin
   Die Titelverbindung wird ausgehend von 1.5 g N-Benzyloxycarbonyl-3(R,S)-methoxycarbonyl-1,2,3,4-tetrahydrochinolin (Beispiel 1s) mittels MitteldruckChromatographie an einer Chiralcel 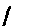 CA-1 Säule mit 95 % Ethanol als Elutionsmittel erhalten: [α]^{D} = -5.4 (c=1.0 in Chloroform).

### Beispiel 22: (S)-Amino-4(S)-hydroxy-2(R),7,7-trimethyl-8-[3(R)- oder 3(S)-methoxycarbonyl-1,2,3,4-tetrahydrochinolin-1-ylcarbonyl]-octansäure(N-butyl)amid

Analog wie in Beispiel 21) beschrieben erhält man die Titelverbindung ausgehend von 59mg 5(S)-Tertiärbutoxycarbonylamino-4(S)-hydroxy-2(R),7,7-trimethyl-8-[3(R)- oder 3(S)-methoxycarbonyl-1,2,3,4-tetrahydrochinolin-1-ylcarbonyl]-octansäure(N-butyl)amid als reines Stereoisomeres: R_{f}(O)= 0.25; Rₜ(I)= 19.8 min; FAB-MS: (M+H)⁺= 490.

Die Ausgangsmaterialien werden folgendermassen hergestellt:
a) 5(S)-Tertiärbutoxycarbonylamino-4(S)-hydroxy-2(R),7,7-trimethyl-8-[3(R)- oder-3(S)-methoxycarbonyl-1,2,3,4-tetrahydrochinolin-1-ylcarbonyl]-octansäure(N-butyl)amid
   Die Titelverbindung wird analog wie in Beispiel 20a) beschrieben, ausgehend von 126 mg (4O,5N-Isopropyliden)-5(S)-tertiärbutoxycarbonylamino-4(S)-hydroxy-2(R),7,7-trimethyl-8-[3(R)- oder 3(S)-methoxycarbonyl-1,2,3,4,-tetrahydrochinolin-1-ylcarbonyl]-octansäure(N-butyl)amid und 5 mg p-Toluolsulfonsäure-Hydrat hergestellt: R_{f}(X)= 0.44; R_{f}(O)= 0.74; Rₜ(II)= 26.5 min.
b) (4O,5N-Isopropyliden)-5(S)-tertiärbutoxycarbonylamino-4(S)-hydroxy-2(R),7,7-trimethyl-8-[3(R)- oder 3(S)-methoxycarbonyl-1,2,3,4-tetrahydrochinolin-1-ylcarbonyl]-octansäure(N-butyl)amid
   Die Titelverbindung wird ausgehend von 183 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl propionsäure(N-butyl)amid und 115 mg 3(R)- oder 3(S)-Methoxycarbonyl-1,2,3,4-tetrahydrochinolin analog wie in Beispiel 1a) beschrieben hergestellt: R_{f}(A)= 0.32.
c) 3(R)- oder 3(S)-Methoxycarbonyl-1,2,3,4-tetrahydrochinolin
   Die Titelverbindung wird analog Beispiel 1r) ausgehend von 495 mg N-Benzyloxycarbonyl-3(R)- oder 3(S)-methoxycarbonyl-1,2,3,4-tetrahydrochinolin hergestellt: Rₜ(II)= 3.5 min; [α]^{D} = +25.1 (c=0.66 in Ethanol).
d) N-Benzyloxycarbonyl-3(R) oder 3(S)-methoxycarbonyl-1,2,3,4-tetrahydrochinolin wird
   Die Titelverbindung wird ausgehend von 1.5 g N-Benzyloxycarbonyl-3(R,S)-methoxycarbonyl-1,2,3,4-tetrahydrochinolin (Beispiel 1s) mittels Mitteldruck-Chromatographie an einer Chiralcel 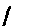 CA-1 Säule mit 95 % Ethanol als Elutionsmittel erhalten: [α]^{D} = +5.5 (c=0.9 in Chloroform).

### Beispiel 23: 5(S)-Amino-4(S)-hydroxy-2(R),7,7-trimethyl-8-[3(R,S)-dimethylaminocarbonyl-1,2,3,4-tetrahydrochinolin-1-ylcarbonyl]-octansäure(N-butyl)amid

Analog wie in Beispiel 21) beschrieben wird die Titelverbindung ausgehend von 29 mg 5(S)-Tertiärbutoxycarbonylamino-4(S)-hydroxy-2(R),7,7-trimethyl-8-[3(R,S)-dimethylaminocarbonyl-1,2,3,4-tetrahydrochinolin-1-ylcarbonyl]-octansäure(N-butyl)amid und 2 ml 4N-Salzsäure in Dioxan erhalten und mittels FC (5 g Kieselgel, Laufmittel N und O) gereinigt: R_{f}(O)=0.11; Rₜ(I)= 18.2 min; FAB-MS: (M+H)⁺= 503.

Die Ausgangsmaterialien werden beispielsweise folgendermassen hergestellt:
a) 5(S)-Tertiärbutoxycarbonylamino-4(S)-hydroxy-2(R),7,7-trimethyl-8-[3(R,S)-dimethylaminocarbonyl-1,2,3,4-tetrahydrochinolin-1-ylcarbonyl]-octansäure(N-butyl)amid
   Die Titelverbindung wird analog wie in Beispiel 20a) beschrieben, ausgehend von 40 mg (4O,5N-Isopropyliden)-5(S)-tertiärbutoxycarbonylamino-4(S)-hydroxy-2(R),7,7-trimethyl-8-[3(R,S)-dimethylaminocarbonyl-1,2,3,4-tetrahydrochinolin-1-ylcarbonyl]-octansäure(N-butyl)amid und Reinigung mittels FC (10 g Kieselgel, Laufmittel N) als Diastereomeren-Gemisch hergestellt: R_{f}(O)= 0.30/0.33.
b) (4O,5N-Isopropyliden)-5(S)-tertiärbutoxycarbonylamino-4(S)-hydroxy-2(R),7,7-trimethyl-8-[3(R,S)-dimethylaminocarbonyl-1,2,3,4-tetrahydrochinolin-1-ylcarbonyl]-octansäure(N-butyl)amid
   Die Titelverbindung wird ausgehend von 92 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 123mg 1,2,3,4-Tetrahydrochinolin3(R,S)-carbonsäure(N,N-dimethyl)amid analog wie in Beispiel 2a) und Reinigung durch FC an 30 g Kieselgel mit einem Laufmittel-Gradienten von A zu Essigester als Gemisch von Stereoisomeren hergestellt: R_{f}(A)= 0.11/0.13; Rₜ(II)= 17.2/17.4 min.
c) 1,2,3,4-Tetrahydrochinolin3(R,S)-carbonsäure(N,N-dimethyl)amid
   Die Titelverbindung wird analog Beispiel 1r) ausgehend von 620 mg N-Benzyloxycarbonyl-1,2,3,4-tetrahydrochinolin3(R,S)-carbonsäure(N,N-dimethyl)amid hergestellt und durch Umkristallisation aus Diethylether/Hexan gereinigt: Smp. 130 °C.
d) N-Benzyloxycarbonyl-1,2,3,4-tetrahydrochinolin-3(R,S)carbonsäure(N,N-dimethyl)amid
   Die Titelverbindung wird analog wie in Beispiel 1a) beschrieben ausgehend von 0.6 g N-Benzyloxycarbonyl-1,2,3,4-tetrahydrochinolin3(R,S)-carbonsäure, 0.55 ml 1-ChlorN,N,2-trimethylpropenylamin und 0.3 ml Dimethylamin erhalten und mittels FC (50 g Kieselgel, LaufmittelGradient von A zu Essigester) gereinigt: R_{f}(A)= 0.15; MS: (M)⁺= 338.

### Beispiel 24: 5(S)-Amino-4(S)-hydroxy-2(R),7,7-trimethyl-8-[2(R,S)-butylaminocarbonyl-3,4-dihydro-2H-1,4-benzoxazin-4-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 205mg 2(R,S)-Butylaminocarbonyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-butylaminocarbonyl-3,4-dihydro-2H-1,4-benzoxazin-4-ylcarbonyl]-octansäure(N-butyl)amid (R_{f}(Y)=0.33) als DiastereomerenGemisch: R_{f}(S)=0.27; FAB-MS: (M+H)⁺=533.

Die als Ausgangsmaterial eingesetzte Amin-Komponente wird beispielsweise wie folgt hergestellt:
Das Gemisch aus 600mg 2(R,S)-Ethoxycarbonyl-3,4-dihydro-2H-1,4-benzoxazin (Beispiel 16)) und 1,2 ml Butylamin wird 1h bei 60° C gerührt. Das Reaktionsgemisch wird eingeengt und direkt mittels FC an 50 g Kieselgel mit einem 20:1-Gemisch aus Methylenchlorid und Diethylether als Laufmittel gereinigt. Man erhält das 2(R,S)-Butylaminocarbonyl-3,4-dihydro-2H-1,4-benzoxazin: R_{f}(20:1-Gemisch aus Methylenchlorid und Diethylether)=0.32.

### Beispiel 25: 5(S)-Amino-4(S)-hydroxy-2(R),7,7-trimethyl-8-[3(R,S)-butylaminocarbonyl-1,2,3,4-tetrahydrochinolin-1-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 204mg 3(R,S)-Butylaminocarbonyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-butylaminocarbonyl-1,2,3,4-tetrahydrochinolin-1-ylcarbonyl]-octansäure(N-butyl)amid (R_{f}(Y)=0.18) als DiastereomerenGemisch: R_{f}(S)=0.16; FAB-MS: (M+H)⁺=531.

Die als Ausgangsmaterial eingesetzte Amin-Komponente wird beispielsweise wie folgt hergestellt:
Das Gemisch von 500 mg 3(R,S)-Methoxycarbonyl-1,2,3,4-tetrahydrochinolin (Beispiel 1r) und 1 ml n-Butylamin wird über 1h bei 60° C gerührt. Reinigung des Rohprodukts mittels FC an 50 g Kieselgel (Laufmittel Z) ergibt 3(R,S)-Butylaminocarbonyl-1,2,3,4-tetrahydrochinolin: R_{f}(Z)=0.21.

### Beispiel 26: 5(S)-Amino-4(S)-hydroxy-2(R),7,7-trimethyl-8-[3(R,S)-morpholinocarbonyl-1,2,3,4-tetrahydrochinolin-1-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 183 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 195 mg 1,2,3,4-Tetrahydrochinolin-3(R,S)-carbonsäure-morpholinamid über das (4O,5N-Isopropyliden)-5(S)-tertiärbutoxycarbonylamino-4(S)-hydroxy-2(R),7,7-trimethyl-8-[3(R,S)-morpholinocarbonyl-1,2,3,4-tetrahydrochinolin-1-ylcarbonyl]-octansäure(N-butyl)amid (R_{f}(Essigester)= 0.22; Rₜ(II)= 17.1/16.8 min) und FC-Reinigung an 5 g Kieselgel (Laufmittel N und O) als DiastereomerenGemisch: R_{f}(O)= 0.08; Rₜ(I)= 18.1 min; FAB-MS: (M+H)⁺= 545.

Die als Ausgangsmaterial eingesetzte Amin-Komponente wird beispielsweise folgendermassen hergestellt:
1.2 g N-Benzyloxycarbonyl-1,2,3,4-tetrahydrochinolin3(R,S)-carbonsäure, 1.1 ml 1-Chlor-N,N,2-trimethylpropenylamin und 0.67 ml Morpholin werden analog wie in Beispiel 1a) beschrieben umgesetzt. Reinigung des Rohprodukts mittels FC (50 g Kieselgel, Laufmittel-Gradient von A zu Essigester) ergibt das N-Benzyloxycarbonyl-1,2,3,4-tetrahydrochinolin-3(R,S)-carbonsäuremorpholinamid: R_{f}(A)= 0.24; MS: (M)⁺= 380. 1.52 g N-Benzyloxycarbonyl-1,2,3,4-tetrahydrochinolin3(R,S)-carbonsäuremorpholinamid werden analog Beispiel 1r) umgesetzt. Durch Umkristallisation aus Methylenchlorid/Hexan erhält man das 1,2,3,4-Tetrahydrochinolin3(R,S)-carbonsäuremorpholinamid: Smp. 155 °C; R_{f}(O)= 0.78.

### Beispiel 27: 5(S)-Amino-4(S)-hydroxy-2(R),7,7-trimethyl-8-[2(R,S)-methylaminocarbonyl-3,4-dihydro-2H-1,4-benzthiazin-4ylcarbonyl]-octansäure(N-butyl)amid

92mg 5(S)-Amino-4(S)-hydroxy-2(R),7,7-trimethyl-8-[2(R,S)-methoxycarbonyl-3,4-dihydro-2H-1,4-benzthiazin-4-ylcarbonyl]-octansäure(N-butyl)amid (Beispiel 18)) werden in 3 ml einer 5N-MethylaminLösung in Dimethylformamid gelöst und während 26h bei Raumtemperatur stehen gelassen. Die Titelverbindung erhält man nach Reinigung des eingeengten Rohproduktes mittels FC (LaufmittelT) an 50 g Kieselgel als DiastereomerenGemisch: R_{f}(T)=0.26/0.31; FAB-MS: (M+H)⁺=507.

### Beispiel 28: 5(S)-Amino-4(S)-hydroxy-2(R),7,7-trimethyl-8-[2(R,S)-methylaminocarbonyl-3,4-dihydro-2H-1,4-benzoxazin-4-ylcarbonyl]-octansäure(N-butyl)amid

Die Titelverbindung wird analog Beispiel27) ausgehend von 30mg 5(S)-Amino-4(S)-hydroxy-2(R),7,7-trimethyl-8-[2(R,S)-ethoxycarbonyl-3,4-dihydro-2H-1,4-benzoxazin-4-ylcarbonyl]-octansäure(N-butyl)amid (Beispiel 16)) und 2 ml einer 5N-MethylaminLösung in Dimethylformamid erhalten: R_{f}(S)=0.14; FAB-MS: (M+H)⁺=491.

### Beispiel 29: 5(S)-Amino-4(S)-hydroxy-2(R),7,7-trimethyl-8-[3(R,S)-acetyl-1,2,3,4-tetrahydrochinolin-4-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 123mg 3(R,S)-Acetyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)acetyl-1,2,3,4-tetrahydrochinolin-4-ylcarbonyl]-octansäure(N-butyl)amid (R_{f}(Y)=0.39) und Reinigung durch FC an 30 g Kieselgel (LaufmittelT) als DiastereomerenGemisch: R_{f}(T)=0.39; FAB-MS: (M+H)⁺=474.

Die Ausgangsmaterialien werden beispielsweise folgendermassen hergestellt:
a) 3(R,S)-Acetyl-1,2,3,4-tetrahydrochinolin
   868mg 1-Benzyl-3(R,S)-acetyl-1,2,3,4-tetrahydrochinolin werden in 100ml Tetrahydrofuran in Gegenwart von 100mg PalladiumKohle (10% Pd) bei Normaldruck und Raumtemperatur bis zur Sättigung hydriert. Das Reaktionsgemisch wird filtriert und das eingeengte Filtrat mittels FC an 50 g Kieselgel (LaufmittelB) gereinigt. Man erhält die Titelverbindung: R_{f}(A)=0.29; R_{f}(D)=0.08.
b) 1-Benzyl-3(R,S)-acetyl-1,2,3,4-tetrahydrochinolin
   0.78ml Oxalylchlorid werden in 15ml Dichlormethan gelöst und auf -65° C gekühlt. Bei dieser Temperatur werden zuerst 0.85ml Dimethylsulfoxid, dann eine Lösung von 1.6 g 1-Benzyl-3(R,S)-(1-hydroxyethyl)-1,2,3,4-tetrahydrochinolin und 3.3 ml Triethylamin in 25ml Dichlormethan zugetropft. Nach 3h werden 10ml einer 20% Kaliumhydrogensulfat-Lösung zugegeben. Das Rohprodukt wird mit Dichlormethan extrahiert und mittels FC an 500 g Kieselgel (LaufmittelD) gereinigt: R_{f}(D)=0.27.
c) 1-Benzyl-3(R,S)-(1hydroxyethyl)-1,2,3,4-tetrahydrochinolin
   Zu 0.435 g Magnesiumpulver, suspendiert in 50ml Diethylether, werden 1.12ml Methyljodid innerhalb von 10 min zugetropft. Nach 0.5 h Nachrühren werden 1.5 g 1-Benzyl-3(R,S)-formyl-1,2,3,4-tetrahydrochinolin, gelöst in 10ml Diethylether, zugetropft. Es wird 0.5 h nachgerührt und anschliessend auf Eiswasser gegossen. Das mit Dichlormethan extrahierte Rohprodukt wird mittels FC an 150 g Kieselgel (LaufmittelB) gereinigt: R_{f}(B)=0.31; R_{f}(D)=0.15.
d) 1-Benzyl-3(R,S)-formyl-1,2,3,4-tetrahydrochinolin
   5.4 g 1-Benzyl-3(R,S)-hydroxymethyl-1,2,3,4-tetrahydrochinolin werden ausgehend von 2.8 ml Oxalylchlorid, 3.0 ml Dimethylsulfoxid und 12ml Triethylamin analog Beispiel29b) umgesetzt. Die Reinigung mittels FC erfolgt an 500 g Kieselgel (LaufmittelD): R_{f}(D)=0.26; R_{f}(B)=0.41.
e) 1-Benzyl-3(R,S)-hydroxymethyl-1,2,3,4-tetrahydrochinolin
   6.24 g 1-Benzyl-3(R,S)-benzyloxycarbonyl-1,2,3,4-tetrahydrochinolin werden in 60ml Tetrahydrofuran gelöst, mit 920mg Lithiumborhydrid versetzt und 4h bei Raumtemperatur gerührt. Anschliessend werden unter Eiskühlung 120ml Methanol zugetropft, das Reaktionsgemisch zur Trockene eingedampft und das Produkt mittels FC an 250 g Kieselgel (LaufmittelZ) gereinigt: R_{f}(Z)=0.36.
f) 1-Benzyl-3(R,S)-benzyloxycarbonyl-1,2,3,4-tetrahydrochinolin:
   5.6 g 1,2,3,4-Tetrahydrochinolin3(R,S)-carbonsäure (Beispiel 1t)) werden in 100ml Dimethylformamid unter Zusatz von 12.7 ml Benzylbromid und 25.1 g Cäsiumcarbonat während 24h bei Raumtemperatur gerührt. Das eingeengte Rohprodukt wird anschliessend mittels FC an 250 g Kieselgel mit einem 1:1-Gemisch aus Methylenchlorid und Hexan als Laufmittel gereinigt. Man erhält die Titelverbindung: R_{f}(1:1-Gemisch aus Methylenchlorid und Hexan)=0.31.

### Beispiel 30: 5(S)-Amino-4(S)-hydroxy-2(R),7,7-trimethyl-8-[2(R,S)-glycinylcarbonyl-3,4-dihydro-2H-1,4-benzoxazin-4-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 384mg 2(R,S)-(Tertiärbutoxycarbonylmethylaminocarbonyl)3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-(tertiärbutoxycarbonylmethylaminocarbonyl-3,4-dihydro-2H-1,4-benzoxazin-4-ylcarbonyl]-octansäure(N-butyl)amid (R_{f}(Y)=0.45) und Reinigung durch FC an 30 g Kieselgel (LaufmittelU) als Gemisch von Diastereomeren: R_{f}(U)=0.26; R_{f}(V)=0.52; FAB-MS: (M+H)⁺=535.

Die als Ausgangsmaterial eingesetzte Amin-Komponente wird beispielsweise folgendermassen hergestellt:
1.0 g 2(R,S)-Ethoxycarbonyl-3,4-dihydro-2H-1,4-benzoxazin und 1.2 g Glycintertiärbutylester werden in 10ml Toluol auf 80° C erhitzt. Nach 5h wird eingedampft und mittels FC an 100 g Kieselgel (LaufmittelZ) das reine 2(R,S)-(Tertiärbutoxycarbonylmethylaminocarbonyl)3,4-dihydro-2H-1,4-benzoxazin isoliert: R_{f}(Z)=0.38.

### Beispiel 31: 5(S)-Amino-4(S)-hydroxy-2(R),7,7-trimethyl-8-[3(R,S)-ethoxycarbonyl-1,2,3,4-tetrahydrochinolin-1-ylcarbonyl]-octansäure(N-butyl)amid

In analoger Weise wie in den Beispielen 20 und 20a) für die entsprechenden Reaktionsstufen beschrieben erhält man die Titelverbindung ausgehend von 58 mg (4O,5N-Isopropyliden)-5(S)-tertiärbutoxycarbonylamino-4(S)-hydroxy-2(R),7,7-trimethyl-8-[3(R,S)-ethoxycarbonyl-1,2,3,4-tetrahydrochinolin-1-ylcarbonyl]-octansäure(N-butyl)amid über das 5(S)-Tertiärbutoxycarbonylamino-4(S)-hydroxy-2(R),7,7-trimethyl-8-[3(R,S)-ethoxycarbonyl-1,2,3,4-tetrahydrochinolin-1-ylcarbonyl]-octansäure(N-butyl)amid (R_{f}(A)= 0.10) und Reinigung mittels FC (5 g Kieselgel, Laufmittel N): R_{f}(O)= 0.20; Rₜ(I)= 20.8 min; FAB-MS: (M+H)⁺= 504.

Die Ausgangsmaterialien werden beispielsweise folgendermassen hergestellt:
a) (4O,5N-Isopropyliden)-5(S)-tertiärbutoxycarbonylamino-4(S)-hydroxy-2(R),7,7-trimethyl-8-[3(R,S)-ethoxycarbonyl-1,2,3,4-tetrahydrochinolin-1-ylcarbonyl]-octansäure(N-butyl)amid
   Die Titelverbindung wird ausgehend von 69 mg 3-[N-Tertiärbutoxycarbonyl-4(S)(3-carboxy-2,2-dimethylpropyloxy-2,2-dimethyl-1,3-oxazolidin-5(S)-yl]-2(R)-methyl-propionsäure(N-butyl)amid, 0.01 ml Oxalylchlorid, 45 mg 3(R,S)-Ethoxycarbonyl-1,2,3,4-tetrahydrochinolin, 0.08 ml Diisopropylethylamin und 3 mg 4-DMAP analog wie in Beispiel 1a) beschrieben erhalten und durch FC (20 g Kieselgel, Laufmittel B) gereinigt. Die Titelverbindung wird als Diastereomerengemisch erhalten: R_{f}(A)= 0.30.
b) 3(R,S)-Ethoxycarbonyl-1,2,3,4-tetrahydrochinolin
   Die Titelverbindung wird analog Beispiel 1r) ausgehend von 1.55 g N-Benzyloxycarbonyl-3(R,S)-ethoxycarbonyl-1,2,3,4-tetrahydrochinolin hergestellt. Man erhält die Titelverbindung: R_{f}(A)= 0.62; Rₜ(II)= 4.7 min.
c) N-Benzyloxycarbonyl-3(R,S)-ethoxycarbonyl-1,2,3,4-tetrahydrochinolin
   Die Titelverbindung wird analog Beispiel 1s) ausgehend von 1.5 g N-Benzyloxycarbonyl-1,2,3,4-tetrahydrochinolin-3(R,S)-carbonsäure und 3.7 ml N,N-Dimethylformamiddiethylacetal hergestellt und mittels FC (100 g Kieselgel, Laufmittel D und B) gereinigt. Man erhält die Titelverbindung: R_{f}(A)= 0.75; MS: (M)⁺= 339.

### Beispiel 32: 5(S)-Amino-4(S)-hydroxy-2(R),7,7-trimethyl-8-[3(R,S)-isopropoxycarbonyl-1,2,3,4-tetrahydrochinolin-1-ylcarbonyl]-octansäure(N-butyl)amid

In analoger Weise wie in den Beispielen 20 und 20a) für die entsprechenden Reaktionsstufen beschrieben erhält man die Titelverbindung ausgehend von 41 mg (4O,5N-Isopropyliden)-5(S)-tertiärbutoxycarbonylamino-4(S)-hydroxy-2(R),7,7-trimethyl-8-[3(R,S)-isopropoxycarbonyl-1,2,3,4-tetrahydrochinolin-1-ylcarbonyl]-octansäure(N-butyl)amid über das 5(S)-Tertiärbutoxycarbonylamino-4(S)-hydroxy-2(R),7,7-trimethyl-8-[3(R,S)-isopropoxycarbonyl-1,2,3,4-tetrahydrochinolin-1-ylcarbonyl]-octansäure(N-butyl)amid (R_{f}(A)= 0.15; Rₜ(II)= 17.7 min): R_{f}(O)= 0.28; Rₜ(I)= 21.7 min; FAB-MS: (M+H)⁺= 518.

Die Ausgangsmaterialien werden beispielsweise folgendermassen hergestellt:
a) (4O,5N-Isopropyliden)-5(S)-tertiärbutoxycarbonylamino-4(S)-hydroxy-2(R),7,7-trimethyl-8-[3(R,S)-isopropoxycarbonyl-1,2,3,4-tetrahydrochinolin-1-ylcarbonyl]-octansäure(N-butyl)amid
   Die Titelverbindung wird ausgehend von 69 mg 3-[N-Tertiärbutoxycarbonyl-4(S)-(3-carboxy-2,2-dimethylpropyloxy-2,2-dimethy-1,3-oxazolidin-5(S)-yl]-2(R)-methyl-propionsäure(N-butyl)amid und 88mg 3(R,S)-Isopropoxycarbonyl-1,2,3,4-tetrahydrochinolin analog Beispiel 31a) als Diastereomerengemisch erhalten und durch FC (20 g Kieselgel, Laufmittel B) gereinigt: R_{f}(A)= 0.34; Rₜ(II)= 32.7/32.8 min.
b) 3(R,S)-Isopropoxycarbonyl-1,2,3,4-tetrahydrochinolin
   Die Titelverbindung wird analog Beispiel 1r) ausgehend von 2.19 g N-Benzyloxycarbonyl-3(R,S)-isopropoxycarbonyl-1,2,3,4-tetrahydrochinolin hergestellt: R_{f}(D)= 0.37; MS: (M)⁺= 219.
c) N-Benzyloxycarbonyl-3(R,S)-isopropoxycarbonyl-1,2,3,4-tetrahydrochinolin
   Die Titelverbindung wird analog Beispiel 1s) ausgehend von 1.87 g N-Benzyloxycarbonyl-1,2,3,4-tetrahydrochinolin3(R,S)-carbonsäure und 5.1 ml N,N-Dimethylformamiddiisopropylacetal hergestellt: R_{f}(A)= 0.78; MS: (M)⁺= 353.

### Beispiel 33: 5(S)-Amino-4(S)-hydroxy-2(R),7,7-trimethyl-8-(6-brom-1,2,3,4-tetrahydrochinolin-1ylcarbonyl)-octansäure(N-butyl)amid

In analoger Weise wie in den Beispielen 20 und 20a) für die entsprechenden Reaktionsstufen beschrieben erhält man die Titelverbindung ausgehend von 74 mg (4O,5N-Isopropyliden)-5(S)-tertiärbutoxycarbonylamino-4(S)-hydroxy-2(R),7,7-trimethyl-8-(6-brom-1,2,3,4-tetrahydrochinolin-1-ylcarbonyl)-octansäure(N-butyl)amid über das 5(S)-Tertiärbutoxycarbonylamino-4(S)-hydroxy-2(R),7,7-trimethyl-8-(6-brom-1,2,3,4-tetrahydrochinolin-1ylcarbonyl]-octansäure(N-butyl)amid (R_{f}(A)= 0.29): R_{f}(O)= 0.20; Rₜ(I)= 21.8 min; FAB-MS: (M+H)⁺= 510.

Die Ausgangsmaterialien werden beispielsweise folgendermassen hergestellt:
a) (4O,5N-Isopropyliden)-5(S)-tertiärbutoxycarbonylamino-4(S)-hydroxy-2(R),7,7-trimethyl-8-(6-brom-1,2,3,4-tetrahydrochinolin-1-ylcarbonyl)-octansäure(N-butyl)amid
   Die Titelverbindung wird ausgehend von 69 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 85mg 6-Brom-1,2,3,4-tetrahydrochinolin wie in Beispiel 31a) beschrieben erhalten und durch FC (20 g Kieselgel, Laufmittel B) gereinigt: R_{f}(A)= 0.50.
b) 6-Brom-1,2,3,4-tetrahydrochinolin wird ausgehend von 6.7 g 1,2,3,4-Tetrahydrochinolin und 2.7 ml Brom analog dem von Z. Hoffman und W. Königs in Chemische Berichte 16, 727 (1883) beschriebenen Verfahren hergestellt.

### Beispiel 34: 5(S)-Amino-4(S)-hydroxy-2(R),7,7-trimethyl-8-[6-brom-3(R,S)-ethoxycarbonyl-1,2,3,4-tetrahydrochinolin-1-ylcarbonyl]-octansäure(N-butyl)amid

In analoger Weise wie in den Beispielen 20 und 20a) für die entsprechenden Reaktionsstufen beschrieben erhält man die Titelverbindung ausgehend von 320 mg (4O,5N-Isopropyliden)-5(S)-tertiärbutoxycarbonylamino-4(S)-hydroxy-2(R),7,7-trimethyl-8-[6-brom-3(R,S)-ethoxycarbonyl-1,2,3,4-tetrahydrochinolin-1-ylcarbonyl]-octansäure(N-butyl)amid (R_{f}(A)= 0.31; Rₜ(I)= 29.6 min), das analog dem Verfahren in Beispiel 1a) ausgehend von 228 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 240 mg 6-Brom-3(R,S)-ethoxycarbonyl-1,2,3,4-tetrahydrochinolin erhalten wird, über das 5(S)-Tertiärbutoxycarbonylamino-4(S)-hydroxy-2(R),7,7-trimethyl-8-[6-brom-3(R,S)-ethoxycarbonyl-1,2,3,4-tetrahydrochinolin-1-ylcarbonyl]-octansäure(N-butyl)amid (R_{f}(X)= 0.50; Rₜ(II)= 26.9/27.1 min) als Gemisch von Diastereomeren: R_{f}(O)= 0.21; Rₜ(I)= 22.7 min; FAB-MS: (M+H)⁺=582.

Die als Ausgangsmaterial eingesetzte Amin-Komponente wird beispielsweise folgendermassen hergestellt:
Analog Beispiel 33b) erhält man die Titelverbindung ausgehend von 410 mg 3(R,S)-Ethoxycarbonyl-1,2,3,4-tetrahydrochinolin und 0.11 ml Brom das 6-Brom-3(R,S)-ethoxycarbonyl-1,2,3,4-tetrahydrochinolin: R_{f}(D)= 0.15; MS: (M)⁺= 285.

### Beispiel 35: 5(S)-Amino-4(S)-hydroxy-7,7-dimethyl-8-[2(R,S)-methoxycarbonyl-3,4-dihydro-2H-1,4-benzthiazin-4-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 205 mg 3-[N-Tertiärbutoxycarbonyl-4(S)-(3-carboxy-2,2-dimethylpropyloxy-2,2-dimethyl-1,3-oxazolidin-5(S)-yl]-propionsäure(N-butyl)amid und 581 mg 2(R,S)-Methoxycarbonyl-3,4-dihydro-2H-1,4-benzthiazin (Beispiel 18) über das (4O,5N-Isopropyliden)-5(S)-tertiärbutoxycarbonylamino-4(S)-hydroxy-7,7-dimethyl-8-[2(R,S)-methoxycarbonyl-3,4-dihydro-2H-1,4-benzthiazin-4-yl- carbonyl]-octansäure(N-butyl)amid (Reinigung an 80 g Kieselgel mit einem 20:1-Gemisch aus Methylenchlorid und Diethylether zu einem 1:1-Gemisch als LaufmittelGradient; R_{f}(1:1-Gemisch aus Methylenchlorid und Diethylether) = 0.43) und Reinigung durch FC an 30 g Kieselgel (Laufmittel S): R_{f}(S) = 0.32; FAB-MS: (M+H)⁺ = 494.

Die Ausgangsmaterialien werden beispielsweise folgendermassen hergestellt:
a) 3-[N-Tertiärbutoxycarbonyl-4(S)-(3-carboxy-2,2-dimethylpropyl)-2,2-dimethvl-1,3-oxazolidin-5(S)-yl]-propionsäure(N-butyl)amid
   Die Titelverbindung wird durch Oxidation analog Beispiel 1b) ausgehend von 268 mg 3-[N-Tertiärbutoxycarbonyl-4(S)-(4-hydroxy-2,2-dimethylbutyl)-2,2-dimethyl-1,3-oxazolidin-5(S)-yl]-propionsäure(N-butyl)amid, 802 mg Natriumperjodat, 17 mg Ruthenium(III)-chloridHydrat in 5 ml Acetonitril, 5 ml Tetrachlorkohlenstoff und 10 ml Wasser erhalten. Gereinigt wird an 50 g Kieselgel (Laufmittel U): R_{f} (U) = 0.33.
b) 3-[N-Tertiärbutoxycarbonyl-4(S)-(4-hydroxy-2,2-dimethylbutyl)-2,2-dimethyl-1,3-oxazolidin-5(S)-yl]-propionsäure(N-butyl)amid
   Die Titelverbindung wird analog Beispiel 1c) ausgehend von 490 mg 3-[N-Tertiärbutoxycarbonyl-4(S)-(2,2-dimethyl-4-triisopropylsilyloxybutyl)-2,2-dimethyl-1,3-oxazolidin-5(S)-yl]-propionsäure(N-butyl)amid und 396 mg TetrabutylammoniumfluoridTrihydrat erhalten. Gereinigt wird an 80 g Kieselgel (Essigester als Laufmittel): R_{f} (Essigester) = 0.40.
c) 3-[N-Tertiärbutoxycarbonyl-4(S)-(2,2-dimethyl-4-triisopropylsilyloxybutyl)-2,2-dimethyl-1,3-oxazolidin-5(S)-yl]-propionsäure(N-butyl)amid
   Die Titelverbindung wird erhalten, indem man 500 mg 3-[N-Tertiärbutoxycarbonyl-2,2-dimethyl-4(S)-(2,2-dimethyl-4-triisopropylsilyloxybutyl)-1,3-oxazolidin-5(S)-yl]-propionsäure in 15 ml Tetrahydrofuran bei -40° C mit 0.2 ml N-Methylmorpholin und 0.26 ml Chlorameisensäureisobutylester umsetzt und das Reaktionsgemisch nach 1 h bei 0° C mit 0.75 ml n-Butylamin versetzt. Nach 1 h bei Raumtemperatur wird dieses Gemisch auf 1N-Salzsäure gegossen und das Amid mit Methylenchlorid extrahiert. Gereinigt wird an 50 g Kieselgel (Laufmittel B). Man erhält die Titelverbindung: R_{f}(B) = 0.28; R_{f}(T) = 0.60.
d) 3-[N-Tertiärbutoxycarbonyl-2,2-dimethyl-4(S)-(2,2-dimethyl-4-triisopropylsilyloxybutyl)-1,3-oxazolidin-5(S)-yl]-propionsäure
   1.48 g 4-[N-Tertiärbutoxycarbonyl-2,2-dimethyl-4(S)-(2,2-dimethyl-4-triisopropylsilyloxybutyl)-1,3-oxazolidin-5(S)-yl]-buten-(1) werden in einer Mischung aus 21 ml Acetonitril, 21 ml Tetrachlorkohlenstoff und 39 ml Wasser mit 78 mg Ruthenium(III)-chloridHydrat und 3.71 g Natriumperjodat analog Beispiel 1b) oxidiert. Das Rohprodukt wird an 250 g Kieselgel gereinigt (Laufmittel: zuerst T, dann U). Man erhält die Titelverbindung als bräunliches Öl: R_{f}(T) = 0.25.
e) 4-[N-Tertiärbutoxycarbonyl-2,2-dimethyl-4(S)-(2,2-dimethyl-4-triisopropylsilyloxybutyl)-1,3-oxazolidin-5(S)-yl]-buten-(1)
   1.80 g 6(S)-Tertiärbutoxycarbonylamino8,8-dimethyl5(S)-hydroxy-10-triisopropylsilyloxydec1-en werden in je 10 ml Methylenchlorid und Dimethoxypropan mit 50 mg p-Toluolsulfonsäure-Hydrat während 2 h bei Raumtemperatur gerührt. Nach Einengen wird an 250 g Kieselgel mit einem 1:20-Gemisch aus Essigester und Hexan als Laufmitel gereinigt: R_{f} (1:20-Gemisch aus Essigester und Hexan) = 0.40.
f) 6(S)-Tertiärbutoxycarbonylamino8,8-dimethyl5(S)-hydroxy-10-triisopropylsilyloxydec1-en
   Aus 3.70 ml 4-Brom-1-buten und 880 mg Magnesiumpulver wird in 50 ml Tetrahydrofuran bei 55° C das GrignardReagens vorbereitet. Diese Lösung wird anschliessend bei 5 - 10° C zu einer Lösung von 3.0 g 2(S)-Tertiärbutoxycarbonylamino-6-triisopropylsilyloxy-4,4-dimethyl-hexanal (Beispiel 1 g)) in 25 ml Tetrahydrofuran getropft. 1 h nach Ende der Zugabe wird auf eine 0.1N-Salzsäure-Lösung gegossen, mit Methylenchlorid extrahiert und eingeengt. Das Rohprodukt enthält die beiden 5(S): 5(R)-Diastereomeren im Verhältnis ca. 5 : 1. Man erhält die Titelverbindung (apolares Diastereomer) durch Reinigung an 250 g Kieselgel (Laufmittel E): R_{f}(E) = 0.30.

### Beispiel 36: 5(S)-Amino-4(S)-hydroxy-2(R),7,7-trimethyl-8-[3,3-bis-(methoxycarbonyl)-1,2,3,4-tetrahydrochinolin-1-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 80 mg 3-[N-Tertiärbutoxycarbonyl-4(S)-(3-carboxy-2,2-dimethylpropyloxy-2,2-dimethyl-1,3-oxazolidin-5(S)-yl]-2(R)- methyl-propionsäure(N-butyl)amid und 380 mg 3,3-Bis-(methoxycarbonyl)-1,2,3,4-tetrahydrochinolin über das (4O,5N-Isopropyliden)-5(S)-tertiärbutoxycarbonylamino-4(S)-hydroxy-2(R),7,7-trimethyl-8-[3,3-bis-(methoxycarbonyl)-1,2,3,4-tetrahydrochinolin-1-yl-carbonyl]-octansäure(N-butyl)amid (R_{f}(A)= 0.22) : R_{f} (S) = 0.18; FAB-MS: (M+H)⁺ = 548.

Die als Ausgangsmaterial eingesetzte Amin-Komponente wird beispielsweise folgendermassen hergestellt:
1.00 g N-Benzyloxycarbonyl-3(R,S)-methoxycarbonyl-1,2,3,4-tetrahydrochinolin (Beispiel 1s)) werden in 10 ml Tetrahydrofuran mit überschüssigem Lithiumdiisopropylamid (1.5 equiv.) unter üblichen Reaktionsbedingungen deprotoniert und anschliessend bei -70° C mit 0.80 ml Chlorameisensäuremethylester versetzt. Nach 30 min wird auf 0.1N-Salzsäure gegossen und mit Methylenchlorid extrahiert. Das nach üblicher weiterer Aufarbeitung erhaltene Rohprodukt wird in 30 ml Tetrahydrofuran gelöst und in Anwesenheit von 1.00 g Palladium auf Kohle (10% Pd) bis zum Ende der Umsetzung hydriert. Nach Reinigung des Produktes an 100 g Kieselgel (Laufmittel B) erhält man das 3,3-Bis-(methoxycarbonyl)-1,2,3,4-tetrahydrochinolin: R_{f} (B) = 0.50.

### Beispiel 37: 5(S)-Amino-4(S)-hydroxy-2(R,S)-isopropyl-7,7-dimethyl-8-[2(R,S)-methoxycarbonyl-3,4-dihydro-2H-1,4-benzthiazin-4-ylcarbonyl]-octansäure(N-butyl)amid

In analoger Weise wie in den Beispielen 2 und 2a) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R,S)-isopropyl-propionsäure(N-butyl)amid und 215 mg 2(R,S)-Methoxycarbonyl-3,4-dihydro-2H-1,4-benzthiazin über das (4O,5N-Isopropyliden)-5(S)-tertiärbutoxycarbonylamino-4(S)-hydroxy-2(R,S)-isopropyl-7,7-dimethyl-8-[2(R,S)-methoxycarbonyl-3,4-dihydro-2H-1,4-benzthiazin-4-ylcarbonyl]-octansäure(N-butyl)amid (Reinigung an 30 g Kieselgel (Laufmittel Z); R_{f}(B) = 0.33) und Reinigung durch FC an 30 g Kieselgel (Laufmittel S) als Diastereromerengemisch: R_{f}(S) = 0.35; FAB-MS: (M+H)⁺ = 536.

Die Ausgangsmaterialien werden beispielsweise folgendermassen hergestellt:
a) 3-[N-Tertiärbutoxycarbonyl-4(S)-(3-carboxy-2,2-dimethylpropyloxy-2,2-dimethyl-1,3-oxazolidin-5(S)-yl]-2(R,S)isopropyl-propionsäure(N-butyl)amid
   970 mg 3-[N-Tertiärbutoxycarbonyl-4(S)-(4-hydroxy-2,2-dimethylbutyloxy-2,2-dimethyl-1,3-oxazolidin-5(S)-yl]-2(R,S)isopropyl-propionsäure(N-butyl)amid werden mit 2.6 g Natriumperjodat und 55 mg Ruthenium(III)chloridHydrat analog Beispiel 1b) oxidiert. Reinigung an 165 g Kieselgel (Laufmittel S) ergibt die reine Titelverbindung als Diastereomerengemisch: R_{f}(S) = 0.44.
b) 3-[N-Tertiärbutoxycarbonyl-4(S)-(4-hydroxy-2,2-dimethylbutyloxy-2,2-dimethyl-1,3-oxazolidin-5(S)-yl]-2(R,S)isopropyl-propionsäure(N-butyl)amid
   1.43 g 3-[N-Tertiärbutoxycarbonyl-4(S)-(4-triisopropylsilyloxy-2,2-dimethylbutyloxy-2,2-dimethyl-1,3-oxazolidin-5(S)-yl]-2(R,S)isopropyl-propionsäure(N-butyl)amid werden analog Beispiel 1c) mit 1.1 g TetrabutylammoniumfluoridTrihydrat in 25 ml Tetrahydrofuran behandelt. Reinigung durch FC an 150 g Kieselgel (Laufmittel A) ergibt die Titelverbindung als Diastereomerengemisch: R_{f}(A) = 0.40.
c) 3-[N-Tertiärbutoxycarbonyl-4(S)-(4-triisopropylsilyloxy-2,2-dimethylbutyloxy-2,2-dimethyl-1,3-oxazolidin-5(S)-yl]-2(R,S)isopropyl-propionsäure(N-butyl)amid
   0.98 g 3[N-Tertiärbutoxycarbonyl-2,2-dimethyl-4(S)-(4-triisopropylsilyloxy-2,2-dimethylbutyl)-1,3-oxazolidin-5(S)-yl]-2(R,S)-isopropyl-propionsäure werden mit 0.4 ml N-Methylmorpholin, 0.5 ml Chlorameisensäureisobutylester und 1.5 ml n-Butylamin analog Beispiel 35c) umgesetzt. Reinigung durch FC (Laufmittel D) an 250 g Kieselgel ergibt das Gemisch aus zwei Diastereomeren: R_{f}(D) = 0.40/0.31.
d) 3-[N-Tertiärbutoxycarbonyl-2,2-dimethyl-4(S)-(4-triisopropylsilyloxy-2,2-dimethylbutyl)-1,3-oxazolidin-5(S)-yl[-2(R,S)-isopropyl-propionsäure
   3.6 g 4-[N-Tertiärbutoxycarbonyl-2,2-dimethyl-4(S)-(4-triisopropylsilyloxy-2,2-dimethylbutyl)-1,3-oxazolidin-5(S)-yl]-3(R,S)-isopropylbuten(1) werden mit 8.3 g Natriumperjodat und 176 mg Ruthenium(III)-chloridHydrat analog Beispiel 35d) oxidiert. Reinigung durch FC (Laufmittel T) an 500 g Kieselgel ergibt die Titelverbindung: R_{f}(T) = 0.36.
e) 4-[N-Tertiärbutoxycarbonyl-2,2-dimethyl-4(S)-(4-triisopropylsilyloxy-2,2-dimethylbutyl)-1,3-oxazolidin-5(S)-yl]-3(R,S)-isopropylbuten(1)
   4.4 g 6(S)-Tertiärbutoxycarbonylamino8,8-dimethyl5(S)-hydroxy3(R,S)-isopropyl-10-triisopropylsilyloxydecen(1) und 134 mg p-Toluolsulfonsäure-Hydrat werden in 25 ml Methylenchlorid und Dimethoxypropan während 2 h bei Raumtemperatur gerührt. Nach Einengen des Reaktionsansatzes wird an 500 g Kieselgel mittels FC (Laufmittel D) gereinigt: R_{f}(D) = 0.61.
f) 6(S)-Tertiärbutoxycarbonylamino5(S)-hydroxy3(R,S)-isopropyl-10-triisopropylsilyloxy-8,8-dimethyldecen(1)
   Die Titelverbindung wird ausgehend von 23.9 g 1-Brom-2(R,S)-isopropyl-3-buten (Herstellung nach D. Mesnard et al., C.R. Acad. Sci. Paris, t. 277, Serie C-567), 3.3 g Magnesiumpulver und 11.25 g 2(S)-Tertiärbutoxycarbonylamino-6-triisopropylsilyloxy-4,4-dimethyl-hexanal (Beispiel 1 g) analog wie in Beispiel 35f) beschriebenhergestellt. Die Reinigung des Rohprodukts erfolgt durch FC an 900 g Kieselgel (Laufmittel E) und ergibt die Titelverbindung als Diastereomerengemisch: R_{f}(E) = 0.31.

### Beispiel 38: 5(S)-Amino-4(S)-hydroxy-2(R,S)-isopropyl-7,7-dimethyl-8-[(R,S)-methoxycarbonyl-3,4-dihydro-2H-1,4-benzoxazin-4ylcarbonyl]-octansäure(N-butyl)amid

In analoger Weise wie in den Beispielen 2 und 2a) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R,S)-isopropyl-propionsäure(N-butyl)amid (Beispiel 37a) und 213 mg 2(R,S)-Methoxycarbonyl-3,4-dihydro-2H-1,4-benzoxazin (Arch. Pharmacol. 315, 538 (1982)) über das (4O,5N-Isopropyliden)-5(S)-tertiärbutoxycarbonylamino-4(S)-hydroxy-2(R,S)-isopropyl-7,7-dimethyl-8-[2(R,S)-methoxycarbonyl-3,4-dihydro-2H-1,4-benzoxazin-4-ylcarbonyl]-octansäure(N-butyl)amid (R_{f}(Z) = 0.30) als Gemisch von Diastereomeren: R_{f}(S) = 0.34; FAB-MS: (M+H)⁺ = 520.

### Beispiel 39: 5(S)-Tertiärbutoxycarbonylamino-4(S)-hydroxy-2(S)-isopropyl-7,7dimethyl-8-[2(R,S)-methylaminocarbonyl-3,4-dihydro-2H-1,4-benzthiazin-4-ylcarbonyl]-octansäure(N-butyl)amid

Analog wie in Beispiel 20a) beschrieben, ausgehend von 535 mg (4O,5N-Isopropyliden)-5(S)-tertiärbutoxycarbonylamino-4(S)-hydroxy-2(S)-isopropyl-7,7-dimethyl-8-[2(R,S)-methylaminocarbonyl-3,4-dihydro-2H-1,4-benzthiazin-4-ylcarbonyl]-octansäure(N-butyl)amid (R_{f}(D)=0.44), das aus 500 mg 3-[N-Tertiärbutoxycarbonyl-4(S)-(3-carboxy-2,2-dimethylpropyloxy-2,2-dimethyl-1,3-oxazolidin-5(S)-yl]-2(S)isopropyl-propionsäure(N-butyl)amid und 859 mg 2(R,S)-Methylaminocarbonyl-3,4-dihydro-2H-1,4-benzthiazin in analoger Weise wie in Beispiel 1a) beschrieben hergestellt und durch FC an 150 g Kieselgel mit einem Laufmittelgradienten von D nach Essigester gereinigt wird, und Reinigung mittels FC an 30 g Kieselgel (Laufmittel D) als Diastereomerengemisch: R_{f}(D)=0.22/0.33; R_{f}(Essigester)=0.39/0.50; FAB-MS: (M+H)⁺=635.

Das als Ausgangsmaterial eingesetze 2(R,S)-Methylaminocarbonyl-3,4-dihydro-2H-1,4-benzthiazin wird beispielsweise folgendermassen hergestellt:
880 mg 2(R,S)-Methoxycarbonyl-3,4-dihydro-2H-1,4-benzthiazin (Beispiel 18) werden in 8 ml einer 3N-MethylaminLösung in Dimethylformamid gelöst und über 20 h bei Raumtemperatur stehengelassen. Anschliessend wird das eingeengte Reaktionsgemisch mittels FC an 80 g Kieselgel (Laufmittel D) zur Titelverbindung gereinigt: R_{f}(D)=0.38.

Das als Ausgangsmaterial eingesetzte diastereomerenreine 3-[N-Tertiärbutoxycarbonyl-4(S)-(3-carboxy-2,2-dimethylpropyloxy-2,2-dimethyl-1,3-oxazolidin-5(S)-yl]-2(S)isopropyl-propionsäure(N-butyl)amid wird beispielsweise wie folgt erhalten:
a) Oxidation von 7.0 g 3-[N-Tertiärbutoxycarbonyl-4(S)-(4-hydroxy-2,2-dimethylbutyloxy-2,2-dimethyl-1,3-oxazolidin-5(S)-yl]-2(S)isopropyl-propionsäure(N-butyl)amid mit 12.8 g Natriumperjodat und 140 mg Ruthenium(III)chloridHydrat analog Beispiel 1b) und Reinigung an 300 g Kieselgel (Laufmittel S) ergibt die Titelverbindung: R_{f}(S)=0.44; R_{f}(O)=0.62.
b) 3-[N-Tertiärbutoxycarbonyl-4(S)-(4-hydroxy-2,2-dimethylbutyloxy-2,2-dimethyl-1,3-oxazolidin-5(S)-yl]-2(S)isopropyl-propionsäure(N-butyl)amid
   Die Titelverbindung wird durch Hydrierung von 10.0 g 3-[N-Tertiärbutoxycarbonyl-4(S)(4-benzyloxy-2,2-dimethylbutyloxy-2,2-dimethyl-1,3-oxazolidin-5(S)-yl]-2(S)isopropyl-propionsäure(N-butyl)amid in Gegenwart von 2.0 g Palladium auf Kohle (5 % Pd, Degussa) in 200 ml Tetrahydrofuran bei Raumtemperatur und Normaldruck für 3 h hergestellt. Das Reaktionsgemisch wird über Hyflo® filtriert und eingeengt. Die Reinigung erfolgt durch FC an 300 g Kieselgel (Laufmittel A und Essigester). Man erhält die reine Titelverbindung als weisse Kristalle: Smp. 132-3 °C; R_{f}(A)=0.22; [α]^{D} = - 46.5 (c=1.0 in Chloroform); FAB-MS: (M+H)⁺= 471; Anal. kalk. für C₂₆H₅₀N₂O₅: C 66.35 %, H 10.71 %, N 5.95 %; gef. C 66.23 %, H 10.86 %, N 5.93 %.
c) 3-[N-Tertiärbutoxycarbonyl-4(S)-(4-benzyloxy-2,2-dimethylbutyloxy-2,2-dimethyl-1,3-oxazolidin-5(S)-yl]-2(S)isopropyl-propionsäure(N-butyl)amid
   27.5 g 3-[N-Butoxycarbonyl-4(S)(4-benzyloxy-2,2-dimethylbutyloxy-2,2-dimethyl-1,3-oxazolidin-5(S)-yl]-2-(R,S)isopropyl-propionsäure werden mit 11.4 ml 1-ChlorN,N,2-trimethylpropenylamin und 10.7 ml n-Butylamin analog Beispiel 1a) umgesetzt. Das Diastereomerengemisch wird mittels FC an 800 g Kieselgel mit einem LaufmittelGradienten (von einem 10:1-Gemisch zu einem 3:1-Gemisch) aus Toluol und Essigester aufgetrennt. Man erhält die reine Titelverbindung (Diastereomer I): R_{f} (3:1-Gemisch aus Toluol und Essigester)=0.36; FAB-MS: (M+H)⁺= 461; sowie Diastereomer II: R_{f} (3:1-Gemisch aus Toluol und Essigester)=0.28; FAB-MS: (M+H)⁺= 461.
d) 3-[N-Tertiärbutoxycarbonyl-4(S)(4-benzyloxy-2,2-dimethylbutyloxy-2,2-dimethyl-1,3-oxazolidin-5(S)-yl]-2-(R,S)isopropyl-propionsäure
   30 g 4-[3-Tertiärbutoxycarbonyl-4(S)(4-benzyloxy-2,2-dimethylbutyloxy-2,2-dimethyl-1,3-oxazolidin-5(S)-yl]3(R,S)isopropylbuten(1) werden mit 52.8 g Natriumperjodat und 600 mg Ruthenium(III)-chloridHydrat analog Beispiel 37d) oxidiert. Reinigung de Titelverbindung erfolgt durch FC (Laufmittel B und D) an 900 g Kieselgel: R_{f}(A)=0.22.
e) 4-[N-Tertiärbutoxycarbonyl-4(S)(4-benzyloxy-2,2-dimethylbutyloxy-2,2-dimethyl-1,3-oxazolidin-5(S)-yl]3(R,S)isopropylbuten(1)
   44.8 g 10-Benzyloxy6(S)-tertiärbutoxycarbonylamino5(S)-hydroxy3(R,S)-isopropyl8,8-dimethyldecen(1) und 1.2 g p-Toluolsulfonsäure-Hydrat werden in 150 ml Methylenchlorid und 60 ml Dimethoxypropan während 20 h bei Raumtemperatur gerührt. Nach Einengen des Reaktionsansatzes wird direkt an 900 g Kieselgel mittels FC (Laufmittel G) gereinigt: R_{f}(D)=0.56; FAB-MS: (M+H)⁺= 488.
f) 10-Benzyloxy6(S)-tertiärbutoxycarbonylamino5(S)-hydroxy3(R,S)-isopropyl8,8-dimethyldecen(1)
   88.5 g 1-Brom-2(R,S)-isopropyl-3-buten, 13 g Magnesiumpulver und 41.9 g 6-Benzyloxy-2(S)-tertiärbutoxycarbonylamino-4,4-dimethyl-hexanal werden analog wie in Beispiel 37f) beschrieben umgesetzt. Die Reinigung erfolgt durch FC an 1 kg Kieselgel (Laufmittel G und E): R_{f}(3:1-Gemisch aus Toluol und Essigester)=0.47; Rₜ(I)=33.8/34.2 min; FAB-MS: (M+H)⁺= 520.
g) 6-Benzyloxy-2(S)-tertiärbutoxycarbonylamino-4,4-dimethyl-hexanal
   Die Titelverbindung wird ausgehend von 28.1 g 6-Benzyloxy-2(S)-tertiärbutoxycarbonylamino-4,4dimethyl-hexan-1-ol, 10.5 ml Oxalylchlorid, 11.4 ml Dimethylsulfoxid und 45 ml Triethylamin analog Beispiel 1 g) hergestellt: R_{f}(A)= 0.62; Rₜ(I)=28.1 min; FAB-MS: (M+H)⁺= 350.
h) 6-Benzyloxy-2(S)-tertiärbutoxycarbonylamino-4,4-dimethyl-hexan-1-ol
   Die Titelverbindung wird ausgehend von 75.2 g 4(S)-(4-Benzyloxy-2,2-dimethylbutyl)3-tertiärbutoxycarbonyl-2,2-dimethyl-1,3-oxazolidin und 2.0 g p-Toluolsulfonsäure-Hydrat in 500 ml Methanol analog Beispiel 20a) hergestellt: R_{f}(A)= 0.31; FAB-MS: (M+H)⁺=352; Anal. kalk. für C₂₀H₃₃NO₄: C68.34%, H9.46%, N3.99%; gef. C68.14%, H9.37%, N3.98%.
i) 4(S)-(4-Benzyloxy-2,2-dimethylbutyl)3-tertiärbutoxycarbonyl-2,2-dimethyl-1,3-oxazolidin
   Zu einer Suspension von 10 g Kaliumhydrid in 300 ml Tetrahydrofuran wird bei 0 °C eine Lösung von 55 g 3-Tertiärbutoxycarbonyl-4(S)-(4-hydroxy-2,2-dimethylbutyloxy-2,2-dimethyl-1,3-oxazolidin (Beispiel 1j), gelöst in 150 ml Tetrahydrofuran, addiert. Nach 1 h Rühren bei 25 °C werden nach Abkühlen auf 0 °C 23 ml Benzylbromid zugetropft und anschliessend 1h bei Raumtemperatur gerührt. Das Reaktionsgemisch wird vorsichtig mit 400 ml Wasser verdünnt und mit Diethylether extrahiert. Das Rohprodukt wird mittels FC (500 g Kieselgel, Laufmittel F) gereinigt: R_{f}(D)= 0.47; [α]^{D} = +27.8 (c=1.0 in Chloroform); FAB-MS: (M+H)⁺=392; Anal. kalk. für C₂₃H₃₇NO₄: C70.55%, H9.52%, N3.58%; gef. C70.74%, H9.59%, N3.64%.

### Beispiel 40: 5(S)-Amino-4(S)-hydroxy-2(S)-isopropyl-7,7-dimethyl-8-[2(R,S)-methylaminocarbonyl-3,4-dihydro-2H-1,4-benzthiazin-4-yloctansäure(N-butyl)amid

Die Titelverbindung erhält man ausgehend von 294 mg 5(S)-Tertiärbutoxycarbonylamino-4(S)-hydroxy-2(S)-isopropyl-7,7dimethyl-8-[2(R,S)-methylaminocarbonyl-3,4-dihydro-2H-1,4-benzthiazin-4-ylcarbonyl]-octansäure(N-butyl)amid (Beispiel 39) erhält man analog wie in Beispiel 1) beschrieben als Diastereomerengemisch: R_{f} (S)= 0.36 (Doppelfleck); FAB-MS: (M+H)⁺= 535.

### Beispiel 41: 5(S)-Amino-4(S)-hydroxy-2(R,S)-isopropyl-7,7-dimethyl-8-(1,2,3,4-tetrahydrochinolin-1-ylcarbonyl)-octansäure(N-butyl)amid

In analoger Weise wie in den Beispielen 1 und 1a) für die entsprechenden Reaktionsstufen beschrieben erhält man die Titelverbindung ausgehend von 200 mg 3-[N-Tertiärbutoxycarbonyl-4(S)-(3-carboxy-2,2-dimethylpropyloxy-2,2-dimethyl-1,3-oxazolidin-5(S)-yl]-2(R,S)isopropyl-propionsäure(N-butyl)amid (Beispiel 37a)) und 0.2 ml 1,2,3,4-Tetrahydrochinolin über das (4O,5N-Isopropyliden)-5(S)-tertiärbutoxycarbonylamino-4(S)-hydroxy-2(R,S)-isopropyl-7,7-dimethyl-8-(1,2,3,4-tetrahydrochinolin-1ylcarbonyl]-octansäure(N-butyl)amid (Reinigung an 50 g Kieselgel mit einem Laufmittelgradienten von einem 20:1- zu einem 9:1-Gemisch aus Methylenchlorid und Diethylether; R_{f}(9:1-Gemisch aus Methylenchlorid und Diethylether)= 0.30) als Diastereomerengemisch: R_{f}(S)=0.34; FAB-MS: (M+H)⁺= 461.

### Beispiel 42: 5(S)-Amino-4(S)-hydroxy-2(R,S)-isopropyl-7,7-dimethyl-8-[2(R,S)-carbamoyl-3,4-dihydro-2H-1,4-benzthiazin-4-ylcarbonyl]-octansäure(N-butyl)amid

In analoger Weise wie in den Beispielen 1 und 1 a) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R,S)isopropyl-propionsäure(N-butyl)amid (Beispiel 37a)) und 160 mg 2(R,S)-carbamoyl-3,4-dihydro-2H-1,4-benzthiazin über das (4O,5N-Isopropyliden)-5(S)-tertiärbutoxycarbonylamino-4(S)-hydroxy-2(R,S)-isopropyl-7,7-dimethyl-8-[2(R,S)-carbamoyl-3,4-dihydro-2H-1,4-benzthiazin-4-ylcarbonyl]-octansäure(N-butyl)amid (R_{f}(Y)=0.42) als Diastereomerengemisch: R_{f}(S)=0.37; FAB-MS: (M+H)⁺= 521.

Die eingesetzte Amin-Komponente wird beispielsweise folgendermassen hergestellt:
580 mg 2(R,S)-methoxycarbonyl-3,4-dihydro-2H-1,4-benzthiazin werden in 3 ml einer 6N-AmmoniakLösung in Methanol gelöst und während 16 h bei 50 °C gerührt. Chromatographische Reinigung an 50 g Kieselgel ergibt das 2(R,S)-carbamoyl-3,4-dihydro-2H-1,4-benzthiazin: R_{f}(Y)= 0.19.

### Beispiel 43: 5(S)-Amino-4(S)-hydroxy-2(R),7,7-trimethyl-8-[2(R,S)-methoxycarbonyl-3,4-dihydro-2H-1,4-benzoxazin-4-ylcarbonyl]-octansäure(N-butyl)amid

In analoger Weise wie in den Beispielen 1 und 1a) für die entsprechenden Reaktionsstufen beschrieben erhält man die Titelverbindung ausgehend von 400 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 1.02 g 2(R,S)-Methoxycarbonyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-methoxycarbonyl-3,4-dihydro-2H-1,4-benzoxazin-4-ylcarbonyl]-octansäure(N-butyl)amid (R_{f}(Y)= 0.55) als Diastereomerengemisch: R_{f}(T)= 0.40; FAB-MS: (M+H)⁺= 492.

### Beispiel 44: 5(S)-Amino-4(S)-hydroxy-2(R),7,7-trimethyl-8-[3(R,S)-hydroxymethyl-1,2,3,4-tetrahydrochinolin-1ylcarbonyl]-octansäure(N-butyl)amid

Die Lösung von 165 mg (4O,5N-Isopropyliden)-5(S)-tertiärbutoxycarbonylamino-4(S)-hydroxy-2(R),7,7-trimethyl-8-[3(R,S)-(tertiärbutyldimethylsilyloxymethyl)-1,2,3,4-tetrahydrochinolin-1-ylcarbonyl]-octansäure(N-butyl)amid in 3 ml Tetrahydrofuran wird mit 137 mg Tetrabutylammoniumfluorid versetzt und über 1 h bei Raumtemperatur gerührt. Der Reaktionsansatz wird eingeengt und anschliessend direkt in 5 ml 95% Trifluoressigsäure analog Beispiel 1) umgesetzt. Reinigung mittels FC an 30 g Kieselgel (Laufmittel S) ergibt die Titelverbindung als Diastereomerengemisch: R_{f}(T)=0.30; FAB-MS: (M+H)⁺= 462.

Die Ausgangsmaterialien werden beispielsweise wie folgt erhalten:
a) (4O,5N-Isopropyliden)-5(S)-tertiärbutoxycarbonylamino-4(S)-hydroxy-2(R),7,7-trimethyl-8-[3(R,S)-(tertiärbutyldimethylsilyloxymethyl)-1,2,3,4-tetrahydrochinolin-1ylcarbonyl]-octansäure(N-butyl)amid
   Die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2-(R)-methylpropionsäure(N-butyl)-amid und 303 mg 3(R,S)-(Tertiärbutyldimethylsilyloxymethyl)-1,2,3,4-tetrahydrochinolin: R_{f}(Z)=0.30.
b) 3(R,S)-(Tertiärbutyldimethysilyloxymethyl)-1,2,3,4-tetrahydrochinolin
   25.2 g 1-Benzyl-3(R,S)-(tertiärbutyldimethylsilyloxymethyl)-1,2,3,4-tetrahydrochinolin in 250 ml Tetrahydrofuran werden in Gegenwart von 2.0 g 10% Palladium auf Kohle unter üblichen Reaktionsbedingungen hydriert. Reinigung des Rohproduktes an 500 g Kieselgel mit einem 1:1-Gemisch aus Methylenchlorid und Hexan als Laufmittel ergibt 7.6 g der Titelverbindung: R_{f}(1:1-Gemisch aus Methylenchlorid und Hexan)= 0.19.
c) 1-Benzyl-3(R,S)-(tertiärbutyldimethylsilyloxymethyl)-1,2,3,4-tetrahydrochinolin
   11.6 g 1-Benzyl-3(R,S)-hydroxymethyl-1,2,3,4-tetrahydrochinolin (Beispiel 29e), 8.54 g Tertiärbutyldimethylchlorsilan und 4.05 g Imidazol werden in 120 ml Methylenchlorid über 20 h bei Raumtemperatur gerührt. Der Niederschlag wird anschliessend abfiltriert und das nach Einengen des Filtrats erhaltene Rohprodukt an 900 g Kieselgel (Laufmittel Z) gereinigt. Man erhält 16 g der Titelverbindung als gelbliches Öl: R_{f}(Z)= 0.66.

### Beispiel 45: 4(S)-Amino-3(S)-hydroxy-1(R),6,6-trimethyl-heptandicarbonsäure-1-(N-butyl)amid-7-[N-[2-(3-pyridyl)phenyl]]amid

Unter Eiskühlung werden 30mg 4(S)-Tertiärbutoxycarbonylamino-3(S)-hydroxy-1(R),6,6-trimethyl-heptandicarbonsäure-1-(N-butyl)amid-7-[N-[2-(3-pyridyl)phenyl]]amid mit 2 ml einer 25%igen Lösung von Trifluoressigsäure in Methylenchlorid versetzt. Es wird über 2h bei 0°C gerührt. Nach Zugabe von 2 ml Toluol wird das Reaktionsgemisch eingeengt und der Rückstand durch FC an 15 g Kieselgel mit einem Laufmittelgradienten aus Methylenchlorid und Methanol (vom 90:10-Gemisch zum 80:20-Gemisch) gereinigt. Man erhält die Titelverbindung: R_{f}(S)=0.22; R_{f}(IV)=27.4min; FAB-MS: (M+M)⁺=469.

Die Ausgangsmaterialien werden beispielsweise folgendermassen hergestellt:
a) 4(S)-Tertiärbutoxycarbonylamino-3(S)-hydroxy-1(R),6,6trimethyl-heptandicarbonsäure-1-(N-butyl)amid-7-[N-[2-(3-pyridyl)phenyl]]amid
   Die Titelverbindung wird ausgehend von 143mg (3O,4N-Isopropyliden)4(S)-tertiärbutoxycarbonylamino-3(S)-hydroxy-1(R),6,6trimethyl-heptandicarbonsäure-1-(N-butyl)amid-7-[N-[2-(3-pyridyl)phenyl]]amid analog 20a) hergestellt und durch FC an 15 g Kieselgel mit einem Laufmittelgradienten aus Methylenchlorid und Methanol (vom 97:3-Gemisch zum 94:6-Gemisch) gereinigt: R_{f}(P)=0.42.
b) (3O,4N-Isopropyliden)4(S)-tertiärbutoxycarbonylamino-3(S)-hydroxy-1(R),6,6trimethylheptandicarbonsäure-1-(N-butyl)amid-7-[N-[2-(3-pyridyl)phenyl]]amid
   Zu einer Lösung von 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid in 3 ml Methylenchlorid werden bei 0° C 0.06ml 1-ChlorN,N,2trimethylpropenylamin addiert. Nach 30min wird eine Lösung von 2-(3-Pyridyl)anilin in 1 ml Methylenchlorid und 3mg 4-DMAP zugefügt und der Reaktionsansatz über 4h bei Raumtemperatur gerührt. Das Reaktionsgemisch wird eingeengt und der Rückstand mittels FC an 80 g Kieselgel (LaufmittelB und A) chromatographiert: R_{f}(H)=0.16.

### Beispiel 46: 4(S)-Amino-3(S)-hydroxy-1(R),6,6-trimethyl-heptandicarbonsäure-1-(N-butyl)amid-7-[N-[2-(N-oxido3-pyridyl)phenyl]]amid

31mg 4(S)-Tertiärbutoxycarbonylamino-3(S)-hydroxy-1(R),6,6trimethylheptandicarbonsäure-1-(N-butyl)amid-7-[N-[2-(N-oxido3-pyridyl)phenyl]]amid werden analog wie in Beispiel45) beschrieben umgesetzt und das erhaltene Rohprodukt mittels FC an 10 g Kieselgel (Laufmittel P) gereinigt. Man erhält die Titelverbindung: R_{f}(S)=0.12; R_{f}(IV)=26.9min.

Das Ausgangsmaterial wird beispielsweise folgendermassen hergestellt:
a) 4(S)-Tertiärbutoxycarbonylamino-3(S)-hydroxy-1(R),6,6trimethyl-heptandicarbonsäure-1-(N-butyl)amid-7-[N-[2-(N-oxido3-pyridyl)phenyl]]amid
   Zu einer Lösung von 30mg 4(S)-Tertiärbutoxycarbonylamino-3(S)-hydroxy-1(R),6,6trimethylheptandicarbonsäure-1-(N-butyl)amid-7-[N-[2-(3-pyridyl)phenyl]]amid in 2 ml Methylenchlorid werden 19mg metaChlorperbenzoesäure addiert und über Nacht bei Raumtemperatur gerührt. Es werden erneut 5mg meta-Chlorperbenzoesäure zugegeben und bis zur Vollständigkeit der Reaktion bei Raumtemperatur gerührt. Der Reaktionsansatz wird ohne weitere Aufarbeitung direkt an 10 g Kieselgel (Laufmittel M) chromatographiert. Man erhält die Titelverbindung: R_{f}(P)=0.15; FAB-MS: (M+M)⁺=585.

### Beispiel 47: 5(S)-Formylamino-4(S)-hydroxy-2(R),7,7-trimethyl-8-[2(R,S)-methoxycarbonyl-3,4-dihydro-2H-1,4-benzthiazin-4-ylcarbonyl]-octansäure(N-butyl)amid

195 mg 5(S)-Amino-4(S)-hydroxy-2(R),7,7-trimethyl-8-[2(R,S)-methoxycarbonyl-3,4-dihydro-2H-1,4-benzthiazin-4-ylcarbonyl]-octansäure(N-butyl)amid (Beispiel 18)) werden analog wie in Beispiel 17) beschrieben mit 128 mg Ameisensäure-4-nitrophenylester umgesetzt und an 30 g Kieselgel (Laufmittel N) gereinigt. Man erhält die Titelverbindung als gelblich gefärbten Festkörper: R_{f}(N)=0.20; FAB-MS: (M+H)⁺= 536.

### Beispiel 48: 5(S)-Amino-4(S)-hydroxy-2(R),7,7-trimethyl-8-(S,S-dioxo-3,4-dihydro-1H-2,4-benzthiazin-4-ylcarbonyl)-octansäure(N-butyl)amid

Analog zu den in den Beispielen 21, 21a und 21b) für die entsprechenden Reaktionsstufen beschriebenen Verfahren erhält man die Titelverbindung ausgehend von 228 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 120 mg 3,4-Dihydro-1H-2,4-benzthiazin über das (4O,5N-Isopropyliden)-5(S)-tertiärbutoxycarbonylamino-4(S)-hydroxy-2(R),7,7-trimethyl-8-(3,4-dihydro-1H-2,4-benzthiazin-4-ylcarbonyl)-octansäure(N-butyl)amid (R_{f}(A)= 0.32) und das 5(S)-Tertiärbutoxycarbonylamino-4(S)-hydroxy-2(R),7,7-trimethyl-8-(S,S-dioxo-3,4-dihydro-1H-2,4-benzthiazin-4-ylcarbonyl]-octansäure(N-butyl)amid. Nach beendeter Reaktion mit p-Toluolsulfonsäure-Hydrat in Methanol, wie in Beispiel 21a) beschrieben, wird das Reaktionsgemisch wird mit 2 ml Wasser verdünnt und mit 228 mg Oxone oxidiert, und das Produkt nach üblichen Verfahren durch Extraktion isoliert: R_{f}(K)= 0.51; Rₜ(I)= 23.6 min) und Reinigung mittels FC (10 g Kieselgel, Laufmittel O): R_{f}(O)= 0.14; Rₜ(I)= 17.3 min; FAB-MS: (M+H)⁺= 482.

Die als Ausgangsmaterial eingesetzte Amin-Komponente wird beispielsweise folgendermassen hergestellt:
a) 3,4-Dihydro-1H-2,4-benzthiazin
   Zu einer Lösung von 1.0 g N-Tertiärbutoxycarbonyl-3,4-dihydro-1H-2,4-benzthiazin in 35 ml Methylenchlorid werden bei Raumtemperatur 1.47 ml Trifluormethansulfonsäure-trimethylsilylester zugetropft und anschliessend 1.41 ml Lutidin2,6 addiert. Das Reaktionsgemisch wird für 20 min bei Raumtemperatur gerührt und dann mit 5 ml Methanol verdünnt und eingeengt. Das Rohprodukt wird mittels FC (30 g Kieselgel, Laufmittel G) gereinigt. Man erhält die reine Titelverbindung: R_{f}(D)= 0.26; Rₜ(I)= 19.5 min; Anal. kalk. für C₈H₉NS: C 63.54 %, H 6.00 %, N 9.26 %; gef. C 63.22 %. H 6.01 %, N 8.99 %.
b) N-Tertiärbutoxycarbonyl-3,4-dihydro-1H-2,4-benzthiazin
   5.78 g 2-Acetylthiomethyl(N-tertiärbutoxycarbonyl)anilin werden in 10 ml Tetrahydrofuran gelöst und bei Raumtemperatur mit 5.72 g Kaliumcarbonat und 16.4 ml einer 35 %igen FormaldehydLösung versetzt. Das Reaktionsgemisch wird für 3 h bei 40-50 °C gerührt und anschliessend mit KaliumhydrogensulfatLösung neutralisiert. Das Zwischenprodukt wird mit Chloroform extrahiert, über Natriumsulfat getrocknet, anschliessend mit 80 mg p-Toluolsulfonsäure-Hydrat versetzt und für 20 h bei Raumtemperatur gerührt. Das Reaktionsgemisch wird eingedampft und das Rohprodukt mittels FC (100 g Kieselgel,Laufmittel G) gereinigt: R_{f}(D)= 0.45.
c) 2-Acetylthiomethyl(N-tertiärbutoxycarbonyl)anilin
   Zu einer Lösung von 51.4 g Triphenylphosphin in 50 ml Tetrahydrofuran werden bei 0 °C 38.6 ml Azocarbonsäurediisopropylester addiert. Nach 30 min wird eine Lösung von 21.0 g N-(Tertiärbutoxycarbonyl)-2-(hydroxymethyl)anilin und 15 ml Thioessigsäure in 20 ml Tetrahydrofuran dazugetropft. Das Reaktionsgemisch wird für 2 h bei 0 °C und anschliessend für 22 h bei Raumtemperatur gerührt. Das Rohprodukt wird mittels FC (500 g Kieselgel,LaufmittelG)geeinigt: R_{f}(3:1-Gemisch aus Toluol und Eisessig)= 0.43; MS: M⁺= 281.
d) N-(Tertiärbutoxycarbonyl)2-(hydroxymethyl)anilin
   Zu einer Lösung von 12.3 g 2-Aminobenzylalkohol in 100 ml Methylenchlorid werden 22.5 g Di-tertiärbutyldicarbonat und 21 ml Triethylamin addiert und während 24 h bei Raumtemperatur gerührt. Das Reaktionsgemisch wird mehrmals mit 10 %iger Citronensäure-Lösung, gesättigter Natriumhydrogencarbonat-Lösung und gesättigter Natriumchloridlösung gewaschen und dann eingeengt. Man erhält die Titelverbindung: R_{f}(A) 0.61.

### Beispiel 49: 5(S)-Amino-4(S)-hydroxy-2(R),7,7-trimethyl-8-(7-nitro-1,2,3,4-tetrahydrochinolin-1ylcarbonyl)-octansäure(N-butyl)amid

Analog zu den in den Beispielen 21, 21a und 21b) für die entsprechenden Reaktionsstufen beschriebenen Verfahren erhält man die Titelverbindung ausgehend von 114 mg 3-[N-Tertiärbutoxycarbonyl-4(S)-(3-carboxy-2,2-dimethylpropyloxy-2,2-dimethyl-1,3-oxazolidin- 5(S)-yl]-2(R)-methyl-propionsäure(N-butyl)amid und 129mg 7-Nitro-1,2,3,4-tetrahydrochinolin (A.P. Terent'ev et al. Khim. Geter. Soedin 12, 1663(1969)) analog der in Beispiel 20b) beschriebenen Methode über das (4O,5N-Isopropyliden)-5(S)-tertiärbutoxycarbonylamino-4(S)-hydroxy-2(R),7,7-trimethyl-8-(7-nitro-1,2,3,4-tetrahydrochinolin-1-yl-carbonyl)-octansäure(N-butyl)amid (R_{f}(A)= 0.16; R_{f}(Essigester)= 0.55) und das 5(S)-Tertiärbutoxycarbonylamino-4(S)-hydroxy-2(R),7,7-trimethyl-8-(7-nitro-1,2,3,4-tetrahydrochinolin-1-ylcarbonyl]-octansäure(N-butyl)amid (Reinigung an 10 g Kieselgel, Laufmittel A und Essigester: R_{f}(X)= 0.43): R_{f}(W)= 0.63; Rₜ(I)= 20.5 min; FAB-MS: (M+H)⁺= 477.

### Beispiel 50: 5(S)-Amino-4(S)-hydroxy-2(R),7,7-trimethyl-8-[3(R,S)-ethoxycarbonyl-7-nitro-1,2,3,4-tetrahydrochinolin-1-ylcarbonyl]-octansäure(N-butyl)amid

Analog zu den in den Beispielen 21, 21a und 21b) für die entsprechenden Reaktionsstufen beschriebenen Verfahren erhält man die Titelverbindung ausgehend von 183 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 125 mg 3(R,S)-Ethoxycarbonyl-7-nitro-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-ethoxycarbonyl-7-nitro-1,2,3,4-tetrahydrochinolin-1-ylcarbonyl]-octansäure(N-butyl)amid (Reinigung an 20 g Kieselgel, Laufmittel A : R_{f}(X)= 0.68; Rₜ(I)= 31.6 min) und das 5(S)-Tertiärbutoxycarbonylamino-4(S)-hydroxy-2(R),7,7-trimethyl-8-[3(R,S)-ethoxycarbonyl-7-nitro-1,2,3,4-tetrahydrochinolin-1-ylcarbonyl]-octansäure(N-butyl)amid (Reinigung an 10 g Kieselgel, Laufmittel A und G: R_{f}(X)= 0.47; Rₜ(I)= 24.9 min): R_{f}(W)= 0.55; Rₜ(I)= 21.3 min; FAB-MS: (M+H)⁺= 549.

Das eingesetzte 3(R,S)-Ethoxycarbonyl-7-nitro-1,2,3,4-tetrahydrochinolin wird analog dem Verfahren von A.P. Terent'ev et al. Khim. Geter. Soedin 12, 1663 (1969) ausgehend von 246 mg 3(R,S)-Ethoxycarbonyl-1,2,3,4-tetrahydrochinolin (Beispiel 31b) hergestellt: R_{f}(A)= 0.57; R_{f}(3:1-Gemisch aus Toluol und Essigester)= 0.49.

### Beispiel 51: 5(S)-Amino-4(S)-hydroxy-2(R),7,7-trimethyl-8-[2(R,S)-ethoxycarbonyl-2(R,S)-methyl-3,4-dihydro-2H-1,4-benzoxazin-4-ylcarbonyl]-octansäure(N-butyl)amid

Analog zu den in den Beispielen 21, 21a und 21b) für die entsprechenden Reaktionsstufen beschriebenen Verfahren erhält man die Titelverbindung ausgehend von 205 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 200 mg 2(R,S)-Ethoxycarbonyl-2(R,S)-methyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-ethoxycarbonyl-2(R,S)-methyl-3,4-dihydro-2H-1,4-benzoxazin-4-ylcarbonyl]-octansäure(N-butyl)amid (R_{f}(A)= 0.24) und das 5(S)-Tertiärbutoxycarbonylamino-4(S)-hydroxy-2(R),7,7-trimethyl-8-[2(R,S)-ethoxycarbonyl-2(R,S)-methyl-3,4-dihydro-2H-1,4-benzoxazin-4-ylcarbonyl]-octansäure(N-butyl)amid (Reinigung an 10 g Kieselgel, Laufmittel A und G: R_{f}(X)= 0.50; FAB-MS: (M+H)⁺= 620): R_{f}(W)= 0.25; Rₜ(I)= 20.7/20.9 min; FAB-MS: (M+H)⁺= 520.

Die Ausgangsmaterialien werden beispielsweise folgendermassen hergestellt:
a) 2(R,S)-Ethoxycarbonyl-2(R,S)-methyl-3,4-dihydro-2H-1,4-benzoxazin
   Die Titelverbindung wird analog Beispiel 1r) ausgehend von 800 mg N-Benzyloxycarbonyl-2(R,S)-ethoxycarbonyl-2(R,S)-methyl-3,4-dihydro-2H-1,4-benzoxazin erhalten. Das Rohprodukt wird mittels FC an 50 g Kieselgel (Laufmittel B) gereinigt. Man erhält die reine Titelverbindung: R_{f}(A)= 0.55; MS: M⁺= 221.
b) N-Benzyloxycarbonyl-2(R,S)-ethoxycarbonyl-2(R,S)-methyl-3,4-dihydro-2H-1,4-benzoxazin
   Zu einer Lösung von 8.8 ml einer 2 M Lithiumdiisopropylamid-Lösung in Tetrahydrofuran wird bei -20 °C eine Lösung von 5.0 g N-Benzyloxycarbonyl-2(R,S)-ethoxycarbonyl-3,4-dihydro-2H-1,4-benzoxazin in 20 ml Tetrahydrofuran addiert. Nach 20 min werden 1.6 ml Methyljodid zugetropft und das Reaktionsgemisch für 4 h bei 0 °C gerührt. Anschliessend wird nach Addition von 30 ml einer gesättigten Ammoniumchloridlösung mit Essigester extrahiert. Das Rohprodukt wird mittels FC an 200 g Kieselgel (Laufmittel E) gereinigt. Man erhält die reine Titelverbindung: R_{f}(D)= 0.25; FAB-MS: (M+H)⁺= 356.

### Beispiel 52: 5(S)-Amino-4(S)-hydroxy-2(R),7,7-trimethyl-8-[3(R,S)-methylsulfonyl-1,2,3,4-tetrahydrochinolin-1ylcarbonyl]-octansäure(N-butyl)amid

95mg (S)-Tertiärbutoxycarbonylamino-4(S)-hydroxy-2(R),7,7-trimethyl-8-[3(R,S)-methylsulfonyl-1,2,3,4-tetrahydrochinolin-1ylcarbonyl]-octansäure(N-butyl)amid werden in 2 ml einer 20%igen TrifluoressigsäureLösung in Methylenchlorid bei 0°C über 2h gerührt. Aufarbeitung analog wie in Beispiel45) beschrieben und Reinigung des Rohprodukts durch FC an 10 g Kieselgel (Laufmittel M) ergibt die Titelverbindung als Diastereomerengemisch: R_{f}(S)=0.16/0.20; Rₜ(IV)=35.0/35.3min; FAB-MS: (M+H)⁺=510.

Die Ausgangsmaterialien werden beispielsweise folgendermassen hergestellt:
a) 5(S)-Tertiärbutoxycarbonylamino-4(S)-hydroxy-2(R),7,7-trimethyl-8-[3(R,S)-methylsulfonyl-1,2,3,4-tetrahydrochinolin-1ylcarbonyl]-octansäure(N-butyl)amid
   Die Titelverbindung wird ausgehend von 170mg (4O,5N-Isopropyliden)-5(S)-tertiärbutoxycarbonylamino-4(S)-hydroxy-2(R),7,7-trimethyl-8-[3(R,S)-methylsulfonyl-1,2,3,4-tetrahydrochinolin-1ylcarbonyl]-octansäure(N-butyl)amid in analoger Weise wie in Beispiel21a) erhalten und durch FC an 25 g Kieselgel mit einem Laufmittelgradienten aus Methylenchlorid und Methanol (von J zu K) gereinigt. Man erhält die Titelverbindung: R_{f}(P)-=0.56.
b) (4O,5N-Isopropyliden)-5(S)-tertiärbutoxycarbonylamino-4(S)-hydroxy-2(R),7,7-trimethyl-8-[3(R,S)-methylsulfonyl-1,2,3,4-tetrahydrochinolin-1ylcarbonyl]-octansäure(N-butyl)amid
   Die Titelverbindung wird durch Umsetzung von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methylpropionsäure(N-butyl)amid, 0.06ml 1-ChlorN,N,2-trimethylpropenylamin, 140mg 3(R,S)-methylsulfonyl-1,2,3,4-tetrahydrochinolin und 4mg 4-DMAP analog wie in Beispiel45b) beschrieben und nachfolgende Reinigung des Rohprodukts mittels FC an 80 g Kieselgel mit einem Laufmittelgradienten aus Hexan und Essigester (von B zu A) erhalten: R_{f}(A)=0.15.
c) 3(R,S)-Methylsulfonyl-1,2,3,4-tetrahydrochinolin
   Eine Lösung von 6.5 g 3-Methylsulfonylchinolin in 200ml Eisessig wird in Gegenwart von 690mg Platinoxid bei 50°C über 13h hydriert. Nach Filtration des Reaktionsgemischs über Celite 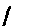 545 wird eingeengt und das Rohprodukt mittels FC an 200 g Kieselgel mit einem 99:1-Gemisch aus Methylenchlorid und Methanol als Laufmittel gereinigt: R_{f}(K)=0.45; Anal. kalk. für C₁₀H₁₃NO₂S: C56.85%, H6.20%, N6.63%; gef. C56.95%, H6.17%, N6.77%.
d) 3-Methylsulfonylchinolin
   Zu einer Lösung von 6.1 g 3-Methylthiochinolin in 11 ml Dioxan wird eine Mischung aus 28mg NatriumwolframatDihydrat, 8 ml Wasser und 2Tropfen Eisessig addiert. Unter kräftigem Rühren werden bei 65°C 7.1 ml einer 30%igen Wasserstoffperoxidlösung über 30min zugetropft. Anschliessend wird das Reaktionsgemisch auf 80°C erwärmt und noch 1h gerührt. Nach dem Abkühlen wird mit 100ml Methylenchlorid verdünnt, die organische Phase abgetrennt und die wäßrige Phase mit Methylenchlorid extrahiert. Nach FC des Rohprodukts an 150 g Kieselgel mit einem Laufmittelgradienten aus Hexan und Essigester (von D zu A) erhält man die reine Titelverbindung: R_{f}(A)=0.31.
e) 3-Methylthiochinolin
   Unter Rühren werden zu einer Lösung von 7.4 g 3-Bromchinolin in 110ml Dimethylformamid 5.0 g Natriummethanthiolat addiert und 30min bei Raumtemperatur gerührt. Anschliessend wird das Reaktionsgemisch eingeengt, in 100ml Wasser aufgenommen und mit Methylenchlorid extrahiert. Die organische Phase wird mit gesättigter Natriumchloridlösung gewaschen, über Natriumsulfat getrocknet und der Rückstand durch FC an 100 g Kieselgel mit einem 1:2-Gemisch aus Diethylether und Petrolether als Laufmittel gereinigt. Man erhält die reine Titelverbindung: Smp.40°C; R_{f}(F)=0.24; Anal. kalk. für C₁₀H₉NS: C68.53%, H5.18%, N7.99%; gef. C68.76%, H5.16%, N7.97%.

### Beispiel 53: 5(S)-Amino-4(S)-hydroxy-2(R),7,7-trimethyl-8-[3-methylsulfonyl-1,2,3,4-tetrahydrochinazolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methylpropionsäure(N-butyl)amid und 140mg 3-Methylsulfonyl-1,2,3,4-tetrahydrochinazolin über das (4O,5N-Isopropyliden)-5(S)-tertiärbutoxycarbonylamino-4(S)-hydroxy-2(R),7,7-trimethyl-8-[3-methylsulfonyl-1,2,3,4-tetrahydrochinazolin-1ylcarbonyl]-octansäure(N-butyl)amid (R_{f}(A)=0.33) und das 5(S)-Tertiärbutoxycarbonylamino-4(S)-hydroxy-2(R),7,7-trimethyl-8-[3-methylsulfonyl-1,2,3,4-tetrahydrochinazolin-1ylcarbonyl]-octansäure(N-butyl)amid (R_{f}(J)=0.37): R_{f}(S)=0.32; Rₜ(IV)=35.5min; FAB-MS: (M+H)⁺=511.

Das als Ausgangsmaterial eingesetzte 3-Methylsulfonyl-1,2,3,4-tetrahydrochinazolin wird beispielsweise folgendermassen hergestellt:
Zu einer Mischung von 300mg 1,2,3,4-Tetrahydrochinazolin (hergestellt nach der von R.F.Smith et al. in J.Heterocycl. Chem.2 (1965) beschriebenen Methode) und 0.47ml Triethylamin in 14ml Methylenchlorid werden bei 0° C 0.17ml Methansulfochlorid unter Rühren addiert. Nach 5min wird mit gesättigter Natriumchloridlösung versetzt, kurz nachgerührt und die organische Phase nach Trocknen über Natriumsulfat eingeengt. Reinigung des Rohprodukts mittels FC an 30 g Kieselgel (Laufmittel P) ergibt 3-Methylsulfonyl-1,2,3,4-tetrahydrochinazolin: R_{f}(K)=0.65; IR(KBr): 3360(m), 1600(m), 1500(m), 1320(s), 1145(s), 770(s).

### Beispiel 54: 5(S)-Amino-4(S)-hydroxy-2(R),7,7-trimethyl-8-[3(R,S)-methylcarbonylamino-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 70mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 88mg 3(R,S)-methylcarbonylamino-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methylcarbonylamino-1,2,3,4-tetrahydrochinolin-1-ylcarbonyl]-octansäure(N-butyl)amid (R_{f} (P)=0.55) und das 5(S)-Tertiärbutoxycarbonylamino-4(S)-hydroxy-2(R),7,7-trimethyl-8-[3(R,S)-methylcarbonylamino-1,2,3,4-tetrahydrochinolin-1-ylcarbonyl]-octansäure(N-butyl)amid (R_{f}(S)=0.24) als Diastereomerengemisch: R_{f}(S)=0.10; Rₜ(IV)=33.5min; FAB-MS: (M+H)⁺=489.

Das als Ausgangsmaterial eingesetzte 3(R,S)-Methylcarbonylamino-1,2,3,4-tetrahydrochinolin wird beispielsweise folgendermassen hergestellt:
Zu einer Lösung von 5.3 g 3-Aminochinolin in 150ml Methylenchlorid werden nacheinander 5.6 ml Pyridin und 2.96ml Acetylchlorid bei 0° C unter Rühren addiert. Nach 45min bei 0°C läßt man auf Raumtemperatur aufwärmen, rührt weitere 30min nach und gießt anschliessend das Reaktionsgemisch auf 150ml Eiswasser. Nach Extraktion der alkalischen wäßrigen Phase mit Essigester werden die vereinigten organischen Phasen mit 5%iger NatriumhydrogencarbonatLösung und gesättigter Natriumchloridlösung gewaschen und über Natriumsulfat getrocknet.
300mg des nach einmaligem Umkristallisieren aus Essigester/Hexan erhaltenen 3-(Acetylamino)chinolin (R_{f}(D)=0.43) werden in 10ml Ethanol in Gegenwart von 60 mg Palladium auf Kohle (10%Pd) bei 50° C und unter Normaldruck für 20h hydriert. Nach Filtration des Reaktionsgemischs über Celite 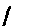 545 und chromatographischer Reinigung des Rohprodukts an 25 g Kieselgel (Laufmittel P) erhält man das 3(R,S)-Methylcarbonylamino-1,2,3,4-tetrahydrochinolin: R_{f}(P)=0.49; MS: (M)⁺=190.

### Beispiel 55: 4(S)-Amino-3(S)-hydroxy-1(R),6,6-trimethyl-heptandicarbonsäure-1-(N-butyl)amid-7-[N-(2carbamoylmethoxyphenyl)]amid

Analog zu den in den Beispielen 21, 21a und 21b) für die entsprechenden Reaktionsstufen beschriebenen Verfahren erhält man die Titelverbindung ausgehend von 120mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 83mg 2-(Carbamoylmethoxy)anilin über das (3O,4N-Isopropyliden)4(S)-tertiärbutoxycarbonylamino-3(S)-hydroxy-1(R),6,6trimethylheptandicarbonsäure-1-(N-butyl)amid-7-[N-(2carbamoylmethoxyphenyl)]amid (R_{f}(X)=0.33) und das 4(S)-Tertiärbutoxycarbonylamino-3(S)-hydroxy-1(R),6,6trimethylheptandicarbonsäure-1-(N-butyl)amid-7-[N-(2carbamoylmethoxyphenyl)]amid (R_{f}(X)=0.11; R_{f}(I)=21.5min): R_{f}(W)=0.33; Rₜ(I)=15.7min; FAB-MS: (M+H)⁺=465.

Das als Ausgangsmaterial eingesetzte 2-(Carbamoylmethoxy)anilin wird beispielsweise wie folgt hergestellt:
Zu einer Lösung von 4.0 g 2-Nitrophenol in 50ml Aceton werden 8.0 g Kaliumcarbonat und 11.4 g Jodacetamid addiert. Das Reaktionsgemisch wird für 7h unter Rückfluss erhitzt und dann eingeengt. Der Rückstand wird in Chloroform gelöst und mit 0.05N Natronlauge extrahiert. Umkristallisation des Rohprodukts aus Tetrahydrofuran ergibt 2-(Carbamoylmethoxy)nitrobenzol vom Smp.190-1°C; R_{f}(O)=0.55.

2.0 g 2-(Carbamoylmethoxy)nitrobenzol werden in Gegenwart von 100mg PalladiumKohle (10%Pd) in 50ml Tetrahydrofuran bei Raumtemperatur und Normaldruck für 2h hydriert. Das Reaktionsgemisch wird über Hyflo® filtriert und eingeengt. Man erhält das 2-(Carbamoylmethoxy)anilin als weisse Kristalle: Smp. 117°C; R_{f}(O)=0.36; FAB-MS: (M+H)⁺=167; Anal. kalk. für C₈H₁₀N₂O₂: C57.82%, H6.07%, N16.86%; gef. C57.71%, H6.04%, N16.87%.

### Beispiel 56: 4(S)-Amino-3(S)-hydroxy-1(R),6,6-trimethyl-heptandicarbonsäure-1-(N-butyl)amid-7-[N-(2carbamoylmethoxy-4methoxphenyl]amid

Analog zu den in den Beispielen 21, 21a und 21b) für die entsprechenden Reaktionsstufen beschriebenen Verfahren erhält man die Titelverbindung ausgehend von 120mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 100mg 2-Carbamoylmethoxy-4-methoxyanilin über das (3O,4N-Isopropyliden)4(S)-tertiärbutoxycarbonylamino-3(S)-hydroxy-1(R),6,6trimethyl-heptandicarbonsäure-1-(N-butyl)amid-7-[N-(2carbamoylmethoxy-4methoxyphenyl)]amid (R_{f}(X)=0.31; Rₜ(I)=25.4min) und das 4(S)-Tertiärbutoxycarbonylamino-3(S)-hydroxy-1(R),6,6trimethyl-heptandicarbonsäure-1-(N-butyl)amid-7-[N-(2carbamoylmethoxy-4methoxyphenyl)]amid (R_{f}(X)=0.16): R_{f}(W)=0.57; Rₜ(I)=16.0min; FAB-MS: (M+H)⁺=495.

Das als Ausgangsmaterial eingesetzte 2-Carbamoylmethoxy-4methoxyanilin wird beispielsweise folgendermassen hergestellt:
Umsetzung von 0.84 g 5-Methoxy-2nitrophenol, 1.66 g Kaliumcarbonat und 1.84 g Jodacetamid nach dem in Beispiel55) beschriebenen Verfahren ergibt das 2-Carbamoylmethoxy-4methoxynitrobenzol: Smp.185-7°C; R_{f}(O)=0.58.

930mg 2-Carbamoylmethoxy-4-methoxynitrobenzol werden in Gegenwart von 300mg RaneyNickel in 30ml Methanol bei Raumtemperatur und Normaldruck für 12h hydriert. Das Reaktionsgemisch wird über Hyflo® filtriert und eingeengt. Durch Umkristallisation des Rohprodukts aus Methanol erhält man 2-Carbamoylmethoxy-4methoxyanilin vom Smp.139-140°C; R_{f}(O)=0.25.

### Beispiel 57: 4(S)-Amino-3(S)-hydroxy-1(R),6,6-trimethyl-heptandicarbonsäure-1-(N-butyl)amid-7-[N-(2-carbamoylmethoxy5methoxyphenyl)]amid

Analog zu den in den Beispielen 21, 21a und 21b) für die entsprechenden Reaktionsstufen beschriebenen Verfahren erhält man die Titelverbindung ausgehend von 120mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 100mg 2-Carbamoylmethoxy5methoxyanilin über das (3O,4N-Isopropyliden)4(S)-tertiärbutoxycarbonylamino-3(S)-hydroxy-1(R),6,6trimethyl-heptandicarbonsaure-1-(N-butyl)amid-7-[N-(2-carbamoylmethoxy5-methoxyphenyl)]amid (R_{f}(X)=0.35; Rₜ(I)=25.6min) und das 4(S)-Tertiärbutoxycarbonylamino-3(S)-hydroxy-1(R),6,6trimethyl-heptandicarbonsäure-1-(N-butyl)amid-7-[N-(2-carbamoyl methoxy5methoxyphenyl)]amid (R_{f}(X)=0.11): R_{f}(W)=0.69; Rₜ(I)=16.3min; FAB-MS: (M+H)⁺=495.

Das als Ausgangsmaterial eingesetzte AnilinDerivat wird beispielsweise folgendermassen hergestellt:
Durch Umsetzung von 1.41 g 4-Methoxy-2nitrophenol, 3.04 g Kaliumcarbonat und 3.61 g Jodacetamid analog dem in Beispiel55) beschriebenen Verfahren erhält man das 2-Carbamoylmethoxy5methoxynitrobenzol: Smp.190-1°C; R_{f}(O)=0.58; FAB-MS: (M+H)⁺=227; Anal. kalk. für C₉H₁₀N₂O₅: C47.79%, H4.46%, N12.38%; gef. C47.90%, H4,48%, N12.58%.

Ausgehend von 1.13 g 2-Carbamoylmethoxy5methoxynitrobenzol erhält man in analoger Weise wie im Beispiel55) beschrieben das 2-Carbamoylmethoxy5methoxyanilin: Smp.121-2°C; R_{f}(O)=0.37; FAB-MS: (M+H)⁺=197.

### Beispiel 58: 4(S)-Amino-3(S)-hydroxy-1(R),6,6-trimethyl-heptandicarbonsäure-1-(N-butyl)amid-7-[N-[2-carbamoylmethoxy-4-(methoxycarbonyl)phenyl]]amid

Analog zu den in den Beispielen 21, 21a und 21b) für die entsprechenden Reaktionsstufen beschriebenen Verfahren erhält man die Titelverbindung ausgehend von 120mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 100mg 2-Carbamoylmethoxy-4-(methoxycarbonyl)anilin über das (3O,4N-Isopropyliden)4(S)-tertiärbutoxycarbonylamino-3(S)-hydroxy-1(R),6,6trimethyl-heptandicarbonsäure-1(N-butyl)amid-7-[N-[2-carbamoylmethoxy-4-(methoxycarbonyl)phenyl]]amid (R_{f}(X)=0.35; Rₜ(I)=26.5min) und das 4(S)-Tertiärbutoxycarbonylamino-3(S)-hydroxy-1(R),6,6trimethyl-heptandicarbonsäure-1-(N-butyl)amid-7-[N-[2-carbamoylmethoxy-4-(methoxycarbonyl)phenyl]]amid (R_{f}(X)=0.09; Rₜ(I)=22.0min): R_{f}(W)=0.70; Rₜ(I)=16.6min; FAB-MS: (M+H)⁺=523.

Das als Ausgangsmaterial eingesetzte AnilinDerivat wird beispielsweise in analoger Weise wie im Beispiel 55) für die entsprechenden Reaktionsstufen beschrieben hergestellt:
Umsetzung von 1.08 g 5-Methoxycarbonyl-2-nitrophenol, 1.82 g Kaliumcarbonat und 2.02 g Jodacetamid ergibt das 2-Carbamoylmethoxy-4-(methoxycarbonyl)nitrobenzol vom Smp. 138-9°C; R_{f}(O)=0.63; FAB-MS: (M+H)⁺=255; Anal. kalk. für C₁₀H₁₀N₂O₆: C47.25%, H3.97%, N11.02%; gef. C47.08%, H4.04%, N10.79%.

Ausgehend von 1.16 g 2-Carbamoylmethoxy-4(methoxycarbonyl)nitrobenzol erhält man das 2-Carbamoylmethoxy-4-(methoxycarbonyl)anilin vom Smp.178-80° C; R_{f}(O)=0.37; MS: M⁺=224; Anal. kalk. für C₁₀H₁₂N₂O₄: C53.57%, H5.39%, N12.49%; gef. C53.41%. H5.32%, N12.53%.

### Beispiel 59: 4(S)-Amino-3(S)-hydroxy-1(R),6,6-trimethyl-heptandicarbonsäure-1-(N-buty)amid-7-[N-[2-carbamoylmethoxy-5-(methoxycarbonyl)phenyl]]amid

Analog zu den in den Beispielen 21, 21a und 21b) für die entsprechenden Reaktionsstufen beschriebenen Verfahren erhält man die Titelverbindung ausgehend von 120 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 100 mg 2-Carbamoylmethoxy5-(methoxycarbonyl)anilin über das (3O,4N-Isopropyliden)4(S)-tertiärbutoxycarbonylamino-3(S)-hydroxy-1(R),6,6trimethyl-heptandicarbonsäure-1-(N-butyl)amid-7-[N-[2-carbamoylmethoxy5-(methoxycarbonyl)phenyl]]amid (Reinigung an 20 g Kieselgel, Laufmittel Essigester: R_{f}(X)= 0.26; Rₜ(I)= 25.7 min) und das 4(S)-Tertiärbutoxycarbonylamino-3(S)-hydroxy-1(R),6,6trimethyl-heptandicarbonsäure-1-(N-butyl)amid-7-[N-[2-carbamoylmethoxy5-(methoxycarbonyl)phenyl]]amid (Reinigung an 10 g Kieselgel, Laufmittel Essigester und S: R_{f}(X)= 0.10; Rₜ(I)= 21.5 min; FAB-MS: (M+H)⁺= 623): R_{f}(W)= 0.68; Rₜ(I)= 16.4 min.; FAB-MS: (M+H)⁺= 523.

Die Ausgangsmaterialien werden beispielsweise folgendermassen hergestellt:
a) 2-Carbamoylmethoxy5-(methoxycarbonyl)anilinwird ausgehend von 1.0 g 2-Carbamoylmethoxy5-(methoxycarbonyl)nitrobenzol analog Beispiel 56) erhalten: Smp. 181-2 °C; R_{f}(O)= 0.35; MS: M⁺= 224.
b) 2-Carbamoylmethoxy5-(methoxycarbonyl)nitrobenzol wird ausgehend von 3.66 g 4-Methoxycarbonyl-2-nitrophenol, 6.91 g Kaliumcarbonat und 4.62 g Jodacetamid analog Beispiel 55) erhalten: Smp. 185-7 °C; R_{f}(O)= 0.56.

### Beispiel 60: 5(S)-Amino-4(S)-hydroxy-2(S)-isopropyl-7,7-dimethyl-8-[3(R,S)-methylaminocarbonyl-1,2,3,4-tetrahydrochinolin-1-ylcarbonyl]-octansäure(N-butyl)amid

Analog wie in den Beispielen 21, 21a) und 21b) für die entsprechenden Reaktionsstufen beschrieben erhält man die Titelverbindung ausgehend von 300 mg 3-[N-Tertiärbutoxycarbonyl-4(S)-(3-carboxy-2,2-dimethylpropyloxy-2,2-dimethyl-1,3-oxazolidin- 5(S)-yl]-2(S)isopropyl-propionsäure(N-butyl)amid und 236 mg 3(R,S)-methylaminocarbonyl-1,2,3,4-tetrahydrochinolin über das (4O,5N-Isopropyliden)-5(S)-tertiärbutoxycarbonylamino-4(S)-hydroxy-2(S)-isopropyl-7,7-dimethyl-8-[3(R,S)-methylaminocarbonyl-1,2,3,4-tetrahydrochinolin-1-ylcarbonyl]-octansäure(N-butyl)amid (Reinigung an 20 g Kieselgel mit Laufmittel A: Rₜ(I)= 29.6/29.9 min) und das 5(S)-Tertiärbutoxycarbonylamino-4(S)-hydroxy-2(S)-isopropyl-7,7dimethyl-8-[3(R,S)-methylaminocarbonyl-1,2,3,4-tetrahydrochinolin-1-yl-carbonyl]-octansäure(N-butyl)amid (Reinigung, nach Extraktion mit Essigester aus NatriumcarbonatLösung, mittels FC an Kieselgel (Laufmittel H): R_{f}(P)= 0.50) als Diastereomerengemisch: R_{f}(V)= 0.33; Rₜ(I)= 19.1/19.3 min; FAB-MS: (M+H)⁺= 517.

Das als Ausgangsmaterial eingesetzte 3(R,S)-Methylaminocarbonyl-1,2,3,4-tetrahydrochinolin wird analog Beispiel 1r über das N-Benzyloxycarbonyl-3(R,S)-methylaminocarbonyl-1,2,3,4-tetrahydrochinolin, hergestellt aus N-Benzyloxycarbonyl-1,2,3,4-tetrahydrochinolin3(R,S)carbonsäure und Methylamin wie in Beispiel 1a) beschrieben, erhalten: R_{f}(P)= 0.46.

### Beispiel 61: 5(S)-Amino-4(S)-hydroxy-2(R),7,7-trimethyl-8-[3(R,S)-methoxymethyl-1,2,3,4-tetrahydrochinolin-1-ylcarbonyl]-octansäure(N-butyl)amid

Ausgehend von 300 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 465 mg 3(R,S)-Methoxymethyl-1,2,3,4-tetrahydrochinolin und anschliessende Umsetzung des so erhaltenen (4O,5N-Isopropyliden)-5(S)-tertiärbutoxycarbonylamino-4(S)-hydroxy-2(R),7,7-trimethyl-8-[3(R,S)-methoxymethyl-1,2,3,4-tetrahydrochinolin-1-ylcarbonyl]-octansäure(N-butyl)amid (analog wie in Beispiel 1a) beschrieben; R_{f}(A)=0.38) nach der in Beispiel 1) angegebenen Methode erhält man die Tietelverbindung als Diastereomerengemisch: R_{f}(S)-=0.31; FAB-MS: (M+H)⁺= 476.

Die als Ausgangsmaterial eingesetzte Amin-Komponente wird beispielsweise folgendermassen hergestellt:
Zu einer Lösung von 2.6 g 1-Benzyl-3(R,S)-hydroxymethyl-1,2,3,4-tetrahydrochinolin in 30 ml Tetrahydrofuran werden bei 0 °C unter Rühren 2.3 g einer KaliumhydridSuspension (20% in Öl) und anschliessend 1.9 ml Methyljodid addiert. Nach 1 h wird das Reaktionsgemisch eingeengt und der Rückstand an 160 g Kieselgel mit einem 20:1-Gemisch aus Hexan und Essigester gereinigt. Man erhält das 1-Benzyl-3(R,S)-methoxymethyl-1,2,3,4-tetrahydrochinolin als gelblichen Festkörper: R_{f}(20:1-Gemisch aus Hexan und Essigester)= 0.32.
Die Lösung von 2.9 g des so erhaltenen Produktes in 30 ml Tetrahydrofuran wird in Gegenwart von 0.6 g Palladium auf Kohle unter üblichen Bedingungen hydriert. Reinigung des Rohproduktes an 80 g Kieselgel mit Methylenchlorid als Elutionsmittel ergibt das 3(R,S)-Methoxymethyl-1,2,3,4-tetrahydrochinolin: R_{f}(Methylenchlorid)= 0.32; R_{f}(A)=0.56.

### Beispiel 62: 5(S)-Formylamino-4(S)-hydroxy-2(R),7,7-trimethyl-8-[2(R,S)-methylaminocarbonyl-3,4-dihydro-2H1,4-benzoxazin-4-ylcarbonyl]-octansäure(N-butyl)amid

140 mg 5(S)-Amino-4(S)-hydroxy-2(R),7,7-trimethyl-8-[2(R,S)-methylaminocarbonyl-3,4-dihydro-2H1,4-benzoxazin-4-ylcarbonyl]-octansäure(N-butyl)amid (Beispiel 28)) werden analog wie in Beispiel 17) beschrieben mit 100 mg Ameisensäure-4-nitrophenylester umgesetzt: R_{f}(S)=0.33; FAB-MS: (M+H)⁺= 519.

### Beispiel 63: 5(S)-Amino-4(S)-hydroxy-2(R),7,7-trimethyl-8-(3-acetyl-2,3-dihydro-1H-benzimidazo1ylcarbonyl)-octansäure(N-butyl)amid

In analoger Weise wie in den Beispielen 52, 52a und 52b) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 113mg 1-Acetyl-2,3-dihydro-1H-benzimidazol, das nach der von I. Butula in Liebigs Ann. Chem.718, 260 (1968) beschriebenen Methode hergestellt wird, über das (4O,5N-Isopropyliden)-5(S)-tertiärbutoxycarbonylamino-4(S)-hydroxy-2(R),7,7-trimethyl-8-(3-acetyl-2,3-dihydro-1H-benzimidazo1-yl-carbonyl)-octansäure(N-butyl)amid (R_{f}(H)=0.46) und das 5(S)-Tertiärbutoxycarbonylamino-4(S)-hydroxy-2(R),7,7-trimethyl-8-(3-acetyl-2,3-dihydro-1H-benzimidazo1-ylcarbonyl)-octansäure(N-butyl)amid (R_{f}(L)=0.20): R_{f}(S)=0.24; Rₜ(IV)=34.4min; FAB-MS: (M+H)⁺=461.

### Beispiel 64: 5(S)-Amino-4(S)-hydroxy-2(R),7,7-trimethyl-8-[3(R,S)-ethylsulfonyl-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 178mg 3(R,S)-Ethylsulfonyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-ethylsulfonyl-1,2,3,4-tetrahydrochinolin-1ylcarbonyl]-octansäure(N-butyl)amid (Diastereomerengemisch: R_{f}(A)=0.09/0.14) und das 5(S)-Tertiärbutoxycarbonylamino-4(S)-hydroxy-2(R),7,7-trimethyl-8-[3(R,S)-ethylsulfonyl-1,2,3,4-tetrahydrochinolin-1ylcarbonyl]-octansäure(N-butyl)amid (R_{f}(A)=0.08) als Gemisch von Diastereomeren: R_{f}(S)=0.13/0.16; Rₜ(IV)=35.9/36.3min; FAB-MS: (M+H)⁺=524.

Die als Ausgangsmaterial eingesetzte Amin-Komponente wird beispielsweise folgendermassen hergestellt:
a) 3(R,S)-Ethylsulfonyl-1,2,3,4-tetrahydrochinolin
   Eine Lösung von 0.3 g 3-Ethylsulfonylchinolin in 15ml Eisessig wird in Gegenwart von 30mg Platinoxid bei 50° C über 22h hydriert. Nach Aufarbeitung analog Beispiel52c) und FC an 30 g Kieselgel (Laufmittel I) erhält man die reine Titelverbindung: Smp.88-90°C, R_{f}(K)=0.51; IR(KBr): 3380(s), 1590, 1500(m), 1270(s), 1130(s), 750(s); Anal. kalk. für C₁₁H₁₅NO₂S: C58.64%, H6.71%, N6.22%; gef. C58.95,6.75, N6.21.
b) 3-Ethylsulfonylchinolin
   Analog Beispiel52d) werden zu einem Gemisch von 1.2 g 3-Ethylthiochinolin und 14mg NatriumwolframatDihydrat in 6 ml DioxanWasser (2:1-Gemisch) und 1Tropfen Eisessig 1.3 ml einer 30%igen Wasserstoffperoxidlösung bei 65°C unter kräftigem Rühren addiert. Es wird noch 1h bei 80°C gerührt und nach dem Abkühlen wie üblich aufgearbeitet. FC an 30 g Kieselgel (LaufmittelA) ergibt die reine Titelverbindung: R_{f}(A)=0.34; IR(KBr): 1305(s), 1150 und 1130(s); Anal. kalk. für C₁₁H₁₁NO₂S: C59.71%, H5.01%, N6.33%; gef. C59.82%, H5.03%, N6.49%.
c) 3-Ethylthiochinolin
   wird ausgehend von 2.1 g 3-Bromchinolin und 4.2 g Natriumethanthiolat in 30ml Dimethylformamid analog Beispiel52e) hergestellt und mittels FC an 30 g Kieselgel mit einem 1:2-Gemisch aus Diethylether und Petrolether als Laufmittel gereinigt. Man erhält die Titelverbindung: R_{f}(F)=0.31.

### Beispiel 65: 5(S)-Amino-4(S)-hydroxy-2(R),7,7-trimethyl-8-[3(R,S)-methylcarbonyloxy-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 50mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 63mg 3(R,S)-Methylcarbonyloxy-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methylcarbonyloxy-1,2,3,4-tetrahydrochinolin-1ylcarbonyl]-octansäure(N-butyl)amid (R_{f} (A)=0.30) und das 5(S)-Tertiärbutoxycarbonylamino-4(S)-hydroxy-2(R),7,7-trimethyl-8-[3(R,S)-methylcarbonyloxy-1,2,3,4-tetrahydrochinolin-1 ylcarbonyl]-octansäure(N-butyl)amid (R_{f}(A)=0.07) als Diastereomerengemisch: R_{f}(S)-=0.29/0.31; Rₜ(IV)=18.6/18.9min; FAB-MS: (M+H)⁺=490.

Das als Ausgangsmaterial eingesetzte 3(R,S)-Methylcarbonyloxy-1,2,3,4-tetrahydrochinolin wird beispielsweise folgendermassen hergestellt
Zu einer Mischung von 2.5 g 3-Hydroxychinolin (hergestellt nach der von Mills und Watson in J.Chem.Soc., 97, 753 (1910) beschriebenen Methode), 8.6 ml Pyridin und 50ml Methylenchlorid werden unter Eiskühlung 1.35ml Acetylchlorid tropfenweise addiert. Nach 10min Rühren bei Raumtemperatur wird der Reaktionsansatz mit 50ml Wasser versetzt. Übliche Aufarbeitung und Reinigung des Rohprodukts durch FC an 50 g Kieselgel (LaufmittelC) ergibt 3-Methylcarbonyloxychinolin: R_{f}(A)=0.55; IR (CH₂Cl₂): 1770(s) cm⁻¹.

2.80 g des oben beschriebenen Esters, gelöst in 280ml Dioxan, werden in Gegenwart von 1.12 g Palladium auf Kohle (10% Palladium) bei 55°C unter Normaldruck über 2h hydriert. Das Reaktionsgemisch wird über Celite 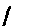 545 filtriert und eingeengt. Durch Reinigung des Rückstandes mittels FC an 80 g Kieselgel (Laufmittel F) erhält man 3(R,S)-Methylcarbonyloxy-1,2,3,4-tetrahydrochinolin: R_{f}(A)=0.65.

### Beispiel 66: 5(S)-Amino-4(S)-hydroxy-2(R),7,7-trimethyl-8-[2(R,S)-methoxycarbonyl-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin- 5(S)-yl]-2(R)-methyl-propionsäure(N-butyl)amid und 101mg 2(R,S)-Methoxycarbonyl-1,2,3,4-tetrahydrochinolin über das (4O,5N-Isopropyliden)-5(S)-tertiärbutoxycarbonylamino-4(S)-hydroxy-2(R),7,7-trimethyl-8-[2(R,S)-methoxycarbonyl-1,2,3,4-tetrahydrochinolin-1ylcarbonyl]-octansäure(N-butyl)amid (R_{f}(A)=0.35) und das 5(S)-Tertiärbutoxycarbonylamino-4(S)-hydroxy-2(R),7,7-trimethyl-8-[2(R,S)-methoxycarbonyl-1,2,3,4-tetrahydrochinolin-1ylcarbonyl]-octansäure(N-butyl)amid (R_{f}(P)=0.53) als Diastereomerengemisch: R_{f}(S)=0.23/0.30; Rₜ(IV)=38.1/39.3min; FAB-MS: (M+H)⁺=490.

Das als Ausgangsmaterial eingesetzte 2(R,S)-Methoxycarbonyl-1,2,3,4-tetrahydrochinolin wird beispielsweise wie folgt hergestellt:
Umsetzung von 10.0 g Chinaldinsäure mit 5.4 ml Methyljodid nach der in Beispiel97f) beschriebenen Methode über 20h bei Raumtemperatur und Reinigung des Rohprodukts durch FC an 80 g Kieselgel (Laufmittel C) ergibt Chinaldinsäuremethylester als weissen Festkörper: R_{f}(50:50:6-Gemisch aus Hexan, Essigester und Eisessig)=0.65.

1.0 g des obengenannten Esters in 9 ml Ethanol wird in Gegenwart von 140mg Platinoxid über 1h bei Raumtemperatur hydriert. Reinigung des Rohprodukts durch FC an 60 g Kieselgel (Laufmittel P) ergibt 2(R,S)-Methoxycarbonyl-1,2,3,4-tetrahydrochinolin: R_{f}(A)=0.80; MS: (M)⁺=191.

### Beispiel 67: 5(S)-Amino-4(S)-hydroxy-2(R),7,7-trimethyl-8-[3(R,S)-methylaminocarbonyl-1,2,3,4-tetrahydrochinoxalin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 32 mg 5(S)-Tertiärbutoxycarbonylamino-4(S)-hydroxy-2(R),7,7-trimethyl-8-[3(R,S)-methylaminocarbonyl-1,2,3,4-tetrahydrochinoxalin-1ylcarbonyl]-octansäure(N-butyl)amid durch Umsetzung in 2 ml 4N-Salzsäure in Dioxan (1 h bei 0 °C) und FC-Reinigung an 10 g Kieselgel (Laufmittel P) als DiastereomerenGemisch: R_{f}(S)= 0.07; Rₜ(IV)= 31.7/32.0 min; FAB-MS: (M+H)⁺= 490.

Die Ausgangsmaterialien werden beispielsweise folgendermassen hergestellt:
a) 5(S)-Tertiärbutoxycarbonylamino-4(S)-hydroxy-2(R),7,7-trimethyl-8-[3(R,S)-methylaminocarbonyl-1,2,3,4-tetrahydrochinoxalin-1ylcarbonyl]-octansäure(N-butyl)amid
   Die Titelverbindung wird ausgehend von 40 mg (4O,5N-Isopropyliden)-5(S)-tertiärbutoxycarbonylamino-4(S)-hydroxy-2(R),7,7-trimethyl-8-[3(R,S)-methylaminocarbonyl-1,2,3,4-tetrahydrochinoxalin-1ylcarbonyl]-octansäure(N-butyl)amid analog Beispiel 20a) erhalten: R_{f}(P)= 0.19; FAB-MS: (M+H)⁺= 590.
b) (4O,5N-Isopropyliden)-5(S)-tertiärbutoxycarbonylamino-4(S)-hydroxy-2(R),7,7-trimethyl-8-[3(R,S)-methylaminocarbonyl-1,2,3,4-tetrahydrochinoxalin-1ylcarbonyl]-octansäure(N-butyl)amid
   Die Titelverbindung wird ausgehend von 237 mg 3-[N-Tertiärbutoxycarbonyl-4(S)-(3-carboxy-2,2-dimethylpropyloxy-2,2-dimethyl-1,3-oxazolidin-5(S)-yl]-2(R)-methylpropionsäure(N-butyl)amid und 268 mg 2(R,S)-methylaminocarbonyl-1,2,3,4-tetrahydrochinoxalin analog Beispiel 1a) und Reinigung mittels FC an 30 g Kieselgel mit einem 97:3:0.5-Gemisch aus Methylenchlorid, Methanol und Ammoniak konz. als Laufmittel erhalten: R_{f}(95:5:1-Gemisch aus Methylenchlorid, Methanol und Eisessig)= 0.50; FAB-MS: (M+H)⁺= 630.
c) 2(R,S)-methylaminocarbonyl-1,2,3,4-tetrahydrochinoxalin
   0.33 g 2-(Methylaminocarbonyl)chinoxalin in 15 ml Ethanol werden in Gegenwart von 0.07 g Palladium auf Kohle (10% Pd) über 6 h bei Raumtemperatur hydriert: R_{f}(P)= 0.45.
d) 2-(Methylaminocarbonyl)chinoxalin wird ausgehend von 0.5 g 2-Chinoxaloylchlorid durch Umsetzung mit Methylaminhydrochlorid unter üblichen Reaktionsbedingungen erhalten: R_{f}(P)= 0.69.

### Beispiel 68: 5(S)-Amino-4(S)-hydroxy-2(R),7,7-trimethyl-8-[4-acetyl-3(R,S)-methylaminocarbonyl-1,2,3,4-tetrahydrochinoxalin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 38 mg (4O,5N-Isopropyliden)-5(S)-tertiärbutoxycarbonylamino-4(S)-hydroxy-2(R),7,7-trimethyl-8-[4-acetyl-3(R,S)-methylaminocarbonyl-1,2,3,4-tetrahydrochinoxalin-1ylcarbonyl]-octansäure(N-butyl)amid über das 5(S)-Tertiärbutoxycarbonylamino-4(S)-hydroxy-2(R),7,7-trimethyl-8-[4-acetyl-3(R,S)-methylaminocarbonyl-1,2,3,4-tetrahydrochinoxalin-1ylcarbonyl]-octansäure(N- butyl)amid (R_{f}(P)= 0.39) als Gemisch von Diastereomeren: R_{f}(P)= 0.07; Rₜ(IV)= 30.7/31.2 min; FAB-MS: (M+H)⁺= 532.

Das Ausgangsmaterial wird beispielsweise folgendermassen hergestellt:
40 mg (4O,5N-Isopropyliden)-5(S)-tertiärbutoxycarbonylamino-4(S)-hydroxy-2(R),7,7-trimethyl-8-[3(R,S)-methylaminocarbonyl-1,2,3,4-tetrahydrochinoxalin-1ylcarbonyl]-octansäure(N-butyl)amid (Beispiel 67b) werden zusammen mit 0.09 ml Acetanhydrid über 1 h auf 120 °C erhitzt. Anschliessend wird das Reaktionsgemisch direkt an 50 g Kieselgel mit einem 97:3:1-Gemisch aus Methylenchlorid, Methanol und Ammoniak konz. als Laufmittel chromatographiert. Man erhält die Titelverbindung: R_{f}(97:5:1-Gemisch aus Methylenchlorid, Methanol und Ammoniak konz.)= 0.55; FAB-MS: (M+H)⁺= 672.

### Beispiel 69: 5(S)-Amino-4(S)-hydroxy-2(R),7,7-trimethyl-8-[3(R,S)-methylaminocarbonyl-1,2,3,4-tetrahydrochinolin-1ylcarbonyl]-octansäure(N-butyl)amid

Analog wie in den Beispielen 1) und 1a) für die entsprechenden Reaktionsstufen beschrieben erhält man die Titelverbindung ausgehend von 300 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 570 mg 3(R,S)-methylaminocarbonyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methylaminocarbonyl-1,2,3,4-tetrahydrochinolin-1-ylcarbonyl]-octansäure(N-butyl)amid (R_{f}(Essigester)= 0.36; R_{f}(N)= 0.27) und Reinigung an 80 g Kieselgel (Laufmittel S) als Diastereomerengemisch: R_{f}(S)= 0.21; FAB-MS: (M+H)⁺= 489.

Die eingesetzte Amin-Komponente wird hergestellt ausgehend von 1.2 g 3(R,S)-Methoxycarbonyl-1,2,3,4-tetrahydrochinolin und Umsetzung in 10 ml einer 3N-MethylaminLösung in Dimethylformamid über 30 h bei 50 °C. Reinigung des Rohprodukts nach Einengen des Reaktionsansatzes mittels FC an Kieselgel (Laufmittel N) ergibt das 3(R,S)-methylaminocarbonyl-1,2,3,4-tetrahydrochinolin als weissen Festkörper: R_{f}(N)= 0.25.

### Beispiel 70: 5(S)-Amino-4(S)-hydroxy-2(S)-isopropyl-7,7-dimethyl-8-[2(R,S)-methyaminocarbonyl-3,4-dihydro-2H-1,4-benzoxazin-4-ylcarbonyl]-octansäure(N-butyl)amid

Analog wie in den Beispielen 21, 21a) und 21b) für die entsprechenden Reaktionsstufen beschrieben erhält man die Titelverbindung ausgehend von 300 mg 3-[N-Tertiärbutoxycarbonyl-4(S)-(3-carboxy-2,2-dimethylpropyloxy-2,2-dimethyl-1,3-oxazolidin-5(S)-yl]-2(S)isopropyl-propionsäure(N-butyl)amid und 240 mg 2(R,S)-methylaminocarbonyl-3,4-dihydro-2H-1,4-benzoxazin über das (4O,5N-Isopropyliden)-5(S)-tertiärbutoxycarbonylamino-4(S)-hydroxy-2(S)-isopropyl-7,7-dimethyl-8-[(R,S)-methylaminocarbonyl-3,4-dihydro-2H-1,4-benzoxazin-4-ylcarbonyl]-octansäure(N-butyl)amid (R_{f}(X)= 0.45) und das 5(S)-Tertiärbutoxycarbonylamino-4(S)-hydroxy-2(S)-isopropyl-7,7dimethyl-8-[2(R,S)-methylaminocarbonyl-3,4-dihydro-2H-1,4-benzoxazin-4-ylcarbonyl]-octansäure(N-butyl)amid (R_{f}(X)= 0.28) als Diastereomerengemisch: R_{f}(V)= 0.55; Rₜ(I)= 23.2 min; FAB-MS: (M+H)⁺= 519.

### Beispiel 71: 5(S)-Amino-4(S)-hydroxy-2(R),7,7-trimethyl-8-[2(R,S)-methoxyethylaminocarbonyl-3,4-dihydro-2H-1,4-benzoxazin-4-ylcarbonyl]-octansäure(N-butyl)amid

Analog wie in den Beispielen 21, 21a) und 21b) für die entsprechenden Reaktionsstufen beschrieben erhält man die Titelverbindung ausgehend von 205 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 237 mg 2(R,S)-Methoxyethylaminocarbonyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-methoxyethylaminocarbonyl-3,4-dihydro-2H-1,4-benzoxazin-4-ylcarbonyl]-octansäure(N-butyl)amid (analog Beispiel 1a): R_{f}(A)= 0.30) und das 5(S)-Tertiärbutoxycarbonylamino-4(S)-hydroxy-2(R),7,7-trimethyl-8-[2(R,S)-methoxyethylaminocarbonyl-3,4-dihydro-2H-1,4-benzoxazin-4-ylcarbonyl]-octansäure(N-butyl)amid (R_{f}(P)= 0.53; FAB-MS: (M+H)⁺= 635) analog wie in Beispiel 1) beschrieben als Diastereomerengemisch: R_{f}(V)= 0.42; Rₜ(I)= 18.0 min; FAB-MS: (M+H)⁺= 535.

Die als Ausgangsmaterial eingesetzte Amin-Komponente wird beispielsweise folgendermassen hergestellt:
Zu einer Lösung von 0.92 ml 2-Methoxyethylamin in 30 ml Methylenchlorid wird bei Raumtemperatur 5.3 ml einer 2 M Trimethylaluminiumlösung in Hexan addiert. Das Reaktionsgemisch wird 1 h bei 25 °C gerührt und dann innerhalb von 15 min eine Lösung von 1.22 g 2(R,S)-Ethoxycarbonyl-3,4-dihydro-2H-1,4-benzoxazin in 20 ml Methylenchlorid zugetropft. Nach 16 h Rühren bei 40 °C wird das Reaktionsgemisch mit Wasser versetzt und mit Chloroform extrahiert. Man erhält das 2(R,S)-Methoxyethylaminocarbonyl-3,4-dihydro-2H-1,4-benzoxazin durch Umkristallisation aus Essigester/Hexan: R_{f}(P)= 0.59.

### Beispiel 72: 5(S)-Amino-4(S)-hydroxy-2(R),7(S)-dimethyl-8-[2(R,S)-ethoxycarbonyl-3,4-dihydro-2H1,4-benzoxazin-4-ylcarbonyl]-octansäure(N-butyl)amid

Unter Eiskühlung werden 40mg 5(S)-Tertiärbutoxycarbonylamino-4(S)-hydroxy-2(R),7(S)-dimethyl-8-[2(R,S)-ethoxycarbonyl-3,4-dihydro-2H1,4-benzoxazin-4-ylcarbonyl]-octansäure(N-butyl)amid mit 2 ml einer 25%igen Lösung von Trifluoressigsäure in Methylenchlorid versetzt. Es wird bei 0°C weitergerührt. Nach beendeter Reaktion (DC-Kontrolle) wird zum Ansatz 1 ml Toluol addiert, das Lösungsmittel im Vakuum rasch abgedampft und der ölige Rückstand direkt mittels FC an 10 g Kieselgel (Laufmittel P) gereinigt. Man erhält die Titelverbindung als Diastereomerengemisch: R_{f}(S)=0.27; Rₜ(IV)=35.0/35.2min; FAB-MS: (M+H)⁺=478.

Die Ausgangsmaterialien werden beispielsweise folgendermassen hergestellt:
a) 5(S)-Tertiärbutoxycarbonylamino-4(S)-hydroxy-2(R),7(S)-dimethyl-8-[2(R,S)-ethoxycarbonyl-3,4-dihydro-2H1,4-benzoxazin-4-ylcarbonyl]-octansäure(N-butyl)amid
   Die Titelverbindung wird analog wie in Beispiel21a) beschrieben ausgehend von 114mg (4O,5N-Isopropyliden)-5(S)-tertiärbutoxycarbonylamino-4(S)-hydroxy-2(R),7(S)-dimethy-8-[2(R,S)-ethoxycarbonyl-3,4-dihydro-2H1,4-benzoxazin-4-ylcarbonyl]-octansäure(N-butyl)amid erhalten und mittels FC an 25 g Kieselgel (LaufmittelA und B) gereinigt: R_{f}(A)=0.15; FAB-MS: (M+H)⁺=578.
b) (4O,5N-Isopropyliden)-5(S)-tertiärbutoxycarbonylamino-4(S)-hydroxy-2(R),7(S)-dimethyl-8-[2(R,S)-ethoxycarbonyl-3,4-dihydro-2H1,4-benzoxazin-4-ylcarbonyl]-octansäure(N-butyl)amid
   Zu einer Lösung von 90mg 3-[N-Tertiärbutoxycarbonyl-4(S)-[3-carboxy-2(S)-methylpropyl]-2,2-dimethyl-1,3-oxazolidin-5(S)-yl]-2(R)-methyl-propionsäure(N-butyl)amid in 2 ml Methylenchlorid werden unter Stickstoff bei 0°C 0.07ml 1ChlorN,N,2trimethylpropenylamin addiert und 30min nachgerührt. Dann werden 157mg 2(R,S)Ethoxycarbonyl-3,4-dihydro-2H-1,4-benzoxazin, in wenig Methylenchlorid gelöst, und 4-DMAP hinzugefügt. Nach Aufwärmen auf Raumtemperatur wird noch 5h weitergerührt und anschliessend das Reaktionsgemisch direkt über 80 g Kieselgel mit einem Elutionsmittelgradienten von E nach A chromatographiert. Man erhält die reine Titelverbindung als Diastereomerengemisch: R_{f}(A)=0.30/0.40.
c) 3-[N-Tertiärbutoxycarbonyl-4(S)-[3-carboxy-2(S)-methylpropyl]-2,2-dimethyl-1,3-oxazolidin-5(S)-yl]-2(R)-methyl-propionsäure(N-butyl)amid
   Eine Lösung von 311mg 3-[N-Tertiärbutoxycarbonyl-4(S)[4-hydroxy-2(S)-methylbutyl]-2,2-dimethyl-1,3-oxazolidin-5(S)-yl]-2-(R)-methyl-propionsäure(N-butyl)amid in einem Gemisch aus 4.8 ml Tetrachlorkohlenstoff und 4.8 ml Acetonitril wird unter kräftigem Rühren zu einer Mischung aus 928mg Natriummetaperjodat, 19mg Ruthenium(III)-chloridHydrat und 0.6 ml Wasser addiert. Nach 1h wird mit 30ml Methylenchlorid und 5 ml Isopropanol verdünnt, die wäßrige Phase abgetrennt und mit Methylenchlorid extrahiert. Die vereinigte organische Phase wird eingeengt, in 10ml Toluol aufgenommen und erneut eingeengt. Der dunkel gefärbte Rückstand wird durch FC an 25 g Kieselgel mit einem 50:50:1-Gemisch aus Hexan, Essigester und Eisessig gereinigt: R_{f}(B)=0.18; FAB-MS: (M+H)⁺=443.
d) 3-[N-Tertiärbutoxycarbonyl-4(S)-[4-hydroxy-2(S)-methylbutyl]-2,2-dimethyl-1,3-oxazolidin-5(S)-yl]-2(R)-methyl-propionsäure(N-butyl)amid
   475mg 3-[N-Tertiärbutoxycarbonyl-4(S)[4triisopropylsilyloxy-2(S)-methylbutyl]-2,2-dimethyl-1,3-oxazolidin-5(S)-yl]-2(R)-methyl-propionsäure(N-butyl)amid, in 12ml Tetrahydrofuran gelöst, werden zusammen mit 1.63ml einer 1M Tetrabutylammoniumfluoridlösung in Tetrahydrofuran bei Raumtemperatur über 3 h gerührt. Die organische Phase wird mit gesättigter Natriumchloridlösung gewaschen, über Natriumsulfat getrocknet und eingeengt. Das Rohprodukt wird durch FC an 50 g Kieselgel (LaufmittelH) gereinigt. Man erhält die Titelverbindung als reines Diastereomer: R_{f}(H)=0.48.
e) 3-[N-Tertiärbutoxycarbonyl-4(S)-[4triisoproylsilyloxy-2(S)-methylbutyl]-2,2-dimethyl-1,3-oxazolidin-5(S)-yl]-2(R)-methyl-propionsäure(N-butyl)amid
   Die Titelverbindung wird analog wie in Beispiel 1d) beschrieben ausgehend von 450mg 5(S)-Tertiärbutoxycarbonylamino-4(S)-hydroxy-9triisopropylsilyloxy-2(R),7(S)-dimethylnonansäure(N-butyl)amid hergestellt: R_{f}(A)=0.63.
f) 5(S)-Tertiärbutoxycarbonylamino-4(S)-hydroxy-9triisopropylsilyloxy-2(R),7(S)-dimethylnonansäure(N-butyl)amid
   550 mg 5(S)-Tertiärbutoxycarbonylamino-4(S)-hydroxy-7(S)-methyl-2-methylen9triisopropylsilyloxynonansäure(N-butyl)amid, in 30 ml wasserfreiem Methanol gelöst, werden unter Argon bei einem Druck von 25 bar in Gegenwart von 10 mg Bis[(S)-(-)-2,2'-bis(diphenylphosphino)-1,1'-binaphtyl](trimethylamino)dirutheniumtetrachlorid bis zur Vollständigkeit der Reaktion hydriert. Das Reaktionsgemisch wird eingeengt und das so erhaltene Rohprodukt an 25 g Kieselgel (Laufmittel A) gereinigt. Man erhält die Titelverbindung: R_{f}(A)=0.34.
g) 5(S)-Tertiärbutoxycarbonylamino-4(S)-hydroxy-7(S)-methyl-2-methylen9triisopropylsilyloxynonansäure(N-butyl)amid
   Zu einer Lösung von 2.7 g Methacrylsäurebutylamid in 80ml Tetrahydrofuran werden bei -75°C 23.9 ml einer 1.6Mn-Butyllithiumlösung in Hexan über 30min unter Argon addiert. Anschliessend wird das Reaktionsgemisch für 30min bei 0°C gerührt. Zu der klaren Lösung werden nach Abkühlen auf -75°C 29.0ml einer 1.0MChlortriisopropyloxytitanLösung in Hexan über 30min hinzugetropft, das dunkel gefärbte Reaktionsgemisch für weitere 15min bei -75°C gerührt und anschliessend eine Lösung von 3.1 g 2(S)-Tertiärbutoxycarbonylamino-4(S)-methyl6triisopropylsilyloxyhexanal in 18ml Tetrahydrofuran während 10min addiert. Nach 1h Rühren bei -75°C wird tropfenweise mit 19ml gesättigter Ammoniumchloridlösung versetzt und die weisse Suspension nach Aufwärmen auf Raumtemperatur mit Diethylether extrahiert. Die organische Phase wird mit 25ml Wasser und 25ml gesättigter Natriumchloridlösung gewaschen, über Natriumsulfat getrocknet und eingeengt. Das Rohprodukt wird mittels FC an 400 g Kieselgel (Laufmittelgradient von D zu B) unter Trennung der Stereoisomeren gereinigt. Man erhält die Titelverbindung (Diastereomer I): R_{f}(A)=0.53; sowie Diastereomer II: R_{f}(A)=0.45.
h) 2(S)-Tertiärbutoxycarbonylamino-4(S)-methyl6triisopropylsilyloxyhexanal
   Die Lösung von 3.35 g 2(S)-Tertiärbutoxycarbonylamino-4(S)-methyl6triisopropylsilyloxyhexansäuremethylester in 44ml Toluol wird unter Argon auf -75°C gekühlt, und anschliessend werden 12.9 ml einer 20%igen Lösung von Diisobutylaluminiumhydrid in Toluol über 30min hinzugetropft. Es wird noch 45min bei -75°C gerührt. Die Reaktion wird anschliessend durch rasche Zugabe von 2.8 ml Methanol gequencht und der Ansatz auf 50ml einer halbgesättigten wäßerigen KaliumNatriumtartratLösung gegossen. Es wird mit Diethylether extrahiert, die vereinigten organischen Phasen über Natriumsulfat getrocknet und eingeengt. Die Titelverbindung (R_{f}(E)=0.34) wird als Rohprodukt direkt weiter umgesetzt.
i) 2(S)-Tertiärbutoxycarbonylamino-4(S)-methyl6triisopropylsilyloxyhexansäuremethylester
   Zu einer Mischung aus 2.63 g 2(S)-Amino-4(S)-methyl6triisopropylsilyloxyhexansäuremethylester in 11ml Dioxan und 5.5 ml Wasser werden bei 0°C unter Rühren 2.40 g Ditertiärbutyldicarbonat addiert. Anschliessend läßt man auf Raumtemperatur aufwärmen und rührt über Nacht weiter. Nach Entfernen des Dioxan unter Vakuum wird die verbleibende wäßrige Phase durch Zugabe von 1 M KaliumhydrogensulfatLösung auf pH=2 gebracht und mit Essigester extrahiert. Die organische Phase wird mit einer 5%igen NatriumhydrogencarbonatLösung und mit gesättigter Natriumchloridlösung gewaschen, über Natriumsulfat getrocknet und eingeengt. Das Rohprodukt wird durch FC an 400 g Kieselgel (ElutionsmittelGradienten von J zu L) gereinigt. Man erhält die Titelverbindung: R_{f}(C)=0.63.
j) 2(S)-Amino-4(S)-methyl6triisopropylsilyloxyhexansäuremethylester
   Die Lösung von 10.0 g 3,6-Dihydro3(S)-[4triisoproylsilyloxy-2(S)-methylbutyl]6(R)-isopropyl-2,5dimethoxypyrazin in 100ml Tetrahydrofuran wird mit 100ml 0.5N Salzsäure versetzt und 40min bei Raumtemperatur gerührt. Das Reaktionsgemisch wird durch Zugabe von 1N Natriumhydroxidlösung auf pH 9 gebracht und anschliessend mit Methylenchlorid extrahiert. Die vereinigte organische Phase wird durch Watte filtriert und eingeengt. Das Rohprodukt wird durch FC an 900 g Kieselgel mit einem 500:10:1-Gemisch aus Methylenchlorid, Methanol und 25%iger AmmoniakLösung gereinigt. Neben zurückisoliertem Ausgangsmaterial erhält man die Titelverbindung: R_{f}(MethylenchloridMethanolAmmoniak konz. (140:10:1))=0.34.
k) 3,6-Dihydro3(S)-[4triisopropylsilyloxy-2(S)-methylbutyl]6(R)-isopropyl-2,5dimethoxypyrazin
   Unter Argon werden zu einer Mischung von 10.2 ml 2(R)-2,5Dihydro3,6dimethoxy-2isopropylpyrazin in 15ml Tetrahydrofuran bei -75°C bis -65°C langsam 34.1 ml einer 1.6M n-Butyllithiumlösung in Hexan zugetropft. Anschliessend wird das Reaktionsgemisch weitere 30min bei -75°C gerührt und dann eine auf -75°C gekühlte Lösung von 16.6 g 4-Triisopropylsilyloxy-2(R)-methylbutylbromid in 70ml Tetrahydrofuran über eine Kanüle zugetropft. Man läßt das Reaktionsgemisch auf -20°C aufwärmen, rührt weitere 2h bei dieser Temperatur und bringt schliesslich den Ansatz innerhalb 1h auf Raumtemperatur. Nach üblicher Aufarbeitung wird das Rohprodukt durch FC an 900 g Kieselgel (Laufmittel J) gereinigt. Man erhält die Titelverbindung als reines Diastereomer: R_{f}(G)=0.73.
l) 4-Triisopropylsilyloxy-2(R)-methylbutylbromid
   Zu einer Lösung von 8.7 g (R)-4Brom3-methylbutan-1ol in 320ml Methylenchlorid werden bei 0°C 15.4 ml Triisopropylsilyltriflat und dann während 30min 6.6 ml 2,6-Lutidin addiert. Das Reaktionsgemisch wird anschliessend auf 100ml Eiswasser gegossen und die wäßrige Phase durch Zugabe von 2N Salzsäure angesäuert (pH 3). Es wird mit Methylenchlorid extrahiert, die organische Phase mit einer 5%igen NatriumhydrogencarbonatLösung und gesättigter Natriumchloridlösung gewaschen und über Natriumsulfat getrocknet. Nach Reinigung des Rohprodukts durch FC an 200 g Kieselgel (Laufmittel J) erhält man die Titelverbindung: R_{f}(J)=0.48.
m) (R)-4-Brom-3-methylbutan-1-ol
   Die Titelverbindung wird ausgehend von 18.8 g (R)-4-Brom-3-methylbuttersäureethylester durch Reduktion mit Dibutylaluminiumhydrid nach dem von Schmid und Barner in Helv. Chim. Acta, 62, 464 (1979) beschriebenen Verfahren hergestellt und mittels FC an 200 g Kieselgel mit einem Laufmittelgradienten aus Hexan und Diethylether (von einem 9:1-Gemisch zu einem 3:1-Gemisch) gereinigt: R_{f}(C)=0.24.
n) (R)-4-Brom-3-methylbuttersäureethylester
   Ausgehend von 11.7 g (R)-3-Methylg-butyrolakton, das nach der von Mattes et al. in J. Med. Chem., 30, 1948 (1987) beschriebenen Methode hergestellt wird, wird analog dem für die Herstellung des 3(S)-Enantiomeren angegebenen Verfahren von Schmid und Bauer (Helv. Chim. Acta, 62, 464 (1979)) und anschliessender Reinigung mittels FC an 100 g Kieselgel mit einem 9:1-Laufmittelgemisch aus Hexan und Diethylether die Titelverbindung erhalten: R_{f}(C)=0.77.

### Beispiel 73: 5(S)-Formylamino-4(S)-hydroxy-2(R),7,7-trimethyl-8-[2(R,S)-methylaminocarbonyl-3,4-dihydro-2H-1,4-benzthiazin-4-ylcarbonyl]-octansäure(N-butyl)amid

Ausgehend von 100 mg 5(S)-Amino-4(S)-hydroxy-2(R),7,7-trimethyl-8-[2(R,S)-methylaminocarbonyl-3,4-dihydro-2H-1,4-benzthiazin-4-ylcarbonyl]-octansäure(N-butyl)amid (Beispiel 27)) erhält man nach der im Beispiel 47) beschriebenen Methode und anschliessender Reinigung durch FC an 30 g Kieselgel (Laufmittel S) als Diastereomerengemisch: R_{f}(S)= 0.24; FAB-MS: (M+H)⁺= 535.

### Beispiel 74: 5(S)-Amino-2(R)-benzyl-4(S)-hydroxy-7,7-dimethyl-8-[3(R)- oder-3(S)-methoxycarbonylamino-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 80 mg 3-[N-Tertiärbutoxycarbonyl-4(S)(3-carboxy-2,2-dimethylpropyloxy-2,2-dimethyl-1,3-oxazolidin-5(S)-yl]-2(R)-benzyl-propionsäure(N-butyl)amid und 77 mg 3(R)-oder 3(S)-Methoxycarbonylamino-1,2,3,4-tetrahydrochinolin (Stereoisomer II, Beispiel 74h)) über das (4O,5N-Isopropyliden)-5(S)-tertiärbutoxycarbonylamino-2(R)-benzyl-4(S)-hydroxy-7,7-dimethyl-8-[3(R)- oder 3(S)-methoxycarbonylamino-1,2,3,4-tetrahydrochinolin-1ylcarbonyl]-octansäure(N-butyl)amid (R_{f}(L)= 0.27; FAB-MS: (M+H)⁺= 721) und das 5(S)-Tertiärbutoxycarbonylamino-2(R)-benzyl-4(S)-hydroxy-7,7dimethyl-8-[3(R)- oder 3(S)-methoxycarbonylamino-1,2,3,4-tetrahydrochinolin-1ylcarbonyl]-octansäure(N-butyl)amid (R_{f}(L)= 0.26; FAB-MS: (M+H)⁺= 681) analog Beispiel 92) als reines Diastereomer: R_{f}(P)= 0.39; Rₜ(IV)= 41.8 min; FAB-MS: (M+H)⁺= 581.

Die Ausgangsmaterialien werden beispielsweise folgendermassen hergestellt:
a) 3-[N-Tertiärbutoxycarbonyl-4(S)(3-carboxy-2,2-dimethylpropyloxy-2,2-dimethyl-1,3-oxazolidin-5(S)-yl]-2(R)-benzyl-propionsäure(N-butyl)amid
   576 mg 3-[N-Tertiärbutoxycarbonyl-4(S)(4-benzyloxy-2,2-dimethylbutyloxy-2,2-dimethyl-1,3-oxazolidin-5(S)-yl]-2(R)-benzyl-propionsäure(N-butyl)amid in 10 ml Essigesterwerden in Gegenwart von Palladium auf Kohle (5% Pd) hydriert und das Rohprodukt an Kieselgel gereinigt: R_{f}(2:1-Gemisch aus Essigester und Hexan)= 0.44; FAB-MS: (M+H)⁺= 519. Die weitere Umsetzung analog wie in Beispiel 1b) beschrieben ergibt die Titelverbindung: R_{f}(P)= 0.55; FAB-MS: (M+H)⁺= 533.
b) 3-[N-Tertiärbutoxycarbonyl-4(S)(4-benzyloxy-2,2-dimethylbutyloxy-2,2-dimethyl-1,3-oxazolidin-5(S)-yl]-2(R)-benzyl-propionsäure(N-butyl)amid
   Eine Mischung aus 580 mg 2(R)-Benzyl9benzyloxy5(S)-tertiärbutoxycarbonylamino-4(S)-hydroxy-7,7-dimethyl-nonansäure(N-butyl)amid und 13 mg p-Toluolsulfonsäure in 3 ml 2,2-Dimethoxypropan und 6 ml Methylenchlorid wird bei Raumtemperatur über 2 h gerührt. Uebliche Aufarbeitung ergibt die Titelverbindung: R_{f}(B)= 0.60; FAB-MS: (M+H)⁺= 609.
c) 2(R)-Benzyl9benzyloxy5(S)-tertiärbutoxycarbonylamino-4(S)-hydroxy-7,7-dimethyl-nonansäure(N-butyl)amid
   Die Lösung von 502 mg 3(R)-Benzyl5(S)-[5-benzyloxy-1(S)-tertiärbutoxycarbonylamino-3,3-dimethyl-pentyl]-2-oxo-tetrahydrofuran in 8.5 ml Butylamin wird während 20 h bei Raumtemperatur gerührt, eingeengt und der Rückstand an Kieselgel (Laufmittel A) chromatographisch gereinigt: R_{f}(A)= 0.47; FAB-MS: (M+H)⁺= 569.
d) 3(R)-Benzyl5(S)-[5-benzyloxy-1(S)-tertiärbutoxycarbonylamino-3,3-dimethyl-pentyl]-2-oxo-tetrahydrofuran
   Die Lösung von 1.76 g 5(S)-[5-Benzyloxy-1(S)-tertiärbutoxycarbonylamino-3,3-dimethyl-pentyl]-2-oxo-tetrahydrofuran in 30 ml Tetrahydrofuran wird unter Argon bei -78 °C zu einem Gemisch aus 9.14 ml einer 1 M Lithiumbis-(trimethylsilyl)amid-Lösung in Tetrahydrofuran und 30 ml Tetrahydrofuran addiert und 30 min gerührt. Anschliessend werden 0.63 ml Benzylbromid in 30 ml Tetrahydrofuran tropfenweise addiert. Nach 3.5 h Rühren bei -78 °C wird die Reaktion durch Zugabe von 1.6 ml Propionsäure gequencht und mit Diethylether verdünnt. Die organische Phase wird mit einer 10%igen wäßerigen Citronensäure-Lösung und einer 1 M NatriumbicarbonatLösung gewaschen, über Natriumsulfat getrocknet und eingeengt. Reinigung an Kieselgel (laufmittel D) ergibt die Titelverbindung: R_{f}(D)= 0.23; FAB-MS: (M+H)⁺= 496.
e) 5(S)-[5-Benzyloxy-1(S)-tertiärbutoxycarbonylamino-3,3-dimethyl-pentyl]-2-oxo-tetrahydrofuran
   5.52 g des als Rohprodukt erhaltenen 9-Benzyloxy5(S)-tertiärbutoxycarbonylamino-4(R,S)-hydroxy-7,7-dimethyl-nonansäureethylester werden in 100 ml Toluol und 4 ml Eisessig unter Argon über 4.5 h bei 110 °C rückflussiert. Nach dem Abkühlen des Reaktionsansatzes wird mit Essigester verdünnt und wie üblich aufgearbeitet. Das Rohprodukt wird durch Chromatographie an Kieselgel (Laufmittel B) unter Auftrennung der beiden 5(S)- und 5(R)-Stereoisomeren gereinigt. Man erhält die reine Titelverbindung (Diastereomer I) als gelbliches Öl: R_{f}(B)= 0.26; FAB-MS: (M+H)⁺= 406; sowie Diastereomer II: R_{f}(B)= 0.22.
f) 9-Benzyloxy5(S)-tertiärbutoxycarbonylamino-4(R,S)-hydroxy-7,7-dimethylnonansäureethylester
   Die Lösung von 5.85 g 9-Benzyloxy5(S)-tertiärbutoxycarbonylamino-4(R,S)-hydroxy-7,7-dimethyl-non-2-insäureethylester in 100 ml Tetrahydrofuran wird in Gegenwart von 1.2 g Platin auf Kohle (5% Pt; nach 24 h werden weitere 1.2 g Katalysator addiert) bei Raumtemperatur unter Normaldruck über 33 h hydriert. Filtration des Reaktionsansatzes über Hyflo® und Einengen des Filtrats ergibt die rohe Titelverbindung als gelbes Öl: R_{f}(B)=0.32; FAB-MS: (M+H)⁺= 452.
g) 9-Benzyloxy5(S)-tertiärbutoxycarbonylamino-4(R,S)-hydroxy-7,7-dimethyl-non-2-insäureethylester
   Zu einer Lösung von 5.1 ml Diisopropylamin in 40 ml Tetrahydrofuran werden unter Argon bei -78 °C 22.2 ml einer 1.6 M n-Butyllithiumlösung in Hexan unter Rühren addiert. Nach 30 min wird via Kanüle eine auf -78 °C gekühlte Lösung von 2.5 ml Ethylpropiolat in 20 ml Tetrahydrofuran tropfenweise addiert. Die gelbe Reaktionslösung wird noch 1 h bei -78 °C gerührt und anschliessend 5.0 g 6-Benzyloxy-2(S)-tertiärbutoxycarbonylamino-4,4-dimethylhexanal (Beispiel 39f) in 30 ml Tetrahydrofuran zugetropft. Nach Rühren über 4.5 h wird die Reaktion durch Zugabe von gesättigter wäßriger Ammoniumchloridlösung gequencht, der Reaktionsansatz auf Raumtemperatur aufwärmen gelassen und in üblicher Weise aufgearbeitet. Das Rohprodukt wird durch FC an Kieselgel (Elutionsmittel C) gereinigt. Man erhält die Titelverbindung als ein Gemisch der 4(S)- und 4(R)-Diastereomeren im Verhältnis von ca.(5.4:1): R_{f}(C)=0.28; FAB-MS: (M+H)⁺= 448.
h) 3(R)- oder 3(S)-Methoxycarbonylamino-1,2,3,4-tetrahydrochinolin
   Die Lösung von 6.54 g 3-(Methoxycarbonylamino)chinolin in 240 ml Ethanol wird in Gegenwart von 0.68 g Palladium auf Kohle (10% Pd) bei 50 °C über 20 h unter Normaldruck hydriert. Nach üblicher Aufarbeitung wird das Rohprodukt an 400 g Kieselgel mit einem 8:1-Gemisch aus Methylenchlorid und Diethylether als Laufmittel chromatographiert. Man erhält das 3(R,S)-Methoxycarbonylamino-1,2,3,4-tetrahydrochinolin als kristallinen Festkörper: R_{f}(P)= 0.84; MS: M⁺= 206.
   1.0 g des so erhaltenen Produkts wird an einer Chiralcel^{R} OD-Prep-Säule (500 x 50 mm) mit einem 8:2-Gemisch aus Heptan und Propanol als Elutionsmittel chromatographiert. Man erhält die Titelverbindung in Form der beiden reinen Stereoisomere als kristalline Festkörper: Stereoisomer I: [α]^{DRT}= + 28.7 (c=1; Methylenchlorid); Stereoisomer II: [α]^{DRT}= - 28.5 (c=1; Methylenchlorid).
i) 3-(Methoxycarbonylamino)chinolin
   Zu 5.0 g 3-Aminochinolin in 30 ml Methylenchlorid und 15 ml Pyridin werden bei 0 °C 3.2 ml Chlorameisensäuremethylester über 30 min zugetropft. Es wird noch 30 min nachgerührt und anschliessend in üblicher Weise aufgearbeitet. Man erhält die Titelverbindung als kristallinen Festkörper: R_{f}(H)= 0.41.

### Beispiel 75: 5(S)-Amino-2(R)-butyl-4(S)-hydroxy-7,7-dimethyl-8-[3(R)- oder-3(S)-methoxycarbonylamino-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 81 mg 3-[N-Tertiärbutoxycarbonyl-4(S)(3-carboxy-2,2-dimethylpropyloxy-2,2-dimethyl-1,3-oxazolidin-5(S)-yl]-2(R)-butyl-propionsäure(N-butyl)amid und 83 mg 3(R)-oder 3(S)-Methoxycarbonylamino-1,2,3,4-tetrahydrochinolin (Stereoisomer II, Beispiel 74h)) über das (4O,5N-Isopropyliden)-5(S)-tertiärbutoxycarbonylamino-2(R)-butyl-4(S)-hydroxy-7,7dimethyl-8-[3(R)- oder 3(S)-methoxycarbonylamino-1,2,3,4-tetrahydrochinolin-1ylcarbonyl]-octansäure(N-butyl)amid (R_{f}(L)= 0.32; FAB-MS: (M+H)⁺= 687) und das 5(S)-Tertiärbutoxycarbonylamino-2(R)-butyl-4(S)-hydroxy-7,7dimethyl-8-[3(R)- oder 3(S)-methoxycarbonylamino-1,2,3,4-tetrahydrochinolin-1ylcarbonyl]-octansäure(N-butyl)amid (R_{f}(L)= 0.15; FAB-MS: (M+H)⁺= 647) analog Beispiel 92) als reines Diastereomer: R_{f}(P)= 0.48; FAB-MS: (M+H)⁺= 547.

Die Ausgangsmaterialien werden beispielsweise folgendermassen erhalten:
a) 3-[N-Tertiärbutoxycarbonyl-4(S)(3-carboxy-2,2-dimethylpropyloxy-2,2-dimethy-1,3-oxazolidin-5(S)-yl]-2(R)-butyl-propionsäure(N-butyl)amid
   Die Titelverbindung erhält man analog wie in Beispiel 74a) ausgehend von 338 mg 3-[N-Tertiärbutoxycarbonyl-4(S)(4-hydroxy-2,2-dimethylbutyloxy-2,2-dimethyl-1,3-oxazolidin-5(S)-yl]-2(R)-butyl-propionsäure(N-butyl)amid: R_{f}(P)= 0.77; FAB-MS: (M+H)⁺= 499.
b) 3-[N-Tertiärbutoxycarbonyl-4(S)(4-hydroxy-2,2-dimethylbutyloxy-2,2-dimethyl-1,3-oxazolidin-5(S)-yl]-2(R)-butyl-propionsäure(N-butyl)amid
   Analog den Beispielen 74b und 74c) erhält man die Titelverbindung ausgehend von 455 mg 3(R)-But-2-enyl-5(S)-[5-benzyloxy-1(S)-tertiärbutoxycarbonylamino-3,3-dimethyl-pentyl]-2-oxo-tetrahydrofuran das 3-[N-Tertiärbutoxycarbonyl-4(S)(4-benzyloxy-2,2-dimethylbutyloxy-2,2-dimethyl-1,3-oxazolidin-5(S)-yl]-2(R)-but-2-enyl-propionsäure(N-butyl)amid (471 mg; R_{f}(C)= 0.32; FAB-MS: (M+H)⁺= 573), das anschliessend in 10 ml Essigester in Gegenwart von Palladium auf Kohle (5 % Pd) über 1 h bei Raumtemperatur zur Titelverbindung hydriert wird: R_{f}(A)= 0.32; FAB-MS: (M+H)⁺= 485.
c) 3(R)-But-2-enyl-5(S)-[5-benzyloxy-1(S)-tertiärbutoxycarbonylamino-3,3-dimethyl-pentyl]-2-oxo-tetrahydrofuran
   Die Titelverbindung wird ausgehend von 546 mg 5(S)-[5-Benzyloxy-1(S)-tertiärbutoxycarbonylamino-3,3-dimethyl-pentyl]-2-oxo-tetrahydrofuran und 0.17 ml Crotylbromid nach dem in Beispiel 74d) beschriebenen Verfahren erhalten: R_{f}(D)= 0.32; FAB-MS: (M+H)⁺= 460.

### Beispiel 76: 5(S)-Amino-4(S)-hydroxy-2(R),7,7-trimethyl-8-[3(R,S)-1,2,3,4-tetrahydrochinolin-1-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 150 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 181 mg 3(R,S)-methoxymethylcarbonylamino-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxymethylcarbonylamino-1,2,3,4-tetrahydrochinolin-1-ylcarbonyl]-octansäure(N-butyl)amid (R_{f}(P)= 0.58) und das 5(S)-Tertiärbutoxycarbonylamino-4(S)-hydroxy-2(R),7,7-trimethyl-8-[3(R,S)-methoxymethylcarbonylamino-1,2,3,4-tetrahydrochinolin-1-ylcarbonyl]- octansäure(N-butyl)amid (R_{f}(P)=0.49) analog wie in Beispiel 92) beschrieben als Diastereomerengemisch: R_{f}(S)=0.17; Rₜ(V)=14.7 min; FAB-MS: (M+H)⁺= 519.

Das 3(R,S)-methoxymethylcarbonylamino-1,2,3,4-tetrahydrochinolin erhält man aus 3(R,S)-Amino1,2,3,4-tetrahydrochinolin durch Umsetzung mit Methoxyessigsäurechlorid analog Beispiel 109b) als kristallinen Festkörper: R_{f}(P)=0.63; MS: M⁺= 220.

### Beispiel 77: 5(S)-Amino-4(S)-hydroxy-2(R),7,7-trimethyl-8-[3-ethoxycarbonyl-1,2,3,4-tetrahydrochinazolin-1-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 90 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 163 mg 3-Ethoxycarbonyl-1,2,3,4-tetrahydrochinazolin über das (4O,5N-Isopropyliden)-5(S)-tertiärbutoxycarbonylamino-4(S)-hydroxy-2(R),7,7-trimethyl-8-(3-ethoxycarbonyl-1,2,3,4-tetrahydrochinazolin-1-ylcarbonyl)-octansäure(N-butyl)amid (R_{f}(K)=0.24) und das 5(S)-Tertiärbutoxycarbonylamino-4(S)-hydroxy-2(R),7,7-trimethyl-8-(3-ethoxycarbonyl-1,2,3,4-tetrahydrochinazolin-1-ylcarbonyl)-octansäure(N-butyl)amid (R_{f}(P)=0.27): R_{f}(S)=0.19; Rₜ(IV)=38.0min; FAB-MS: (M+H)⁺=505.

Das als Ausgangsmaterial eingesetzte 3-Ethoxycarbonyl-1,2,3,4-tetrahydrochinazolin wird beispielsweise folgendermassen hergestellt:
Zu einer Mischung von 473mg 1,2,3,4-Tetrahydrochinazolin (vgl. Beispiel53)) und 1.03ml Triethylamin in 30ml Methylenchlorid werden bei 0°C unter Rühren 0.35ml Chlorameisensäureethylester addiert. Nach 5min wird mit gesättigter wäßriger Natriumchloridlösung versetzt, kurz nachgerührt und der Reaktionsansatz wie üblich aufgearbeitet. Man erhält nach Reinigung mittels FC an Kieselgel das 3-Ethoxycarbonyl-1,2,3,4-tetrahydrochinazolin: R_{f}(K)=0.57; FAB-MS: (M+H)⁺=207.

### Beispiel 78: 5(S)-Amino-4(S)-hydroxy-2(R),7,7-trimethyl-8-[3(R,S)-phenylcarbonyloxy-1,2,3,4-tetrahydrochinolin-1-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 50mg 3-[N-Tertärbutoxycarbonyl-4(S)-(3-carboxy-2,2-dimethylpropyloxy-2,2-dimethyl-1,3-oxazolidin- 5(S)-yl]-2(R)-methyl-propionsäure(N-butyl)amid und 83mg 3(R,S)-Phenylcarbonyloxy-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)phenylcarbonyloxy-1,2,3,4-tetrahydrochinolin-1ylcarbonyl]-octansäure(N-butyl)amid (R_{f}(A)=0.47) und das 5(S)-Tertiärbutoxycarbonylamino-4(S)-hydroxy-2(R),7,7-trimethyl-8-[3(R,S)phenylcarbonyloxy-1,2,3,4-tetrahydrochinolin-1ylcarbonyl]-octansäure(N-butyl)amid (R_{f}(A)=0.20) als Diastereomerengemisch: R_{f}(S)=0.30; Rₜ(V)=28.1/28.6min; FAB-MS: (M+H)⁺=552.

Das als Ausgangsmaterial verwendete 3(R,S)-Phenylcarbonyloxy-1,2,3,4-tetrahydrochinolin wird beispielsweise nach der in J.Am. Chem.Soc., 66, 1168(1944) beschrieben Methode hergestellt: R_{f}(1:1-Gemisch HexanMethylenchlorid)=0.13; R_{f}(A)=0.80.

### Beispiel 79: 5(S)-Amino-4(S)-hydroxy-2(R),7,7-trimethyl-8-[2(R,S)-cyano-3,4-dihydro-2H1,4-benzoxazin-4-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 65mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 68mg 2(R,S)-Cyano-3,4-dihydro-2H1,4-benzoxazin, das nach dem von Bartsch und Schwarz in J.HeterocyclicChem., 20, 45 (1983) beschriebenen Verfahren hergestellt wird, über das (4O,5N-Isopropyliden)-5(S)-tertiärbutoxycarbonylamino-4(S)-hydroxy-2(R),7,7-trimethyl-8-[3(R,S)-cyano-3,4-dihydro-2H-1,4-benzoxazin-4-ylcarbonyl]-octansäure(N-butyl)amid (R_{f}(A)=0.47) und das 5(S)-Tertiärbutoxycarbonylamino-4(S)-hydroxy-2(R),7,7-trimethyl-8-[3(R,S)-cyano-3,4-dihydro-2H1,4-benzoxazin-4-ylcarbonyl]-octansäure(N-butyl)amid (R_{f}(A)=0.16) als Diastereomerengemisch: R_{f}(S)=0.34; Rₜ(V)=17.3min; FAB-MS: (M+H)⁺=459.

### Beispiel 80: 5(S)-Tertiärbutoxycarbonylamino-4(S)-hydroxy-2(R),7,7-trimethyl-8-[3(R,S)-methoxycarbonyl-3,4-dihydro-2H-1,4-benzoxazin-4-ylcarbonyl]-octansäure(N-butyl)amid

166 mg (4O,5N-Isopropyliden)-5(S)-tertiärbutoxycarbonylamino-4(S)-hydroxy-2(R),7 ,7-trimethyl-8-[3(R,S)-methoxycarbonyl-3,4-dihydro-2H-1,4-benzoxazin-4-ylcarbonyl]-octansäure(N-butyl)amid werden in 3 ml Methanol in Gegenwart von 5 mg p-Toluolsulfonsäurehydrat bei Raumtemperatur über Nacht gerührt. Nach dem Einengen wird das Rohprodukt an Kieselgel (Laufmittel N) gereinigt: R_{f}(N)= 0.30; FAB-MS: (M+H)⁺= 592.

Die Ausgangsgangsmaterialien werden beispielsweise wie folgt hergestellt:
a) (4O,5N-Isopropyliden)-5(S)-tertiärbutoxycarbonylamino-4(S)-hydroxy-2(R),7,7-trimethyl-8-[3(R,S)-methoxycarbonyl-3,4-dihydro-2H-1,4-benzoxazin-4-ylcarbonyl]-octansäure(N-butyl)amid
   Die Titelverbindung wird ausgehend von 120 mg 3-[N-Tertiärbutoxycarbonyl-4(S)-(3-carboxy-2,2-dimethylpropyloxy-2,2-dimethyl-1,3-oxazolidin-5(S)-yl]-2(R)-methylpropionsäure(N-butyl)amid und 203 mg 3(R,S)-methoxycarbonyl-3,4-dihydro-2H-1,4-benzoxazin analog wie in Beispiel 1a) und Reinigung an Kieselgel (Laufmittel Y) erhalten: R_{f}(Y)= 0.64.
b) 3(R,S)-methoxycarbonyl-3,4-dihydro-2H-1,4-benzoxazin
   4.0 g 3(R,S)Ethoxycarbonyl-3,4-dihydro-2H-1,4-benzoxazin (Beispiel 20)) werden zusammen mit 0.8 g MagnesiumPulver in 100 ml Methanol während 2 h bei Raumtemperatur gerührt. Nach beendeter Exothermie wird eingeengt, der Rückstand in Methylenchlorid aufgenommen und die organische Phase mit 0.1N-Salzsäure gewaschen. Nach üblicher Aufarbeitung wird das Rohprodukt an 250 g Kieselgel (Laufmittel B) gereinigt: R_{f}(B)= 0.26.

### Beispiel 81: 5(S)-Amino-4(S)-hydroxy-2(R),7,7-trimethyl-8-[3(R,S)-methoxycarbonyl-3,4-dihydro-2H-1,4-benzoxazin-4-ylcarbonyl]-octansäure(N-butyl)amid

ausgehend von 78 mg 5(S)-Tertiärbutoxycarbonylamino-4(S)-hydroxy-2(R),7,7-trimethyl-8-[-3(R,S)-methoxycarbonyl-3,4-dihydro-2H-1,4-benzoxazin-4-ylcarbonyl]-octansäure(N-butyl)amid (Beispiel 80)) analog wie in Beispiel 1) beschrieben: R_{f}(S)= 0.37; FAB-MS: (M+H)⁺= 492.

### Beispiel 82: 5(S)-Amino-4(S)-hydroxy-2(R),7(R)dimethyl-8-[2(R,S)-ethoxycarbonyl-3,4-dihydro-2H1,4-benzoxazin-4-ylcarbonyl]-octansäure(N-butyl)amid

Ausgehend von 91 mg 5(S)-Tertiärbutoxycarbonylamino-4(S)-hydroxy-2(R),7(R)dimethyl-8-[2(R,S)-ethoxycarbonyl-3,4-dihydro-2H1,4-benzoxazin-4-ylcarbonyl]-octansaure(N-butyl)amid erhält man analog wie in Beispiel72) beschrieben als Diastereomerengemisch: R_{f}(S)=0.26; Rₜ(IV)=35.2min; FAB-MS: (M+H)⁺=478.

Die Ausgangsmaterialien werden beispielsweise folgendermassen hergestellt:
a) 5(S)-Tertiärbutoxycarbonylamino-4(S)-hydroxy-2(R),7(R)dimethyl-8-[2(R,S)-ethoxycarbonyl-3,4-dihydro-2H1,4-benzoxazin-4-ylcarbonyl]-octansäure(N-butyl)amid
   Die Titelverbindung wird analog wie in Beispiel72a) beschrieben ausgehend von 123mg (4O,5N-Isopropyliden)-5(S)-tertiärbutoxycarbonylamino-4(S)-hydroxy-2(R),7(R)dimethyl-8-[2(R,S)-ethoxycarbonyl-3,4-dihydro-2H1,4-benzoxazin-4-ylcarbonyl]-octansäure(N-butyl)amid hergestellt: R_{f}(A)=0.12; FAB-MS: (M+H)⁺=578.
b) (4O,5N-Isopropyliden)-5(S)-tertiärbutoxycarbonylamino-4(S)-hydroxy-2(R),7(R)dimethyl-8-[2(R,S)-ethoxycarbonyl-3,4-dihydro-2H1,4-benzoxazin-4-ylcarbonyl]-octansäure(N-butyl)amid
   Die Titelverbindung wird analog wie in Beispiel72b) beschrieben ausgehend von 90mg 3-[N-Tertiärbutoxycarbonyl-4(S)-[3-carboxy-2(R)-methylpropyl]-2,2-dimethyl-1,3-oxazolidin-5(S)-yl]-2(R)-methyl-propionsäure(N-butyl)amid als DiastereomerenGemisch erhalten: R_{f}(A)=0.31.
c) 3-[N-Tertiärbutoxycarbonyl-4(S)-[3-carboxy-2(R)-methylpropyl]-2,2-dimethyl-1,3-oxazolidin-5(S)-yl]-2(R)-methyl-propionsäure(N-butyl)amid
   In analoger Weise wie in Beispiel72c) beschrieben wird die Titelverbindung aus 425mg 3-[N-Tertiärbutoxycarbonyl-4(S)[4-hydroxy-2(R)-methylbutyl]-2,2-dimethyl-1,3-oxazolidin-5(S)-yl]-2-(R)-methyl-propionsäure(N-butyl)amid hergestellt und durch FC an 35 g Kieselgel mit einem 50:50:1-Gemisch aus Hexan, Essigester und Eisessig als Laufmittel gereinigt: R_{f}(B)=0.22.
d) 3-[N-Tertiärbutoxycarbonyl-4(S)[4-hydroxy-2(R)-methylbutyl]-2,2-dimethyl-1,3-oxazolidin-5(S)-yl]-2-(R)-methyl-propionsäure(N-butyl)amid
   Die Titelverbindung wird analog wie in Beispiel72d) beschrieben ausgehend von 694mg 3-[N-Tertiärbutoxycarbonyl-4(S)[4triisopropylsilyloxy-2(R)-methylbutyl]-2,2-dimethyl-1,3-oxazolidin-5(S)-yl]-2-(R)-methyl-propionsäure(N-butyl)amid hergestellt und das Rohprodukt durch FC an 60 g Kieselgel (LaufmittelA) gereinigt: R_{f}(H)=0.45.
e) 3-[N-Tertiärbutoxycarbonyl-4(S)[4triisopropylsilyloxy-2(R)-methylbutyl]-2,2-dimethyl-1,3-oxazolidin-5(S)-yl]-2-(R)-methyl-propionsäure(N-butyl)amid
   Die Titelverbindung wird analog wie in Beispiel72e) beschrieben ausgehend von 800mg 5(S)Tertiärbutoxycarbonylamino-4(S)-hydroxy-9triisopropylsilyloxy-2(R),7(R)dimethyl-nonansäure(N-butyl)amid hergestellt: R_{f}(A)=0.69.
f) 5(S)Tertiärbutoxycarbonylamino-4(S)-hydroxy-9triisopropylsilyloxy-2(R),7(R)dimethylnonansäure(N-butyl)amid
   Die Titelverbindung wird analog wie in Beispiel72f) beschrieben aus 885mg 5(S)-Tertiärbutoxycarbonylamino-4(S)-hydroxy-7(R)-methyl-2-methylen9triisopropylsilyloxynonansäure(N-butyl)amid hergestellt: R_{f}(A)=0.36.
g) 5(S)Tertiärbutoxycarbonylamino-4(S)-hydroxy-7(R)-methyl-2-methylen9triisopropylsilyloxynonansäure(N-butyl)amid
   In analoger Weise wie in Beispiel 72g) beschrieben wird die Titelverbindung ausgehend von 3.2 g 2(S)Tertiärbutoxycarbonylamino-4(R)-methyl6triisopropylsilyloxyhexanal (R_{f}(D)=0.36), das analog den in den Beispielen 72h bis 72n) beschriebenen Verfahren ausgehend von (S)-3-methylbutyrolakton hergestellt wird, erhalten und durch FC an 400 g Kieselgel (Laufmittelgradient von E zu B) unter Trennung der beiden Stereoisomeren gereinigt. Man erhält die Titelverbindung (Diastereomer I): R_{f}(A)=0.58; sowie Diastereomer II: R_{f}(A)=0.47.

### Beispiel 83: 5(S)-Amino-4(S)-hydroxy-2(R),7,7-trimethyl-8-[3(R,S)-methoxy-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 50mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 54mg 3(R,S)-Methoxy-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxy-1,2,3,4-tetrahydrochinolin-1ylcarbonyl]-octansäure(N-butyl)amid (R_{f}(A)=0.38) und das 5(S)-Tertiärbutoxycarbonylamino-4(S)-hydroxy-2(R),7,7-trimethyl-8-[3(R,S)-methoxy-1,2,3,4-tetrahydrochinolin-1ylcarbonyl]- octansäure(N-butyl)amid (R_{f}(A)=0.08; FAB-MS: (M+H)⁺=562) als Diastereomerengemisch: R_{f}(S)=0.24; Rₜ(V)=16.6/17.5min; FAB-MS: (M+H)⁺=462.

Das als Ausgangsmaterial eingesetzte 3(R,S)-Methoxy-1,2,3,4-tetrahydrochinolin wird beispielsweise wie folgt hergestellt:
Zu einer Lösung von 1.0 g 3-Hydroxychinolin in 8.5 ml Dimethylformamid werden 1.05 g wasserfreies Kaliumcarbonat und 0.48ml Methyljodid addiert und das Reaktionsgemisch über 18h bei Raumtemperatur gerührt. Man verdünnt mit Diethylether, filtriert die erhaltene Suspension und engt das Filtrat ein. Das Rohprodukt wird durch FC an 60 g Kieselgel (LaufmittelF) zum 3-Methoxychinolin gereinigt: R_{f}(A)=0.53.

Die Lösung von 0.34 g 3-Methoxychinolin in 15ml Ethanol wird in Gegenwart von 0.07 g Palladium auf Kohle (10%Palladium) bei 50°C über 8h hydriert und das Rohprodukt nach üblicher Aufarbeitung durch FC an Kieselgel (LaufmittelK) gereinigt. Man erhält das 3(R,S)-Methoxy-1,2,3,4-tetrahydrochinolin: R_{f}(A)=0.61.

### Beispiel 84: 5(S)-Amino-4(S)-hydroxy-2(R),7,7-trimethyl-8-[2(R,S)-methoxymethyl-3,4-dihydro-2H1,4-benzthiazin-4-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 107mg 2(R,S)-methoxymethyl-3,4-dihydro-2H1,4-benzthiazin über das (4O,5N-Isopropyliden)-5(S)-tertiärbutoxycarbonylamino-4(S)-hydroxy-2(R),7,7-trimethyl-8-[2(R,S)-methoxymethyl-3,4-dihydro-2H1,4-benzthiazin-4-ylcarbonyl]-octansäure(N-butyl)amid (R_{f}(A)=0.41) und das 5(S)-Tertiärbutoxycarbonylamino-4(S)-hydroxy-2(R),7,7-trimethyl-8-[2(R,S)-methoxymethyl-3,4-dihydro-2H1,4-benzthiazin-4-ylcarbonyl]-octansäure(N-butyl)amid (R_{f}(K)=0.30) als Diastereomerengemisch: R_{f}(S)=0.26/0.28; Rₜ(IV)=39.9/40.4min; FAB-MS: (M+H)⁺=494.

Die oben als Ausgangsmaterial eingesetzte Aminkomponente wird beispielsweise wie folgt hergestellt:
a) 2(R,S)-Methoxymethyl-3,4-dihydro-2H1,4-benzthiazin
   435mg 4-Tertiärbutoxycarbonyl-2(R,S)-methoxymethyl-3,4-dihydro-2H1,4-benzthiazin werden bei 0°C mit 3 ml einer 25%igen TrifluoressigsäureLösung in Methylenchlorid versetzt und 3h gerührt. Der Reaktionsansatz wird eingeengt und der Rückstand an 30 g Kieselgel mit einem 99:1-Gemisch aus Methylenchlorid und Methanol chromatographiert. Man erhält die Titelverbindung: R_{f}(99:1-Gemisch aus Methylenchlorid/Methanol)=0.67.
b) 4-Tertiärbutoxycarbonyl-2(R,S)-methoxymethyl-3,4-dihydro-2H1,4-benzthiazin
   1.9 g 4-Tertiärbutoxycarbonyl-2(R,S)-hydroxymethyl-3,4-dihydro-2H1,4-benzthiazin werden in 20 ml Tetrahydrofuran gelöst und bei 0 °C mit 1.5 g einer 20 %igen Kaliumhydrid-Suspension in Oel versetzt. Anschliessend werden 1.3 ml Methyljodid zugetropft, die Mischung auf Raumtemperatur aufwärmen gelassen und nach beendeter Reaktion die Mischung auf Wasser gegossen. Nach üblicher Aufarbeitung wird das Rohprodukt an 250 g Kieselgel mittels FC (Methylenchlorid als Laufmittel) gereinigt: R_{f}(D) = 0.45.
c) 4-Tertiärbutoxycarbonyl-2(R,S)-hydroxymethyl-3,4-dihydro-2H1,4-benzthiazin
   1.47 g 2(R,S)Hydroxymethyl-3,4-dihydro-2H1,4-benzthiazin werden in 10 ml Tetrahydrofuran mit 2.3 g Di-tertiärbutyldicarbonat und 10 mg 4-DMAP versetzt und über 17 h gerührt. Der Reaktionsansatz wird eingeengt und direkt an 150 g Kieselgel chromatographiert (Laufmittel D). Man erhält die Titelverbindung: R_{f}(B) = 0.52.
d) 2(R,S)Hydroxymethyl-3,4-dihydro-2H1,4-benzthiazin
   1.6 g 2(R,S)Ethoxycarbonyl-3,4-dihydro-2H1,4-benzthiazin werden in 20 ml Tetrahydrofuran mit 1.06 g Lithiumborhydrid umgesetzt. Nach Zugabe von 10 ml Methanol wird eingeengt und mittels FC an 80 g Kieselgel (Laufmittel S) gereinigt. Man erhält die Titelverbindung: R_{f}(S)= 0.29.

### Beispiel 85: 5(S)-Amino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid

Die Titelverbindung wird ausgehend von (4O,5N-Isopropyliden)-5(S)-tertiärbutoxycarbonylamino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid (Diastereomer I) über das 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid (R_{f}(P)=0.34) als reines Diastereomer erhalten: R_{f}(S)=0.24; Rₜ(IV)=36.4min; FAB-MS: (M+H)⁺=505.

Die Ausgangsmaterialien werden beispielsweise folgendermassen erhalten:
a) (4O,5N-Isopropyliden)-5(S)-tertiärbutoxycarbonylamino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid
   Die Titelverbindung wird ausgehend von 50mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 68mg 3(R,S)-methoxycarbonylamino-1,2,3,4-tetrahydrochinolin und Reinigung des erhaltenen Rohpodukts mittels FC an 25 g Kieselgel (Laufmittelgradient von B zu A) unter Trennung der beiden 3(R)-bzw. 3(S)-Stereoisomere eerhalten. Man erhält die reine Titelverbindung (Diastereomer I): R_{f}(A)=0.19; sowie Diastereomer II: R_{f}(A)=0.15.

### Beispiel 86: 5(S)-Amino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von (4O,5N-Isopropyliden)-5(S)-tertiärbutoxycarbonylamino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid (Diastereomer II; Beispiel 85a)) über das 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid (R_{f}(P) = 0.32) als reines Diastereomer: R_{f}(S) = 0.25; Rₜ(IV) = 36.6 min; FAB-MS: (M+H)⁺ = 505.

### Beispiel 87: 5(S)-Amino-4(S)-hydroxy-2(R),7,7-trimethyl-8-[2(R,S)-allyloxymethyl-3,4-dihydro-2H-1,4-benzoxazin-4-ylcarbonyl]-octansäure(N-butyl)amid

Analog den in den Beispielen 21, 21a und 21b) angegebenen Methoden erhält man die Titelverbindung ausgehend von 205 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 205 mg 2(R,S)-Allyloxymethyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)- allyloxymethyl-3,4-dihydro-2H-1,4-benzoxazin-4-ylcarbonyl]-octansäure(N-butyl)amid (R_{f}(A)= 0.39) und das 5(S)-Tertiärbutoxycarbonylamino-4(S)-hydroxy-2(R),7,7-trimethyl-8-[2(R,S)-allyloxymethyl-3,4-dihydro-2H-1,4-benzoxazin-4-ylcarbonyl]-octansäure(N-butyl)amid (Reinigung an 10 g Kieselgel, Laufmittel A und Essigester: R_{f}(X)= 0.50; Rₜ(I)= 28.5 min; FAB-MS: (M+H)⁺= 604) als Diastereomerengemisch: R_{f}(V)= 0.38; Rₜ(I)= 21.5/21.7 min; FAB-MS: (M+H)⁺= 504.

Die Amin-Komponente wird beispielsweise wie folgt hergestellt:
a) 2(R,S)-Allyloxymethyl-3,4-dihydro-2H-1,4-benzoxazin
   Zu einer Lösung von 1.37 g N-Benzyloxycarbonyl-2(R,S)allyloxymethyl-3,4-dihydro-2H-1,4-benzoxazin in 25 ml Methylenchlorid werden unter Argon 0.8 ml Jodtrimethylsilan addiert. Das Reaktionsgemisch wird für 2 h bei Raumtemperatur gerührt und dann mit 10 ml Methanol versetzt und eingeengt. Der Rückstand wird zweimal mit 50 ml eines 10:1-Gemisches aus Hexan und Diethylether extrahiert, anschliessend in Essigester gelöst und mit einer 1N Natriumcarbonat-Lösung und gesättigter Natriumchloridlösung gewaschen. Das Rohprodukt wird mittels FC (100 g Kieselgel, Laufmittel D) gereinigt. Man erhält die reine Titelverbindung: R_{f}(D)= 0.17.
b) N-Benzyloxycarbonyl-2(R,S)-allyloxymethyl-3,4-dihydro-2H-1,4-benzoxazin
   Die Titelverbindung wird ausgehend von 3.0 g N-Benzyloxycarbonyl-2(R,S)-hydroxymethyl-3,4-dihydro-2H-1,4-benzoxazin, 2.75 g Natriumbis(trimethylsilyl)amid und 1.7 ml 3-Brom-1-propen analog Beispiel 61) hergestellt: R_{f}(D)= 0.32; R_{f}(3:1-Gemisch aus Toluol und Essigester)= 0.61.
c) N-Benzyloxycarbonyl-2(R,S)-hydroxymethyl-3,4-dihydro-2H-1,4-benzoxazin
   Die Titelverbindung wird analog Beispiel 84) ausgehend von 50.0 g N-Benzyloxycarbonyl-2(R,S)-ethoxycarbonyl-3,4-dihydro-2H-1,4-benzoxazin und 5.4 g Lithiumborohydrid analog Beispiel 61) erhalten: R_{f}(3:1-Gemisch aus Toluol und Essigester)= 0.26; Rₜ(I)= 23.8 min; MS : M⁺= 299.
d) N-Benzyloxycarbonyl-2(R,S)-ethoxycarbonyl-3,4-dihydro-2H-1,4-benzoxazin
   Die Titelverbindung wird analog Beispiel 1t) ausgehend von 40.0 g 2(R,S)Ethoxycarbonyl-3,4-dihydro-2H-1,4-benzoxazin, 39 ml Chlorameisensäurebenzylester und 22.3 g Natriumhydrogencarbonat erhalten: R_{f}(3:1-Gemisch aus Toluol und Essigester)= 0.55; Rₜ(I)= 27.8 min.

### Beispiel 88: 5(S)-Amino-4(S)-hydroxy-2(R),7,7-trimethyl-8-[2(R,S)-propyloxymethyl-3,4-dihydro-2H-1,4-benzoxazin-4-ylcarbonyl]-octansäure(N-butyl)amid

Analog den in den Beispielen 21, 21a und 21b) angegebenen Methoden erhält man die Titelverbindung ausgehend von 205 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 291 mg 2(R,S)-Propyloxymethyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-propyloxymethyl-3,4-dihydro-2H-1,4-benzoxazin-4-ylcarbonyl]-octansäure(N-butyl)amid (R_{f}(A)= 0.38; FAB-MS: (M+H)⁺= 646) und das 5(S)-Tertiärbutoxycarbonylamino-4(S)-hydroxy-2(R),7,7-trimethyl-8-[2(R,S)-propyloxymethyl-3,4-dihydro-2H-1,4-benzoxazin-4-ylcarbonyl]-octansäure(N-butyl)amid (R_{f}(V)= 0.58; Rₜ(I)= 29.3 min; FAB-MS: (M+H)⁺= 606) als Diastereomerengemisch: R_{f}(V)= 0.29; Rₜ(I)= 22.0/22.2 min; FAB-MS: (M+H)⁺= 506.

Das eingesetzte 2(R,S)-Propyloxymethyl-3,4-dihydro-2H-1,4-benzoxazin erhält man analog Beispiel 1r) ausgehend von 1.8 g N-Benzyloxycarbonyl-2(R,S)allyloxymethyl-3,4-dihydro-2H-1,4-benzoxazin (Beispiel 87b) und Reinigung des Rohprodukts mittels FC (80 g Kieselgel, Laufmittelgradient von einem 10:1- zu einem 3:1-Gemisch aus Toluol und Essigester: R_{f}(3:1-Gemisch aus Toluol und Essigester)= 0.50; FAB-MS: (M+H)⁺= 208.

### Beispiel 89: 5(S)-Amino-4(S)-hydroxy-2(R),7,7-trimethyl-8-[2(R,S)-methoxymethyl-3,4-dihydro-2H-1,4-benzoxazin-4-ylcarbonyl]-octansäure(N-butyl)amid

Analog den in den Beispielen 21, 21a und 21b) angegebenen Methoden erhält man die Titelverbindung ausgehend von 300 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 359 mg 2(R,S)-Methoxymethyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-methoxymethyl-3,4-dihydro-2H-1,4-benzoxazin-4-ylcarbonyl]-octansäure(N-butyl)amid (R_{f}(A)= 0.22; Rₜ(I)= 31.3/31.6 min) und das 5(S)-Tertiärbutoxycarbonylamino-4(S)-hydroxy-2(R),7,7-trimethyl-8-[2(R,S)-methoxymethyl-3,4-dihydro-2H-1,4-benzoxazin-4-ylcarbonyl]- octansäure(N-butyl)amid (R_{f}(X)= 0.31; Rₜ(I)= 26.6 min; FAB-MS: (M+H)⁺= 578) als Diastereomerengemisch: R_{f}(V)= 0.33; Rₜ(I)= 19.3/19.5 min; FAB-MS: (M+H)⁺= 478.

Die Amin-Komponente wird beispielsweise folgendermassen hergestellt:
a) 2(R,S)-Methoxymethyl-3,4-dihydro-2H-1,4-benzoxazin wird analog Beispiel 1r) ausgehend von 1.7 g N-Benzyloxycarbonyl-2(R,S)-methoxymethyl-3,4-dihydro-2H-1,4-benzoxazin erhalten: R_{f}(3:1-Gemisch aus Toluol und Essigester)= 0.47; Rₜ(I)= 12.9 min; FAB-MS: (M+H)⁺= 180.
b) N-Benzyloxycarbonyl-2(R,S)-methoxymethyl-3,4-dihydro-2H-1,4-benzoxazin
   Die Titelverbindung wird analog Beispiel 87b) ausgehend von 3.0 g N-Benzyloxycarbonyl-2(R,S)-hydroxymethyl-3,4-dihydro-2H-1,4-benzoxazin, 2.4 g Natriumbis(trimethylsilyl)amid und 0.94 ml Methyljodid hergestellt: R_{f}(3:1-Gemisch aus Toluol und Essigester)= 0.56; Rₜ(I)= 27.9 min.

### Beispiel 90: 5(S)-Amino-4(S)-hydroxy-2(S)-isopropyl-7,7-dimethyl-8-[2(R)- oder 2(S)-methylaminocarbonyl-3,4-dihydro-2H1,4-benzthiazin-4-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyl)-2,2-dimethyl-1,3-oxazolidin-5(S)-yl]-2(S)isopropyl-propionsäure(N-butyl)amid und 107 mg 2(R)- oder 2(S)-methylaminocarbonyl-3,4-dihydro-2H1,4-benzthiazin (Stereoisomer I) über das (4O,5N-Isopropyliden)-5(S)-tertiärbutoxycarbonylamino-4(S)-hydroxy-2(S)-isopropyl-7,7-dimethyl-8-[2(R)- oder 2(S)-methylaminocarbonyl-3,4-dihydro-2H1,4-benzthiazin-4-ylcarbonyl]-octansäure(N-butyl)amid (R_{f}(Essigester)=0.64) und das 5(S)-Tertiärbutoxycarbonylamino-4(S)-hydroxy-2(S)-isopropyl-7,7dimethyl-8-[2(R)- oder 2(S)-methylaminocarbonyl-3,4-dihydro-2H1,4-benzthiazin-4-ylcarbonyl]-octansaure(N-butyl)amid (R_{f}(P)=0.50) als reines Diastereomer: R_{f}(S)=0.34; Rₜ(IV)=37.0 min; FAB-MS: (M+H)⁺=535.

Die als Ausgangsmaterial eingesetzte Amin-Komponente wird beispielsweise wie folgt hergestellt:
0.2 g 2(R,S)-methylaminocarbonyl-3,4-dihydro-2H1,4-benzthazin werden an Cellulose-Triacetat (ChromatographieSäule 50 x 1000 mm) mit einem 95:5-Gemisch aus Ethanol und Wasser als Elutionsmittel chromatographiert. Nach Einengen der stereoisomerenreinen Substanzfraktionen erhält man das 2(R)- oder 2(S)-methylaminocarbonyl-3,4-dihydro-2H-1,4-benzthiazin (Stereoisomer I) als gelbliches Oel: [α]^{D} = + 8.3 (c=1; Methylenchlorid); sowie das 2(R)- oder 2(S)-methylaminocarbonyl-3,4-dihydro-2H1,4-benzthiazin (Stereoisomer II) als gelbliches Oel: [α]^{D} = -9.1 (c=1; Methylenchlorid).

### Beispiel 91: 5(S)-Amino-4(S)-hydroxy-2(S)-isopropyl-7,7-dimethyl-8-[2(R)- oder 2(S)-methylaminocarbonyl-3,4-dihydro-2H1,4-benzthiazin-4-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyl)-2,2-dimethyl-1,3-oxazolidin-5(S)-yl]-2(S)isopropyl-propionsäure(N-butyl)amid und 107 mg 2(R)- oder 2(S)-methylaminocarbonyl-3,4-dihydro-2H1,4-benzthiazin (Stereoisomer II; Beispiel 90) über das (4O,5N-Isopropyliden)-5(S)-tertiärbutoxycarbonylamino-4(S)-hydroxy-2(S)-isopropyl-7,7-dimethyl-8-[2(R)- oder 2(S)-methylaminocarbonyl-3,4-dihydro-2H1,4-benzthiazin-4-ylcarbonyl]-octansäure(N-butyl)amid (R_{f}(Essigester)=0.60) und das 5(S)-Tertiärbutoxycarbonylamino-4(S)-hydroxy-2(S)-isopropyl-7,7dimethyl-8-[2(R)- oder 2(S)-methylaminocarbonyl-3,4-dihydro-2H1,4-benzthiazin-4-ylcarbonyl]-octansäure(N-butyl)amid (R_{f}(P)=0.58) als reines Diastereomer: R_{f}(S)=0.36; Rₜ(IV)=37.7 min; FAB-MS: (M+H)⁺=535.

### Beispiel 92: 5(S)-Amino-4(S)-hydroxy6-[1-[3(R,S)-methylaminocarbonyl-1,2,3,4-tetrahydrochinolin-1-ylcarbonylmethyl]cyclopropyl]-2(R)-methyl-hexansäure(N-butyl)amid

116mg 5(S)-Tertiärbutoxycarbonylamino-4(S)-hydroxy6-[1-[3(R,S)-methylaminocarbonyl-1,2,3,4-tetrahydrochinolin-1ylcarbonylmethyl]cyclopropyl]-2(R)-methyl-hexansäure(N-butyl)amid werden bei 0°C mit 2 ml 4N-Salzsäure in Dioxan versetzt und über 2h gerührt. Es werden 2 ml Toluol addiert und das Reaktionsgemisch eingeengt. Der ölige Rückstand wird an 10 g Kieselgel (LaufmittelK und L) chromatographiert. Man erhält die Titelverbindung als DiastereoisomerenGemisch: R_{f}(P)=0.11; Rₜ(IV)=32.6min; FAB-MS: (M+H)⁺=487.

Die Ausgangsmaterialien werden beispielsweise folgendermassen hergestellt:
a) 5(S)-Tertiärbutoxycarbonylamino-4(S)-hydroxy6-[1-[3R,S)-methylaminocarbonyl-1,2,3,4-tetrahydrochinolin-1ylcarbonylmethyl]cyclopropyl]-2(R)-methyl-hexansaure(N-butyl)amid
   Die Titelverbindung wird analog wie in Beispiel21a) beschrieben ausgehend von 180mg (4O,5N-Isopropyliden)-5(S)-tertiärbutoxycarbonylamino-4(S)-hydroxy6-[1-[3(R,S)-methylaminocarbonyl-1,2,3,4-tetrahydrochinolin-1ylcarbonylmethyl]cyclopropyl]-2(R)-methyl-hexansäure(N-butyl)amid hergestellt und mittels FC an 25 g Kieselgel (LaufmittelJ) gereinigt: R_{f}(P)=0.48.
b) (4O,5N-Isopropyliden)-5(S)-tertiärbutoxycarbonylamino-4(S)-hydroxy6-[1-[3(R,S)-methylaminocarbonyl-1,2,3,4-tetrahydrochinolin-1ylcarbonylmethyl]cyclopropyl]-2(R)-methyl-hexansäure(N-butyl)amid
   100mg 3-[N-Tertiärbutoxycarbonyl-4(S)[1-(carboxymethyl)cyclopropylmethyl]-2,2-dimethyl-1,3-oxazolidin-5(S)-yl]-2(R)-methyl-propionsäure(N-butyl)amid werden analog dem in Beispiel72b) beschriebenen Verfahren mit 126mg 2(R,S)-methylaminocarbonyl-1,2,3,4-tetrahydrochinolin umgesetzt und mittels FC an 30 g Kieselgel mit einem 96:4-Gemisch aus Methylenchlorid und Methanol als Laufmittel gereinigt. Man erhält die Titelverbindung: R_{f}(P)=0.54.
c) 3-[N-Tertiärbutoxycarbonyl-4(S)[1-(carboxymethyl)cyclopropylmethyl]-2,2-dimethyl-1,3-oxazolidin-5(S)-yl]-2(R)-methyl-propionsäure(N-butyl)amid
   Eine Lösung von 788mg 3-[NTertiärbutoxycarbonyl-4(S)[1-(2-hydroxyethyl)cyclopropylmethyl]-2,2-dimethyl-1,3-oxazolidin-5(S)-yl]-2-(R)-methyl-propionsäure(N-butyl)amid in 24ml eines 1:1-Gemisches aus Tetrachlorkohlenstoff und Acetonitril wird zu einem Gemisch aus 2.2 g Natriummetaperjodat, 47mg Ruthenium(III)-chloridHydrat und 24ml Wasser addiert und das Reaktionsgemisch während 1h kräftig gerührt. Nach Aufarbeitung analog wie in Beispiel72c) beschrieben wird das Rohprodukt mittels FC an 80 g Kieselgel mit einem 50:50:1 Gemisch aus Hexan,Essigester und Eisessig als Laufmittel gereinigt: R_{f}(1:2:0.1 Gemisch aus Hexan, Essigester und Eisessig)=0.66; FAB-MS: (M+H)⁺=455.
d) 3-[NTertiärbutoxycarbonyl-4(S)[1-(2-hydroxyethyl)cyclopropylmethyl]-2,2-dimethyl-1,3-oxazolidin-5(S)-yl]-2-(R)-methyl-propionsäure(N-butyl)amid
   Die Titelverbindung wird durch Hydrierung analog der in Beispiel39a) beschriebenen Methode aus 1.2 g 3-[NTertiärbutoxycarbonyl-4(S)[1-(2-benzyloxyethyl)cyclopropylmethyl]-2,2-dimethyl-1,3-oxazolidin-5(S)-yl]-2-(R)-methyl-propionsäure(N-butyl)amid erhalten: R_{f}(A)=0.24.
e) 3-[NTertiärbutoxycarbonyl-4(S)[1-(2-benzyloxyethyl)cyclopropylmethyl]-2,2-dimethyl-1,3-oxazolidin-5(S)-yl]-2-(R)-methyl-propionsäure(N-butyl)amid
   Eine Lösung von 3.1 g 5(S)-Tertiärbutoxycarbonylamino-6-[1-(2-benzyloxyethyl)cyclopropyl]4(S)-hydroxy-2-methylenhexansäure(N-butyl)amid in 30ml absolutem Methanol wird in Gegenwart von 30 mg Bis[(S)-(-)-2,2'-bis(diphenylphosphino)-1,1'-binaphtyl]-(triethylamino)dirutheniumtetrachlorid analog wie in Beispiel72f) beschrieben hydriert und das Rohprodukt mittels FC an 100 g Kieselgel (LaufmittelA) gereinigt: R_{f}(A)=0.40; MS: M⁺=491; Anal. Kalk. für: C68.54%, H9,45%, N5.71%; gef. C68.29%, H9.72%, N5.70%.
   Das so erhaltene 5(S)-Tertiärbutoxycarbonylamino-6-[1-(2-benzyloxyethyl)cyclopropyl]4(S)-hydroxy-2(R)-methyl-hexansäure(N-butyl)amid wird analog wie in Beispiel 1d) beschrieben mit Dimethoxypropan und p-Toluolsulfonsäurehydrat umgesetzt und mittels FC an 80 g Kieselgel (Laufmittel C) gereinigt. Man erhält die Titelverbindung als reines Diastereomer: R_{f}(A)=0.58.
f) 5(S)-Tertiärbutoxycarbonylamino-6-[1-(2-benzyloxyethyl)cyclopropyl]4(S)-hydroxy-2-methylenhexansäure(N-butyl)amid
   Durch Umsetzung von 6.5 g 2(S)Tertiärbutoxycarbonylamino-3-[1-(2-benzyloxyethyl)-cyclopropyl]-propanal in analoger Weise wie in Beispiel 1f) beschrieben und anschliessender Reinigung des Rohprodukts mittels FC an 500 g Kieselgel (Laufmittel von D zu A) unter Auftrennung der beiden Stereoisomeren erhält man die reine Titelverbindung (Diastereomer I): R_{f}(A)=0.52; sowie Diastereomer II: R_{f}(A)=0.41.
g) 2(S)Tertiärbutoxycarbonylamino-3-[1-(2-benzyloxyethyl)cyclopropyl]-propanal
   7.0 g 2(S)Tertiärbutoxycarbonylamino-3-[1(2-benzyloxyethyl)cyclopropyl]-propan-1-ol werden analog dem Verfahren 1g) umgesetzt. Nach üblicher Aufarbeitung erhält man die Titelverbindung: R_{f}(A)=0.84.
h) 2(S)Tertiärbutoxycarbonylamino-3-[1(2-benzyloxyethyl)cyclopropyl]-propan-1-ol
   Die Mischung aus 23.9 g 3-Tertiärbutoxycarbonyl-2,2-dimethyl-4(S)[1(2-benzyloxyethyl)cyclopropylmethyl]1,3-oxazolidin, 0.67 g p-Toluolsulfonsäurehydrat und 100ml Methanol wird bei Raumtemperatur 5h gerührt. Nach Einengen des Reaktionsgemischs wird mit Diethylether versetzt. Die organische Phase wird mit gesättigter Natriumhydrogencarbonat-Lösung und gesättigter Natriumchloridlösung gewaschen und über Natriumsulfat getrocknet. Das Rohprodukt wird mittels FC an 900 g Kieselgel (Laufmittelgradient von E zu A) gereinigt: R_{f}(C)=0.26; Anal. Kalk. für C₂₀H₃₁NO₄: C68.74%, H8.94%, N4.01%; gef. C68.44%, H8.93%, N4.09%.
i) 3-Tertiärbutoxycarbonyl-2,2-dimethyl-4(S)[1(2-benzyloxyethyl)cyclopropylmethyl]1,3-oxazolidin
   15.1 g 3-Tertiärbutoxycarbonyl-2,2-dimethyl-4(S)[1(2-hydroxyethyl)cyclopropylmethyl]1,3-oxazolidin werden in 50 ml Tetrahydrofuran gelöst und bei 0 °C mit 14.2 g einer KaliumhydridSuspension (20% in Oel) versetzt. Es wird 1 h bei 0 °C gerührt und anschliessend 6.3 ml Benzylbromid unter Rühren zugetropft. Nach 90 min werden langsam 100 ml Wasser zum Reaktionsansatz addiert und die wäßerige Phase mit Diethylether extrahiert. Man erhält die Titelverbindung: R_{f}(A)= 0.8; FAB-MS: (M+H)⁺= 390.
j) 3-Tertiärbutoxycarbonyl-2,2-dimethyl-4(S)[1(2-hydroxyethyl)cyclopropylmethyl]1,3-oxazolidin
   Zu 18.0 g 3-Tertiärbutoxycarbonyl-2,2-dimethyl-4(S)[(1vinylcyclopropyl)methyl]1,3-oxazolidin, gelöst in 300 ml Tetrahydrofuran werden bei 0 °C 2.5 ml DiboranDimethylsulfidKomplex innerhalb von 3 Minuten addiert und der Ansatz anschliessend während 3 h unter Eiskühlung gerührt. Es werden nacheinander 5 ml Wasser, 16 ml 2N Natronlauge und 8.5 ml einer 30%igen Wasserstoffperoxidlösung zugegeben und 1 h lang bei Raumtemperatur nachgerührt. Dann wird mit eiskalter KaliumcarbonatLösung verdünnt und die wäßerige Phase mit Essigester extrahiert. Die Reinigung des Rohprodukts erfolgt an 900 g Kieselgel (Laufmittel B): R_{f}(B)= 0.25; Anal. kalk. für C₁₆H₃₅NO₄: C 64.18%, H 9.76%, N 4.68%; gef. C 64.05%, H 9.76%, N 4.73%.
k) 3-Tertiärbutoxycarbonyl-2,2-dimethyl-4(S)[(1vinylcyclopropyl)methyl]1,3-oxazolidin
   Die Suspension von 52.7 g Methyltriphenylphosphoniumbromid in 470 ml Tetrahydrofuran wird anteilweise innerhalb von 10 min mit 27.05 g Natriumhexamethyldisilazan versetzt. Zu dieser Mischung werden bei 5 °C innerhalb von 10 min 20.9 g 3-Tertiärbutoxycarbonyl-2,2-dimethyl-4(S)[(1formylcyclopropyl)methyl]1,3-oxazolidin addiert, und der Ansatz nach dem Aufwärmen auf Raumtemperatur 1 h gerührt. Die Mischung wird auf Eiswasser gegossen und die wäßerige Phase mit Methylenchlorid extrahiert. Die Titelverbindung wird mittels FC an 900 g Kieselgel (Laufmittel G) gereinigt: R_{f}(G)= 0.56.
l) 3-Tertiärbutoxycarbonyl-2,2-dimethyl-4(S)[(1-formylcyclopropyl)methyl]1,3-oxazolidin
   Zu einer Lösung von 10.1 ml Oxalylchlorid in 130 ml Methylenchlorid wird bei -60 °C 11.1 ml Dimethylsulfoxid in 150 ml Methylenchlorid zugetropft. Nach beendeter Zugabe wird noch 10 min nachgerührt, anschliessend eine Lösung von 22.3 g 3-Tertiärbutoxycarbonyl-2,2-dimethyl-4(S)[(1-hydroxymethylcyclopropyl)methyl]1,3-oxazolidin in 270 ml Methylenchlorid innerhalb von 20 min bei -60 °C addiert und weitere 30 min gerührt. Dann werden 43.6 ml Triethylamin bei gleicher Temperatur zugegeben und nach 30 min schliesslich das Reaktionsgemisch mit 140 ml einer 20%igen KaliumhydrogensulfatLösung versetzt. Nach üblicher Aufarbeitung erhält man die Titelverbindung als Rohprodukt: R_{f}(A)= 0.73.
m) 3-Tertiärbutoxycarbonyl-2,2-dimethyl-4(S)[(1-hydroxymethylcyclopropyl)methyl]1,3-oxazolidin
   31.3 g 3-Tertiärbutoxycarbonyl-2,2-dimethyl-4(S)[[(1-benzyloxymethyl)cyclopropyl]methyl]1,3-oxazolidin werden in 300 ml Tetrahydrofuran gelöst und unter Zusatz von 3 g Palladium auf Kohle (10% Pd) bei Raumtemperatur hydriert. Das Rohprodukt wird mittels FC an 900 g Kieselgel (Laufmittel A) gereinigt (22.3 g der Titelverbindung): R_{f}(A)= 0.32.
n) 3-Tertiärbutoxycarbonyl-2,2-dimethyl-4(S)[[(1-benzyloxymethyl)cyclopropyl]methyl]1,3-oxazolidin
   Die Mischung aus 31.4 g 1-Benzyloxymethyl-1[2(S)-tertiärbutoxycarbonylamino-3hydroxypropan-1yl]cyclopropan, 80 ml 2-Methoxypropen und 0.5 g p-Toluolsulfonsäure-Hydrat in 170 ml Methylenchlorid wird bei Raumtemperatur während 16 h gerührt. Der Ansatz wird eingeengt und mittels FC an 900 g Kieselgel (Laufmittel G) gereinigt: R_{f}(G)= 0.23.
o) 1-Benzyloxymethyl-1[2(S)-tertiärbutoxycarbonlamino-3hydroxypropan-1yl]cyclopropan
   Zur Lösung von 30.9 g 1-Benzyloxymethyl-1[2(S)-tertiärbutoxycarbonylamino-2-methoxycarbonyleth1yl]cyclopropan in 300 ml Tetrahydrofuran werden unter Rühren 4.3 g Lithiumborhydrid in Anteilen addiert. Es wird noch 2 h bei Raumtemperatur gerührt, anschliessend langsam 500 ml Methanol zugetropft, bis fast zur Trockene eingeengt und der Rückstand in Methylenchlorid aufgenommen. Die organische Phase wird mit 1N-Salzsäure gewaschen. Die weitere übliche Aufarbeitung ergibt die reine Titelverbindung: R_{f}(B)= 0.50.
p) 1-Benzyloxymethyl-1[2(S)-tertiärbutoxycarbonylamino-2-methoxycarbonyleth1-yl]cyclopropan
   Die Lösung von 27.7 g 1-Benzyloxymethyl-1[2(S)-amino-2-methoxycarbonyleth 1-yl]cyclopropan in 250 ml Tetrahydrofuran wird unter Eiskühlung mit 25.3 g Di-tertiärbutyldicarbonat versetzt und nach Aufwärmen während 4 h bei Raumtemperatur gerührt. Den nach Einengen des Reaktionsansatzes erhaltenen Rückstand chromatographiert man an 900 g Kieselgel (Laufmittel D). Man erhält die reine Titelverbindung (34.8 g) als gelbliches Oel: R_{f}(D)= 0.33.
q) 1-Benzyloxymethyl-1[2(S)-amino-2-methoxycarbonyleth1yl]cyclopropan
   Zur Lösung von 32 ml (2R)-(-)-2,5-Dihydro3,6dimethoxy-2isopropylpyrazin in 180 ml Tetrahydrofuran werden unter Argon bei -70 °C 105 ml einer 1.6 M n-Butyllithiumlösung in Hexan addiert. Es wird 30 min gerührt und dann bei gleicher Temperatur eine Lösung von 39.1 g 1-Benzyloxymethyl-1-(brommethyl)cyclopropan in 180 ml Tetrahydrofuran zugetropft. Nach 2 h ist die Reaktion beendet. Der Ansatz wird auf eine 5N-Ammoniumchloridlösung gegossen und die wäßerige Phase mit Methylenchlorid extrahiert. Das so erhalten rohe Alkylierungsprodukt (38.04 g) wird in 425 ml Acetonitril gelöst, mit 425 ml 1N-Salzsäure versetzt und 2 h bei Raumtemperatur gerührt. Man gießt anschliessend die Mischung auf 500 ml einer 1N-NatriumbicarbonatLösung und extrahiert mit Methylenchlorid. Das Rohprodukt wird an 900 g Kieselgel (Laufmittel N) chromatographiert: R_{f}(N)= 0.28.
r) 1-Benzyloxymethyl-1-(brommethyl)cyclopropan
   58.9 g 1-Benzyloxymethyl-1-(hydroxymethyl)cyclopropan und 88.5 g Triphenylphosphin werden in 600 ml Methylenchlorid gelöst und bei 0 °C portionsweise mit 60 g N-Bromsuccinimid versetzt. Man rührt 16 h bei Raumtemperatur, engt den Reaktionsansatz ein und reinigt den Rückstand mittels FC an 3 kg Kieselgel mit einem 1:1-Gemisch aus Methylenchlorid und Hexan als Elutionsmittel. Man erhält 68 g der reinen Titelverbindung: R_{f}(1:1-Gemisch aus Methylenchlorid und Hexan)= 0.50.
s) 1-Benzyloxymethyl-1-(hydroxymethyl)cyclopropan
   Die Lösung von 23.2 g 1,1-(Bis-hydroxymethyl)cyclopropan (hergestellt nach der in J. Org. Chem. 21, 1490(1956) beschriebenen Methode) in 250 ml Dimethylformamid wird unter Rühren nacheinander mit 9.1 g einer NatriumhydridSuspension (60% in Oel) und 29.7 g Benzylbromid versetzt. Nach 2 h wird der Reaktionsansatz eingeengt und der Rückstand an 900 g Kieselgel (Laufmittel B) gereinigt: R_{f}(B)= 0.33.

### Beispiel 93: 5(S)-Amino-4(S)-hydroxy-2(R),7,7-trimethyl-8-[3(R,S)-N,N-dimethylaminocarbonylamino-1,2,3,4-tetrahydrochinolin-1-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 144mg 3(R,S)-N,N-dimethylaminocarbonylamino-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)N,N-dimethylaminocarbonylamino-1,2,3,4-tetrahydrochinolin-1ylcarbonyl]-octansäure(N-butyl)amid [R_{f}(L, zweifache Laufstrecke)=0.42] und das 5(S)-Tertiärbutoxycarbonylamino-4(S)-hydroxy-2(R),7,7-trimethyl-8-[3(R,S)N,N-dimethylaminocarbonylamino-1,2,3,4-tetrahydrochinolin-1ylcarbonyl]octansäure(N-butyl)amid [R_{f}(L, zweifache Laufstrecke)=0.37; FAB-MS: (M+H)⁺=618) als Diastereomerengemisch: R_{f}(S)=0.27; Rₜ(V)=17.8/18.1min; FAB-MS: (M+H)⁺=518.

Das als Ausgangsmaterial eingesetzte 3(R,S)N,N-dimethylaminocarbonylamino-1,2,3,4-tetrahydrochinolin wird beispielsweise folgendermassen hergestellt:
Zu einer Lösung von 1.65ml N,N-Dimethylcarbamoylchlorid in 3.9 ml Pyridin werden 2.0 g 3-Aminochinolin in Anteilen addiert und das Reaktionsgemisch über 24h bei Raumtemperatur gerührt. Der Reaktionsansatz wird anschliessend auf 50ml Eiswasser gegossen. Nach üblicher Aufarbeitung und chromatographischer Reinigung des Rohprodukts an 100 g Kieselgel (LaufmittelL) erhält man 3-(N,N-dimethylaminocarbonylamino)chinolin: R_{f}(L)=0.23; MS:M⁺=215.

1.4 g des oben beschriebenen HarnstoffDerivats, gelöst in 40ml Ethanol, werden in Gegenwart von 0.28 g Palladium auf Kohle (10%Palladium) bei 50°C über 40 h, und nach erneuter Zugabe von 0.14 g des Katalysators weitere 7h hydriert. Nach üblicher Aufarbeitung und FCReinigung an 170 g Kieselgel mit einem 96:4 Gemisch aus Methylenchlorid und Methanol als Laufmittel erhält man 3(R,S)-N,N-dimethylaminocarbonylamino-1,2,3,4-tetrahydrochinolin: R_{f}(L)=0.36; MS: (M)⁺=219.

### Beispiel 94: 5(S)-Amino-4(S)-hydroxy-2(S)-isopropyl-7,7-dimethyl-8-[2(R,S)-ethylaminocarbonyl-3,4-dihydro-2H1,4-benzthiazin-4-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 80mg 3-[N-Tertiärbutoxycarbonyl-4(S)-(3-carboxy-2,2-dimethylpropyloxy-2,2-dimethyl-1,3-oxazolidin-5(S)-yl]-2(S)isopropyl-propionsäure(N-butyl)amid und 100mg 2(R,S)Ethylaminocarbonyl-3,4-dihydro-2H1,4-benzthiazin über das (4O,5N-Isopropyliden)-5(S)-tertiärbutoxycarbonylamino-4(S)-hydroxy-2(S)-isopropyl-7,7-dimethyl-8-[2(R,S)ethylaminocarbonyl-3,4-dihydro-2H1,4-benzthiazin-4-ylcarbonyl]-octansäure(N-butyl)amid (R_{f}(L)=0.40) und das 5(S)-Tertiärbutoxycarbonylamino-4(S)-hydroxy-2(S)-isopropyl-7,7dimethyl-8-[2(R,S)ethylaminocarbonyl-3,4-dihydro-2H1,4-benzthiazin-4-ylcarbonyl]-octansäure(N-butyl)amid (R_{f}(L)=0.38; FAB-MS: (M+H)⁺=649) als Diastereomerengemisch: R_{f}(S)=0.30/0.27; Rₜ(VI)=38.9/39.5min; FAB-MS: (M+H)⁺=549.

Das als Ausgangsmaterial eingesetzte 2(R,S)-Ethylaminocarbonyl-3,4-dihydro-2H1,4-benzthiazin wird ausgehend von 2.0 g 2(R,S)-methoxycarbonyl-3,4-dihydro-2H1,4-benzthiazin durch Umsetzung in 5 ml einer 2.3N-EthylaminLösung in Dimethylformamid bei 60 °C über 17 h und Reinigung mittels FC an 80 g Kieselgel (Laufmittel A) erhalten: R_{f}(A)= 0.29.

### Beispiel 95: 5(S)-Amino-4(S)-hydroxy-2(R),7,7-trimethyl-8-[2(R,S)-methoxyethylaminocarbonyl-3,4-dihydro-2H1,4-benzthiazin-4-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin- 5(S)-yl]-2(R)-methyl-propionsäure(N-butyl)amid und 138mg 2(R,S)-Methoxyethylaminocarbonyl-3,4-dihydro-2H1,4-benzthiazin über das (4O,5N-Isopropyliden)-5(S)-tertiärbutoxycarbonylamino-4(S)-hydroxy-2(R),7,7-trimethyl-8-[2(R,S)-methoxyethylaminocarbonyl-3,4-dihydro-2H1,4-benzthiazin-4-ylcarbonyl]-octansäure(N-butyl)amid (R_{f}(K)=0.38) und das 5(S)-Tertiärbutoxycarbonylamino-4(S)-hydroxy-2(R),7,7-trimethyl-8-[2(R,S)-methoxyethylaminocarbonyl-3,4-dihydro-2H1,4-benzthiazin-4-ylcarbonyl]-octansäure(N-butyl)amid (R_{f}(K)=0.23) als Diastereomerengemisch: R_{f}(P)=0.45; Rₜ(IV)=36.0/36.4min; FAB-MS: (M+H)⁺=551.

Die als Ausgangsmaterial eingesetzte Amin-Komponente wird beispielsweise wie folgt hergestellt:
839mg 2(R,S)-Ethoxycarbonyl-3,4-dihydro-2H1,4-benzthiazin in 6.6 ml einer 6 M 2-MethoxyethylaminLösung in Dimethylformamid werden bei 50°C über Nacht gerührt. Das Reaktionsgemisch wird eingeengt und der Rückstand durch FC an 25 g Kieselgel mit einem 99:1:0.1 Gemisch aus Methylenchlorid, Methanol und Ammoniak(konz.) als Laufmittel gereinigt: R_{f}(H)=0.21.

### Beispiel 96: 5(S)-Amino-4(S)-hydroxy-2(S)-isopropyl-7,7-dimethyl-8-[3(R,S)-methoxymethyl-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 188mg 3-[N-Tertiärbutoxycarbonyl-4(S)-(3-carboxy-2,2-dimethylpropyloxy-2,2-dimethyl-1,3-oxazolidin-5(S)-yl]-2(S)isopropyl-propionsäure(N-butyl)amid und 206mg 3(R,S)-Methoxymethyl-1,2,3,4-tetrahydrochinolin über das (4O,5N-Isopropyliden)-5(S)-tertiärbutoxycarbonylamino-4(S)-hydroxy-2(S)-isopropyl-7,7-dimethyl-8-[3(R,S)-methoxymethyl-1,2,3,4-tetrahydrochinolin-1-ylcarbonyl]-octansäure(N-butyl)amid (R_{f}(A)=0.54) und über das 5(S)-Tertiärbutoxycarbonylamino-4(S)-hydroxy-2(S)-isopropyl-7,7dimethyl-8-[3(R,S)-methoxymethyl-1,2,3,4-tetrahydrochinolin-1-ylcarbonyl]-octansäure(N-butyl)amid (R_{f}(A)=0.20) als Diastereomerengemisch: R_{f}(S)=0.29; Rₜ(V)=24.5min; FAB-MS: (M+H)⁺=504.

### Beispiel 97: 5(S)-Amino-4(S)-hydroxy-2(R),7,7-trimethyl-8-[3(R)- oder 3(S)-ethoxycarbonylindolin-1ylcarbonyl]-octansäure(N-butyl)amid

Die Titelverbindung wird ausgehend von 58mg 5(S)-Tertiärbutoxycarbonylamino-4(S)-hydroxy-2(R),7,7-trimethyl-8-[3(R)- oder 3(S)-ethoxycarbonylindolin-1ylcarbonyl]-octansäure(N-butyl)amid analog Beispiel 52) hergestellt und durch FC an Kieselgel (LaufmittelM) gereinigt: R_{f}(S)=0.13; Rₜ(IV)=39.5min; FAB-MS: (M+H)⁺=490.

Die Ausgangsmaterialien werden beispielsweise folgendermassen hergestellt:
a) 5(S)-Tertiärbutoxycarbonylamino-4(S)-hydroxy-2(R),7,7-trimethyl-8-[3(R)- oder-3(S)-ethoxycarbonylindolin-1ylcarbonyl]-octansäure(N-butyl)amid
   Die Titelverbindung wird ausgehend von 73mg (4O,5N-Isopropyliden)-5(S)-tertiärbutoxycarbonylamino-4(S)-hydroxy-2(R),7,7-trimethyl-8-[3(R)- oder 3(S)-ethoxycarbonylindolin-1ylcarbonyl]-octansäure(N-butyl)amid (Diastereomer I) analog Beispiel20a) erhalten und durch FC an 25 g Kieselgel (LaufmittelJ) gereinigt. Man erhält die Titelverbindung: R_{f}(P)=0.62.
b) (4O,5N-Isopropyliden)-5(S)-tertiärbutoxycarbonylamino-4(S)-hydroxy-2(R),7,7-trimethyl-8-[3(R)- oder 3(S)-ethoxycarbonylindolin-1ylcarbonyl]-octansäure(N-butyl)amid
   Die Titelverbindung wird ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methylpropionsäure(N-butyl)amid und 126mg Indolin3(R,S)-carbonsäureethylester in analoger Weise wie in Beispiel45b) beschrieben hergestellt und mittels FC an 80 g Kieselgel (LaufmittelB) unter Trennung der beiden Stereoisomeren gereinigt. Man erhält die beiden reinen Diastereomere der Titelverbindung: R_{f}(A)=0.48 für Diastereomer I; R_{f}(A)=0.41 für Diastereomer II.
c) Indolin3(R,S)-carbonsäureethylester
   Bei 0°C werden 0.83 g 1-(Tertiärbutoxycarbonyl)indolin3(R,S)carbonsäureethylester in 5 ml 4N Salzsäure in Dioxan über 1h gerührt. Das Reaktionsgemisch wird eingeengt, der ölige Rückstand in Methylenchlorid aufgenommen und mit wenig gesättigter NatriumhydrogencarbonatLösung und gesättigter Natriumchloridlösung gewaschen. Nach Trocknen über Natriumsulfat wird die organische Phase eingedampft, und der Rückstand mittels FC an 80 g Kieselgel (LaufmittelB) gereinigt: R_{f}(A)=0.65; IR(Chloroform): 1730(s).
d) 1-(Tertiärbutoxycarbonyl)indolin3(R,S)-carbonsäurethylester
   Eine Lösung von 1.0 g 1-(Tertiärbutoxycarbonyl)indol3-carbonsäureethylester in 20ml wasserfreiem Ethanol wird in Gegenwart von 0.50 g Palladium auf Kohle (5% Pd) bei 80°C bis zum Ende der Umsetzung hydriert. Nach Filtration des Reaktionsgemisches über Celite 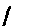 545 und Einengen des Filtrats wird der ölige Rückstand mittels FC an 15 g Kieselgel mit einem 15:1-Gemisch aus Hexan und Essigester als Laufmittel gereinigt. Man erhält die Titelverbindung: R_{f}(G)=0.39; Anal. Kalk. für C₁₆H₂₁NO₄: C65.96%, H7.27%, N4.81%; gef. C65.67%, H7.18%, N4.77%.
e) 1-(Tertiärbutoxycarbonyl)indol3-carbonsäureethylester
   Eine Lösung von 3.15 g Indol3-carbonsäureethylester, 4.36 g Di-tertiärbutylcarbonat und 0.02 g 4-DMAP in 30ml Acetonitril wird bei Raumtemperatur über 15min gerührt. Danach wird das Reaktionsgemisch mit 200ml Essigester verdünnt. Die organische Phase wird nacheinander mit verdünnter Salzsäure, Wasser und gesättigter Natriumchloridlösung gewaschen, über Natriumsulfat getrocknet und eingeengt. Reinigung mittels FC an 80 g Kieselgel mit einem 15:1-Gemisch aus Hexan und Essigester als Laufmittel ergibt 5.1 g der Titelverbindung: R_{f}(G)=0.41.
f) Indol3-carbonsäureethylester
   Die Mischung von 3.23 g Indol3-carbonsäure in 85ml Methanol und 8.5 ml Wasser wird durch Zugabe einer 20%igen wäßrigen CäsiumcarbonatLösung auf pH=7 gebracht, und anschliessend im Vakuum eingedampft. Der Rückstand wird zweimal mit jeweils 30ml Dimethylformamid aufgenommen und erneut eingeengt. Anschliessend versetzt man mit 30ml Dimethylformamid, addiert 1.75ml Jodethan bei Raumtemperatur und rührt über 4h nach. Nach Einengen des Reaktionsgemischs wird der Rückstand an 30 g Kieselgel (LaufmittelD) mittels FC gereinigt. Man erhält die Titelverbindung (3.15 g): R_{f}(E)= 0.11.

### Beispiel 98: 5(S)-Amino-4(S)-hydroxy-2(R),7,7-trimethyl-8-[3(R)- oder 3(S)-ethoxycarbonylindolin-1-ylcarbonyl]-octansäure(N-butyl)amid

Die Titelverbindung wird ausgehend von 41 mg 5(S)-Tertiärbutoxycarbonylamino-4(S)-hydroxy-2(R),7,7-trimethyl-8-[3(R)- oder 3(S)-ethoxycarbonylindolin-1ylcarbonyl]-octansäure(N-butyl)amid analog Beispiel97) hergestellt und durch FC an Kieselgel (LaufmittelM) gereinigt. Man erhält die Titelverbindung: R_{f}(S)=0.26; Rₜ(IV)=39.5min; FAB-MS: (M+H)⁺=490.

Das Ausgangsmaterial wird folgendermassen hergestellt:
a) 5(S)-Tertiärbutoxycarbonylamino-4(S)-hydroxy-2(R),7,7-trimethyl-8-[3(R)- oder-3(S)-ethoxycarbonylindolin-1ylcarbonyl]-octansäure(N-butyl)amid
   Die Titelverbindung wird ausgehend von 55mg (4O,5N-Isopropyliden)-5(S)-tertiärbutoxycarbonylamino-4(S)-hydroxy-2(R),7,7-trimethyl-8-[3(R)- oder 3(S)-ethoxycarbonylindolin-1ylcarbonyl]-octansäure(N-butyl)amid (Beispiel97b), Diastereomer II) analog wie in Beispiel97a) beschrieben erhalten und durch FC an 25 g Kieselgel (LaufmittelJ) gereinigt: R_{f}(P)=0.58.

### Beispiel 99: 5(S)-Amino-4(S)-hydroxy-2(R),7,7-trimethyl-8-[3(R)- oder-3(S)-methylaminocarbonylamino-1,2,3,4-tetrahydrochinolin-1-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 40 mg (4O,5N-Isopropyliden)-5(S)-tertiärbutoxycarbonylamino-4(S)-hydroxy-2(R),7,7-trimethyl-8-[3(R)-oder 3(S)-methylaminocarbonylamino-1,2,3,4-tetrahydrochinolin-1ylcarbonyl]-octansäure(N-butyl)amid (Diastereomer I) über das 5(S)-Tertiärbutoxycarbonylamino-4(S)-hydroxy-2(R),7,7-trimethyl-8-[3(R)- oder 3(S)-methylaminocarbonylamino-1,2,3,4-tetrahydrochinolin-1ylcarbonyl]-octansäure(N-butyl)amid (R_{f}(P)=0.49; FAB-MS: (M+H)⁺= 604) als reines Stereoisomer: R_{f}(S)=0.13; Rₜ(IV)=13.6 min; FAB-MS: (M+H)⁺= 504.

Die Ausgangsmaterialen wird beispielsweise folgendermassen hergestellt:
a) (4O,5N-Isopropyliden)-5(S)-tertiärbutoxycarbonylamino-4(S)-hydroxy-2(R),7,7-trimethyl-8-[3(R)- oder-3(S)-methylaminocarbonylamino-1,2,3,4-tetrahydrochinolin-1ylcarbonyl]-octansäure(N-butyl)amid
   Ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 135 mg 3(R,S)-Methylaminocarbonylamino-1,2,3,4-tetrahydrochinolin und chromatographischer Reinigung des Rohproduktes unter Auftrennung der beiden 3(R)- und 3(S)-Diastereomeren erhält man die reine Titelverbindung (Diastereomer I): R_{f}(P)=0.55; sowie Diastereomer II: R_{f}(P)=0.50.
b) 3(R,S)-Methylaminocarbonylamino-1,2,3,4-tetrahydrochinolin
   1.4 g 3-(Methylaminocarbonylamino)chinolin in 40 ml Ethanol werden analog wie in Beispiel 93) beschrieben hydriert. Das Rohprodukt wird an 200 g Kieselgel mit einem 99:1:1-Gemisch aus Methylenchlorid, Methanol und Eisessig als Laufmittel gereinigt: R_{f}(L)=0.34; MS: M⁺= 205.
c) 3-(Methylaminocarbonylamino)chinolin
   Zu einer Lösung von 2.0 g 3-Aminochinolin in 15 ml Methylenchlorid werden unter Argon 0.99 ml Methylisocyanat addiert und über 2 h unter Rückfluss erhitzt. Es werden erneut 0.95 ml Methylisocyanat zugesetzt und das Reaktionsgemisch erneut 2 h rückflussiert. Das Rohprodukt wird an 100 g Kieselgel (Laufmittelgradient von L zu P) chromatographiert. Man erhält die Titelverbindung als kristallinen Festkörper: R_{f}(L)=0.2; M⁺= 201.

### Beispiel 100: 5(S)-Amino-4(S)-hydroxy-2(R),7,7-trimethyl-8-[3(R)- oder 3(S)-methylaminocarbonylamino-1,2,3,4-tetrahydrochinolin-1-ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 40 mg (4O,5N-Isopropyliden)-5(S)-tertiärbutoxycarbonylamino-4(S)-hydroxy-2(R),7,7-trimethyl-8-[3(R)- oder 3(S)-methylaminocarbonylamino-1,2,3,4-tetrahydrochinolin-1ylcarbonyl]-octansäure(N-butyl)amid (Diastereomer II; Beispiel 99a)) über das 5(S)-Tertiärbutoxycarbonylamino-4(S)-hydroxy-2(R),7,7-trimethyl-8-[3(R)- oder 3(S)-methylaminocarbonylamino-1,2,3,4-tetrahydrochinolin-1ylcarbonyl]-octansäure(N-butyl)amid (R_{f}(95:5:1-Gemisch aus Methylenchlorid, Methanol und Ammoniak konz.)=0.22; FAB-MS: (M+H)⁺= 604): R_{f}(S)=0.17; Rₜ(IV)=13.3 min; FAB-MS: (M+H)⁺= 504.

### Beispiel 101: 5(S)-Amino-4(S)-hydroxy-2(R),7,7-trimethyl-8-[3(R,S)-phenyl-1,2,3,4-tetrahydrochinolin-1ylcarbonyl]-octansäure(N-butyl)amid

In analoger Weise wie in den Beispielen 52, 52a und 52b) für die entsprechenden Reaktionsstufen beschrieben erhält man die Titelverbindung ausgehend von 100mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 137mg 3(R,S)-Phenyl-1,2,3,4-tetrahydrochinolin über das (4O,5N-Isopropyliden)-5(S)tertiärbutoxycarbonylamino-4(S)-hydroxy-2(R),7,7-trimethyl-8-[3(R,S)phenyl-1,2,3,4-tetrahydrochinolin-1ylcarbonyl]- octansäure(N-butyl)amid (R_{f}(A)=0.57) und das 5(S)-Tertiärbutoxycarbonylamino-4(S)-hydroxy-2(R),7,7-trimethyl-8-[3(R,S)phenyl-1,2,3,4-tetrahydrochinolin-1ylcarbonyl]-octansäure(N-butyl)amid (R_{f}(P)=0.59) als Diastereomerengemisch: R_{f}(S)=0.28; Rₜ(IV)=32.0min; FAB-MS: (M+H)⁺=508.

Das als Ausgangsmaterial eingesetzte 3-(R,S)-Phenyl-1,2,3,4-tetrahydrochinolin wird durch Hydrierung von 410mg 3-Phenylchinolin, das nach der von J.Stavennuiter et al. in Heterocycles 26, 2711(1987), beschriebenen Methode hergestellt wird, gelöst in 10ml Ethanol, in Gegenwart von 100mg Palladium auf Kohle (10%Palladium) bei 70°C unter Normaldruck und anschliessender Reinigung des Rohprodukts mittels FC an 30 g Kieselgel mit einem 9:1 Gemisch aus Hexan und Essigester als Laufmittel hergestellt: Smp.84-6°C; R_{f}(F)=0.39; MS: M⁺=209.

### Beispiel 102: 5(S)-Amino-4(S)-hydroxy-2(R),7,7-trimethyl-8-[3(R,S)-methoxycarbonyl-4-oxo-1,2,3,4-tetrahydrochinolin-1-ylcarbonyl]-octansäure(N-butyl)amid

Analog den in den Beispielen 20, 20a und 20b) für die entsprechenden Reaktionsstufen beschriebenen Verfahren erhält man die Titelverbindung ausgehend von 301 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 410 mg 3(R,S)-Methoxycarbonyl-4-oxo-1,2,3,4-tetrahydrochinolin (hergestellt nach G. R. Proctor et al., J.Chem.Soc. Perkin Trans. I, 1803 (1972)) über das (4O,5N-Isopropyliden)-5(S)-tertiärbutoxycarbonylamino-4(S)-hydroxy-2(R),7,7-trimethyl-8-[3(R,S)-methoxycarbonyl-4-oxo1,2,3,4-tetrahydrochinolin-1-yl-carbonyl]-octansäure(N-butyl)amid (analog Beispiel 1a) und Reinigung an 20 g Kieselgel mit einem 1:3-Gemisch aus Essigester und Toluol als Laufmittel: R_{f}(1:3-Gemisch aus Essigester und Toluol)= 0.15; Rₜ(I)= 27.3 min) und das 5(S)-Tertiärbutoxycarbonylamino-4(S)-hydroxy-2(R),7,7-trimethyl-8-[3(R,S)-methoxycarbonyl-4-oxo1,2,3,4-tetrahydrochinolin-1-ylcarbonyl]-octansäure(N-butyl)amid (Reinigung an 10 g Kieselgel mit Laufmittel A und Essigester) als Diastereomerengemisch: R_{f}(V)= 0.35; Rₜ(I)= 20.5/21.3 min; FAB-MS: (M+H)⁺= 504.

### Beispiel 103: 5(S)-Amino-4(S)-hydroxy-2(R),7,7-trimethyl-8-[3(R,S)allylaminocarbonyl-1,2,3,4-tetrahydrochinolin-1-ylcarbonl]-octansäure(N-butyl)amid

Analog den in den Beispielen 20, 20a und 20b) für die entsprechenden Reaktionsstufen beschriebenen Verfahren erhält man die Titelverbindung ausgehend von 205 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 487 mg 3(R,S)-Allylaminocarbonyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-allylaminocarbonyl-1,2,3,4-tetrahydrochinolin-1-yl-carbonyl]-octansäure(N-butyl)amid (R_{f}(X)= 0.45; Rₜ(I)= 29.1/29.5 min) und das 5(S)-Tertiärbutoxycarbonylamino-4(S)-hydroxy-2(R),7,7-trimethyl-8-[3(R,S)-allylaminocarbonyl-1,2,3,4-tetrahydrochinolin-1-ylcarbonyl]-octansäure(N-butyl)amid (R_{f}(X)= 0.30; Rₜ(I)= 25.2 min) als Diastereomerengemisch: R_{f}(V)= 0.28/0.34; Rₜ(I)= 19.1/19.2 min; FAB-MS: (M+H)⁺= 515.

### Beispiel 104: 5(S)-Amino-4(S)-hydroxy-2(R),7,7-trimethyl-8-[3(R,S)-methoxycarbonylaminomethyl-1,2,3,4-tetrahydrochinolin-1ylcarbonyl]-octansäure(N-butyl)amid

Analog den in den Beispielen 20, 20a und 20b) für die entsprechenden Reaktionsstufen beschriebenen Verfahren erhält man die Titelverbindung ausgehend von 600 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 866 mg 3(R,S)-Methoxycarbonylaminomethyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxycarbonylaminomethyl-1,2,3,4-tetrahydrochinolin-1-ylcarbonyl]-octansäure(N-butyl)amid (R_{f}(X)= 0.45) und das 5(S)-Tertiärbutoxycarbonylamino-4(S)-hydroxy-2(R),7,7-trimethyl-8-[3(R,S)-methoxycarbonylaminomethyl-1,2,3,4-tetrahydrochinolin-1-ylcarbonyl]-octansäure(N-butyl)amid (R_{f}(X)= 0.25; Rₜ(I)= 29.6 min; FAB-MS: (M+H)⁺= 619) als Diastereomerengemisch: R_{f}(V)= 0.23; Rₜ(I)= 21.9 min; FAB-MS: (M+H)⁺= 519.

Die Amin-Komponente wird beispielsweise wie folgt hergestellt:
a) 3(R,S)-methoxycarbonylaminomethyl-1,2,3,4-tetrahydrochinolin
   Die Titelverbindung wird aus 3.1 g 3(R,S)-Aminomethyl-1,2,3,4-tetrahydrochinolin und 2.2 ml Chlorameisensäuremethylester in analoger Weise wie in Beispiel54) beschrieben (2h Rühren bei 0°C) hergestellt und chromatographisch an Kieselgel mit einem 1:2-Gemisch aus Hexan und Essigester als Laufmittel gereinigt: R_{f}(A)=0.25; FAB-MS: (M+H)⁺= 221.
b) 3(R,S)-Aminomethyl-1,2,3,4-tetrahydrochinolin
   Eine Lösung von 10 g Chinolin3-carbonsäurenitril in 500ml Ethanol wird in Gegenwart von 1.0 g Palladium auf Kohle (10%Palladium) bei 45°C über 40h unter Normaldruck hydriert: R_{f}(A)=0.61.

### Beispiel 105: 4(S)-Hydroxy-2(S)-isopropyl5(S)-methansulfonylamino-7,7-dimethyl-8-[2(R)- oder 2(S)-methylaminocarbonyl-3,4-dihydro-2H-1,4-benzthiazin-4-ylcarbonyl]-octansäure(N-butyl)amid

Zu einer Lösung von 5(S)-Amino-4(S)-hydroxy-2(S)-isopropyl-7,7-dimethyl-8-[2(R)-oder 2(S)-methylaminocarbonyl-3,4-dihydro-2H-1,4-benzthiazin-4-ylcarbonyl]-octansäure(N-butyl)amid (Beispiel 90) in Methylenchlorid-Dimethylformamid werden nachfolgend 27 µl Triethylamin und 15 mg Methansulfonsäureanhydrid bei 0 °C addiert. Das Reaktionsgemisch wird über Nacht gerührt und anschliessend direkt über 10 g Kieselgel (Laufmittel P) chromatographiert. Man erhält die Titelverbindung: R_{f}(P)= 0.44; Rₜ(IV)= 41.6 min; FAB-MS: (M+H)⁺= 613.

### Beispiel 106: 5(S)-Amino-4(S)-hydroxy-2(R),-7,7-trimethyl-8-[3(R,S)-N,N-dimethylaminomethyl-1,2,3,4-tetrahydrochinolin-1-ylcarbonyl]-octansäure(N-butyl)amid

Analog den in den Beispielen 20, 20a und 20b) für die entsprechenden Reaktionsstufen beschriebenen Verfahren erhält man die Titelverbindung ausgehend von 151 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 146 mg 3(R,S)-N,N-Dimethylaminomethyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-N,N-dimethylaminomethyl-1,2,3,4-tetrahydrochinolin-1-ylcarbonyl]-octansäure(N-butyl)amid (analog Beispiel 1a) und Reinigung an 20 g Kieselgel (Laufmittel N): R_{f}(N)= 0.39; FAB-MS: (M+H)⁺=629) und das 5(S)-Tertiärbutoxycarbonylamino-4(S)-hydroxy-2(R),7,7-trimethyl-8-[3(R,S)-N,N-dimethylaminomethyl-1,2,3,4-tetrahydrochinolin-1-ylcarbonyl]-octansäure(N-butyl)amid (Reinigung, nach Extraktion mit Essigester aus Natriumcarbonat-Lösung, mittels FC an 10 g Kieselgel (Laufmittel J): R_{f}(V)= 0.18) nach der in Beispiel 1) beschriebenen Methode als Diastereomerengemisch: R_{f}(V)= 0.06; Rₜ(I)= 15.3 min; FAB-MS: (M+H)⁺= 489. Das als Ausgangsmaterial eingesetzte 3(R,S)-N,N-Dimethylaminomethyl-1,2,3,4-tetrahydrochinolin wird analog Beispiel 1m) ausgehend von 165 mg (R,S)-N,N-Dimethylaminocarbonyl-1,2,3,4-tetrahydrochinolin (Beispiel 23c) und 80 mg Lithiumaluminiumhydrid erhalten. Nach üblicher Aufarbeitung und Reinigung des Rohprodukts erhält man das 3(R,S)-N,N-Dimethylaminomethyl-1,2,3,4-tetrahydrochinolin: R_{f}(O)= 0.20; MS: M⁺= 190.

### Beispiel 107: 5(S)-Amino-4(S)-hydroxy-2(R),7,7-trimethyl-8-[2(R,S)-methoxyiminomethyl-3,4-dihydro-2H1,4-benzoxazin-4-ylcarbonyl]-octansäure(N-butyl)amid

Analog den in den Beispielen 20, 20a und 20b) für die entsprechenden Reaktionsstufen beschriebenen Verfahren erhält man die Titelverbindung ausgehend von 205 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 220 mg 2(R,S)-Methoxyiminomethyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-methoxyiminomethyl-3,4-dihydro-2H-1,4-benzoxazin-4-ylcarbonyl]-octansäure(N-butyl)amid (analog Beispiel 1a): R_{f}(A)= 0.44) und das 5(S)-Tertiärbutoxycarbonylamino-4(S)-hydroxy-2(R),7,7-trimethyl-8-[2(R,S)-methoxyiminomethyl-3,4-dihydro-2H-1,4-benzoxazin-4-ylcarbonyl]-octansäure(N-butyl)amid (R_{f}(V)= 0.64; FAB-MS: (M+H)⁺= 591) analog wie in Beispiel 1) beschrieben als Diastereomerengemisch: R_{f}(V)= 0.25; Rₜ(I)= 20.5/20.7 min; FAB-MS: (M+H)⁺= 491.

Die als Ausgangsmaterial eingesetzte Amin-Komponente wird beispielsweise folgendermassen hergestellt:
Zu einer Lösung von 600 mg N-Benzyloxycarbonyl-2(R,S)-formyl-3,4-dihydro-2H-1,4-benzoxazin (R_{f} = 0.31; hergestellt mittels SwernOxidation (Beispiel 1g) aus Beispiel 87c) in 30 ml Pyridin werden 3.42 g O-MethylhydroxylaminHydrochlorid addiert, das Reaktionsgemisch anschliessend für 36 h bei Raumtemperatur gerührt und dann eingeengt. Das Rohprodukt wird in Essigester gelöst und mit einer 1 M NatriumhydrogencarbonatLösung, Wasser und gesättigter Natriumchloridlösung gewaschen. Man erhält das N-Benzyloxycarbonyl-2(R,S)-methoxyiminomethyl-3,4-dihydro-2H-1,4-benzoxazin (540 mg): R_{f}(A)= 0.61. Die weitere Umsetzung analog Beispiel 87a) und Reinigung des Rohprodukts mittels FC an 50 g Kieselgel (Laufmittel N) ergibt das 2(R,S)-Methoxyiminomethyl-3,4-dihydro-2H-1,4-benzoxazin: R_{f}(A)= 0.51.

### Beispiel 108: 5(S)-Amino-4(S)-hydroxy-2(R),7,7-trimethyl-8-[3(R,S)-carbamoyl-3,4-dihydro-2H-1,4-benzoxazin-4-ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 70mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 82mg 3(R,S)Carbamoyl-3,4-dihydro-2H1,4-benzoxazin über das (4O,5N-Isopropyliden)-5(S)-tertiärbutoxycarbonylamino-4(S)-hydroxy-2(R),7,7-trimethyl-8-[3(R,S)carbamoyl-3,4-dihydro-2H1,4-benzoxazin-4-ylcarbonyl]-octansäure(N-butyl)amid (R_{f}(P)=0.47) und das 5(S)-Tertiärbutoxycarbonylamino-4(S)-hydroxy-2(R),7,7-trimethyl-8-[3(R,S)carbamoyl-3,4-dihydro-2H1,4-benzoxazin-4-ylcarbonyl]-octansäure(N-butyl)amid (R_{f}(P)=0.38) analog wie in Beispiel 52) beschrieben als Diastereomerengemisch: R_{f}(S)=0.12/0.10; Rₜ(IV)=32.2/33.6min; FAB-MS: (M+H)⁺=477.

Die als Ausgangsmaterial eingesetzte Amin-Komponente wird beispielsweise folgendermassen hergestellt:
1.20 g 3(R,S)-Ethoxycarbonyl-3,4-dihydro-2H-1,4-benzoxazin (erhalten als Nebenprodukt bei der Herstellung von 2(R,S)-Ethoxycarbonyl-3,4-dihydro-2H-1,4-benzoxazin; Beispiel 16a)) werden in 20 ml einer 6N-AmmoniakLösung in Methanol gelöst und während 60 h im Bombenrohr bei 50 °C gehalten. Nach Einengen und Reinigung des Rohprodukts mittels FC an 180 g Kieselgel (Laufmittel O) erhält man das 3(R,S)Carbamoyl-3,4-dihydro-2H1,4-benzoxazin: R_{f}(T)= 0.26.

### Beispiel 109: 5(S)-Amino-4(S)-hydroxy-2(R),7,7-trimethyl-8-[3(R)-oder 3(S)-ethoxycarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 125 mg (4O,5N-Isopropyliden)-5(S)-tertiärbutoxycarbonylamino-4(S)-hydroxy-2(R),7,7-trimethyl-8-[3(R)- oder 3(S)-ethoxycarbonylamino-1,2,3,4-tetrahydrochinolin-1ylcarbonyl]-octansäure(N-butyl)amid (Diastereomer I) über das 5(S)-Tertiärbutoxycarbonylamino-4(S)-hydroxy-2(R),7,7-trimethyl-8-[3(R)- oder 3(S)-ethoxycarbonylamino-1,2,3,4-tetrahydrochinolin-1-ylcarbonyl]-octansäure(N-butyl)amid (R_{f}(P)=0.43) als reines Stereoisomer: R_{f}(S)=0.21; Rₜ(IV)=37.6 min; FAB-MS: (M+H)⁺=519.

Die Ausgangsmaterialien werden beispielsweise folgendermassen hergestellt:
a) (4O,5N-Isopropyliden)-5(S)-tertiärbutoxycarbonylamino-4(S)-hydroxy-2(R),7,7-trimethyl-8-[3(R)- oder 3(S)-ethoxycarbonylamino-1,2,3,4-tetrahydrochinolin-1ylcarbonyl]-octansäure(N-butyl)amid
   Die Titelverbindung wird ausgehend von 200 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 289 mg 2(R,S)Ethoxycarbonylamino-1,2,3,4-tetrahydrochinolin und FC an 25 g Kieselgel (LaufmittelGradient von C zu A) unter Auftrennung der beiden 3(R)- und 3(S)-Stereoisomeren. Man erhält die reine Titelverbindung (Diastereomer I): R_{f}(A)=0.28; sowie Diastereomer II: R_{f}(A)=0.20.
b) 3(R,S)Ethoxycarbonylamino-1,2,3,4-tetrahydrochinolin
   Zu einer Lösung von 0.5 g 3(R,S)-Amino1,2,3,4-tetrahydrochinolin in 6 ml Toluol werden unter kräftigem Rühren nacheinander 10 ml einer 10%igen Natronlauge und 0.34 ml Ethylchloroformat addiert und nach beendeter Zugabe noch 5 min gerührt. Nach üblicher Aufarbeitung und Chromatographie des Rohprodukts an 25 g Kieselgel mit Methylenchlorid als Laufmittel erhält man die Titelverbindung: R_{f}(P)=0.78; MS: M⁺= 220.
c) 3(R,S)-Amino1,2,3,4-tetrahydrochinolin
   Die Lösung von 35 g 3-Aminochinolin in 330 ml Ethanol wird in Gegenwart von insgesamt 27 g RaneyNickel bei 55 °C und einem Druck von 90 bar über 44 h hydriert. Nach üblicher Aufarbeitung wird das Rohprodukt an 900 g Kieselgel (Laufmittel S) gereinigt: R_{f}(S)= 0.40; MS: M⁺= 148.

### Beispiel 110: 5(S)-Amino-4(S)-hydroxy-2(R),7,7-trimethyl-8-[3(R)- oder 3(S)-ethoxycarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 150 mg (4O,5N-Isopropyliden)-5(S)-tertiarbutoxycarbonylamino-4(S)-hydroxy-2(R),7,7-trimethyl-8-[3(R)- oder 3(S)-ethoxycarbonylamino-1,2,3,4-tetrahydrochinolin-1ylcarbonyl]-octansäure(N-butyl)amid (Diastereomer II, Beispiel 109a)) über das 5(S)-Tertiärbutoxycarbonylamino-4(S)-hydroxy-2(R),7,7-trimethyl-8-[3(R)- oder 3(S)-ethoxycarbonylamino-1,2,3,4-tetrahydrochinolin-1ylcarbonyl]-octansäure(N-butyl)amid (R_{f}(P)=0.45) als reines Stereoisomer: R_{f}(S)=0.18; Rₜ(IV)=37.4 min; FAB-MS: (M+H)⁺=519.

### Beispiel 111: 5(S)-Amino-4(S)-hydroxy-2(R),7,7-trimethyl-8-[3(R)- oder 3(S)-propyloxycarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 118 mg (4O,5N-Isopropyliden)-5(S)-tertiärbutoxycarbonylamino-4(S)-hydroxy-2(R),7,7-trimethyl-8-[3(R)- oder 3(S)propyloxycarbonylamino-1,2,3,4-tetrahydrochinolin-1ylcarbonyl]-octansäure(N-butyl)amid (Diastereomer I) über das 5(S)-Tertiärbutoxycarbonylamino-4(S)-hydroxy-2(R),7,7-trimethyl-8-[3(R)- oder 3(S)propyloxycarbonylamino-1,2,3,4-tetrahydrochinolin-1ylcarbonyl]-octansäure(N-butyl)amid (R_{f}(P)=0.44) als reines Stereoisomer: R_{f}(S)=0.29; Rₜ =(IV)=39.7 min; FAB-MS: (M+H)⁺= 533.

Die Ausgangsmaterialien werden beispielsweise folgendermassen hergestellt:
a) (4O,5N-Isopropyliden)-5(S)-tertiärbutoxycarbonylamino-4(S)-hydroxy-2(R),7,7-trimethyl-8-[3(R)- oder 3(S)propyloxycarbonylamino-1,2,3,4-tetrahydrochinolin-1ylcarbonyl]-octansäure(N-butyl)amid
   Die Titelverbindung wird ausgehend von 150 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure-(N-butyl)amid und 231 mg 3(R,S)Propyloxycarbonylamino-1,2,3,4-tetrahydrochinolin und chromatographischer Trennung der beiden 3(R)- und 3(S)-Stereoisomeren an 25 g Kieselgel (Laufmittelgradient von C zu A). Man erhält die Titelverbindung (Diastereomer I): R_{f}(A)=0.37; sowie Diastereomer II: R_{f}(A)=030
b) 3(R,S)Propyloxycarbonylamino-1,2,3,4-tetrahydrochinolin
   analog der in Beispiel 108b) beschriebenen Methode aus 3(R,S)-Amino1,2,3,4-tetrahydrochinolin und Propylchloroformat: R_{f}(P)=0.84; MS: M⁺=234.

### Beispiel 112: 5(S)-Amino-4(S)-hydroxy-2(R),7,7-trimethyl-8-[3(R)- oder 3(S)propyloxycarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 94 mg (4O,5N-Isopropyliden)-5(S)-tertiärbutoxycarbonylamino-4(S)-hydroxy-2(R),7,7-trimethyl-8-[3(R)- oder 3(S)propyloxycarbonylamino-1,2,3,4-tetrahydrochinolin-1ylcarbonyl]-octansäure(N-butyl)amid (Diastereomer II; Beispiel 111a)) über das 5(S)-Tertiärbutoxycarbonylamino-4(S)-hydroxy-2(R),7,7-trimethyl-8-[3(R)- oder 3(S)propyloxycarbonylamino-1,2,3,4-tetrahydrochinolin-1ylcarbonyl]-octansäure(N-butyl)amid (R_{f}(P)=0.40) als reines Stereoisomer: R_{f}(S)=0.27; Rₜ =(IV)=39.8 min; FAB-MS: (M+H)⁺= 533.

### Beispiel 113: 5(S)-Amino-4(S)-hydroxy-2(R),7,7-trimethyl-8-[2(R,S)(dioxolan2-yl)-3,4-dihydro-2H-1,4-benzoxazin-4-ylcarbonyl]-octansäure(N-butyl)amid

Analog wie in den Beispielen 21a und 21b) beschrieben erhält man die Titelverbindung ausgehend von 205 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 207 mg 2(R,S)(Dioxolan2-yl)-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)(dioxolan2-yl)3,4-dihydro-2H-1,4-benzoxazin-4-ylcarbonyl]-octansäure(N-butyl)amid (R_{f}(A)= 0.22) und anschliessender Umsetzung mit Jod-trimethylsilan analog wie in Beispiel 87a) beschrieben als Diastereomerengemisch: R_{f}(V)= 0.27; Rₜ(I)= 23.1/23.3 min; FAB-MS: (M+H)⁺= 506.

Das als Ausgangsmaterial eingesetzte 2(R,S)(Dioxolan2-yl)-3,4-dihydro-2H-1,4-benzoxazin wird durch Umsetzung von 500 mg N-Benzyloxycarbonyl-2(R,S)-formyl-3,4-dihydro-2H-1,4-benzoxazin (Beispiel 107) mit 0.28 ml Ethylenglykol in 25 ml Toluol unter Rückfluss und azeotroper Entfernung von Wasser über Nacht in Gegenwart von 25 mg p-Toluolsulfonsäure-Hydrat hergestellt. Nach üblicher Aufarbeitung und FC des Rohprodukts an Kieselgel mit einem 6:1-Gemisch aus Toluol und Essigester erhält man das N-Benzyloxycarbonyl-2(R,S)-(dioxolan2-yl)3,4-dihydro-2H-1,4-benzoxazin (R_{f}(3:1-Gemisch aus Toluol und Essigester)= 0.52). Anschliessende Hydrogenolyse analog Beispiel 1r) ergibt das 2(R,S)(Dioxolan2-yl)-3,4-dihydro-2H-1,4-benzoxazin: R_{f}(P)= 0.74; FAB-MS: (M+H)⁺= 208.

### Beispiel 114: 5(S)-Amino-4(S)-hydroxy-2,2,7,7-tetramethyl-8-[3(R)- oder 3(S)-methoxycarbonylamino-1,2,3,4-tetrahydrochinolin-1ylcarbonyl]-octansäure(N-butyl)amid

Ausgehend von 92 mg 3-[N-Tertiärbutoxycarbonyl-4(S)(3-carboxy-2,2-dimethylpropyloxy-2,2-dimethyl-1,3-oxazolidin-5(S)-yl]-2,2-dimethylpropionsäure(N-butyl)amid und 109 mg 3(R)- oder 3(S)-Methoxycarbonylamino-1,2,3,4-tetrahydrochinolin (Stereoisomer II, Beispiel 74h)) erhält man über das (4O,5N-Isopropyliden)-5(S)-tertiärbutoxycarbonylamino-4(S)-hydroxy-2,2,7,7tetramethyl-8-[3(R)- oder 3(S)-methoxycarbonylamino-1,2,3,4-tetrahydrochinolin-1ylcarbonyl]-octansäure(N-butyl)amid (R_{f}(L)= 0.31; FAB-MS: (M+H)⁺= 659) und das 5(S)-Tertiärbutoxycarbonylamino-4(S)-hydroxy-2,2,7,7tetramethyl-8-[3(R)- oder 3(S)-methoxycarbonylamino-1,2,3,4-tetrahydrochinolin-1ylcarbonyl]-octansäure(N-butyl)amid (R_{f}(L)= 0.20; FAB-MS: (M+H)⁺= 619) analog Beispiel 92) als reines Diastereomer: R_{f}(P)= 0.43; Rₜ(IV)= 35.3 min; FAB-MS: (M+H)⁺= 519.

Die Ausgangsmaterialien werden beispielsweise folgendermassen erhalten:
a) 3-[N-Tertiärbutoxycarbonyl-4(S)(3-carboxy-2,2-dimethylpropyloxy-2,2-dimethyl-1,3-oxazolidin-5(S)-yl]-2,2-dimethyl-propionsäure(N-butyl)amid
   Die Titelverbindung erhält man analog wie in Beispiel 74a) ausgehend von 204 mg 3-[N-Tertiärbutoxycarbonyl-4(S)(4-hydroxy-2,2-dimethylbutyloxy-2,2-dimethyl-1,3-oxazolidin-5(S)-yl]-2,2-dimethyl-propionsäure(N-butyl)amid: R_{f}(L)= 0.15; FAB-MS: (M+H)⁺= 471.
b) 3-[N-Tertiärbutoxycarbonyl-4(S)(4-hydroxy-2,2-dimethylbutyloxy-2,2-dimethyl-1,3-oxazolidin-5(S)-yl]-2,2-dimethylpropionsäure(N-butyl)amid
   Analog den Beispielen 74b und 74c) erhält man die Titelverbindung ausgehend von 398 mg 5(S)-[5-Benzyloxy-1(S)-tertiärbutoxycarbonylamino-3,3-dimethyl-pentyl]3,3-dimethyl-2-oxotetrahydrofuran das 3-[N-Tertiärbutoxycabonyl-4(S)(4-benzyloxy-2,2-dimethylbutyloxy-2,2-dimethyl-1,3-oxazolidin-5(S)-yl]-2,2-dimethyl-propionsäure(N-butyl)amid (309 mg; R_{f}(B)= 0.41; FAB-MS: (M+H)⁺= 547), das anschliessend in 5 ml Essigester in Gegenwart von Palladium auf Kohle (5 % Pd) über 2 h bei Raumtemperatur zur Titelverbindung hydriert wird: R_{f}(L)= 0.33; FAB-MS: (M+H)⁺= 457.
c) 5(S)-[5-Benzyloxy-1(S)-tertiärbutoxycarbonylamino-3,3-dimethyl-pentyl]3,3-dimethyl-2-oxo-tetrahydrofuran
   Die Titelverbindung wird ausgehend von 518 mg 5(S)-[5-Benzyloxy-1(S)-tertiärbutoxycarbonylamino-3,3-dimethylpentyl]-2-oxo-tetrahydrofuran und 0.18 ml Methyljodid analog dem in Beispiel 74d) beschriebenen Verfahren erhalten. Nach 3 h Rühren bei - 78 °C läßt man das Reaktionsgemisch auf 0 °C aufwärmen, rührt noch weitere 2 h und arbeitet nach üblichen Verfahren auf. Man erhält die Titelverbindung: R_{f}(D)= 0.30; FAB-MS: (M+H)⁺= 434.

### Beispiel 115: 5(S)-Amino-4(S)-hydroxy-2(R),7,7-trimethyl-8-[3(R,S)-(2oxo-oxazolidin3-yl)-1,2,3,4-tetrahydrochinolin-1ylcarbonyl]-octansäure(N-butyl)amid

Die Titelverbindung wird analog wie in den Beispielen 92, 52a und 52b) beschrieben, ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 144 mg 3(R,S)-(2Oxo-oxazolidin3-yl)-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-(2oxo-oxazolidin-3-yl)-1,2,3,4-tetrahydrochinolin-1ylcarbonyl]-octansäure(N-butyl)amid (R_{f}(P)=0.57) und das 5(S)-Tertiärbutoxycarbonylamino-4(S)-hydroxy-2(R),7,7-trimethyl-8-[3(R,S)-(2oxo-oxazolidin3-yl)-1,2,3,4-tetrahydrochinolin-1ylcarbonyl]-octansäure(N-butyl)amid (R_{f}(P)=0.48) als Diastereomerengemisch: R_{f}(P)= 0.15; Rₜ(V)= 15.7 min; FAB-MS: (M+H)⁺= 517.

Die eingesetzte Amin-Komponente wird beispielsweise folgendermassen hergestellt:
26.0 g 3-Bromchinolin und 4.35 g 2-Oxazolidinon in 200 ml Xylol werden in Gegenwart von 7.4 g Kaliumacetat und 4.8 g Kupferpulver analog dem Verfahren von B.Renger in Synthesis (1985) 856 über 9 h unter Rückfluss erhitzt. Nach Aufarbeitung des Ansatzes wird das Rohprodukt an Kieselgel mit einem 500:10:1-Gemisch aus Methylenchlorid, Methanol und Ammoniak konz. als Laufmittel gereinigt und man erhält das 3-(Chinolin3-yl)-2-oxazolidinon als kristallinen Festköper: R_{f}(500:10:1-Gemisch aus Methylenchlorid, Methanol und Ammoniak konz.)= 0.39; FAB-MS: (M+H)⁺= 215. Hydrierung von 0.67 g des erhaltenen Produkts in 10 ml Ethanol in Gegenwart von 0.1 g Palladium auf Kohle (10% Palladium) bei 50 °C über 20 h und FC an 80 g Kieselgel (Laufmittel N) ergibt das 3(R,S)-(2Oxo-oxazolidin3-yl)-1,2,3,4-tetrahydrochinolin als weissen Festkörper: R_{f}(N)= 0.61; MS: M⁺= 218.

### Beispiel 116: 5(S)-Amino-4(S)-hydroxy-2(R),7,7-trimethyl-8-[3(R,S)-methoxymethoxymethyl-1,2,3,4-tetrahydrochinolin-1ylcarbonyl]-octansäure(N-butyl)amid

Analog wie in den Beispielen 21, 21a) und 21b) für die entsprechenden Reaktionsstufen beschrieben erhält man die Titelverbindung ausgehend von 600 mg 3-[N-Tertiärbutoxycarbonyl-4(S)-(3-carboxy-2,2-dimethylpropyloxy-2,2-dimethyl-1,3-oxazolidin- 5(S)-yl]-2(R)-methyl-propionsäure(N-butyl)amid und 815 mg 3(R,S)-methoxymethoxymethyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxymethoxymethyl-1,2,3,4-tetrahydrochinolin-1ylcarbonyl]-octansäure(N-butyl)amid (R_{f}(A)= 0.14; FAB-MS: (M+H)⁺= 646) und das 5(S)-Tertiärbutoxycarbonylamino-4(S)-hydroxy-2(R),7,7-trimethyl-8-[3(R,S)-methoxymethoxymethyl-1,2,3,4-tetrahydrochinolin-1ylcarbonyl]-octansäure(N-butyl)amid (R_{f}(X)= 0.36; FAB-MS: (M+H)⁺= 606) als Diastereomerengemisch: R_{f}(X)= 0.40; Rₜ(I)= 23.5 min; FAB-MS: (M+H)⁺= 506.

Das als Ausgangsmaterial eingesetzte 3(R,S)-methoxymethoxymethyl-1,2,3,4-tetrahydrochinolin wird analog wie im Beispiel 105) für die Amin-Komponente beschrieben ausgehend von N-Benzyloxycarbonyl-3(R,S)-hydroxymethyl-1,2,3,4-tetrahydrochinolin hergestellt: R_{f}(A)= 0.44; FAB-MS: (M+H)⁺= 208.

### Beispiel 117: 5(S)-Amino-4(S)-hydroxy-2(R),7,7-trimethyl-8-[3(R,S)allyloxymethyl-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dmethyl-1,3-oxazolidin-5(S)-yl]-2(R)-methyl-propionsäure(N-butyl)amid und 134 mg 3(R,S)-Allyloxymethyl-1,2,3,4-tetrahydrochinolin über das (4O,5N-Isopropyliden)-5(S)tertiärbutoxycarbonylamino-4(S)-hydroxy-2(R),7,7-trimethyl-8-[3(R,S)allyloxymethyl-1,2,3,4-tetrahydrochinolin-1ylcarbonyl]-octansäure(N-butyl)amid (R_{f}(A)=0.48) und das 5(S)-Tertiärbutoxycarbonylamino-4(S)-hydroxy-2(R),7,7-trimethyl-8-[3(R,S)allyloxymethyl-1,2,3,4-tetrahydrochinolin-1ylcarbonyl]-octansäure(N-butyl)amid (R_{f}(A)=0.24) als Diastereomerengemisch: R_{f}(S)= 0.25; Rₜ(IV)= 42.3 min; FAB-MS: (M+H)⁺= 502.

Die eingesetzte Aminkomponente wird beispielsweise wie folgt erhalten:
Zu einer Lösung von 0.6 g 1-Tertiärbutoxycarbonyl-3(R,S)-hydroxymethyl-1,2,3,4-tetrahydrochinolin in 7 ml Tetrahydrofuran werden bei 0 °C 0.5 g einer Kaliumhydrid-Suspension (20% in Oel) addiert, 30 min gerührt und anschliessend 0.58 ml Allylbromid hinzugefügt. Nach 45 min Rühren bei 0 °C wird der Ansatz wie üblich aufgearbeitet und das Rohprodukt an 50 g Kieselgel (Laufmittel F) gereinigt. Man erhält das 1-Tertiärbutoxycarbonyl-3(R,S)-allyloxymethyl-1,2,3,4-tetrahydrochinolin als farbloses Oel (R_{f}(F)= 0.40), das anschliessend analog Beispiel 20a) umgesetzt wird. Reinigung mittels FC an 50 g Kieselgel mit Methylenchlorid als Laufmittel ergibt das 3(R,S)-Allyloxymethyl-1,2,3,4-tetrahydrochinolin: R_{f}(F)= 0.40; MS: M⁺= 203.

### Beispiel 118: 5(S)-Amino-4(S)-hydroxy-2(R),7,7-trimethyl-8-[3(R,S)-methoxyethoxymethyl-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 146 mg 3(R,S)-methoxyethoxymethyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxyethoxymethyl-1,2,3,4-tetrahydrochinolin-1ylcarbonyl]-octansäure(N-butyl)amid (R_{f}(A)= 0.22) und das 5(S)-Tertiärbutoxycarbonylamino-4(S)-hydroxy-2(R),7,7-trimethyl-8-[3(R,S)-methoxyethoxymethyl-1,2,3,4-tetrahydrochinolin-1ylcarbonyl]-octansäure(N-butyl)amid (R_{f}(H)= 0.12): R_{f}(S)= 0.22; Rₜ(IV)= 38.6 min; FAB-MS: (M+H)⁺= 520.

Die eingesetzte Aminkomponente wird beispielsweise wie folgt erhalten:
Umsetzung von 0.6 g 1-Tertiärbutoxycarbonyl-3(R,S)-hydroxymethyl-1,2,3,4-tetrahydrochinolin analog dem in Beispiel 117) beschriebenen Verfahren mit 0.64 ml (2-Bromethyl)methylether über 5 h bei Raumtemperatur und übliche Aufarbeitung ergibt das 1-Tertiärbutoxycarbonyl-3(R,S)-methoxyethoxymethyl-1,2,3,4-tetrahydrochinolin (R_{f}(A)= 0.67), das wie in Beispiel 117) für die entsprechende Reaktionsstufe beschrieben zum 3(R,S)-methoxyethoxymethyl-1,2,3,4-tetrahydrochinolin umgesetzt wird: R_{f}(A)= 0.53; MS: M⁺= 221.

### Beispiel 119: 5(S)-Amino-4(S)-hydroxy-2(R),7,7-trimethyl-8-[3(R,S)-(2-oxopyrrolidin-1-yl)-1,2,3,4-tetrahydrochinolin-1-ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 142 mg 3(R,S)-(2-Oxopyrrolidin-1-yl)-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-(2-oxopyrrolidin-1-yl)-1,2,3,4-tetrahydrochinolin-1- ylcarbonyl]-octansäure(N-butyl)amid (R_{f}(L)=0.31) und das 5(S)-Tertiärbutoxycarbonylamino-4(S)-hydroxy-2(R),7,7-trimethyl-8-[3(R,S)-(2-oxopyrrolidin-1-yl)-1,2,3,4-tetrahydrochinolin-1-ylcarbonyl]-octansäure(N-butyl)amid (R_{f}(L)=0.23) als Diastereomerengemisch : R_{f}(S)= 0.24; Rₜ(IV)= 35.4 min; FAB-MS: (M+H)⁺= 515.

Die eingesetzte Aminkomponente wird beispielsweise wie folgt hergestellt:
Die Mischung von 4.26 g 2-Pyrrolidon, 26.0 g 3-Bromchinolin, 7.4 g Kaliumacetat und 4.8 g Kupferpulver in 200 ml Xylol werden analog wie in Beispiel 115) über 12 h unter Rückfluss erhitzt. Nach Aufarbeitung des Ansatzes wird das Rohprodukt an 250 g Kieselgel (Laufmittel K) gereinigt. Man erhält das 1-(Chinolin3-yl)-2-oxopyrrolidin als kristallinen Festkörper: R_{f}(L)= 0.32.
Die Lösung von 1.0 g des so erhaltenen Produkts in 20 ml Ethanol werden in Gegenwart von 0.2 g Palladium auf Kohle (10% Palladium) bei 50 °C über 22 h hydriert. Nach üblicher Aufarbeitung reinigt man das Rohprodukt mittels FC an 30 g Kieselgel (Laufmittel K) zum kristallinen 3(R,S)-(2-Oxopyrrolidin-1-yl)-1,2,3,4-tetrahydrochinolin: R_{f}(K)= 0.36; MS: M⁺= 216.

### Beispiel 120: 5(S)-Amino-4(S)-hydroxy6-[1-[2(R)- oder 2(S)-methylaminocarbonyl-3,4-dihydro-2H-1,4-benzthiazin-4-ylcarbonylmethyl]cyclopropyl]-2(R)-methyl-hexansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)[1-(carboxymethyl)cyclopropylmethyl]-2,2-dimethyl-1,3-oxazolidin-5(S)-yl]-2(R)-methyl-propionsäure(N-butyl)amid und 137 mg 2(R)- oder 2(S)-methylaminocarbonyl-3,4-dihydro-2H-1,4-benzthiazin (Stereoisomer I; Beispiel 90) über das (4O,5N-Isopropyliden)-5(S)-tertiärbutoxycarbonylamino-4(S)-hydroxy6-[1-[2(R)- oder 2(S)-methylaminocarbonyl-3,4-dihydro-2H-1,4-benzthiazin-4-ylcarbonylmethyl]cyclopropyl]-2(R)-methyl-hexansäure(N-butyl)amid (R_{f}(P)= 0.54) und das 5(S)-Tertiärbutoxycarbonylamino-4(S)-hydroxy6-[1-[2(R)- oder 2(S)-methylaminocarbonyl-3,4-dihydro-2H-1,4-benzthiazin-4-ylcarbonylmethyl]cyclopropyl]-2(R)-methyl-hexansäure(N-butyl)amid (R_{f}(P)= 0.41) als reines Stereoisomer: R_{f}(P)= 0.11; Rₜ(IV)= 33.3 min; FAB-MS: (M+H)⁺= 505.

### Beispiel 121: 5(S)-Amino-4(S)-hydroxy6-[1-[3(R)- oder 3(S)-methoxycarbonylamino-1,2,3,4-tetrahydrochinolin-1-ylcarbonylmethyl]cyclopropyl]-2(R)-methyl-hexansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-[1-(carboxymethyl)cyclopropylmethyl]-2,2-dimethyl-1,3-oxazolidin-5(S)-yl]-2(R)-methyl-propionsäure(N-butyl)amid und 113 mg 3(R)- oder 3(S)-methoxycarbonylamino-1,2,3,4-tetrahydrochinolin (Stereoisomeres I in Beispiel 74h) über das (4O,5N-Isopropyliden)-5(S)-tertiärbutoxycarbonylamino-4(S)-hydroxy6-[1-[3(R)- oder 3(S)-methoxycarbonylamino-1,2,3,4-tetrahydrochinolin-1ylcarbonylmethyl]cyclopropyl]-2(R)-methyl-hexansäure(N-butyl)amid (R_{f}(P)= 0.57) und das 5(S)-Tertiärbutoxycarbonylamino-4(S)-hydroxy6-[1-[3(R)- oder 3(S)-methoxycarbonylamino-1,2,3,4-tetrahydrochinolin-1-ylcarbonylmethyl]cyclopropyl]-2(R)-methyl-hexansäure(N-butyl)amid (R_{f}(P)= 0.51) als reines Stereoisomer: R_{f}(S)= 0.20; FAB-MS: (M+H)⁺=503.

### Beispiel 122: 5(S)-Amino-4(S)-hydroxy6-[1-[3(R)- oder 3(S)-methoxycarbonylamino-1,2,3,4-tetrahydrochinolin-1-ylcarbonylmethyl]cyclopropyl]-2-(R)-methyl-hexansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-[1-(carboxymethyl)cyclopropylmethyl]-2,2-dimethyl-1,3-oxazolidin-5(S)-yl]-2(R)-methyl-propionsäure(N-butyl)amid und 3(R)- oder 3(S)-methoxycarbonylamino-1,2,3,4-tetrahydrochinolin (Stereoisomeres II in Beispiel 74h) über das (4O,5N-Isopropyliden)-5(S)-tertiärbutoxycarbonylamino-4(S)-hydroxy6-[1-[3(R)- oder 3(S)-methoxycarbonylamino-1,2,3,4-tetrahydrochinolin-1ylcarbonylmethyl]cyclopropyl]-2-(R)-methyl-hexansäure(N-butyl)amid (R_{f}(P)= 0.60) und das 5(S)-Tertiärbutoxycarbonylamino-4(S)-hydroxy6-[1-[3(R)- oder 3(S)-methoxycarbonylamino-1,2,3,4-tetrahydrochinolin-1-ylcarbonylmethyl]cyclopropyl]-2(R)-methyl-hexansäure(N-butyl)amid(R_{f}(P)=0.50) als reines Stereoisomer: R_{f}(S)=0.19; Rₜ(IV)=35.1min; FAB-MS: (M+H)⁺=503.

### Beispiel 123: 5(S)-Amino-4(S)-hydroxy-2(R),7,7-trimethyl-8-[3(R,S)-methoxyethoxy-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin- 5(S)-yl]-2(R)-methyl-propionsäure(N-butyl)amid und 136 mg 3(R,S)-Methoxyethoxy-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxyethoxy-1,2,3,4-tetrahydrochinolin-1ylcarbonyl]-octansäure(N-butyl)amid (R_{f}(P)= 0.44) und das 5(S)-Tertiärbutoxycarbonylamino-4(S)-hydroxy-2(R),7,7-trimethyl-8-[3(R,S)-methoxyethoxy-1,2,3,4-tetrahydrochinolin-1ylcarbonyl]-octansäure(N-butyl)amid (R_{f}(L)= 0.28) als Diastereomerengemisch: R_{f}(S)= 0.32; Rₜ(IV)= 37.4/38.0 min; FAB-MS: (M+H)⁺= 506.

Die eingesetzte Amin-Komponente wird beispielsweise wie folgt hergestellt:
Zu einer Lösung von 0.5 g 3-Hydroxychinolin in 10 ml Tetrahydrofuran werden unter Eiskühlung 0.75 g einer KaliumhydridSuspension (20% in Oel) addiert und über 1 h gerührt. Anschliessend gibt man 1.25 ml (2-Brom-methyl)methylether, gelöst in 10 ml Dimethylformamid, hinzu, läßt auf Raumtemperatur aufwärmen und rührt schliesslich für 1 h bei 60 °C. Nach üblicher Aufarbeitung wird das so erhaltene 3-(Methoxyethoxy)chinolin mittels FC an 80 g Kieselgel (Laufmittel B) gereinigt: R_{f}(97:3:0.5-Gemisch aus Methylenchlorid, Methanol und Ammoniak konz)= 0.31.
1.0 g des genannten ChinolinDerivates in 10 ml Ethanol werden in Gegenwart von 0.1 g Palladium auf Kohle (10 % Pd) bei 50 °C über 4 h hydriert. FC an 50 g Kieselgel mit einem 100:1-Gemisch aus Methylenchlorid und Methanol als Elutionsmittel ergibt das 3(R,S)-Methoxyethoxy-1,2,3,4-tetrahydrochinolin: R_{f}(97:3:0.5-Gemisch aus Methylenchlorid, Methanol und Ammoniak konz)= 0.49; MS: M⁺= 207.

### Beispiel 124: 5(S)-Amino-4(S)-hydroxy-2(R),7,7-trimethyl-8-[3(R,S)-propyloxy-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 126 mg 3(R,S)-Propyloxy-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-propyloxy-1,2,3,4-tetrahydrochinolin-1ylcarbonyl]-octansäure(N-butyl)amid (R_{f}(P)= 0.83) und das 5(S)-Tertiärbutoxycarbonylamino-4(S)-hydroxy-2(R),7,7-trimethyl-8-[3(R,S)-propyloxy-1,2,3,4-tetrahydrochinolin-1ylcarbonyl]-octansäure(N-butyl)amid (R_{f}(P)= 0.57) als DiastereomerenGemisch: R_{f}(S)= 0.33; Rₜ(IV)= 42.2/42.6 min; FAB-MS: (M+H)⁺= 490.

Das eingesetzte 3(R,S)-Propyloxy-1,2,3,4-tetrahydrochinolin wird nach den unter Beispiel 123) beschriebenen Verfahren über das 3-Propyloxychinolin (R_{f}(A)= 0.58; MS: (M+H)⁺= 187) erhalten: R_{f}(Methylenchlorid)= 0.35; MS: M⁺= 191.

### Beispiel 125: 5(S)-Amino-4(S)-hydroxy-2(S)-isopropyl-7,7-dimethyl-8-[3(R)- oder-3(S)-methoxycarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 80 mg 3-[N-Tertiärbutoxycarbonyl-4(S)-(3-carboxy-2,2-dimethylpropyloxy-2,2-dimethyl-1,3-oxazolidin-5(S)-yl]-2(S)isopropyl-propionsäure(N-butyl)amid und 85 mg 3(R)- oder 3(S)-Methoxycarbonylamino-1,2,3,4-tetrahydrochinolin (Stereoisomer II, Beispiel 74h) über das (4O,5N-Isopropyliden)-5(S)-tertiärbutoxycarbonylamino-4(S)-hydroxy-2(S)-isopropyl-7,7-dimethyl-8-[3(R)- oder 3(S)-methoxycarbonylamino-1,2,3,4-tetrahydrochinolin-1ylcarbonyl]-octansäure(N-butyl)amid (R_{f}(P)= 0.67) und das 5(S)-Tertiärbutoxycarbonylamino-4(S)-hydroxy-2(S)-isopropyl-7,7dimethyl-8-[3(R)- oder 3(S)-methoxycarbonylamino-1,2,3,4-tetrahydrochinolin-1ylcarbonyl]-octansäure(N-butyl)amid (R_{f}(P)= 0.59) als reines Stereoisomer: R_{f}(S)= 0.23; Rₜ(IV)= 38.5 min; FAB-MS: (M+H)⁺= 533.

### Beispiel 126: 5(S)-Amino-4(S)-hydroxy-2(R),7,7-trimethyl-8-[3(R,S)-methoxymethyloxy-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 127 mg 3(R,S)-methoxymethyloxy-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxymethyloxy-1,2,3,4-tetrahydrochinolin-1-ylcarbonyl]-octansäure(N-butyl)amid (R_{f}(A)= 0.30) und das 5(S)-Tertiärbutoxycarbonylamino-4(S)-hydroxy-2(R),7,7-trimethyl-8-[3(R,S)-methoxymethyloxy-1,2,3,4-tetrahydrochinolin-1ylcarbonyl]-octansäure(N-butyl)amid (R_{f}(L)= 0.29; FAB-MS: (M+H)⁺= 592) durch Umsetzung in 6 ml 50% Trifluoressigsäure in Methylenchlorid bei 0 °C über 15 min als DiastereomerenGemisch: R_{f}(S)= 0.36; Rₜ(IV)= 38.0/38.4 min; FAB-MS: (M+H)⁺= 492.

Das eingesetzte 3(R,S)-methoxymethyloxy-1,2,3,4-tetrahydrochinolin wird nach den unter Beispiel 123) beschriebenen Verfahren ausgehend von 0.7 g 3-Hydroxychinolin und 1.09 ml Methoxymethylchlorid über das 3-(Methoxymethyloxy)chinolin (R_{f}(A)= 0.46) erhalten: R_{f}(P)= 0.84; MS: M⁺= 193.

### Beispiel 127: 5(S)-Amino-4(S)-hydroxy-2(R),7,7-trimethyl-8-[3(R,S)cyclopropylcarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 178 mg 3(R,S)-Cyclopropylcarbonylamino-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-cyclopropylcarbonylamino-1,2,3,4-tetrahydrochinolin-1ylcarbonyl]-octansäure(N-butyl)amid (R_{f}(P)= 0.51) und das 5(S)-Tertiärbutoxycarbonylamino-4(S)-hydroxy-2(R),7,7-trimethyl-8-[3(R,S)cyclopropylcarbonylamino-1,2,3,4-tetrahydrochinolin-1ylcarbonyl]-octansäure(N-butyl)amid (R_{f}(P)= 0.37) als Diastereomerengemisch : R_{f}(S)= 0.13; Rₜ(V)= 16.6/17.0 min; FAB-MS: (M+H)⁺= 515.

Das 3(R,S)Cyclopropylcarbonylamino-1,2,3,4-tetrahydrochinolin erhält man durch Umsetzung von 1.0 g 3-Amino1,2,3,4-tetrahydrochinolin in einer Mischung von 11 ml Toluol und 3.2 ml einer 10%igen wäßrigen Natriumhydroxidlösung mit 0.65 ml Cyclopropancarbonsäurechlorid analog Beispiel 109b) als weissen Festkörper: R_{f}(P)= 0.61; MS: M⁺= 216.

### Beispiel 128: 5(S)-Amino-4(S)-hydroxy-2(R),7,7-trimethyl-8-[3(R)- oder 3(S)allyloxycarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 64 mg (4O,5N-Isopropyliden)-5(S)-tertiärbutoxycarbonylamino-4(S)-hydroxy-2(R),7,7-trimethyl-8-[3(R,S)-allyloxycarbonylamino-1,2,3,4-tetrahydrochinolin-1ylcarbonyl]-octansäure(N-butyl)amid (Diastereomer I) über das 5(S)-Tertiärbutoxycarbonylamino-4(S)-hydroxy-2(R),7,7-trimethyl-8-[3(R)- oder 3(S)-allyloxycarbonylamino-1,2,3,4-tetrahydrochinolin-1ylcarbonyl]-octansäure(N-butyl)amid (R_{f}(P)= 0.50) als reines Stereoisomer: R_{f}(S)= 0.25; Rₜ(IV)= 39.9 min; FAB-MS: (M+H)⁺= 531.

Die Ausgangsmaterialien werden beispielsweise wie folgt hergestellt:
a) (4O,5N-Isopropyliden)-5(S)-tertiärbutoxycarbonylamino-4(S)-hydroxy-2(R),7,7-trimethyl-8-[3(R)- oder 3(S)-allyloxycarbonylamino-1,2,3,4-tetrahydrochinolin-1ylcarbonyl]-octansäure(N-butyl)amid
   Die Titelverbindung wird ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 127 mg 3(R,S)-Allyloxycarbonylamino-1,2,3,4-tetrahydrochinolin und FC an 25 g Kieselgel (Laufmittel C) unter Auftrennung der 3(R)- und 3(S)-Stereoisomeren erhalten. Man erhält die Titelverbindung (Diastereomer I): R_{f}(A)= 0.46; sowie Diastereomer II: R_{f}(A)= 0.34.
b) 3(R,S)-Allyloxycarbonylamino-1,2,3,4-tetrahydrochinolin
   analog wie in Beispiel 109b) beschrieben aus 1.0 g 3(R,S)-Amino1,2,3,4-tetrahydrochinolin und 0.75 ml Allylchloroformat: R_{f}(P)= 0.81; MS: M⁺= 232.

### Beispiel 129: 5(S)-Amino-4(S)-hydroxy-2(R),7,7-trimethyl-8-[3(R)- oder 3(S)allyloxycarbonylamino-1,2,3,4-tetrahydrochinolin-1ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 45 mg (4O,5N-Isopropyliden)-5(S)-tertiärbutoxycarbonylamino-4(S)-hydroxy-2(R),7,7-trimethyl-8-[3(R)- oder 3(S)-allyloxycarbonylamino-1,2,3,4-tetrahydrochinolin-1ylcarbonyl]-octansäure(N-butyl)amid (Diastereomer II; Beispiel 128a)) über das 5(S)-Tertiärbutoxycarbonylamino-4(S)-hydroxy-2(R),7,7-trimethyl-8-[3(R)- oder 3(S)-allyloxycarbonylamino-1,2,3,4-tetrahydrochinolin-1ylcarbonyl]-octansäure(N-butyl)amid (R_{f}(P)= 0.53) als reines Stereoisomer: R_{f}(S)= 0.22; Rₜ(IV)= 39.7 min; FAB-MS: (M+H)⁺= 531.

### Beispiel 130: 4(S)-Amino-3(S)-hydroxy-1(R),6,6-trimethyl-heptandicarbonsäure-1-(N-butyl)-amid-7(N-phenyl,N-phenethyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyloxy-2,2-dimethyl-1,3-oxazolidin-5(S)-yl]-2(R)-methyl-propionsäure(N-butyl)amid und 130 mg N-Phenethylanilin über das (3O,4N-Isopropyliden)4(S)-tertiärbutoxycarbonylamino-3(S)-hydroxy-1(R),6,6trimethylheptandicarbonsäure7-(N-butyl)amid-7(N-phenyl,N-phenethyl)amid (R_{f}(K)= 0.40) und das 4(S)-Tertiärbutoxycarbonylamino-3(S)-hydroxy-1(R),6,6trimethyl-heptandicarbonsäure7-(N-butyl)amid-7(N-phenyl,N-phenethyl)amid (R_{f}(L)= 0.43): R_{f}(S)= 0.20; Rₜ(V)= 29.3 min; FAB-MS: (M+H)⁺= 496.

### Beispiel 131: 5(S)-Amino-4(S)-hydroxy-2(R),7,7-trimethyl-8-[3(R,S)-methylaminocarbonyloxy-1,2,3,4-tetrahydrochinolin-1-ylcarbonyl]-octansäure(N-butyl)amid

In analoger Weise wie in den Beispielen 92, 52a und 52b) für die entsprechenden Reaktionsstufen beschrieben erhält man die Titelverbindung ausgehend von 100 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 136 mg 3(R,S)-methylaminocarbonyloxy-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methylaminocarbonyloxy-1,2,3,4-tetrahydrochinolin-1ylcarbonyl]-octansäure(N-butyl)amid (R_{f}(K)= 0.23) und das 5(S)-Tertiärbutoxycarbonylamino-4(S)-hydroxy-2(R),7,7-trimethyl-8-[3(R,S)-methylaminocarbonyloxy-1,2,3,4-tetrahydrochinolin-1ylcarbonyl]-octansäure(N-butyl)amid (R_{f}(L)= 0.26) als DiastereomerenGemisch: R_{f}(S)= 0.27; Rₜ(V)= 13.0/13.4 min; FAB-MS: (M+H)⁺= 505.

Die eingesetzte Amin-Komponente wird beispielsweise wie folgt erhalten:
Zu einer Suspension von 1.0 g 3-Hydroxychinolin in 8 ml Methylenchlorid werden 0.98 ml Methylisocyanat addiert und das Reaktionsgemisch über 5 h unter Rückfluss erhitzt. Chromatographische Reinigung an 50 g Kieselgel (Laufmittel J) ergibt das 3-(Methylaminocarbonyloxy)chinolin als Festkörper: R_{f}(L)= 0.26.
1.17 g des genannten Garbamates, in 30 ml Ethanol gelöst, werden in Gegenwart von 0.24 g Palladium auf Kohle (10% Pd) bei 40 °C über 17 h hydriert. Chromatographische Reinigung an Kieselgel (Laufmittel C und I) ergibt das 3(R,S)-methylaminocarbonyloxy-1,2,3,4-tetrahydrochinolin als Festkörper: R_{f}(P)= 0.72; MS: M⁺= 206.

### Beispiel 132: 5(S)-Amino-4(S)-hydroxy-2(R),7,7-trimethyl-8-[2(R)- oder-2(S)-methoxymethyloxymethyl-3,4-dihydro-2H-1,4-benzoxazin-4-ylcarbonyl]-octansäure(N-butyl)amid

Ausgehend von 500 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 314 mg 2(R)- oder 2(S)-Methoxymethoxymethyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R)- oder 2(S)-methoxymethoxymethyl-3,4-dihydro-2H-1,4-benzoxazin-4-ylcarbonyl]-octansäure(N-butyl)amid (analog Beispiel 1a); R_{f}(A)= 0.25; Rₜ(VI)= 34.4 min) und das 5(S)-Tertiärbutoxycarbonylamino-4(S)-hydroxy-2(R),7,7-trimethyl-8-[2(R,S)-methoxymethoxymethyl-3,4-dihydro-2H-1,4-benzoxazin-4-ylcarbonyl]-octansäure(N-butyl)amid (R_{f}(X)= 0.38; FAB-MS: (M+H)⁺= 608) analog wie in Beispiel 1) beschrieben als reines Diastereomer: R_{f}(V)= 0.38; Rₜ(VI)= 22.5 min; FAB-MS: (M+H)⁺= 508.

Die als Ausgangsmaterial eingesetzte Amin-Komponente wird beispielsweise folgendermassen hergestellt:
Zu einer Lösung von 2.5 g enantiomerenreinem N-Benzyloxycarbonyl[2(R)- oder 2(S)-hydroxymethyl]3,4-dihydro-2H-1,4-benzoxazin ([α]_{D}= +28.8 (c= 1.0 in CHCl₃)), erhalten durch säulenchromatographische Trennung des Racemats (Beispiel 87c) an Chiracel OD (Hexan/Isopropanol 80:20), in 10 ml Methylenchlorid werden unter Argon 4.4 ml Diisopropylethylamin und anschliessend 1.8 ml Chlormethylmethylether zugetropft. Das Reaktionsgemisch wird für 4 h bei Raumtemperatur gerührt und dann eingeengt. Das Rohprodukt wird mittels FC an 100 g Kieselgel (Laufmittel D) gereinigt und man erhält das N-Benzyloxycarbonyl[2(R)- oder 2(S)-methoxymethoxymethyl]3,4-dihydro-2H-1,4-benzoxazin: R_{f}(A)= 0.56. Die weitere Umsetzung analog Beispiel 1r) ergibt das 2(R)- oder 2(S)-Methoxymethoxymethyl-3,4-dihydro-2H-1,4-benzoxazin: R_{f}(A)= 0.35; Rₜ(I)= 13.8 min; [α]_{D}= +31.7 (c= 1.0 in CHCl₃).

### Beispiel 133: 5(S)-Amino-4(S)-hydroxy-2(R),7,7-trimethyl-8-[2(R)- oder 2(S)-methoxymethyloxymethyl-3,4-dihydro-2H-1,4-benzoxazin-4-ylcarbonyl]-octansäure(N-butyl)amid:

Ausgehend von 500 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 314 mg 2(R)- oder 2(S)-Methoxymethoxymethyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R)- oder 2(S)-methoxymethoxymethyl-3,4-dihydro-2H-1,4-benzoxazin-4-ylcarbonyl]-octansäure(N-butyl)amid (analog Beispiel 1a; R_{f}(A)= 0.25; Rₜ(VI)= 35.3 min) und das 5(S)-Tertiärbutoxycarbonylamino-4(S)-hydroxy-2(R),7,7-trimethyl-8-[2(R,S)-methoxymethoxymethyl-3,4-dihydro-2H-1,4-benzoxazin-4-ylcarbonyl]-octansäure(N-butyl)amid (R_{f}(X)= 0.38; FAB-MS: (M+H)⁺= 608) analog wie in Beispiel 1) beschrieben als reines Diastereomer: R_{f}(V)= 0.39; Rₜ(VI)= 23.2 min; FAB-MS: (M+H)⁺= 508.

Die als Ausgangsmaterial eingesetzte Amin-Komponente wird beispielsweise folgendermassen hergestellt:
Zu einer Lösung von 2.5 g N-Benzyloxycarbonyl[2(R)- oder 2(S)-hydroxymethyl]3,4-dihydro-2H-1,4-benzoxazin (Beispiel 87c), ([α]_{D}= -28.8 (c= 1.0 in CHCl₃)) in 10 ml Methylenchlorid werden unter Argon 4.4 ml Diisopropylethylamin und anschliessend 1.8 ml Chlormethylmethylether zugetropft. Das Reaktionsgemisch wird für 4 h bei Raumtemperatur gerührt und dann eingeengt. Das Rohprodukt wird mittels FC an 100 g Kieselgel (Laufmittel D) gereinigt, und man erhält das N-Benzyloxycarbonyl[2(R)- oder 2(S)-methoxymethoxymethyl]3,4-dihydro-2H-1,4-benzoxazin (2.5 g): R_{f}(A)= 0.56. Die weitere Umsetzung analog Beispiel 1r) ergibt das 2(R)- oder 2(S)-Methoxymethoxymethyl-3,4-dihydro-2H-1,4-benzoxazin: R_{f}(A)= 0.35; Rₜ(I)= 13.8 min; [α]_{D}= -32.8 (c= 1.0 in CHCl₃).

### Beispiel 134: 5(S)-Amino-4(S)-hydroxy-2(R),7,7-trimethyl-8-[3(R,S)(methylaminocarbonyl)indolin-1ylcarbonyl]-octansäure(N-butyl)amid

Analog den in den Beispielen 92, 20a und 20b) für die entsprechenden Reaktionsstufen beschriebenen Verfahren erhält man die Titelverbindung ausgehend von 150 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 87 mg Indolin-3(R,S)carbonsäure(N-methyl)amid über das (40,5N-Isopropyliden)-5(S)-tertiärbutoxycarbonylamino-4(S)-hydroxy-2(R),7,7-trimethyl-8-[3(R,S)(methylaminocarbonyl)indolin-1ylcarbonyl]-octansäure(N-butyl)amid (R_{f}(P)= 0.58) und das 5(S)-Tertiärbutoxycarbonylamino-4(S)-hydroxy-2(R),7,7-trimethyl-8-[3(R,S)(methylaminocarbonyl)indolin-1ylcarbonyl]-octansäure(N-butyl)amid (R_{f}(P)= 0.44) die Titelverbindung: R_{f}(S)= 0.07; Rₜ(VI)= 31.4/31.8 min; FAB-MS: (M+H)⁺= 475.

Die als Ausgangsmaterial eingesetzte Amin-Komponente wird beispielsweise folgendermassen hergestellt:

a) Indolin-3(R,S)-carbonsäure(N-methyl)amid
   Zur Lösung von 216 mg 1-(Tertiärbutoxycarbonyl)indolin-3(R,S)-carbonsäure(N-methyl)amid in 1 ml Methylenchlorid werden bei 0 °C unter Rühren 3 ml einer 25%igen Lösung von Trifluoressigsäure in Methylenchlorid addiert. Nach 90 min läßt man die Mischung auf Raumtemperatur aufwärmen und rührt weitere 2 h. Einengen des Ansatzes und Reinigung des Rohproduktes an 50 g Kieselgel (Laufmittel K) ergibt die Titelverbindung: R_{f}(P)= 0.52.
b) 1-(Tertiärbutoxycarbonyl)indolin-3(R,S)-carbonsäure(N-methyl)amid
   Zu einer Lösung von 0.59 g 1-(Tertiärbutoxycarbonyl)indolin-3(R,S)carbonsäure in Methylenchlorid werden bei 0 °C 0.35 ml 1-Chlor-N,N,2-trimethylpropenylamin addiert. Nach 30 min werden 0.93 ml Triethylamin, 0.23 g Methylamin Hydrochlorid und 4-DMAP als Katalysator zugegeben und der Ansatz bei Raumtemperatur über 3 h gerührt. Das Reaktionsgemisch wird anschliessend direkt über 80 g Kieselgel (Laufmittel J) chromatographiert. Man erhält die Titelverbindung: R_{f}(P)= 0.65.
c) 1-(Tertiärbutoxycarbonyl)indolin-3(R,S)-carbonsäure
   Die Mischung aus 0.7 g 1-(Tertiärbutoxycarbonyl)indolin-3(R,S)-carbonsäureethylester (Beispiel 97d) in 18 ml Methanol-Wasser (1:1) und 2.9 ml einer 1N-Natriumhydroxid-Lösung wird über 3 h bei 50 °C gerührt. Nach Entfernen des Methanol unter Vakuum wird die wäßerige Phase auf pH 2 gestellt und mit Ethylacetat extrahiert. Die übliche weitere Aufarbeitung ergibt die Titelverbindung als weissen Festkörper: R_{f}(Hexan-Ethylacetat-Eisessig (75:25:1)) = 0.23.

### Beispiel 135: 5(S)-Amino-4(S)-hydroxy-2(R),7,7-trimethyl-8-[3(R,S)formylamino-1,2,3,4-tetrahydrochinolin-1ylcarbonyl]-octansäure(N-butyl)amid

Analog den in den Beispielen 92, 20a und 20b) für die entsprechenden Reaktionsstufen beschriebenen Verfahren erhält man die Titelverbindung ausgehend von 100 mg 3-[N-Tertiarbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 115 mg 3(R,S)Formylamino-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)formylamino-1,2,3,4-tetrahydrochinolin-1ylcarbonyl]- octansäure(N-butyl)amid (R_{f}(K)=0.07) und das 5(S)-Tertiärbutoxycarbonylamino-4(S)-hydroxy-2(R),7,7-trimethyl-8-[3(R,S)formylamino-1,2,3,4-tetrahydrochinolin-1ylcarbonyl]-octansäure(N-butyl)amid (R_{f}(N)= 0.23) die Titelverbindung: R_{f}(S)= 0.05; Rₜ(V)= 10.9 min; FAB-MS: (M+H)⁺= 475.

Die als Ausgangsmaterial eingesetzte Amin-Komponente wird beispielsweise folgendermassen hergestellt:
Die Lösung von 0.50 g 3(R,S)-Amino-1,2,3,4-tetrahydrochinolin und 0.62 g Ameisensäure-4-nitrophenylester in 10 ml Methylenchlorid wird 1 h bei Raumtemperatur gerührt und anschliessend eingeengt. Chromatographie an 65 g Kieselgel (Laufmittel J) ergibt einen weissen Festkörper: R_{f}(P)= 0.57.

### Beispiel 136: 5(S)-Amino-4(S)-hydroxy-2(R),7,7-trimethyl-8-[3(R,S)ethylcarbonylamino-1,2,3,4-tetrahydrochinolin-1ylcarbonyl]-octansäure(N-butyl)amid

Analog den in den Beispielen 92, 20a und 20b) für die entsprechenden Reaktionsstufen beschriebenen Verfahren erhält man die Titelverbindung ausgehend von 100 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 134 mg 3(R,S)Ethylcarbonylamino-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)ethylcarbonylamino-1,2,3,4-tetrahydrochinolin-1-ylcarbonyl]-octansäure(N-butyl)amid (R_{f}(K)=0.23) und das 5(S)-Tertiärbutoxycarbonylamino-4(S)-hydroxy-2(R),7,7-trimethyl-8-[3(R,S)ethylcarbonylamino-1,2,3,4-tetrahydrochinolin-1-ylcarbonyl]-octansäure(N-butyl)amid (R_{f}(N)= 0.22) die Titelverbindung: R_{f}(S)= 0.17; Rₜ(V)= 14.1/14.4 min; FAB-MS: (M+H)⁺= 503.

### Beispiel 137: 5(S)-Amino-4(S)-hydroxy-2(R),7,7-trimethyl-8-[3(R,S)pentyl-1,2,3,4-tetrahydrochinolin-1ylcarbonyl]-octansäure(N-butyl)amid

Ausgehend von 230 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 284 mg 3(R,S)-Pentyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-pentyl-1,2,3,4-tetrahydrochinolin-1-ylcarbonyl]-octansäure(N-butyl)amid (analog Beispiel 1a); R_{f}(A)= 0.44) und das 5(S)-Tertiärbutoxycarbonylamino-4(S)-hydroxy-2(R),7,7-trimethyl-8-[3(R,S)-pentyl-1,2,3,4- tetrahydrochinolin-1-ylcarbonyl]-octansäure(N-butyl)amid (R_{f}(X)= 0.47; FAB-MS: (M+H)⁺= 602) analog wie in Beispiel 1) beschrieben als Diastereomerengemisch: R_{f}(V)= 0.33; Rₜ(VI)= 30.1 min; FAB-MS: (M+H)⁺= 502.

Die Amin-Komponente wird beispielsweise wie folgt hergestellt:
a) 3(R,S)-Pentyl-1,2,3,4-tetrahydrochinolin
   Die Titelverbindung wird analog wie in Beispiel 1r) beschrieben ausgehend von 1.33 g N-Benzyloxycarbonyl[3(R,S)-penten-1-yl]-1,2,3,4-tetrahydrochinolin hergestellt: R_{f}(A)= 0.68.
b) N-Benzyloxycarbonyl-[3(R,S)-penten-1-yl]-1,2,3,4-tetrahydrochinolin
   Zu einer Suspension von 3.0 g n-Butyl-triphenylphosphoniumbromid in 40 ml Tetrahydrofuran werden 2.0 g Natrium-bis-(trimethylsilyl)amid addiert und 30 min bei 35 °C gerührt. Danach wird eine Lösung von 1.48 g N-Benzyloxycarbonyl-3(R,S)-formyl-1,2,3,4-tetrahydrochinolin in 10 ml Tetrahydrofuran zugetropft und für 2 h bei 25 °C gerührt. Das Rohprodukt wird mittels FC an 100 g Kieselgel (Laufmittel F) gereinigt: R_{f}(D)= 0.50; MS(EI): M⁺= 335.
c) N-Benzyloxycarbonyl-3(R,S)-formyl-1,2,3,4-tetrahydrochinolin
   Die Titelverbindung wird mittels Swern-Oxidation analog Beispiel 1 g) ausgehend von 7.0 g N-Benzyloxycarbonyl-3(R,S)-hydroxymethyl-1,2,3,4-tetrahydrochinolin erhalten: R_{f}(A)= 0.42.
d) N-Benzyloxycarbonyl-3(R,S)-hydroxymethyl-1,2,3,4-tetrahydrochinolin
   14.0 g N-Benzyloxycarbonyl-3(R,S)-carboxy-1,2,3,4-tetrahydrochinolin werden in 400 ml Tetrahydrofuran mit 9.0 ml 10 M Boran-Dimethylsulfid Komplex für 16 h bei 25 °C gerührt. Das Reaktiongemisch wird mehrmals mit Methanol verdünnt und eingedampft. Das Rohprodukt wird aus Essigester/Hexan umkristallisiert: Smp 62-4 °C; R_{f}(X)= 0.50; FAB-MS: (M+H)⁺= 298; Anal. kalk. für C₁₈H₁₉NO₃: C 72.71 %, H 6.44 %, H 4.71 %; gef. C 72.84 %, H 6.35 %, N 4.75 %.

### Beispiel 138: 5(S)-Amino-4(S)-hydroxy-2(R),7,7-trimethyl-8-[3(R,S)-methoxyethyl-1,2,3,4-tetrahydrochinolin-1ylcarbonl]-octansäure N-butyl)amid

Ausgehend von 230 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 287 mg 3(R,S)-Methoxyethyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxyethyl-1,2,3,4-tetrahydrochinolin-1-ylcarbonyl]-octansäure(N-butyl)amid (analog Beispiel 1a); R_{f}(A)= 0.17; Rₜ(VI)= 35.8 min) und das 5(S)-Tertiärbutoxycarbonylamino-4(S)-hydroxy-2(R),7,7-trimethyl-8-[3(R,S)-methoxyethyl-1,2,3,4-tetrahydrochinolin-1-ylcarbonyl]-octansäure(N-butyl)amid (R_{f}(V)= 0.81; Rₜ(VI)= 31.0 min) analog wie in Beispiel 1) beschrieben als Diastereomerengemisch: R_{f}(V)= 0.44; Rₜ(VI)= 23.6 min; FAB-MS: (M+H)⁺= 490.

Die Amin-Komponente wird beispielsweise wie folgt hergestellt:
a) 3(R,S)-Methoxyethyl-1,2,3,4-tetrahydrochinolin
   Die Titelverbindung wird analog wie in Beispiel 1r) beschrieben ausgehend von 2.3 g N-Benzyloxycarbonyl-3(R,S)-(1-methoxyethen-2-yl)-1,2,3,4-tetrahydrochinolin hergestellt: R_{f}(A)= 0.40; FAB-MS: (M+H)⁺= 192.
b) N-Benzyloxycarbonyl-3(R,S)-(1-methoxyethen-2-yl)-1,2,3,4-tetrahydrochinolin
   Die Titelverbindung wird analog wie in Beispiel 137b) beschrieben ausgehend von 4.8 g N-Benzyloxycarbonyl-3(R,S)-formyl-1,2,3,4-tetrahydrochinolin ,7.2 g Methoxymethyl-triphenylphosphoniumchlorid und 5.4 g Natrium-bis-(trimethylsilyl)amid hergestellt: R_{f}(A)= 0.67.

### Beispiel 139: 5(S)-Formylamino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid

Ausgehend von 80 mg 5(S)-Amino-4(S)-hydroxy-2(R),7,7-trimethyl-8[3(R)- oder 3(S)-methoxycarbonylamino-1,2,3,4-tetrahydrochinolin-1ylcarbonyl]-octansäure(N-butyl)amid (Beispiel 86) erhält man nach der im Beispiel 47) beschriebenen Methode und Reinigung durch FC an 10 g Kieselgel (Laufmittelgradient von J nach K) die Titelverbindung: R_{f}(P)= 0.46; Rₜ(VI)= 36.6 min; FAB-MS: (M+H)⁺= 533.

### Beispiel 140: 5(S)-Acetamido-4(S)-hydroxy-2(R),7,7-trimethyl-8-[3(R)- oder 3(S)-methyoxycarbonylamino-1,2,3,4-tetrahydrochinolin-1ylcarbonyl]-octansäure(N-butyl)amid

Die Mischung aus 80 mg 5(S)-Amino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid (Beispiel 86) und 13.9 µL Acetanhydrid in 2 ml Methylenchlorid wird über Nacht bei Raumtemperatur gerührt und anschliessend direkt über 10 g Kieselgel (Laufmittelgradient J nach L) chromatographiert. Man erhält die Titelverbindung als amorphen Festkörper: R_{f}(P)= 0.42; Rₜ(VI)= 37.5 min; FAB-MS: (M+H)⁺= 547.

### Beispiel 141: 5(S)-Amino-2(R)-ethyl-4(S)-hydroxy-7,7-dimethyl-8-[3(R)- oder-3(S)-methyoxycarbonylamino-1,2,3,4-tetrahydrochinolin-1-ylcarbonyl]-octansäure(N-butyl)amid

ausgehend von 82 mg 3-[N-Tertiärbutoxycarbonyl-4(S)-(3-carboxy-2,2-dimethyl-propyl)-2,2-dimethyl-1,3-oxazolidin-5(S)-yl]-2(R)-ethyl-propionsäure(N-butyl)amid und 91 mg 3(R)- oder 3(S)-Methoxycarbonylamino-1,2,3,4-tetrahydrochinolin (Stereoisomer II, Beispiel 74h) über das (4O,5N-Isopropyliden)-5(S)-tertiärbutoxycarbonylamino-2(R)-ethyl-4(S)-hydroxy-7,7-dimethyl-8-[3(R)-oder 3(S)-methoxycarbonylamino-1,2,3,4-tetrahydrochinolin-1ylcarbonyl]-octansäure(N-butyl)amid (R_{f}(L)= 0.24; FAB-MS:(M+H)⁺= 659) und das 5(S)-Tertiärbutoxycarbonylamino-2(R)-ethyl-4(S)-hydroxy-7,7-dimethyl-8-[3(R)-oder 3(S)-methyoxy- carbonylamino-1,2,3,4-tetrahydrochinolin-1-ylcarbonyl]-octansäure(N-butyl)amid (R_{f}(L)= 0.23; FAB-MS:(M+H)⁺= 619) analog Beispiel 92) als reines Diastereomer: R_{f}(P)=0.23; Rₜ(IV)=38.8 min; FAB-MS:(M+H)⁺= 519.

Die Ausgangsmaterialien wurden folgendermassen hergestellt:
a) 3-[N-Tertiärbutoxycarbonyl-4(S)-(3-carboxy-2,2-dimethylpropyl)-2,2-dimethyl-1,3-oxazolidin-5(S)-yl]-2(R)-ethyl-propionsäure(N-butyl)amid
   Ausgehend von 200 mg 3(R)-Ethyl-5(S)-[5-benzyloxy-1(S)-tertiärbutoxy- carbonylamino-3,3-dimethyl-pentyl]-2-oxo-tetrahydrofuran über das 2(R)-Ethyl-9-benzyloxy5(S)-tertiärbutoxycarbonylamino-4(S)-hydroxy- 7,7-dimethyl-nonansäure(N-butyl)amid (R_{f}(A)= 0.28; FAB-MS: (M+H)⁺= 507), das 3-[N-Tertiärbutoxycarbonyl-4(S)-(4-benzyloxy-2,2-dimethylbutyl)- 2,2-dimethyl-1,3-oxazolidin-5(S)-yl]-2(R)-ethyl-propionsäure(N-butyl)amid (R_{f}(B)= 0.46; FAB-MS:(M+H)⁺= 547) und das 3-[N-Tertiärbutoxycarbonyl- 4(S)(2,2-dimethyl-4-hydroxybutyl)-2,2-dimethyl-1,3-oxazolidin-5(S)- yl]-2(R)-ethyl-propionsäure(N-butyl)amid (R_{f}(A)= 0.24; FAB-MS:(M+H)⁺= 457) analog wie in den Beispielen 74a,74b und 74c) beschrieben als reines Diastereomer: R_{f}(P)= 0.58; FAB-MS:(M+H)⁺= 471.
b) 3(R)-Ethyl-5(S)-[5-benzyloxy-1(S)-tertiärbutoxycarbonylamino-3,3- dimethyl-pentyl]-2-oxo-tetrahydrofuran
   Die Lösung von 496 mg 5(S)-[5-Benzyloxy-1(S)-tertiärbutoxycarbonyl- amino-3,3dimethyl-pentyl]-2-oxotetrahydrofuran (Beispiel 74 e)) und 0.44 ml Hexamethylphosphorsäuretriamid in 15 ml Tetrahydrofuran wird unter Argon bei -78° C zu einer Lösung von 2.48 mmol Lithiumdiisopropylamid in 10 ml Tetrahydofuran addiert und 30 min gerührt. Anschliessend werden 0.10 ml Ethyljodid in 10 ml Tetrahydrofuran tropfenweise addiert. Nach 8.5 h Rühren bei -78° wird die Reaktion durch Zugabe von 0.45 ml Propionsäure gequencht und aufgearbeitet analog Beispiel 74d). Reinigung an Kieselgel (Laufmittel D) ergibt die Titelverbindung: R_{f}(D)= 0.28; FAB-MS:(M+H)⁺= 434.

### Beispiel 142: 5(S)-Amino-4(S)-hydroxy-2(R)-(2-methyl)propyl-7,7-dimethyl-8-[3(R)-oder 3(S)-methyoxycarbonylamino-1,2,3,4-tetrahydrochinolin-1-yl-carbonyl]-octansäure(N-butyl)-amid

Ausgehend von 80 mg 3-[N-Tertiärbutoxycarbonyl-4(S)-(3-carboxy- 2,2-dimethylpropyl)-2,2-dimethyl-1,3-oxazolidin-5(S)-yl]-2(R)-(2- methyl)propyl-propionsäure(N-butyl)amid und 85 mg 3(R)-oder 3(S)-Methoxycarbonylamino-1,2,3,4-tetrahydrochinolin (Stereoisomer II, Beispiel 74h) über das (4O,5N-Isopropyliden)-5(S)-tert- butoxycarbonylamino-2(R)-(2-methyl)propyl-4(S)-hydroxy-7,7dimethyl-8-[3(R)-oder 3(S)-methoxycarbonylamino-1,2,3,4-tetrahydrochinolin- 1-ylcarbonyl]-octansäure(N-butyl)amid (R_{f}(L)= 0.38; FAB-MS:(M+H)⁺= 687) und das 5(S)-Tertiärbutoxycarbonylamino-2(R)(2-methyl)propyl-4(S)-hydroxy-7,7-dimethyl-8-[3(R)- oder 3(S)-methyoxycarbonylamino-1,2,3,4- tetrahydrochinolin-1-yl-carbonyl]-octansäure(N-butyl)amid (R_{f}(L)= 0.21; FAB-MS:(M+H)⁺= 647) analog Beispiel 92) als reines Diastereomer: R_{f}(P)= 0.17; Rₜ(IV)= 37.2 min; FAB-MS:(M+H)⁺= 547.

Die Ausgangsmaterialien wurden folgendermassen hergestellt:
a)3-[N-Tertiärbutoxycarbonyl-4(S)-(3-carboxy-2,2-dimethylpropyl)-2,2-dimethyl-1,3-oxazolidin-5(S)-yl]-2(R)-(2-methyl)propyl-propionsäure (N-butyl)amid
   ausgehend von 207 mg 3(R)-[(2-Methyl)propen-3-yl]-5(S)-[5-benzyloxy-1(S)-tertiärbutoxycarbonylamino-3,3-dimethyl-pentyl]-2-oxotetrahydrofuran über das 2(R)-[(2-methyl)propen-3-yl]-9-benzyloxy- 5(S)-tertiärbutoxycarbonylamino-4(S)-hydroxy-7,7-dimethyl-nonansäure(N-butyl)amid (R_{f}(A)= 0.38; FAB-MS:(M+H)⁺= 533), das 3-[N-Tertiärbutoxy- carbonyl-4(S)-(4-benzyloxy-2,2-dimethylbutyl)-2,2-dimethyl-1,3- oxazolidin-5(S)-yl]-2(R)-[(2-methyl)propen-3-yl]-propionsäure(N-butyl)amid (R_{f}(B)= 0.48; FAB-MS:(M+H)⁺= 573) und das 3-[N-Tertiärbutoxycarbonyl-4(S)-(2,2-dimethyl-4-hydroxybutyloxy-2,2-dimethyl- 1,3-oxazolidin-5(S)-yl]-2(R)-(2-methyl)propyl-propionsäure(N-butyl)amid (R_{f}(A)= 0.35; FAB-MS:(M+H)⁺= 485) analog wie in den Beispielen 74a,74b und 74c) beschrieben als reines Diastereomer: R_{f}(P)= 0.65; FAB-MS:(M+H)⁺= 499.
b)3(R)-[(2-Methyl)propen-3-yl]-5(S)-[5-benzyloxy-1(S)-tertiärbutoxy carbonylamino-3,3-dimethyl-pentyl]-2-oxo-tetrahydrofuran
   Die Lösung von 480 mg 5(S)-[5-Benzyloxy-1(S)-tertiärbutoxycarbonyl amino-3,3dimethylpentyl]-2-oxotetrahydrofuran in 15 ml Tetrahydrofuran wird unter Argon bei -78°C zu einem Gemisch aus 2.4 ml einer 1 M Lithium-bis-(trimethylsilyl)amid-Lösung und 10 ml Tetrahydofuran addiert und 30 min gerührt. Anschliessend werden 0.13 ml 3-Brom-2-methylpropen in 10 ml Tetrahydrofuran tropfenweise addiert. Nach 6 h Rühren bei -78° wird die Reaktion durch Zugabe von 0.44 ml Propionsäure gequencht und aufgearbeitet analog Beispiel 74d). Reinigung an Kieselgel (Laufmittel D) ergibt die Titelverbindung: R_{f}(D)= 0.34; FAB-MS:(M+H)⁺= 460.

### Beispiel 143: 5(S)-Amino-4(S)-hydroxy-2(R)-(2-methyl)propyl- 7,7-dimethyl-8-[3(R)-oder 3(S)-methyoxycarbonylamino-1,2,3,4-tetrahydrochinolin-1-ylcarbonyl]-octansäure(N-methyl)amid

Ausgehend von 57 mg 3-[N-Tertiärbutoxycarbonyl-4(S)-(3-carboxy- 2,2-dimethylpropyloxy-2,2-dimethyl-1,3-oxazolidin-5(S)-yl]-2(R)-(2- methyl)propyl-propionsäure(N-methyl)amid und 64 mg 3(R)-oder 3(S)-Methoxycarbonylamino-1,2,3,4-tetrahydrochinolin (Stereoisomer II, Beispiel 74h) über das (4O,5N-Isopropyliden)-5(S)-tertiärbutoxycarbonylamino-2(R)(2-methyl)propyl-4(S)-hydroxy-7,7dimethyl-8- [3(R)-oder 3(S)-methoxycarbonylamino-1,2,3,4-tetrahydrochinolin- 1-ylcarbonyl]-octansäure(N-methyl)amid (R_{f}(L)= 0.25; FAB-MS:(M+H)⁺= 645) und das 5(S)-Tertiärbutoxycarbonylamino-2(R)(2-methyl)propyl-4(S)- hydroxy-7,7-dimethyl-8-[3(R)- oder 3(S)-methyoxycarbonylamino-1,2,3,4- tetrahydrochinolin-1-ylcarbonyl]-octansäure(N-methyl)amid (R_{f}(L)= 0.12; FAB-MS:(M+H)⁺= 605) analog Beispiel 92) als reines Diastereomer: R_{f}(P)= 0.07; Rₜ(IV)= 42.8 min; FAB-MS:(M+H)⁺= 505.

Die Ausgangsmaterialien wurden folgendermassen hergestellt:
a)3-[N-Tertiärbutoxycarbonyl-4(S)-(3-carboxy-2,2-dimethylpropyl)-2,2-dimethyl-1,3-oxazolidin-5(S)-yl]-2(R)-(2-methyl)propyl-propionsäure(N-methyl)amid
   ausgehend von 156 mg 2(R)-[(2-Methyl)propen-3-yl]-9-benzyloxy-5(S)- tertiärbutoxycarbonylamino-4(S)-hydroxy-7,7-dimethyl-nonansäure(N-methyl)amid (R_{f}(L)= 0.33; FAB-MS:(M+H)⁺= 491) über das 3-[N-tert- Butoxy-carbonyl-4(S)-(4-benzyloxy-2,2-dimethylbutyl)-2,2-dimethyl- 1,3-oxazolidin-5(S)-yl]-2(R)-[(2-methyl)propen-3-yl]-propionsaure(N-methyl)amid (R_{f}(L)= 0.48; FAB-MS:(M+H)⁺= 531) und das 3-[N-Tertiärbutoxycarbonyl-4(S)-(2,2-dimethyl-4-hydroxybutyloxy-2,2-dimethyl-1,3-oxazolidin-5(S)-yl]-2(R)-(2-methyl)propyl-propionsäure(N-methyl)amid (R_{f}(P)= 0.48; FAB-MS:(M+H)⁺= 443) analog wie in den Beispielen 74a und 74b) beschrieben als reines Diastereomer: R_{f}(P)= 0.59; FAB-MS:(M+H)⁺=457.
b)2(R)-[(2-Methyl)propen-3-yl]-9-benzyloxy-5(S)-tertiärbutoxycarbonyl-amino-4(S)-hydroxy-7,7-dimethyl-nonansäure(N-methyl)amid
   10 ml Methylamin werden in eine Lösung von 194 mg 3(R)-[(2-Methyl)propen-3-yl]-5(S)[5-benzyloxy-1(S)-tertiärbutoxycarbonyl- amino-3,3dimethyl-pentyl]-2-oxo-tetrahydrofuran (Beispiel 142b)) in 4 ml Tetrahydrofuran bei 0°C eingeleitet und kondensiert. Die Lösung wird während 24 h bei Raumtemperatur gerührt, eingeengt und der Rückstand an Kieselgel (Laufmittel L) chromatographisch gereinigt: R_{f}(L)= 0.33; FAB-MS:(M+H)⁺= 491.

### Beispiel 144: 5(S)-Amino-2(R)-benzyl-4(S)-hydroxy-7,7-dimethyl-8- [3(R)- oder-3(S)-methyoxycarbonylamino-1,2,3,4-tetrahydrochinolin-1-ylcarbonyl]-octansäure(N-methyl)amid

Ausgehend von 80 mg 3-[N-Tertiärbutoxycarbonyl-4(S)-(3-carboxy-2,2-dimethylpropyl)-2,2-dimethyl-1,3-oxazolidin-5(S)-yl]-2(R)-benzyl- propionsäure(N-methyl)amid und 84 mg 3(R)- oder 3(S)-Methoxy- carbonylamino-1,2,3,4-tetrahydrochinolin (Stereoisomer II, Beispiel 74h) über das (4O,5N-Isopropyliden)-5(S)-tertiärbutoxycarbonylamino- 2(R)-benzyl-4(S)-hydroxy-7,7-dimethyl-8-[3(R)-oder 3(S)-methoxycarbonylamino-1,2,3,4-tetrahydrochinolin-1-yl-carbonyl]- octansäure(N-methyl)amid (R_{f}(L)= 0.35; FAB-MS:(M+H)⁺= 679) und das 5(S)-Tertiärbutoxycarbonylamino-2(R)-benzyl-4(S)-hydroxy-7,7-dimethyl 8-[3(R)- oder 3(S)-methoxycarbonylamino-1,2,3,4-tetrahydrochinolin- 1-ylcarbonyl]-octansäure(N-methyl)amid (R_{f}(L)= 0.24; FAB-MS:(M+H)⁺= 639) analog Beispiel 92) als reines Diastereomer: R_{f}(P)= 0.15; Rₜ(IV)= 37.0 min; FAB-MS:(M+H)⁺= 539.

Die Ausgangsmaterialien wurden folgendermassen hergestellt:
a) 3-[N-Tertiärbutoxycarbonyl-4(S)-(3-carboxy-2,2-dimethylpropyl)-2,2-dimethyl-1,3-oxazolidin-5(S)-yl]-2(R)-benzyl-propionsäure(N-methyl)amid
   ausgehend von 505 mg 2(R)-Benzyl-9-benzyloxy-5(S)-tertiärbutoxycarbonyl- amino-4(S)-hydroxy-7,7-dimethyl-nonansäure(N-methyl)amid über das 3-[N-Tertiärbutoxycarbonyl-4(S)-(4-benzyloxy-2,2-dimethylbutyloxy-2,2-dimethyl-1,3-oxazolidin-5(S)-yl]-2(R)-benzyl-propionsäure(N-methyl)amid (R_{f}(A)= 0.43; FAB-MS:(M+H)⁺= 567) und das 3-[N-Tertiärbutoxycarbonyl- 4(S)(2,2-dimethyl-4-hydroxybutyloxy-2,2-dimethyl-1,3-oxazolidin-5(S)- yl]-2(R)-benzyl-propionsäure(N-methyl)amid (R_{f}(B)= 0.14; FAB-MS:(M+H)⁺= 477) analog wie in den Beispielen 74a,74b und 74c) beschrieben als reines Diastereomer: R_{f}(L)= 0.17; FAB-MS:(M+H)⁺= 491.
b) 2(R)-Benzyl-9-benzyloxy-5(S)-tertiärbutoxycarbonylamino-4(S)-hydroxy-7,7-dimethyl-nonansäure(N-methyl)amid
   8 ml Methylamin werden in eine Lösung von 476 mg 3(R)-Benzyl-5(S)-[5-benzyloxy-1 (S)-tertiärbutoxycarbonylamino-3,3dimethyl-pentyl]- 2-oxotetrahydrofuran (Beispiel 74d)) in 4 ml Tetrahydrofuran bei 0°C eingeleitet und kondensiert. Die Lösung wird während 30 h bei Raumtemperatur gerührt, eingeengt und der Rückstand ohne Chromatographie weiter umgesetzt: R_{f}(L)= 0.48; FAB-MS:(M+H)⁺= 527.

### Beispiel 145: 5(S)-Amino-4(S)-hydroxy-2(S)-isopropyl-7,7-dimethyl-8-[2(R)- oder-2(S)-methoxymethoxymethyl-3,4-dihydro-2H-1,4-benzoxazin-4-ylcarbonyl]-octansäure(N-butyl)amid

Analog den in den Beispielen 20, 20a und 20b) für die entsprechenden Reaktionsstufen beschriebenen Verfahren erhält man die Titelverbindung ausgehend von 300 mg 3-[N-Tertiärbutoxycarbonyl-4(S)-(3-carboxy-2,2-dimethylpropyl)-2,2-dimethyl-1,3-oxazolidin-5(S)-yl]-2(S)isopropyl-propionsäure(N-butyl)amid und 195 mg 2(R)- oder 2(S)-Methoxymethoxymethyl-3,4-dihydro-2H-1,4-benzoxazin (Beispiel 132) über das (4O,5N-Isopropyliden)-5(S)-tertiärbutoxycarbonylamino-4(S)-hydroxy-2(S)-isopropyl-7,7-dimethyl-8-[2(R)- oder 2(S)-methoxymethoxymethyl-3,4-dihydro-2H-1,4-benzoxazin-4-ylcarbonyl]-octansäure(N-butyl)amid (R_{f}(A)= 0.36; Rₜ(VI)= 38.4 min) und das 5(S)-Tertiärbutoxycarbonylamino-4(S)-hydroxy-2(S)-isopropyl-7,7dimethyl-8-[2(R)- oder 2(S)-methoxymethoxymethyl-3,4-dihydro-2H-1,4-benzoxazin-4-ylcarbonyl]-octansäure(N-butyl)amid (R_{f}(X)= 0.53; Rₜ(VI)= 32.1 min; FAB-MS: [M+H]⁺= 636) als reines Diastereomer: R_{f}(V)= 0.33; Rₜ(VI)= 26.2 min; FAB-MS: [M+H]⁺= 536. Beispiel 146: 5(S)-Amino-4(S)-hydroxy-2(R),7,7-trimethyl-8-[3(R,S)-methoxypropyl-1,2,3,4-
tetrahydrochinolin-1ylcarbonyl]-octansäure(N-butyl)amid

Ausgehend von 200 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsaure(N-butyl)amid und 90 mg 3(R,S)-Methoxpropyl-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxypropyl-1,2,3,4-tetrahydrochinolin-1-ylcarbonyl]-octansäure(N-butyl)amid (analog Beispiel 1a); R_{f}(X)= 0.57; Rₜ(VI)= 34.3 min) und das 5(S)-Tertiärbutoxycarbonylamino-4(S)-hydroxy-2(R),7,7-trimethyl-8-[3(R,S)-methoxpropyl-1,2,3,4-tetrahydrochinolin-1-ylcarbonyl]-octansäure(N-butyl)amid (R_{f}(X)= 0.37; Rₜ(VI)= 30.3 min; FAB-MS: (M+H)⁺= 604) analog wie in Beispiel 1) beschrieben als Diastereomerengemisch: R_{f}(V)= 0.42; Rₜ(VI)= 23.0 min; FAB-MS: (M+H)⁺= 504.

Die Amin-Komponente wird beispielsweise wie folgt hergestellt:
a) 3(R,S)-Methoxypropyl-1,2,3,4-tetrahydrochinolin
   Die Titelverbindung wird analog wie in Beispiel 1r) beschrieben ausgehend von 0.32 g N-Benzyloxycarbonyl-3(R,S)-(1-methoxyprop-2-en-3-yl)-1,2,3,4-tetrahydrochinolin hergestellt: R_{f}(A)= 0.50.
b) N-Benzyloxycarbonyl-3(R,S)(1-methoxyprop-2-en-3-yl)-1,2,3,4-tetrahydrochinolin
   Die Titelverbindung wird analog wie in Beispiel 137b) beschrieben ausgehend von 1.50 g N-Benzyloxycarbonyl-3(R,S)-formyl-1,2,3,4-tetrahydrochinolin ,3.1 g Methoxyethyl-triphenylphosphoniumbromid und 2.0 g Natrium-bis-(trimethylsilyl)amid hergestellt: R_{f}(A)= 0.55.

### Beispiel 147: 5(S)-Amino-4(S)-hydroxy-2(R),7,7-trimethyl-8-[3(R,S)-methoxycarbonylamino-2,3,4,5tetrahydro-1H-benz[6,7-b]azepin-1ylcarbonyl]-octansäure(N-butyl)amid

Analog den in den Beispielen 92, 20a und 20b) für die entsprechenden Reaktionsstufen beschriebenen Verfahren erhält man die Titelverbindung ausgehend von 100 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 120 mg 3(R,S)-methoxycarbonylamino-2,3,4,5tetrahydro-1H-benz[6,7-b]azepin über das (4O,5N-Isopropyliden)-5(S)-tertiärbutoxycarbonylamino-4(S)-hydroxy-2(R),7,7-trimethyl-8-[3(R,S)-methoxycarbonylamino-2,3,4,5-tetrahydro-1H-benz[6,7-b]azepin-1ylcarbonyl]-octansäure(N-butyl)amid (R_{f}(P)= 0.58) und das 5(S)-Tertiärbutoxycarbonylamino-4(S)-hydroxy-2(R),7,7-trimethyl-8-[3(R,S)-methoxycarbonylamino-2,3,4,5tetrahydro-1H-benz[6,7-b]azepin-1ylcarbonyl]-octansäure(N-butyl)amid (R_{f}(P)= 0.39; FAB-MS: (M+H)⁺= 619) die Titelverbindung: R_{f}(S)= 0.14; Rₜ(VI)= 37.6 min; FAB-MS: (M+H)⁺= 519.

Die als Ausgangsmaterial eingesetzte Amin-Komponente wird beispielsweise folgendermassen hergestellt:
Zu einer Suspension von 0.40 g 3(R,S)-Amino-2,3,4,5tetrahydro-1H-benz[6,7-b]azepin in 6 ml Toluol werden zunächst 1.1 ml einer 10%igen wäßerigen Natriumhydroxid-Lösung und dann tropfenweise eine Lösung von 200 µl Chlorameisensäuremethylester in 1.5 ml Toluol unter starkem Rühren addiert. Nach 15 min trennt man die organische Phase ab, trocknet über Natriumsulfat und engt ein. Chromatographie an 50 g Kieselgel (Laufmittel I) ergibt einen weissen Festkörper: R_{f}(L)= 0.67; R_{f}(Methylenchlorid)= 0.19.

### Beispiel 148: 5(S)-Amino-4(S)-hydroxy-2(R),7,7-trimethyl-8-[3(R,S)-methoxycarbonyl-(N-methyl)amino-1,2,3,4-tetrahydrochinolin-1ylcarbonyl]-octansäure(N-butyl)amid

Analog den in den Beispielen 92, 20a und 20b) für die entsprechenden Reaktionsstufen beschriebenen Verfahren erhält man die Titelverbindung ausgehend von 84 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 95 mg 3(R,S)-methoxycarbonyl-(N-methyl)amino-1,2,3,4-tetrahydrochinolin über das (4O,5N-Isopropyliden)-5(S)-tertiärbutoxycarbonylamino-4(S)-hydroxy-2(R),7,7-trimethyl-8-[3(R,S)-methoxycarbonyl-(N-methyl)amino-1,2,3,4-tetrahydrochinolin-1ylcarbonyl]-octansäure(N-butyl)amid (R_{f}(P)= 0.61) und das 5(S)-Tertiarbutoxycarbonylamino-4(S)-hydroxy-2(R),7,7-trimethyl-8-[3(R,S)methoxycarbonyl-(N- methyl)amino-1,2,3,4-tetrahydrochinolin-1ylcarbonyl]-octansäure(N-butyl)amid (R_{f}(P)= 0.46) die Titelverbindung: R_{f}(S)= 0.14; Rₜ(V)= 19.9 min; FAB-MS: (M+H)⁺= 519.

Die als Ausgangsmaterial eingesetzte Amin-Komponente wird beispielsweise folgendermassen hergestellt:
a) 3(R,S)-methoxycarbonyl-(N-methyl)amino-1,2,3,4-tetrahydrochinolin
   Analog wie in Beispiel 134a) beschrieben werden 110 mg 1-(Tertiärbutoxycarbonyl)-3(R,S)-methoxycarbonyl-(N-methyl)amino-1,2,3,4-tetrahydrochinolin umgesetzt und das Rohprodukt an 10 g Kieselgel (Laufmittel J) gereinigt: R_{f}(L)= 0.68; MS(EI): (M)⁺= 220.
b) 1-(Tertiärbutoxycarbonyl)-3(R,S)-methoxycarbonyl-(N-methyl)amino-1,2,3,4-tetrahydrochinolin
   0.30 g 1-(Tertiärbutoxycarbonyl)-3(R,S)(methoxycarbonyl)amino-1,2,3,4-tetrahydrochinolin, gelöst in 10 ml Tetrahydrofuran, werden bei 0 °C mit 44 mg Natriumhydrid (80%ige Dispersion in Oel) unter Rühren versetzt. Nach 30 min werden 120 µl Methyljodid addiert und der Ansatz über 4 h bei Raumtemperatur gerührt. Nach vorsichtigem Einengen wird direkt über 50 g Kieselgel mit einem (20:1)-Gemisch aus Methylenchlorid und Diethylether als Eluens chromatographiert. Man erhält die Titelverbindung: R_{f}(Methylenchlorid-Diethylether (10:1))=0.57.
c) 1-(Tertiärbutoxycarbonyl)-3(R,S)(methoxycarbonyl)amino-1,2,3,4-tetrahydrochinolin
   1.0 g 3(R,S)-(Methoxycarbonyl)amino-1,2,3,4-tetrahydrochinolin, gelöst in 7 ml Dioxan und 3.5 ml Wasser, werden bei 0 °C mit 1.38 g Di-tertiärbutylcarbonat versetzt. Man rührt über 24 h bei Raumtemperatur und addiert anschliessend weitere 1.4 g Di-tertiärbutylcarbonat. Nach beendeter Reaktion (insgesamt 48 h) wird der Ansatz zur Entfernung des Dioxan eingeengt und die wäßerige Phase mit Methylenchlorid extrahiert. Reinigung des Rohproduktes an 100 g Kieselgel (Laufmittelgradient F nach A) ergibt neben zurückisoliertem Ausgangsmaterial die Titelverbindung: R_{f}(P)= 0.34.

### Beispiel 149: 5(S)-Formylamino-4(S)-hydroxy-2(S)-isopropyl-7,7-dimethyl-8-[2(R)- oder-2(S)-methoxymethoxymethyl-3,4-dihydro-2H-1,4-benzoxazin-4-ylcarbonyl]-octansäure(N-butyl)amid

Analog des im Beispiel 47) beschriebenen Verfahrens wird ausgehend von 50 mg 5(S)-Amino-4(S)-hydroxy-2(S)-isopropyl-7,7-dimethyl-8-[2(R)-oder 2(S)-methoxymethoxymethyl-3,4-dihydro-2H-1,4-benzoxazin-4-ylcarbonyl]-octansäure(N-butyl)amid (Beispiel 145) und anschliessender Reinigung durch FC an 10 g Kieselgel (Laufmittel J) die Titelverbindung erhalten: R_{f}(P)= 0.45; Rₜ(VI)= 27.7; FAB-MS: (M+H)⁺= 564.

### Beispiel 150: 5(S)-Amino-4(S)-hydroxy-2(S)-isopropyl-7,7-dimethyl-8-[3(R)- oder-3(S)-methoxycarbonylamino-1,2,3,4-tetrahydrochinolin-1ylcarbonyl]-octansäure(N-butyl)amid

Ausgehend von 80 mg 3-[N-Tertiärbutoxycarbonyl-4(S)-(3-carboxy-2,2-dimethylpropyl)-2,2-dimethyl-1,3-oxazolidin-5(S)-yl]-2(S)isopropyl-propionsäure(N-butyl)amid und 85 mg 3(R)- oder 3(S)-Methoxycarbonylamino-1,2,3,4-tetrahydrochinolin (Stereoisomer I, Beispiel 74h) erhält man in analoger Weise wie in Beispiel 125) beschrieben die Titelverbindung als reines Stereoisomer: R_{f}(S)= 0.18; Rₜ(IV)= 38.6 min; FAB-MS: (M+H)⁺= 533.

### Beispiel 151: 5(S)-Amino-4(S)-hydroxy-2(R),7,7-trimethyl-8-[2(R,S)-methoxyethyl-3,4-dihydro-2H-1,4-benzoxazin-4-ylcarbonyl]-octansäure(N-butyl)amid

Ausgehend von 230 mg 3-[N-Tertiärbutoxycarbonyl-4(S)-(3-carboxy-2,2-dimethylpropyl)-2,2-dimethyl-1,3-oxazolidin-5(S)-yl]-2(R)-methylpropionsäure(N-butyl)amid und 193 mg 2(R,S)-methoxyethyl-3,4-dihydro-2H-1,4-benzoxazin über das (4O,5N-Isopropyliden)-5(S)-tertiärbutoxycarbonylamino-4(S)-hydroxy-2(R),7,7-trimethyl-8-[2(R,S)-methoxyethyl-3,4-dihydro-2H-1,4-benzoxazin-4-ylcarbonyl]-octansäure(N-butyl)amid (analog Beispiel 1a); R_{f}(A)= 0.19; Rₜ(VI)= 36.6/36.9 min) und das 5(S)-Tertiärbutoxycarbonylamino-4(S)-hydroxy-2(R),7,7-trimethyl-8-[2(R,S)-methoxyethyl-3,4-dihydro-2H-1,4-benzoxazin-4-ylcarbonyl]-octansäure(N-butyl)amid (R_{f}(X)= 0.36; Rₜ(VI)= 30.6 min; FAB-MS: (M+H)⁺= 592) analog wie in Beispiel 1) beschrieben die Titelverbindung als Diastereomerengemisch: R_{f}(V)= 0.32; Rₜ(VI)= 23.7/24.1 min; FAB-MS: (M+H)⁺= 492.

Die Amin-Komponente wird beispielsweise wie folgt hergestellt:
a) 2(R,S)-Methoxyethyl-3,4-dihydro-2H-1,4-benzoxazin
   Die Titelverbindung wird analog wie in Beispiel 1r) beschrieben ausgehend von 1.45 g N-Benzyloxycarbonyl-2(R,S)-(1-methoxyethen-2-yl)3,4-dihydro-2H-1,4-benzoxazin hergestellt und mittels FC an 100 g Kieselgel (Laufmittel B) gereinigt: R_{f}(A)= 0.30; FAB-MS: (M+H)⁺= 194.
b) N-Benzyloxycarbonyl-2(R,S)-(1-methoxyethen-2-yl)3,4-dihydro-2H-1,4-benzoxazin
   Die Titelverbindung wird analog dem in Beispiel 138b) beschriebenen Verfahren ausgehend von 1.25 g N-Benzyloxycarbonyl-2(R,S)-formyl-3,4-dihydro-2H-1,4-benzoxazin (Beispiel 107), 7.2 g Methoxymethyl-triphenylphosphoniumchlorid und 1.57 g Kaliumbis(trimethylsilyl)amid hergestellt: R_{f}(A)= 0.49 und 0.54.

### Beispiel 152: 5(S)-Amino-4(S)-hydroxy-2(S)-isopropyl-7,7-dimethyl-8-[2(R)- oder-2(S)-methoxyethyl-3,4-dihydro-2H-1,4-benzoxazin-4-ylcabonyl]-octansäure(N-butyl)amid

Analog den in den Beispielen 20, 20a und 20b) für die entsprechenden Reaktionsstufen beschriebenen Verfahren erhält man die Titelverbindung ausgehend von 300 mg 3-[N-Tertiärbutoxycarbonyl-4(S)-(3-carboxy-2,2-dimethylpropyl)-2,2-dimethyl-1,3-oxazolidin-5(S)-yl]-2(S)isopropyl-propionsäure(N-butyl)amid und 150 mg 2(R)- oder 2(S)-Methoxyethyl-3,4-dihydro-2H-1,4-benzoxazin über das (4O,5N-Isopropyliden)-5(S)-tertiärbutoxycarbonylamino-4(S)-hydroxy-2(S)-isopropyl-7,7-dimethyl-8-[2(R)- oder 2(S)-methoxyethyl-3,4-dihydro-2H-1,4-benzoxazin-4-ylcarbonyl]-octansäure(N-butyl)amid (R_{f}(A)= 0.41); Rₜ(VI)= 37.8 min) und das 5(S)-Tertiärbutoxycarbonylamino-4(S)-hydroxy-2(S)-isopropyl-7,7-dimethyl-8-[2(R)- oder 2(S)-methoxyethyl-3,4-dihydro-2H-1,4-benzoxazin-4-ylcarbonyl]-octansäure(N-butyl)amid (R_{f}(A)= 0.13; R_{f}(X)= 0.47; Rₜ(VI)= 33.9 min; FAB-MS: [M+H]⁺= 620) die Titelverbindung als reines Diastereomer: R_{f}(V)= 0.34; Rₜ(VI)= 25.3 min; FAB-MS: [M+H]⁺= 520.

Die als Ausgangsmaterial eingesetzte Amin-Komponente wird beispielsweise folgendermassen hergestellt:
Das Racemat 2(R,S)-Methoxyethyl-3,4-dihydro-2H-1,4-benzoxazin (Beispiel 151a) wird mittels präparativer Trennung an einer Chiracel OD Säule (5 X 50 cm, Laufmittel Hexan-Isopropanol (975:25)) in die reinen Enantiomeren 2(R)- und 2(S)-methoxyethyl-3,4-dihydro-2H-1,4-benzoxazine aufgetrennt; zuerst eluierendes Enantiomer: [α]_{D}= +10.4 (c= 1.0 in CHCl₃); zweites Enantiomer: [α]_{D}= -10.5 (c= 1.0 in CHCl₃).

### Beispiel 153: 5(S)-Amino-4(S)-hydroxy-2(R),7,7-trimethyl-8-[3butyl-2,3dihydro-4(H)-quinazolinon-1ylcarbonyl]-octansäure(N-butyl)amid

Analog den in den Beispielen 92, 20a und 20b) für die entsprechenden Reaktionsstufen beschriebenen Verfahren erhält man die Titelverbindung ausgehend von 100 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 134 mg 3-Butyl-2,3dihydro-4(H)-quinazolinon über das (4O,5N-Isopropyliden)-5(S)-tertiärbutoxycarbonylamino-4(S)-hydroxy-2(R),7,7-trimethyl-8-[3-butyl-2,3dihydro-4(H)-quinazolinon-1ylcarbonyl]-octansäure(N-butyl)amid und das 5(S)-Tertiärbutoxycarbonylamino-4(S)-hydroxy-2(R),7,7-trimethyl-8-[3-butyl-2,3dihydro-4(H)-quinazolinon-1ylcarbonyl]-octansäure(N-butyl)amid (R_{f}(N)= 0.19; FAB-MS: (M+H)⁺= 603) die Titelverbindung: R_{f}(O)= 0.27; FAB-MS: (M+H)⁺= 503.

Die als Ausgangsmaterial eingesetzte Amin-Komponente wird analog den in J.Med.Chem. 11 (1968) 1136 bzw. in Synthesis (1982) 266 beschriebenen Verfahren hergestellt.

### Beispiel 154: 5(S)-Amino-4(S)-hydroxy-2(R),7,7-trimethyl-8-[3(R,S)-(2-carbamoyl)ethyl-1,2,3,4-tetrahydrochinolin-1-ylcarbonyl]-octansäure(N-butyl)amid

Ausgehend von 230 mg 3-[N-Tertiärbutoxycarbonyl-4(S)-(3-carboxy-2,2-dimethylpropyl)-2,2-dimethyl-1,3-oxazolidin-5(S)-yl]-2(R)-methyl-propionsäure(N-butyl)amid und 205 mg 3(R,S)-(2-Carbamoyl)ethyl-1,2,3,4-tetrahydrochinolin erhält man über das (4O,5N-Isopropyliden)-5(S)-tertiärbutoxycarbonylamino-4(S)-hydroxy-2(R),7,7-trimethyl-8-[3(R,S)-(2-carbamoyl)ethyl-1,2,3,4-tetrahydrochinolin-1-ylcarbonyl]-octansäure(N-butyl)amid (analog Beispiel 1a); R_{f}(X)= 0.08; R_{f}(V)= 0.56; Rₜ(VI)= 30.8 min) und das 5(S)-Tertiärbutoxycarbonylamino-4(S)-hydroxy-2(R),7,7-trimethyl-8-[3(R,S)-(2-carbamoyl)ethyl-1,2,3,4-tetrahydrochinolin-1-ylcarbonyl]-octansäure(N-butyl)amid (R_{f}(P)= 0.32; Rₜ(VI)= 26.8 min; FAB-MS: (M+H)⁺= 603) analog wie in Beispiel 1) beschrieben die Titelverbindung als Diastereomerengemisch: R_{f}(V)= 0.21; Rₜ(VI)= 20.3 min; FAB-MS: (M+H)⁺= 503.

Die Amin-Komponente wird beispielsweise wie folgt hergestellt:
a) 3(R,S)-2-Carbamoyl)ethyl-1,2,3,4-tetrahydrochinolin
   Die Titelverbindung wird analog wie in Beispiel 1r) beschrieben ausgehend von 825 mg N-Benzyloxycarbonyl-3(R,S)-[(2-carbamoyl)ethen-1-yl]1,2,3,4-tetrahydrochinolin hergestellt und mittels FC an 30 g Kieselgel (Laufmittel H) gereinigt: FAB-MS: (M+H)⁺= 205.
b) N-Benzyloxycarbonyl-3(R,S)-[(2-carbamoyl)ethen-1-yl]1,2,3,4-tetrahydrochinolin
   Die Umsetzung von 1.48 g N-Benzyloxycarbonyl-2(R,S)-formyl-3,4-dihydro-2H-1,4-benzoxazin (Beispiel 107) mit 2.15 ml Trimethylsilyl-diethylphosphonoacetat und 0.3 g Natriumhydrid (80 %ige Dispersion in Oel) ergibt nach üblicher Aufarbeitung 1.7 g des N-Benzyloxycarbonyl-3(R,S)-[(2-carboxy)ethen-1-yl]1,2,3,4-tetrahydrochinolin. Hieraus erhält man analog wie im Beispiel 1a) beschrieben die Titelverbindung: (R_{f}(X)= 0.47.

### Beispiel 155: 5(S)-Formylamino-4(S)-hydroxy-2(S)-isopropyl-7,7-dimethyl-8-[3(R)- oder 3(S)-methoxycarbonylamino-1,2,3,4-tetrahydrochinolin-1-ylcarbonyl]-octansäure(N-butyl)amid:

Ausgehend von 35 mg 5(S)-Amino-4(S)-hydroxy-2(S)-ispropyl-7,7-dimethyl-8-[3(R)- oder 3(S)-methoxycarbonylamino-1,2,3,4-tetrahydrochinolin-1-ylcarbonyl]-octansäure(N-butyl)amid (Beispiel 125) erhält man nach der im Beispiel 47) beschriebenen Methode und Reinigung durch FC an 10 g Kieselgel (Laufmittel K) die Titelverbindung: R_{f}(O)= 0.52; Rₜ(IV)= 40.9 min; FAB-MS: (M+H)⁺=561.

### Beispiel 156: 5(S)-Amino-2(R)-but-2-enyl-4(S)-hydroxy-7,7-dimethyl-8-[3(R)- oder 3(S)-methoxycarbonylamino-1,2,3,4-tetrahydrochinolin-1-ylcarbonyl]-octansäure(N-butyl)amid:

54 mg 5(S)Tertiärbutoxycarbonylamino-2(R)-but-2-enyl-4(S)-hydroxy-7,7-dimethyl-8-[3(R)- oder 3(S)-methoxycabonylamino-1,2,3,4-tetrahydrochinolin-1-ylcarbonyl]-octansäure(N-butyl)amid werden in 1 ml Methylenchlorid gelöst und bei 0°C unter Argon mit 0.16 ml Trifluoressigsäure versetzt. Nach 2.5 h bei 0°C und 2 h bei Raumtemperatur wird das Reaktionsgemisch analog wie in Beispiel 45) aufgearbeitet und gereinigt (Laufmittel P): R_{f}(P)=0.29;Rₜ(IV)=39.0 min;FAB-MS:(M+H)⁺=545.

Die Ausgangsmaterialien werden beispielsweise folgendermassen hergestellt:
a)-5(S)-Tertiärbutoxycarbonylamino-2(R)-but-2-enyl-4(S)-hydroxy-7,7-dimethyl-8-[3(R)- oder 3(S)-methoxycarbonylamino-1,2,3,4-tetrahydrochinolin-1-ylcarbonyl]-octansäure(N-butyl)amid
   Ausgehend von 87 mg 3-[N-Tertiärbutoxycarbonyl-4(S)-(3-carboxy-2,2-dimethylpropyl)-2,2-dimethyl-1,3-oxazolidin-5(S)-yl]-2(R)-but-2-enyl-propionsäure(N-butyl)amid und 91 mg 3(R)- oder 3(S)-Methoxycarbonylamino-1,2,3,4-tetrahydrochinolin (Stereoisomer II, Beispiel 74h) über das (4O,5N-Isopropyliden)-5(S)-tertiärbutoxycarbonylamino-2(R)-but-2-enyl-4(S)-hydroxy-7,7-dimethyl-8-[3(R)- oder 3(S)-methoxycarbonylamino-1,2,3,4-tetrahydrochinolin-1-ylcarbonyl]-octansäure(N-butyl)amid (R_{f}(L)=0.20;FAB-MS:(M+H)⁺=685) analog Beispiel 92): (R_{f}(P)=0.42;FAB-MS:(M+H)⁺=645).
b)3-[N-Tertiärbutoxycarbonyl-4(S)-(3-carboxy-2,2-dimethylpropyl)-2,2-dimethyl-1,3-oxazolidin-5(S)-yl]-2(R)-but-2-enyl-propionsäure(N-butyl)amid
   Die Lösung von 809 mg Pyridinium-dichromat in 2 ml N,H-Dimethylformamid wird unter Argon bei Raumtemperatur zu einer Lösung von 290 mg 3-[N-Tertiärbutoxycarbonyl-4(S)-(4-hydroxy-2,2-dimethylbutyl)-2,2-dimethyl-1,3-oxazolidin-5(S)-yl]-2(R)-but-2-enylpropionsäure(N-butyl)amid in 2 ml N,N-Dimethylformamid via Kanüle tropfenweise addiert. Nach 23 h werden weitere 238 mg Pyridinium-dichromat zugegeben. Nach insgesamt 29 h wird das Reaktionsgemisch ins Wasser gegossen, mit Diethylether extrahiert (3x), getrocknet (Na₂SO₄) und eingeengt. Reinigung an Kieselgel (Laufmittel P) ergibt die Titelverbindung: R_{f}(P)=0.48;FAB-MS:(M+H)⁺=497.
c)3-[N-Tertiärbutoxycarbonyl-4(S)-(4-hydroxy-2,2-dimethylbutyl)-2,2-dimethyl-1,3-oxazolidin-5(S)-yl]-2(R)-but-2-enyl-propionsäure(N-butyl)amid
   474 mg 3-[N-Tertiärbutoxycarbonyl-4(S)-(4-tertiärbutyl-diphenylsilyloxy-2,2-dimethylbutyl)-2,2-dimethyl-1,3-oxazolidin-5(S)-yl]-2(R)-but-2-enyl-propionsäure(N-butyl)amid werden in 5 ml Tetrahydrofuran gelöst und bei Raumtemperatur unter Argon mit 1.2 ml einer 1 M Tetrabutylammoniumfluorid-Lösung in Tetrahydrofuran versetzt. Nach 4 h wird das Reaktionsgemisch mit Diethylether verdünnt, mit Wasser und Sole gewaschen, getrocknet (Na₂SO₄) und eingeengt. Reinigung an Kieselgel (Laufmittel A) ergibt die Titelverbindung: R_{f}(A)=0.40;FAB-MS:(M+H)⁺=483.
d)3-[N-Tertiärbutoxycarbonyl-4(S)-(4-tertiärbutyl-diphenylsilyloxy-2,2-dimethylbutyl)-2,2-dimethyl-1,3-oxazolidin-5(S)-yl]-2(R)-but-2-enyl-propionsäure(N-butyl)amid
   Ausgehend von 472 mg 3(R)-But-2-enyl-5(S)-[5-tertiärbutyl-diphenylsilyloxy-1(S)-tertiärbutoxycarbonylamino-3,3-dimethyl-pentyl]-2-oxo-tetrahydrofuran über das 2(R)-But-2-enyl-9-tertiärbutyl-diphenylsilyloxy-5(S)-tertiärbutoxycarbonylamino-4(S)-hydroxy-7,7-dimethyl-nonansäure(N-butyl)amid (R_{f}(A)=0.64;FAB-MS:(M+H)⁺=681) analog Beispiele 74b und 74c): R_{f}(B)=0.71;FAB-MS:(M+H)⁺=721.
e)3(R)-But-2-enyl-5(S)-[5-tertiärbutyl-diphenylsilyloxy-1(S)-tertiärbutoxycarbonylamino-3,3-dimethyl-pentyl]-2-oxo-tetrahydrofuran
   1.22 g 5(S)-[5-Tertiärbutyl-diphenylsilyloxy-1(S)-tertiärbutoxycarbonylamino-3,3-dimethylpentyl]-2-oxo-tetrahydrofuran werden mit 4.6 ml einer 1 M Lithium-bis-(trimethylsilyl)amid-Lösung in Tetrahydrofuran und 0.26 ml Crotylbromid (89%, 5.7:1 E:Z) analog Beispiel 74d) versetzt. Aufarbeitung nach 5 h und anschliessende Reinigung mittels FC an Kieselgel (Laufmittel C) ergibt die an C-3 einheitlich (R)-konfigurierte Titelverbindung als nicht trennbares 2E/2Z-Isomerengemisch: (R_{f}(C)=0.62;FAB-MS:(M+H)⁺=608).
f)-5(S)-[5-Tertiärbutyl-diphenylsilyloxy-1(S)-tertiärbutoxycarbonylamino-3,3-dimethyl-pentyl]-2-oxo-tetrahydrofuran
   1.37 g 5(S)-[Benzyloxy-1(S)-tertiärbutoxycarbonylamino-3,3-dimethyl-pentyl]-2-oxo-tetrahydrofuran werden analog Beispiel 74a) hydriert und mittels FC an Kieselgel (Laufmittel B) gereinigt: R_{f}(B)=0.14;FAB-MS:(M+H)⁺=316. 825 mg 5(S)-[Hydroxy-1(S)-tertiärbutoxycarbonylamino-3,3-dimethyl-pentyl]-2-oxo-tetrahydrofuran werden in 10 ml N,N-Dimethylformamid gelöst und bei Raumtemperatur unter Argon mit 0.75 ml Tertiärbutyl-diphenylsilylchlorid und 392 mg Imidazol versetzt. Nach 3 h wird das Reaktionsgemisch mit Wasser verdünnt,mit Pentan extrahiert (2x), getrocknet (Na₂SO₄) und eingeengt. Reinigung an Kieselgel (Laufmittel D) ergibt die Titelverbindung: R_{f}(D)=0.27;FAB-MS:(M+H)⁺=554.

### Beispiel 157: 5(S)-Amino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-morpholin-4-yl-ethyl)amid-dihydrochlorid

58 mg 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-morpholin-4-yl-ethyl)amid werden in 3 ml 4N Salzsäure in Dioxan bei 0° C über 3 h gerührt. Man engt das Reaktionsgemisch ein, versetzt erneut mit 2 ml 4N Salzsäure in Dioxan und rührt bei 0° C über 3 h nach. Nach Lyophilisation und Trocknen am Hochvakuum erhält man die Titelverbindung: R_{f} (S) = 0.15; Rₜ (VI) = 8.8 min; FAB-MS: (M+H)⁺ = 562. Das Ausgangsmaterial wird beispielsweise folgendermaßen hergestellt:
a) 5(S)-Tertiärbutoxycarbonylamino-4(S)-hydroxy-2(R),7,7-dimethyl-8-[3(R)- oder 3(S)-methoxycarbonylamino-1,2,3,4-tetrahydrochinolin-1-ylcarbonyl]-octansäure-N-(2-morpholin-4-yl-ethyl)amid
   50 mg {1-[5(S)-Tertiärbutoxycarbonylamino-3,3-dimethyl-5(S)-(4(R)-methyl-5-oxo-tetrahydrofuran-2(S)-yl)-pentanoyl]-1,2,3,4-tetrahydrochinolin-3(R oder S)-yl}-carbaminsäuremethylester werden in N-(2-Aminoethyl)-morpholin (1 ml) bei 80° C über Nacht gerührt. Das Reaktionsgemisch wird eingeengt und der Rückstand mittels FC an Kieselgel (Dichlormethan-Methanol 92.5:7.5) gereinigt. Man erhält die Titelverbindung als gelbliches Oel: R_{f} (P) = 0.30; Rₜ (VI) = 12.6 min; FAB-MS: (M+H)⁺ = 662.
b) {1-[5(S)-Tertiärbutoxycarbonylamino-3,3-dimethyl-5(S)-(4(R)-methyl-5-oxo-tetrahydrofuran-2(S)-yl)-pentanoyl]-1,2,3,4-tetrahydrochinolin-3(R oder S)-yl}-carbaminsäuremethylester
   Zu einer Lösung von 5(S)-Tertiarbutoxycarbonylamino-4(S)-hydroxy-2(R),7,7-tnmethyl-8-[3(R) oder 3(S)-methoxycarbonylamino-1,2,3,4-tetrahydrochinolin-1-ylcarbonyl]-octansäure(N-butyl)amid (300 mg) in 6 ml Toluol-Dichlormethan (1:2) werden unter Rühren bei Raumtemperatur insgesamt 142 mg *para*-Toluolsulfonsäure-Hydrat in Portionen über 72 h addiert. Man rührt die weiße Suspension über weitere 24 h nach, addiert Pyridin (20 µl) und chromatographiert das Gemisch direkt an Kieselgel (30 g, Fließmittel J). Man erhält die Titelverbindung als weiße Festsubstanz: R_{f} (P) = 0.68; FAB-MS: (M+H)⁺ = 532.

### Beispiel 158: 5(S)-Amino-4(S)-hydroxy-2(R),7,7-trimethyl-8-[3(R)- oder 3(S)-methoxy-carbonylamino-1,2,3,4-tetrahydrochinolin-1-ylcarbonyl]-octansaure-N-[2-(1-acetyl)-piperidin-4-yl)-ethyl]amid-hydrochlorid

55 mg 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-[2-(1-acetylpiperidin-4-yl)-ethyl]amid werden analog wie in Bespiel 159) beschrieben in 2 ml 4N Salzsäure in Dioxan bei 0° C umgesetzt. Nach Lyophilisation wird der Rückstand mit 2 ml wasserfreiem Diethylether behandelt, das Lösungsmittel entfernt und das Produkt am Hochvakuum getrocknet. Man erhält die Titelverbindung als Festkörper: R_{f} (S) = 0.25; Rₜ (VI) = 10.5 min; FAB-MS: (M+H)⁺ = 602.

Das Ausgangsmaterial wird beispielsweise folgendermaßen hergestellt:
a) 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-[2-(1-acetylpiperidin-4-yl)-ethyl]amid
   Die Mischung aus 50 mg {1-[5(S)-Tertiärbutoxycarbonylamino-3,3-dimethyl-5(S)-(4(R)-methyl-5-oxo-tetrahydrofuran-2(S)-yl)-pentanoyl]-1,2,3,4-tetrahydrochinolin-3(R oder S)-yl}-carbaminsäuremethylester, 1-Acetyl-4-(2-aminoethyl)-piperidin (48 mg) und 2-Hydroxypyridin (9 mg) in 2 ml Triethylamin wird während 20 h bei 80° C unter inerter Atmosphäre gerührt. Man versetzt den Reaktionsansatz nach dem Abkühlen mit 20 ml einer Natriumhydrogensulfat-Lösung und extrahiert mit Dichlormethan (3 x 20 ml). Die vereinten organischen Phasen werden über Magnesiumsulfat getrocknet und eingeengt. Nach Reinigung des Rohproduktes mittels FC (30 g Kieselgel, Dichlormethan-Methanol 92.5:7.5) erhält man die Titelverbindung als gelbliches Oel: R_{f} (P) = 0.50; Rₜ (VI) = 14.9 min; FAB-MS: (M+H)⁺ = 702.

### Beispiel 159: 5(S)-Amino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-morpholin-4-yl-2-oxo-ethyl)amid-hydrochlorid

51 mg 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-morpholin-4-yl-2-oxo-ethyl)amid werden in 2 ml 4N Salzsäure in Dioxan bei 0° C über 3 h gerührt. Das Reaktionsgemisch wird am Hochvakuum liophilisiert und bei Raumtemperatur bis zur Gewichtskonstanz getrocknet. Der Rückstand wird mit 2 ml wasserfreiem Diethylether behandelt, das Lösungsmittel entfernt und das Produkt am Hochvakuum getrocknet. Man erhält die Titelverbindung als weißen Festkörper: R_{f} (S) = 0.44; R_{f} (P) = 0.56; Rₜ (VI) = 11.3 min; FAB-MS: (M+H)⁺ = 576.

Das Ausgangsmaterial wird beispielsweise folgendermaßen hergestellt:
a) 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-morpholin-4-yl-2-oxo-ethyl)amid
   Durch Umsetzung von 50 mg {1-[5(S)-Tertiärbutoxycarbonylamino-3,3-dimethyl-5(S)-(4(R)-methyl-5-oxo-tetrahydrofuran-2(S)-yl)-pentanoyl]-1,2,3,4-tetrahydrochinolin-3(R oder S)-yl}-carbaminsäuremethylester und 41 mg 2-Amino-1-morpholin-4-yl-ethanon in Gegenwart von 2-Hydroxy-pyridin (9 mg), analog wie in Beispiel 158a) beschrieben, und anschließende FC Reinigung (30 g Kieselgel, Dichlormethan-Methanol 92.5:7.5) erhält man die Titelverbindung als gelbes Oel: R_{f} (P) = 0.39; Rₜ (VI) = 14.0 min; FAB-MS: (M+H)⁺ = 676.

### Beispiel 160: 5(S)-Amino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-carbamoyl-2-methyl-propyl)amid-hydrochlorid

65 mg 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-carbamoyl-2-methyl-propyl)amid werden analog wie in Beispiel 159) beschrieben in 2 ml 4N Salzsäure in Dioxan bei 0° C umgesetzt. Nach Lyophilisation und Trocknen am Hochvakuum erhält man die Titelverbindung als weißen Festkörper: R_{f} (S) = 0.09; Rₜ (VI) = 9.87 min; FAB-MS: (M+H)⁺ = 548.

Das als Ausgangsmaterial eingesetzte 5(S)-Tertiärbutoxycarbonylamino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-carbamoyl-2-methyl-propyl)amid erhält man analog wie in Beispiel 158a) beschrieben aus 50 mg {1-[5(S)-Tertiärbutoxycarbonylamino-3,3-dimethyl-5(S)-(4(R)-methyl-5-oxo-tetrahydrofuran-2(S)-yl)-pentanoyl]-1,2,3,4-tetrahydrochinolin-3(R oder S)-yl}-carbaminsäuremethylester, 33 mg 3-Amino-2,2-dimethyl-propionamid und 9 mg 2-Hydroxy-pyridin in Triethylamin (2 ml) und FC Reinigung des Rohproduktes als blaßgelbes Oel: R_{f} (P) = 0.13; Rₜ (VI) = 13.9 min; FAB-MS: (M+H)⁺ = 648.

### Beispiel 161: Tabletten, enthaltend je 50 mg Wirkstoff, beispielsweise 5(S)-Amino-4(S)-hydroxy-2(R),7,7-trimethyl-8-[3(R,S)-methoxycarbonyl-1,2,3,4-tetrahydrochinolin-1-ylcarbonyl]-octansäure(N-butyl)amid oder ein Salz davon, können wie folgt hergestellt werden:

### Zusammensetzung (10000 Tabletten)

| | |
|---|---|
| Wirkstoff | 500.0 g |
| Laktose | 500.0 g |
| Kartoffelstärke | 352.0 g |
| Gelatine | 8.0 g |
| Talk | 60.0 g |
| Magnesiumstearat | 10.0 g |
| Siliciumdioxid (hochdispers) | 20.0 g |
| Ethanol | q.s. |

Der Wirkstoff wird mit der Laktose und 292 g Kartoffelstärke vermischt, die Mischung mit einer ethanolischen Lösung der Gelatine beleuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 145,0 mg Gewicht und 50,0 mg Wirkstoffgehalt, die gewünschtenfalls mit Teilkerben zur feineren Anpassung der Dosierung versehen sein können.

### Beispiel 162: Gelatine-Lösung:

Eine sterilfiltrierte wäßrige Lösung mit 20 % Cyclodextrinen als Lösungsvermittler von einer der in den vorausgehenden Beispielen genannten Verbindungen der Formel I, beispielsweise 5(S)-Amino-4(S)-hydroxy-2(R),7,7-trimethyl-8-[3(R,S)-methoxycarbonyl-1,2,3,4-tetrahydrochinolin-1-ylcarbonyl]-octansäure(N-butyl)amid oder ein Salz davon, als Wirkstoff wird unter Erwärmen mit einer sterilen Gelatinelösung, welche als Konservierungsmittel Phenol enthält, unter aseptischen Bedingungen so vermischt, dass 1,0 ml Lösung die folgende Zusammensetzung hat:

| | |
|---|---|
| Wirkstoff | 3.0 mg |
| Gelatine | 150.0 mg |
| Phenol | 4.7 mg |
| dest. Wasser mit 20 % Cyclodextrinen als Lösungsvermittler | 1.0 ml |

### Beispiel 163: Sterile Trockensubstanz zur Injektion:

Man löst 5 mg einer der in den vorausgehenden Beispielen genannten Verbindungen der Formel I, beispielsweise 5(S)-Amino-4(S)-hydroxy-2(R),7,7-trimethyl-8-[3(R,S)-methoxycarbonyl-1,2,3,4-tetrahydrochinolin-1-ylcarbonyl]-octansäure(N-butyl)amid oder ein Salz davon, als Wirkstoff in 1 ml einer wäßrigen Lösung mit 20 mg Mannit und 20 % Cyclodextrinen als Lösungsvermittler. Die Lösung wird sterilfiltriert und unter aseptischen Bedingungen in eine 2 ml-Ampulle gefüllt, tiefgekühlt und lyophilisiert. Vor dem Gebrauch wird das Lyophilisat in 1 ml destilliertem Wasser oder 1 ml physiologischer Kochsalzlösung gelöst. Die Lösung wird intramuskulär oder intravenös angewendet. Diese Formulierung kann auch in Doppelkammerspritzampullen abgefüllt werden.

### Beispiel 164: Nasenspray:

In einer Mischung von 3,5 ml "Myglyol 812" und 0,08 g Benzylalkohol werden 500 mg fein gemahlenes (<5,0 µm) Pulver einer der in den vorausgehenden Beispielen genannten Verbindungen der Formel I, beispielsweise 5(S)-Amino-4(S)-hydroxy-2(R),7,7-trimethyl-8-[3(R,S)-methoxycarbonyl-1,2,3,4-tetrahydrochinolin-1-ylcarbonyl]-octansäure(N-butyl)amid oder ein Salz davon, als Wirkstoff suspendiert. Diese Suspension wird in einen Behälter mit Dosierventil eingefüllt. Es werden 5,0 g "Freon 12" unter Druck durch das Ventil in einen Behälter abgefüllt. Durch Schütteln wird das "Freon" in der Myglyol-Benzylalkoholmischung gelöst. Dieser Spraybehälter enthält ca. 100 Einzeldosen, die einzeln appliziert werden können.

### Beispiel 165: Lacktabletten

Für die Herstellung von 10 000 Tabletten enthaltend je 100 mg Wirkstoff, beispielsweise 5(S)-Amino-4(S)-hydroxy-2(R),7,7-trimethyl-8-[3(R,S)-methoxycarbonyl-1,2,3,4-tetrahydrochinolin-1-ylcarbonyl]-octansäure(N-butyl)amid oder ein Salz davon, werden folgende Bestandteile verarbeitet:

| | |
|---|---|
| Wirkstoff | 1000 g |
| Maisstärke | 680 g |
| Kolloidale Kieselsäure | 200 g |
| Magnesiumstearat | 20 g |
| Stearinsäure | 50 g |
| Natriumcarboxymethylstärke | 250 g |
| Wasser | quantum satis |

Ein Gemisch von einer der in den vorausgehenden Beispielen genannten Verbindungen der Formel I als Wirkstoff, 50 g Maisstärke und der kolloidalen Kieselsäure wird mit Stärkekleister aus 250 g Maisstärke und 2,2 kg entmineralisiertem Wasser zu einer feuchten Masse verarbeitet. Diese wird durch ein Sieb von 3 mm Maschenweite getrieben und bei 45° während 30 Min. im Wirbelschichttrockner getrocknet. Das getrocknete Granulat wird durch ein Sieb von 1 mm Maschenweite gedrückt, mit einer vorher gesiebten Mischung (1 mm Sieb) von 330 g Maisstärke, des Magnesiumstearats, der Stearinsäure und der Natriumcarboxymethylstärke gemischt und zu schwach gewölbten Tabletten verpreßt.

Beispiel 166: In analoger Weise wie in den Beispielen 161 bis 165 beschrieben kann man ferner pharmazeutische Präparate enthaltend eine Verbindung gemäss einem der Beispielen 1 bis 160 herstellen.

## Patentansprüche

1. Verbindungen der Formel I 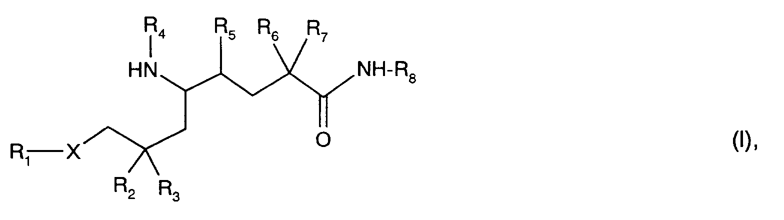 worin
R₁ Arylamino, N-Aryl-N-(niederalkoxyniederalkyl)-amino, N-Aryl-N-arylniederalkyl-amino oder über ein Ringstickstoffatom gebundenes Heterocyclyl bedeutet,
X für eine Carbonyl- oder Methylengruppe steht,
R₂ und R₃ unabhängig voneinander Wasserstoff oder Niederalkyl bedeuten oder zusammen mit dem Kohlenstoffatom, mit dem sie verbunden sind, einen Cycloalkylidenrest darstellen, R₄ Wasserstoff, Niederalkyl, Niederalkanoyl oder Niederalkoxycarbonyl ist,
R₅ Hydroxy, Niederalkanoyloxy oder Niederalkoxycarbonyloxy bedeutet,
R₆ Wasserstoff, Niederalkyl, Niederalkenyl, Niederalkinyl, Cycloalkyl, Cycloalkylniederalkyl, Arylniederalkyl oder Heteroarylniederalkyl mit 5 bis 7 Ringatomen im Heteroarylring und R₇ Wasserstoff oder Niederalkyl bedeutet oder
R₆ und R₇ zusammen mit dem Kohlenstoffatom, mit dem sie verbunden sind, einen Cycloalkylidenrest darstellen und
R₈ einen aliphatischen, cycloaliphatisch-aliphatischen oder heteroarylaliphatischen Rest bedeutet, und ihre Salze.

2. Verbindungen gemäß Anspruch 1 der Formel I, worin R₁ Arylamino, N-Aryl-N-(niederalkoxyniederalkyl)-amino, N-Aryl-N-arylniederalkyl-amino oder über ein Ringstickstoffatom gebundenes Heterocyclyl mit 4 bis 8 Ringatomen und keinem, 1 oder 2 ankondensierten Aryl- und/oder Cycloalkylresten bedeutet, wobei das genannte Heterocyclyl außer dem Ringstickstoffatom, über das es gebunden ist, weitere Ringheteroatome, ausgewählt aus Sauerstoff, Stickstoff, durch Niederalkyl, Niederalkanoyl, Niederalkansulfonyl oder Niederalkoxycarbonyl substituiertem Stickstoff, Schwefel und mit 1 oder 2 Sauerstoffatomen verknüpftem Schwefel enthalten kann,
X für eine Carbonyl- oder Methylengruppe steht,
R₂ und R₃ unabhängig voneinander Wasserstoff oder Niederalkyl bedeuten oder zusammen mit dem Kohlenstoffatom, mit dem sie verbunden sind, einen Cycloalkylidenrest darstellen,
R₄ Wasserstoff, Niederalkyl, Niederalkanoyl oder Niederalkoxycarbonyl ist,
R₅ Hydroxy, Niederalkanoyloxy oder Niederalkoxycarbonyloxy bedeutet, R₆ Wasserstoff, Niederalkyl, Niederalkenyl, Niederalkinyl, Cycloalkyl, Cycloalkylniederalkyl, Arylniederalkyl oder Heteroarylniederalkyl mit 5 bis 7 Ringatomen im Heteroarylring und R₇ Wasserstoff oder Niederalkyl bedeutet oder
R₆ und R₇ zusammen mit dem Kohlenstoffatom, mit dem sie verbunden sind, einen Cycloalkylidenrest darstellen und
R₈ Niederalkyl, Niederalkoxyniederalkyl, Aminoniederalkyl, N-Niederalkylaminoniederalkyl, N,N-Diniederalkylaminoniederalkyl, N,N-Niederalkylenaminoniederalkyl, N,N-(Azaniederalkylenamino)niederalkyl, N,N-(Oxaniederalkylenamino)niederalkyl, N,N-(Thianiederalkylenamino)niederalkyl, Carbamoylniederalkyl, N-Niederalkylcarbamoylniederalkyl, N,N-Diniederalkylcarbamoylniederalkyl, Cyanoniederalkyl oder Heteroarylniederalkyl mit 5 bis 7 Ringatomen im Heteroarylring, das ein Ringstickstoffatom enthält und ein weiteres Ringheteroatom, ausgewählt aus Sauerstoff, Schwefel oder Stickstoff, enthalten kann, bedeutet,
und ihre Salze.

3. Verbindungen gemäß Anspruch 2 der Formel I, worin
R₁ unsubstituiertes oder im Phenyl- oder Naphthylteil wie nachstehend angegeben substituiertes Anilino oder Naphthylamino, unsubstituiertes oder im Phenyl- oder Naphthylteil wie nachstehend angegeben substituiertes N-Phenyl- oder N-Naphthyl-N(niederalkoxyniederalkyl)-amino, unsubstituiertes oder im Phenyl- oder Naphthylteil wie nachstehend angegeben substituiertes N-Phenyl-N-(phenylniederalkyl)-amino oder über ein Ringstickstoffatom gebundenes, gegebenenfalls mit 1 oder 2 ankondensierten Phenyl- oder Cycloalkylresten kondensiertes 5- bis 8-gliedriges Heterocyclyl bedeutet, welches 1 oder 2 weitere Ringheteroatome, ausgewählt aus Sauerstoff, Stickstoff und gegebenenfalls oxydiertem Schwefel enthalten kann,
X für eine Carbonyl- oder Methylengruppe steht,
R₂ und R₃ unabhängig voneinander Wasserstoff oder Niederalkyl bedeuten oder zusammen mit dem Kohlenstoffatom, mit dem sie verbunden sind, 3- bis 8-gliedriges Cycloalkyliden darstellen,
R₄ Wasserstoff, Niederalkyl, Niederalkanoyl oder Niederalkoxycarbonyl bedeutet,
R₅ Hydroxy, Niederalkanoyloxy oder Niederalkoxycarbonyloxy bedeutet,
R₆ Wasserstoff, Niederalkyl, 3- bis 8-gliedriges Cycloalkyl, 3- bis 8-gliedriges Cycloalkylniederalkyl, Niederalkenyl, Niederalkinyl, unsubstituiertes oder wie nachstehend angegeben substituiertes Phenyl, Indenyl, Naphthyl, unsubstituiertes oder im Phenyl- oder Naphthylteil wie nachstehend angegeben substituiertes Phenyl- oder Naphthylniederalkyl, Pyridylniederalkyl oder Imidazolylniederalkyl darstellt,
R₇ Wasserstoff oder Niederalkyl bedeutet oder
R₆ und R₇ zusammen mit dem Kohlenstoffatom, mit dem sie verbunden sind, 3- bis 8-gliedriges Cycloalkyliden darstellen und
R₈ Niederalkyl, Niederalkoxyniederalkyl, Aminoniederalkyl, N-Niederalkylaminoniederalkyl, N,N-Diniederalkylaminoniederalkyl, N,N-Niederalkylenaminoniederalkyl N,N-(Azaniederalkylenamino)niederalkyl, N,N-(Oxaniederalkylenamino)niederalkyl, N,N-(Thianiederalkylenamino)niederalkyl, Heteroarylniederalkyl mit 5 bis 7 Ringatomen im Heteroarylring, welches ein Ringstickstoffatom enthält und ein weiteres Ringheteroatom, ausgewählt aus Sauerstoff, Schwefel oder Stickstoff, enthalten kann,
wobei Phenyl, Naphthyl, und Phenyl- bzw. Naphthylreste als Bestandteil von Naphthylamino, N-Phenyl- oder N-Naphthyl)-N-(niederalkoxyniederalkyl)-amino, N-Phenyl- oder N-Naphthyl)-N-niederalkyl-amino, Indenyl, Phenyl- oder Naphthylniederalkyl und N-Phenyl-N-(phenylniederalkyl)-amino durch Niederalkyl, Hydroxy, Niederalkoxy, Carbamoylniederalkoxy, N-Niederalkylcarbamoylniederalkoxy, N-Niederalkylcarbamoyl, N,N-Diniederalkylcarbamoylniederalkoxy, Amino, N-Niederalkyl- oder N,N-Diniederalkylamino, Carboxy, Niederalkoxycarbonyl, Carbamoyl, Sulfamoyl, Niederalkansulfonyl, Halogen, Nitro, Phenyl, 5- oder 6-gliedriges Heteroaryl, als Heteroatom enthaltend 1 Stickstoff-, Schwefel- oder Sauerstoffatom, 2 N-Atome, 1 N-Atom und 1 S-Atom oder 1 N-Atom und 1 O-Atom, wie Pyridyl, und/oder durch Cyano ein oder mehrfach, z.B. ein- oder zweifach substituiert und Reste R₁ durch Niederalkyl, Niederalkanoyl, Niederalkoxycarbonyl oder Niederalkansulfonyl N-substituiert, durch Oxy S-mono- oder S,S-disubstituiert und/oder durch Niederalkyl, Hydroxyniederalkyl, Niederalkoxyniederalkyl, Niederalkenyloxyniederalkyl, Naphthoxyniederalkyl, Phenyloxyniederalkyl, Phenylniederalkoxyniederalkyl, Niederalkanoyloxyniederalkyl, Benzoyloxyniederalkyl, Niederalkoxycarbonyloxyniederalkyl, Phenyloxycarbonyloxyniederalkyl, Phenylniederalkoxycarbonyloxyniederalkyl, Aminoniederalkyl, N-Niederalkylaminoniederalkyl, N,N-Diniederalkylaminoniederalkyl, Carbamoylniederalkyl, Niederalkanoylaminoniederalkyl, Benzoylaminoniederalkyl, Niederalkoxycarbonylaminoniederalkyl, Niederalkoxycarbonylniederalkyl, Niederalkoxyniederalkoxyniederalkyl, Niederalkylthioniederalkoxyniederalkyl, N-Niederalkoxyiminoniederalkyl, Cycloalkoxyniederalkyl, Cycloalkylniederalkoxyniederalkyl, Niederalkenyl, Niederalkenyloxy, Niederalkoxyniederalkenyl, Niederalkinyl, Niederalkinyloxy, Niederalkanoyl, Oxo, Hydroxy, Niederalkoxy, Carbamoylniederalkoxy, N-Niederalkylcarbamoylniederalkoxy, N-Niederalkylcarbamoyloxy, N,N-Diniederalkylcarbamoyloxy, Niederalkoxyniederalkoxy, Niederalkylthioniederalkoxy, Niederalkanoyloxy, Benzoyloxy, N-Niederalkylcarbamoyl, Amino, N-Niederalkylamino, N,N-Diniederalkylamino, Niederalkanoylamino, Benzoylamino, Cycloalkylcarbonylamino, Cycloalkylniederalkanoylamino, Niederalkoxycarbonylniederalkylamino, Niederalkenyloxycarbonylamino, Niederalkoxyniederalkoxycarbonylamino, Niederalkoxyniederalkanoylamino, N-Niederalkylcarbamoylamino, N,N-Diniederalkylcarbamoylamino, N-Niederalkanoyl-N-niederalkyl-amino, Niederalkoxycarbonylamino, N-Niederalkoxycarbonyl-N-niederalkylamino, N,N-Niederalkylenamino, N,N-(1-Oxoniederalkylen)amino, N,N-(1-Oxo2-oxa-niederalkylen)amino, Carboxy, Niederalkoxycarbonyl, Phenylniederalkoxycarbonyl, Phenyloxycarbonyl, Pyrrolidinylcarbonyl, Piperidinylcarbonyl, Piperazinylcarbonyl, Morpholinylcarbonyl, Thiomorpholinylcarbonyl, S,S-Dioxothiomorpholin-4-ylcarbonyl, Cyano, Carbamoyl, N,N-Diniederalkylcarbamoyl, N-Niederalkenylcarbamoyl, N-Cycloalkylcarbamoyl, N-Cycloalkylniederalkylcarbamoyl, N-Hydroxyniederalkylcarbamoyl, N-Niederalkoxyniederalkylcarbamoyl, N-Carboxyniederalkylcarbamoyl, Carbamoylniederalkylcarbamoyl, Niederalkoxycarbonylniederalkylcarbamoyl, Phenyl, Dioxolan-2-yl, Oxazol-2-yl, Oxazolin-2-yl, Oxazolidin-2-yl, Nitro, Sulfamoyl, Niederalkansulfonyl, Phosphono, Niederalkanphosphono, Diniederalkylphosphono und/oder Halogen ein- oder zweifach C-substituiert sein können,
und ihre Salze, insbesondere ihre pharmazeutisch verwendbaren Salze.

4. Verbindungen gemäß Anspruch 2 der Formel I, worin
R₁ unsubstituiertes oder im Phenyl- oder Naphthylteil durch C₁-C₄-Alkoxy, C₁-C₄-Alkoxycarbonyl, Carbamoyl-C₁-C₄-alkoxy, C₁-C₄-Alkanoylamino-C₁-C₄alkyl, C₁-C₄-Alkoxycarbonylamino-C₁-C₄-alkyl, Halogen und/oder gegebenenfalls N-oxidiertes Pyridyl mono- oder disubstituiertes Anilino, Naphthylamino, N-Phenyl-N-(C₁-C₄-alkoxy-C₁-C₄-alkyl)-amino oder N-Phenyl-N-(phenyl-C₁-C₄-alkyl)-amino oder jeweils unsubstituiertes oder durch C₁-C₄-Alkyl, Hydroxy-C₁-C₄-alkyl, C₁-C₄-Alkoxy-C₁-C₄-alkyl, C₃-C₅-Alkenyloxy-C₁-C₄-alkyl-C₁-C₄-Alkoxy-C₁-C₄-alkoxy-C₁-C₄-alkyl, C₁-C₄-Alkoxycarbonylamino-C₁-C₄-alkyl, C₁-C₄-Alkoxyimino-C₁-C₄-alkyl, Carboxy, C₁-C₇-Alkoxycarbonyl, Cyano, Carbamoyl, N-C₁-C₇-Alkylcarbamoyl, N,N-Di-C₁-C₄-alkylcarbamoyl, C₁-C₄-Alkoxy-C₁-C₄-alkylcarbamoyl, N-Carboxy-C₁-C₄-alkylcarbamoyl, Morpholinocarbonyl, C₁-C₄-Alkoxy, C₁-C₄-Alkoxy-C₁-C₄-alkoxy, C₁-C₇-Alkanoyloxy, Benzoyloxy, C₁-C₄-Alkanoylamino, C₁-C₇-Alkoxycarbonylamino, 3- bis 3- bis 6-gliedriges Cycloalkylcarbonylamino, N-C₁-C₄-Alkoxy-C₁-C₄-alkanoylamino, N-C₁-C₄-Alkylcarbamoylamino oder 5- oder 6-gliedriges N,N-(1-Oxoniederalkylen)amino oder N,N-(1-Oxo2-oxa-niederalkylen)-amino, C₁-C₇-Alkanoyl, Oxo, Nitro, C₁-C₄-Alkansulfonyl und/oder Halogen mono-, di- oder disubstituiertes Pyrrolidino, Piperidino, Piperazino, Morpholino oder Thiomorpholino, Indolin-1-yl, Isoindolin-2-yl, 2,3-Dihydrobenzimidazol-1-yl, 1,2,3,4-Tetrahydrochinol-1-yl, 1,2,3,4-Tetrahydroisochinol-2-yl, 1,2,3,4-Tetrahydro-1,3-benzdiazin-1-yl, 1,2,3,4-Tetrahydro-1,4-benzdiazin-1-yl, 3,4-Dihydro-2H-1,4-benzoxazin-4-yl, gegebenenfalls S,S-dioxydiertes 3,4-Dihydro-2H-1,3-benzthiazin-1-yl, 3,4,5,6,7,8-Hexahydro-2H-1,4-benzoxazin-4-yl, 3,4,5,6,7,8-Hexahydro-2H-1,4-benzthiazin-4-yl, 2,3,4,5-Tetrahydro-1H-1-benz[6,7-b]azepin-1-yl oder 5,6-Dihydrophenanthridin-5-yl bedeutet, X für eine Carbonylgruppe steht, R₂ und R₃ unabhängig voneinander Wasserstoff oder C₁-C₄-Alkyl bedeuten oder zusammen mit dem Kohlenstoffatom, mit dem sie verbunden sind, 3- bis 8-gliedriges Cycloalkyliden darstellen,
R₄ Wasserstoff oder C₁-C₄-Alkanoyl bedeutet,
R₅ Hydroxy bedeutet,
R₆ Wasserstoff, C₁-C₄-Alkyl, 3- bis 8-gliedriges Cycloalkyl oder Phenyl-C₁-C₄-alkyl darstellt und
R₇ Wasserstoff bedeutet oder R₆ und R₇ zusammen mit dem Kohlenstoffatom, mit dem sie verbunden sind, 3- bis 8-gliedriges Cycloalkyliden, darstellen und R₈ C₁-C₇-Alkyl, C₁-C₄-Alkoxy-C₁-C₄-alkyl, Amino-C₁-C₄-alkyl, N-C₁-C₄-Alkylamino-C₁-C₄-alkyl, N,N-Di-C₁-C₄-alkylamino-C₁-C₄-alkyl, 5- oder 6-gliedriges N,N-Niederalkylenamino-C₁-C₄-alkyl bzw. N,N-(Aza)-, N,N-(Oxa)-oder N,N-(Thia)niederalkylenamino-C₁-C₄-alkyl, Carbamoyl-C₁-C₄-alkyl, N-C₁-C₄-Alkylcarbamoyl, N,N-Di-C₁-C₄-alkylcarbamoyl, Cyano-C₁-C₄-alkyl oder Pyridyl-C₁-C₄-alkyl bedeutet,
und ihre Salze, insbesondere ihre pharmazeutisch verwendbaren Salze.

5. Verbindungen gemäß Anspruch 2 der Formel I, worin
R₁ ein Gruppe der Formel Ia oder Ib 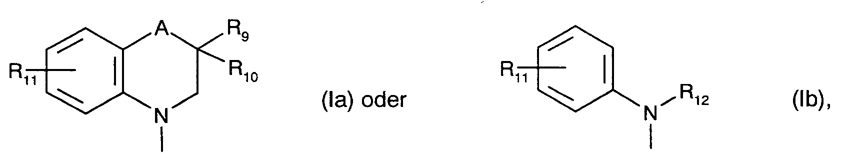
darstellt, in denen
A eine direkte Bindung, Methylen, Ethylen, Imino, Oxy oder Thio darstellt,
R₉ für C₁-C₄-Alkoxy-C₁-C₄-alkyl, C₃-C₅-Alkenyloxy-C₁-C₄-alkyl, C₁-C₄-Alkoxy-C₁-C₄-alkoxy-C₁-C₄-alkyl, C₁-C₄-Alkoxycarbonylamino-C₁-C₄-alkyl, C₁-C₄-Alkoxyimino-C₁-C₄-alkyl, Phenyl, C₁-C₄-Alkoxycarbonyl, Cyano, Carbamoyl, N-C₁-C₄-Alkylcarbamoyl, C₁-C₄-Alkoxy-C₁-C₄-alkylcarbamoyl, C₁-C₄-Alkoxy, C₁-C₄-Alkoxy-C₁-C₄-alkoxy, C₁-C₇-Alkanoyloxy, Benzoyloxy, N-C₁-C₄-Alkylcarbamoylamino, C₁-C₄-Alkanoylamino, C₁-C₇-Alkoxycarbonylamino, 3- bis 3- bis 6-gliedriges Cycloalkylcarbonylamino, C₁-C₄-Alkoxy-C₁-C₄-alkanoylamino, oder 5- oder 6-gliedriges N,N-(1-Oxoniederalkylen)amino oder N,N-(1-Oxo2-oxa-niederalkylen)amino oder für N-C₁-C₄-Alkylcarbamoylamino steht,
R₁₀ Wasserstoff oder C₁-C₄-Alkyl ist,
R₁₁ Wasserstoff oder Halogen ist,
R₁₂ C₁-C₄-Alkoxy-C₁-C₄-alkyl bedeutet,
X für eine Carbonylgruppe steht,
R₂ und R₃ unabhängig voneinander Wasserstoff oder C₁-C₄-Alkyl bedeuten oder zusammen mit dem Kohlenstoffatom, mit dem sie verbunden sind, 3- bis 8-gliedriges Cycloalkyliden darstellen,
R₄ Wasserstoff oder C₁-C₄-Alkanoyl bedeutet,
R₅ Hydroxy bedeutet,
R₆ Wasserstoff, C₁-C₄-Alkyl, 3- bis 8-gliedriges Cycloalkyl oder Phenyl-C₁-C₄-alkyl darstellt,
R₇ Wasserstoff bedeutet und
R₈ C₁-C₇-Alkyl, C₁-C₄-Alkoxy-C₁-C₄-alkyl, Amino-C₁-C₄-alkyl, N-C₁-C₄Alkylamino-C₁-C₄-alkyl, N,N-Di-C₁-C₄-alkylamino-C₁-C₄-alkyl, 5- oder 6-gliedriges N,N-Niederalkylenamino-C₁-C₄-alkyl bzw. N,N-(Aza)-, N,N-(Oxa)- oder N,N-(Thia)niederalkylenamino-C₁-C₄-alkyl oder Pyridyl-C₁-C₄-alkyl bedeutet,
und ihre Salze, insbesondere ihre pharmazeutisch verwendbaren Salze.

6. Verbindungen gemäß Anspruch 2 der Formel Ic 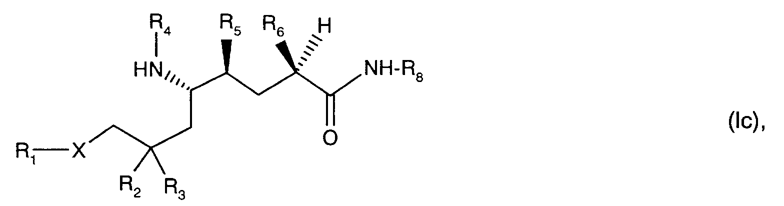
worin R₁, R₂, R₃, R₄, R₅, R₆, R₇, R₈, R₉, R₁₀, R₁₁ und R₁₂ die in einem der Ansprüche 1 bis 4 angegebenen Bedeutungen haben, und ihre Salze.

7. Verbindungen gemäß Anspruch 2 der Formel Id 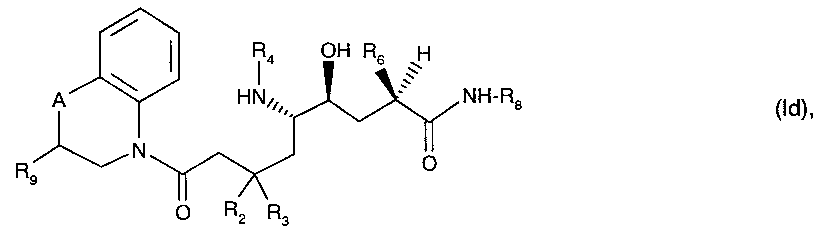 worin
A eine direkte Methylen, Oxy oder Thio darstellt,
R₂ und R₃ C₁-C₄-Alkyl bedeuten,
R₄ Wasserstoff oder C₁-C₄-Alkanoyl bedeutet,
R₆ C₁-C₄-Alkyl oder Phenyl-C₁-C₄-alkyl bedeutet,
R₈ C₁-C₇-Alkyl bedeutet,
R₉ für C₁-C₄-Alkoxycarbonylamino, C₁-C₄-Alkoxy-C₁-C₄-alkoxy-C₁-C₄-alkyl, C₁-C₄-Alkoxy-C₁-C₄-alkyl oder N-C₁-C₄-Alkylcarbamoyl darstellt,
und ihre Salze.

8. 4(S)-Amino-3(S)-hydroxy-1(R),6,6-trimethyl-heptandicarbonsäure-1-(N-butyl)amid-7-[N-(4-bromnaphth-1-yl)]amid;
5(S)-Amino-4(S)-hydroxy-2(R),7,7-trimethyl-8-(5,6-dihydrophenanthridinocarbonyl)-octansäure(N-butyl)amid;
5(S)-Amino-4(S)-hydroxy-2(R),7,7-trimethyl-8-(1,2,3,4-tetrahydroisochinolin-2-ylcarbonyl)-octansäure(N-butyl)amid;
4(S)-Amino-3(S)-hydroxy-1(R),6,6-trimethyl-heptandicarbonsäure-1-(N-butyl)amid-7-[N-[2-(formylaminomethyl)phenyl]]amid;
4(S)-Formylamino-3(S)-hydroxy-1(R),6,6-trimethyl-heptandicarbonsäure-1-(N-butyl)amid-7-(N-phenyl)amid;
5(S)-Amino-4(S)-hydroxy-2(R),7,7-trimethyl-8-[3(R)- oder 3(S)-ethoxycarbonylpiperidin-1-ylcarbonyl]-octansäure(N-butyl)amid;
5(S)-Amino-4(S)-hydroxy-2(R),7,7-trimethyl-8-[3(R)- oder 3(S)-ethoxycarbonylpiperidin-1-ylcarbonyl]-octansäure(N-butyl)amid;
5(S)-Amino-4(S)-hydroxy-2(R),7,7-trimethyl-9-(1,2,3,4-tetrahydrochinolin-1yl)nonansäure(N-butyl)amid;
5(S)-Amino-4(S)-hydroxy-2(R),7,7-trimethyl-8-[3(R,S)-carboxy-1,2,3,4-tetrahydrochinolin-1-ylcarbonyl]-octansäure(N-butyl)amid;
5(S)-Amino-4(S)-hydroxy-2(R),7,7-trimethyl-8-[2(R,S)-ethoxycarbonyl-3,4-dihydro-2H-1,4-benzoxazin-4-ylcarbonyl]-octansäure(N-butyl)amid;
5(S)-Formylamino-4(S)-hydroxy-2(R),7,7-trimethyl-8-[2(R,S)-ethoxycarbonyl-4-dihydro-2H-1,4-benzoxazin-4-ylcarbonyl]-octansäure(N-butyl)amid;
5(S)-Amino-4(S)-hydroxy-2(R),7,7-trimethyl-8-[2(R,S)-methoxycarbonyl-3,4-dihydro-2H-1,4-benzthiazin-4-ylcarbonyl]-octansäure(N-butyl)amid;
5(S)-Amino-4(S)-hydroxy-2(R),7,7-trimethyl-8-[3(R,S)-ethoxycarbonyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-tertiärbutoxycarbonyl-1,2,3,4-tetrahydrochinolin-1-ylcarbonyl]-octansäure(N-butyl)amid;
5(S)-Amino-4(S)-hydroxy-2(R),7,7-trimethyl-8-[3(R)-oder 3(S)-methoxycarbonyl-1,2,3,4-tetrahydrochinolin-1-ylcarbonyl]-octansäure(N-butyl)amid;
(S)-Amino-4(S)-hydroxy-2(R),7,7-trimethyl-8-[3(R)- oder 3(S)-methoxycarbonyl-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-dimethylaminocarbonyl-1,2,3,4-tetrahydrochinolin-1-ylcarbonyl]-octansäure(N-butyl)amid;
5(S)-Amino-4(S)-hydroxy-2(R),7,7-trimethyl-8-[2(R,S)-butylaminocarbonyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-butylaminocarbonyl-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-morpholinocarbonyl-1,2,3,4-tetrahydrochinolin-1-ylcarbonyl]-octansäure(N-butyl)amid;
5(S)-Amino-4(S)-hydroxy-2(R),7,7-trimethyl-8-[2(R,S)-methylaminocarbonyl-3,4-dihydro-2H-1,4-benzthiazin-4ylcarbonyl]-octansäure(N-butyl)amid;
5(S)-Amino-4(S)-hydroxy-2(R),7,7-trimethyl-8-[2(R,S)-methylaminocarbonyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-acetyl-1,2,3,4-tetrahydrochinolin-4-ylcarbonyl]-octansäure(N-butyl)amid;
5(S)-Amino-4(S)-hydroxy-2(R),7,7-trimethyl-8-[2(R,S)-glycinylcarbonyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-ethoxycarbonyl-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-isopropoxycarbonyl-1,2,3,4-tetrahydrochinolin-1-ylcarbonyl]-octansäure(N-butyl)amid;
5(S)-Amino-4(S)-hydroxy-2(R),7,7-trimethyl-8-(6-brom-1,2,3,4-tetrahydrochinolin-1-ylcarbonyl)-octansäure(N-butyl)amid;
5(S)-Amino-4(S)-hydroxy-2(R),7,7-trimethyl-8-[6-brom-3(R,S)-ethoxycarbonyl-1,2,3,4-tetrahydrochinolin-1-ylcarbonyl]-octansäure(N-butyl)amid;
5(S)-Amino-4(S)-hydroxy-7,7-dimethyl-8-[2(R,S)-methoxycarbonyl-3,4-dihydro-2H-1,4-benzthiazin-4-ylcarbonyl]-octansäure(N-butyl)amid;
5(S)-Amino-4(S)-hydroxy-2(R),7,7-trimethyl-8-[3,3-bis-(methoxycarbonyl)-1,2,3,4-tetrahydrochinolin-1-ylcarbonyl]-octansäure(N-butyl)amid;
5(S)-Amino-4(S)-hydroxy-2(R,S)-isopropyl-7,7-dimethyl-8-[2(R,S)-methoxycarbonyl-3,4-dihydro-2H-1,4-benzthiazin-4-ylcarbonyl]-octansäure(N-butyl)amid;
5(S)-Amino-4(S)-hydroxy-2(R,S)-isopropyl-7,7-dimethyl-8-[2(R,S)-methoxycarbonyl-3,4-dihydro-2H-1,4-benzoxazin-4ylcarbonyl]-octansäure(N-butyl)amid;
5(S)-Tertiärbutoxycarbonylamino-4(S)-hydroxy-2(S)-isopropyl-7,7dimethyl-8-[2(R,S)-methylaminocarbonyl-3,4-dihydro-2H-1,4-benzthiazin-4-ylcarbonyl]-octansäure(N-butyl)amid;
5(S)-Amino-4(S)-hydroxy-2(S)-isopropyl-7,7-dimethyl-8-[2(R,S)-methylaminocarbonyl-3,4-dihydro-2H-1,4-benzthiazin-4-yloctansäure(N-butyl)amid;
5(S)-Amino-4(S)-hydroxy-2(R,S)-isopropyl-7,7-dimethyl-8-(1,2,3,4-tetrahydrochinolin-1-ylcarbonyl)-octansäure(N-butyl)amid;
5(S)-Amino-4(S)-hydroxy-2(R,S)-isopropyl-7,7-dimethyl-8-(1,2,3,4-tetrahydrochinolin-1-ylcarbonyl)-octansäure(N-butyl)amid;
5(S)-Amino-4(S)-hydroxy-2(R,S)-isopropyl-7,7-dimethyl-8-[2(R,S)-carbamoyl-3,4-dihydro-2H-1,4-benzthiazin-4-ylcarbonyl]-octansäure(N-butyl)amid;
5(S)-Amino-4(S)-hydroxy-2(R),7,7-trimethyl-8-[2(R,S)-methoxycarbonyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-hydroxymethyl-1,2,3,4-tetrahydrochinolin-1ylcarbonyl]-octansäure(N-butyl)amid;
4(S)-Amino-3(S)-hydroxy-1(R),6,6-trimethyl-heptandicarbonsäure-1-(N-butyl)amid-7-[N-[2-(3-pyridyl)phenyl]]amid;
4(S)-Amino-3(S)-hydroxy-1(R),6,6-trimethyl-heptandicarbonsäure-1-(N-butyl)amid-7-[N-[2-(N-oxido3-pyridyl)phenyl]]amid;
5(S)-Formylamino-4(S)-hydroxy-2(R),7,7-trimethyl-8-[2(R,S)-methoxycarbonyl-3,4-dihydro-2H-1,4-benzthiazin-4-ylcarbonyl]-octansäure(N-butyl)amid;
5(S)-Amino-4(S)-hydroxy-2(R),7,7-trimethyl-8-(S,S-dioxo-3,4-dihydro-1H-2,4-benzthiazin-4-ylcarbonyl)-octansäure(N-butyl)amid;
5(S)-Amino-4(S)-hydroxy-2(R),7,7-trimethyl-8-(7-nitro-1,2,3,4-tetrahydrochinolin-1-ylcarbonyl)-octansäure(N-butyl)amid;
5(S)-Amino-4(S)-hydroxy-2(R),7,7-trimethyl-8-[3(R,S)-ethoxycarbonyl-7-nitro-1,2,3,4-tetrahydrochinolin-1-ylcarbonyl]-octansäure(N-butyl)amid;
5(S)-Amino-4(S)-hydroxy-2(R),7,7-trimethyl-8-[2(R,S)-ethoxycarbonyl-2(R,S)-methyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-methylsulfonyl-1,2,3,4-tetrahydrochinolin-1ylcarbonyl]-octansäure(N-butyl)amid;
5(S)-Amino-4(S)-hydroxy-2(R),7,7-trimethyl-8-[3-methylsulfonyl-1,2,3,4-tetrahydrochinazolin-1ylcarbonyl]-octansäure(N-butyl)amid;
5(S)-Amino-4(S)-hydroxy-2(R),7,7-trimethyl-8-[3(R,S)-methylcarbonylamino-1,2,3,4-tetrahydrochinolin-1ylcarbonyl]-octansäure(N-butyl)amid;
4(S)-Amino-3(S)-hydroxy-1(R),6,6-trimethyl-heptandicarbonsäure-1-(N-butyl)amid-7-[H-(2-carbamoylmethoxyphenyl)]amid;
4(S)-Amino-3(S)-hydroxy-1(R),6,6-trimethyl-heptandicarbonsäure-1-(N-butyl)amid-7-[N-(2-carbamoylmethoxy-4methoxyphenyl)]amid;
4(S)-Amino-3(S)-hydroxy-1(R),6,6-trimethyl-heptandicarbonsäure-1-(N-butyl)amid-7-[N-(2-carbamoylmethoxy5methoxyphenyl)]amid;
4(S)-Amino-3(S)-hydroxy-1(R),6,6-trimethyl-heptandicarbonsäure-1-(N-butyl)amid-7-[N-[2-carbamoylmethoxy-4-(methoxycarbonyl)phenyl]]amid;
4(S)-Amino-3(S)-hydroxy-1(R),6,6-trimethyl-heptandicarbonsäure-1-(N-butyl)amid-7-[N-[2-carbamoylmethoxy5-(methoxycarbonyl)phenyl]]amid;
5(S)-Amino-4(S)-hydroxy-2(S)-isopropyl-7,7-dimethyl-8-[3(R,S)-methylaminocarbonyl-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-methoxymethyl-1,2,3,4-tetrahydrochinolin-1-ylcarbonyl]-octansäure(N-butyl)amid;
5(S)-Formylamino-4(S)-hydroxy-2(R),7,7-trimethyl-8-[2(R,S)-methylaminocarbonyl-3,4-dihydro-2H1,4-benzoxazin-4-ylcarbonyl]-octansäure(N-butyl)amid;
5(S)-Amino-4(S)-hydroxy-2(R),7,7-trimethyl-8-(3-acetyl-2,3-dihydro-1H-benzimidazolylcarbonyl)-octansäure(N-butyl)amid;
5(S)-Amino-4(S)-hydroxy-2(R),7,7-trimethyl-8-[3(R,S)-ethylsulfonyl-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-methylcarbonyloxy-1,2,3,4-tetrahydrochinolin-1ylcarbonyl]-octansäure(N-butyl)amid;
5(S)-Amino-4(S)-hydroxy-2(R),7,7-trimethyl-8-[2(R,S)-methoxycarbonyl-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-methylaminocarbonyl-1,2,3,4-tetrahydrochinoxalin-1ylcarbonyl]-octansäure(N-butyl)amid;
5(S)-Amino-4(S)-hydroxy-2(R),7,7-trimethyl-8-[4-acetyl-3(R,S)-methylaminocarbonyl-1,2,3,4-tetrahydrochinoxalin-1ylcarbonyl]-octansäure(N-butyl)amid;
5(S)-Amino-4(S)-hydroxy-2(R),7,7-trimethyl-8-[3(R,S)-methylaminocarbonyl-1,2,3,4-tetrahydrochinolin-1ylcarbonyl]-octansäure(N-butyl)amid;
5(S)-Amino-4(S)-hydroxy-2(S)-isopropyl-7,7-dimethyl-8-[2(R,S)-methylaminocarbonyl-3,4-dihydro-2H-1,4-benzoxazin-4-ylcarbonyl[-octansäure(N-butyl)amid;
5(S)-Amino-4(S)-hydroxy-2(R),7,7-trimethyl-8-[2(R,S)-methoxyethylaminocarbonyl-3,4-dihydro-2H-1,4-benzoxazin-4-ylcarbonyl]-octansäure(N-butyl)amid;
5(S)-Amino-4(S)-hydroxy-2(R),7(S)-dimethyl-8-[2(R,S)-ethoxycarbonyl-3,4-dihydro-2H1,4-benzoxazin-4-ylcarbonyl]-octansäure(N-butyl)amid;
5(S)-Formylamino-4(S)-hydroxy-2(R),7,7-trimethyl-8-[2(R,S)-methylaminocarbonyl-3,4-dihydro-2H-1,4-benzthiazin-4-ylcarbonyl]-octansäure(N-butyl)amid;
5(S)-Amino-2(R)-benzyl-4(S)-hydroxy-7,7-dimethyl-8-[3(R)- oder-3(S)-methoxycarbonylamino-1,2,3,4-tetrahydrochinolin-1ylcarbonyl]-octansäure(N-butyl)amid;
5(S)-Amino-2(R)-butyl-4(S)-hydroxy-7,7-dimethyl-8-[3(R)- oder-3(S)-methoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-1,2,3,4-tetrahydrochinolin-1-ylcarbonyl]-octansäure(N-butyl)amid;
5(S)-Amino-4(S)-hydroxy-2(R),7,7-trimethyl-8-[3-ethoxycarbonyl-1,2,3,4-tetrahydrochinazolin-1-ylcarbonyl]-octansäure(N-butyl)amid;
5(S)-Amino-4(S)-hydroxy-2(R),7,7-trimethyl-8-[3(R,S)-phenylcarbonyloxy-1,2,3,4-tetrahydrochinolin-1-ylcarbonyl]-octansäure(N-butyl)amid;
5(S)-Amino-4(S)-hydroxy-2(R),7,7-trimethyl-8-[2(R,S)-cyano-3,4-dihydro-2H1,4-benzoxazin-4-ylcarbonyl]-octansäure(N-butyl)amid;
5(S)-Tertiärbutoxycarbonylamino-4(S)-hydroxy-2(R),7,7-trimethyl-8-[3(R,S)-methoxycarbonyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-methoxycarbonyl-3,4-dihydro-2H-1,4-benzoxazin-4-ylcarbonyl]-octansäure(N-butyl)amid;
5(S)-Amino-4(S)-hydroxy-2(R),7(R)dimethyl-8-[2(R,S)-ethoxycarbonyl-3,4-dihydro-2H1,4-benzoxazin-4-ylcarbonyl]-octansäure(N-butyl)amid;
5(S)-Amino-4(S)-hydroxy-2(R),7,7-trimethyl-8-[3(R,S)-methoxy-1,2,3,4-tetrahydrochinolin-1-ylcarbonyl]-octansäure(N-butyl)amid;
5(S)-Amino-4(S)-hydroxy-2(R),7,7-trimethyl-8-[2(R,S)-methoxymethyl-3,4-dihydro-2H1,4-benzthiazin-4-ylcarbonyl]-octansäure(N-butyl)amid;
5(S)-Amino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[2(R,S)-allyloxymethyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[2(R,S)-propyloxymethyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[2(R,S)-methoxymethyl-3,4-dihydro-2H-1,4-benzoxazin-4-ylcarbonyl]-octansäure(N-butyl)amid;
5(S)-Amino-4(S)-hydroxy-2(S)-isopropyl-7,7-dimethyl-8-[2(R)- oder 2(S)-methylaminocarbonyl-3,4-dihydro-2H1,4-benzthiazin-4-ylcarbonyl]-octansäure(N-butyl)amid;
5(S)-Amino-4(S)-hydroxy-2(S)-isopropyl-7,7-dimethyl-8-[2(R)- oder 2(S)-methylaminocarbonyl-3,4-dihydro-2H1,4-benzthiazin-4-ylcarbonyl]-octansäure(N-butyl)amid;
5(S)-Amino-4(S)-hydroxy6-[1-[3(R,S)-methylaminocarbonyl-1,2,3,4-tetrahydrochinolin-1-ylcarbonylmethyl]cyclopropyl]-2(R)-methyl-hexansäure(N-butyl)amid;
5(S)-Amino-4(S)-hydroxy-2(R),7,7-trimethyl-8-[3(R,S)-N,N-dimethylaminocarbonylamino-1,2,3,4-tetrahydrochinolin-1-ylcarbonyl]-octansäure(N-butyl)amid;
5(S)-Amino-4(S)-hydroxy-2(S)-isopropyl-7,7-dimethyl-8-[2(R,S)-ethylaminocarbonyl-3,4-dihydro-2H1,4-benzthiazin-4-ylcarbonyl]-octansäure(N-butyl)amid;
5(S)-Amino-4(S)-hydroxy-2(R),7,7-trimethyl-8-[2(R,S)-methoxyethylaminocarbonyl-3,4-dihydro-2H1,4-benzthiazin-4-ylcarbonyl]-octansäure(N-butyl)amid;
5(S)-Amino-4(S)-hydroxy-2(S)-isopropyl-7,7-dimethyl-8-[3(R,S)-methoxymethyl-1,2,3,4-tetrahydrochinolin-1ylcarbonyl]-octansäure(N-butyl)amid;
5(S)-Amino-4(S)-hydroxy-2(R),7,7-trimethyl-8-[3(R)- oder 3(S)-ethoxycarbonylindolin-1-ylcarbonyl]-octansäure(N-butyl)amid;
5(S)-Amino-4(S)-hydroxy-2(R),7,7-trimethyl-8-[3(R)- oder 3(S)-ethoxycarbonylindolin-1-ylcarbonyl]-octansäure(N-butyl)amid;
5(S)-Amino-4(S)-hydroxy-2(R),7,7-trimethyl-8-[3(R)- oder-3(S)-methylaminocarbonylamino-1,2,3,4-tetrahydrochinolin-1-ylcarbonyl]-octansäure(N-butyl)amid;
5(S)-Amino-4(S)-hydroxy-2(R),7,7-trimethyl-8-[3(R)- oder 3(S)-methylaminocarbonylamino-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-phenyl-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-methoxycarbonyl-4-oxo1,2,3,4-tetrahydrochinolin-1-ylcarbonyl]-octansäure(N-butyl)amid;
5(S)-Amino-4(S)-hydroxy-2(R),7,7-trimethyl-8-[3(R,S)allylaminocarbonyl-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-methoxycarbonylaminomethyl-1,2,3,4-tetrahydrochinolin-1ylcarbonyl]-octansäure(N-butyl)amid;
4(S)-Hydroxy-2(S)-isopropyl5(S)-methansulfonylamino-7,7-dimethyl-8-[2(R)- oder 2(S)-methylaminocarbonyl-3,4-dihydro-2H-1,4-benzthiazin-4-ylcarbonyl]-octansäure(N-butyl)amid;
5(S)-Amino-4(S)-hydroxy-2(R),7,7-trimethyl-8-[3(R,S)-N,N-dimethylaminomethyl-1,2,3,4-tetrahydrochinolin-1-ylcarbonyl]-octansäure(N-butyl)amid;
5(S)-Amino-4(S)-hydroxy-2(R),7,7-trimethyl-8-[2(R,S)-methoxyiminomethyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R,S)-carbamoyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3(R)-oder 3(S)-ethoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R)- oder 3(S)-ethoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R)- oder 3(S)-propyloxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R)- oder 3(S)propyloxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[2(R,S)-(dioxolan2-yl)-3,4-dihydro-2H-1,4-benzoxazin-4-ylcarbonyl]-octansäure(N-butyl)amid;
5(S)-Amino-4(S)-hydroxy-2,2,7,7-tetramethyl-8-[3(R)- oder 3(S)-methoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-(2-oxo-oxazolidin-3-yl)-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-methoxymethoxymethyl-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-allyloxymethyl-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-methoxyethoxymethyl-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-(2-oxopyrrolidin-1-yl)-1,2,3,4-tetrahydrochinolin-1-ylcarbonyl]-octansäure(N-butyl)amid;
5(S)-Amino-4(S)-hydroxy6-[1-[2(R)- oder 2(S)-methylaminocarbonyl-3,4-dihydro-2H-1,4-benzthiazin-4-ylcarbonylmethyl[cyclopropyl]-2(R)-methyl-hexansäure(N-butyl)amid;
5(S)-Amino-4(S)-hydroxy6-[1-[3(R)- oder 3(S)-methoxycarbonylamino-1,2,3,4-tetrahydrochinolin-1-ylcarbonylmethyl]cyclopropyl]-2(R)-methyl-hexansäure(N-butyl)amid;
5(S)-Amino-4(S)-hydroxy6-[1-[3(R)- oder 3(S)-methoxycarbonylamino-1,2,3,4-tetrahydrochinolin-1-ylcarbonylmethyl]cyclopropyl]-2-(R)-methyl-hexansäure(N-butyl)amid;
5(S)-Amino-4(S)-hydroxy-2(R),7,7-trimethyl-8-[3(R,S)-methoxyethoxy-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-propyloxy-1,2,3,4-tetrahydrochinolin-1ylcarbonyl]-octansäure(N-butyl)amid;
5(S)-Amino-4(S)-hydroxy-2(S)-isopropyl-7,7-dimethyl-8-[3(R)- oder 3(S)-methoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-methoxymethyloxy-1,2,3,4-tetrahydrochinolin-1ylcarbonyl]-octansäure(N-butyl)amid;
5(S)-Amino-4(S)-hydroxy-2(R),7,7-trimethyl-8-[3(R,S)cyclopropylcarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R)- oder 3(S)-allyloxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R)- oder 3(S)-allyloxycarbonylamino-1,2,3,4-tetrahydrochinolin-1ylcarbonyl]-octansäure(N-butyl)amid;
4(S)-Amino-3(S)-hydroxy-1(R),6,6-trimethyl-heptandicarbonsäure-1-(N-butyl)amid-7(N-phenyl,N-phenethyl)amid;
5(S)-Amino-4(S)-hydroxy-2(R),7,7-trimethyl-8-[3(R,S)-methylaminocarbonyloxy-1,2,3,4-tetrahydrochinolin-1-ylcarbonyl]-octansäure(N-butyl)amid;
5(S)-Amino-4(S)-hydroxy-2(R),7,7-trimethyl-8-[2(R)- oder-2(S)-methoxymethyloxymethyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[2(R)- oder 2(S)-methoxymethyloxymethyl-3,4-dihydro-2H-1,4-benzoxazin-4-ylcarbonyl]-octansäure(N-butyl)amid.;
5(S)-Amino-4(S)-hydroxy-2(R),7,7-trimethyl-8-[3(R,S)-(methylaminocarbonyl)indolin-1-ylcarbonyl]-octansäure(N-butyl)amid;
5(S)-Amino-4(S)-hydroxy-2(R),7,7-trimethyl-8-[3(R,S)-formylamino-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-ethylcarbonylamino-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-pentyl-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-methoxyethyl-1,2,3,4-tetrahydrochinolin-1ylcarbonyl]-octansäure(N-butyl)amid;
5(S)-Formylamino-4(S)-hydroxy-2(R),7,7-trimethyl-8-[3(R)- oder 3(S)-methoxycarbonylamino-1,2,3,4-tetrahydrochinolin-1ylcarbonyl]-octansäure(N-butyl)amid;
5(S)-Acetamido-4(S)-hydroxy-2(R),7,7-trimethyl-8-[3(R)- oder 3(S)-methyoxycarbonylamino-1,2,3,4-tetrahydrochinolin-1ylcarbonyl]-octansäure(N-butyl)amid;
5(S)-Amino-2(R)-ethyl-4(S)-hydroxy-7,7-dimethyl-8-[3(R)- oder-3(S)-methyoxycarbonylamino-1,2,3,4-tetrahydrochinolin-1-ylcarbonyl]-octansäure(N-butyl)amid;
5(S)-Amino-4(S)-hydroxy-2(R)-(2-methyl)propyl-7,7-dimethyl-8-[3(R)-oder 3(S)-methyoxycarbonylamino-1,2,3,4-tetrahydrochinolin-1-yl-carbonyl]-octansäure(N-butyl)amid;
5(S)-Amino-4(S)-hydroxy-2(R)-(2-methyl)propyl- 7,7-dimethyl-8-[3(R)-oder 3(S)-methyoxycarbonylamino-1,2,3,4-tetrahydrochinolin-1-ylcarbonyl]-octansäure(N-methyl)amid;
5(S)-Amino-2(R)-benzyl-4(S)-hydroxy-7,7-dimethyl-8- [3(R)- oder-3(S)-methyoxycarbonylamino-1,2,3,4-tetrahydrochinolin-1-ylcarbonyl]-octansäure(N-methyl)amid;
5(S)-Amino-4(S)-hydroxy-2(S)-isopropyl-7,7-dimethyl-8-[2(R)- oder-2(S)-methoxymethoxymethyl-3,4-dihydro-2H-1,4-benzoxazin-4-ylcarbonyl]-octansäure(N-butyl)amid;
5(S)Amino-4(S)-hydroxy-2(R),7,7-trimethyl-8-[3(R,S)-methoxypropyl-1,2,3,4-tetrahydrochinolin-1ylcarbonyl]-octansäure(N-butyl)amid;
5(S)-Amino-4(S)-hydroxy-2(R),7,7-trimethyl-8-[3(R,S)-methoxycarbonylamino-2,3,4,5-tetrahydro-1H-benz[6,7-b]azepin-1ylcarbonyl]-octansäure(N-butyl)amid;
5(S)-Amino-4(S)-hydroxy-2(R),7,7-trimethyl-8-[3(R,S)-methoxycarbonyl-(N-methyl)amino-1,2,3,4-tetrahydrochinolin-1ylcarbonyl]-octansäure(N-butyl)amid;
5(S)-Formylamino-4(S)-hydroxy-2(S)-isopropyl-7,7-dimethyl-8-[2(R)- oder-2(S)-methoxymethoxymethyl-3,4-dihydro-2H-1,4-benzoxazin-4-ylcarbonyl]-octansäure(N-butyl)amid;
5(S)-Amino-4(S)-hydroxy-2(S)-isopropyl-7,7-dimethyl-8-[3(R)- oder-3(S)-methoxycarbonylamino-1,2,3,4-tetrahydrochinolin-1ylcarbonyl]-octansäure(N-butyl)amid;
5(S)-Amino-4(S)-hydroxy-2(R),7,7-trimethyl-8-[2(R,S)-methoxyethyl-3,4-dihydro-2H-1,4-benzoxazin-4-ylcarbonyl]-octansäure(N-butyl)amid;
5(S)-Amino-4(S)-hydroxy-2(S)-isopropyl-7,7-dimethyl-8-[2(R)- oder-2(S)-methoxyethyl-3,4-dihydro-2H-1,4-benzoxazin-4-ylcarbonyl]-octansäure(N-butyl)amid;
5(S)-Amino-4(S)-hydroxy-2(R),7,7-trimethyl-8-[3-butyl-2,3dihydro-4(H)-quinazolinon-1-ylcarbonyl]-octansäure(N-butyl)amid;
5(S)-Amino-4(S)-hydroxy-2(R),7,7-trimethyl-8-[3(R,S)-(2-carbamoyl)ethyl-1,2,3,4-tetrahydrochinolin-1-ylcarbonyl]-octansäure(N-butyl)amid;
5(S)-Formylamino-4(S)-hydroxy-2(S)-isopropyl-7,7-dimethyl-8-[3(R)- oder 3(S)-methoxycarbonylamino-1,2,3,4-tetrahydrochinolin-1-ylcarbonyl]-octansäure(N-butyl)amid.;
5(S)-Amino-2(R)-but-2-enyl-4(S)-hydroxy-7,7-dimethyl-8-[3(R)- oder 3(S)-methoxycarbonylamino-1,2,3,4-tetrahydrochinolin-1-ylcarbonyl]-octansäure(N-butyl)amid;
5(S)-Amino-4(S)-hydroxy-2(R),7,7-trimethyl-8-[3(R,S)-methoxycarbonyl-1,2,3,4-tetrahydrochinolin-1-ylcarbonyl]-octansäure(N-butyl)amid;
4(S)-Amino-3(S)-hydroxy-1(R),6,6-trimethyl-heptandicarbonsäure-1-(N-butyl)amid-7-(N-naphth-1-yl)amid;
5(S)-Amino-4(S)-hydroxy-2(R),7,7-trimethyl-8-(1,2,3,4-tetrahydrochinolin-1-ylcarbonyl)octansäure(N-butyl)amid;
5(S)-Amino-4(S)-hydroxy-2(R),7,7-trimethyl-8-(2,3-dihydroindol1-ylcarbonyl)-octansäure(N-butyl)amid;
4(S)-Amino-3(S)-hydroxy-1(R),6,6-trimethyl-heptandicarbonsäure-1-(N-butyl)amid-7-(N-phenyl)amid oder
4(S)-Amino-3(S)-hydroxy-1(R),6,6-trimethyl-heptandicarbonsäure-1-(N-butyl)amid-7-{N-[2-(methoxycarbonylaminomethyl)phenyl]}amid gemäß Anspruch 2 oder jeweils ein Salz davon.

9. 15(S)-Amino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-morpholin-4-yl-ethyl)amid;
15(S)-Amino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-[2-(1-acetyl)-piperidin-4-yl)-ethyl]amid;
15(S)-Amino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-morpholin-4-yl-2-oxo-ethyl)amid oder
15(S)-Amino-4(S)-hydroxy-2(R),7,7-trimethyl-8-[3(R)- oder 3(S)-methoxycarbonylamino-1,2,3,4-tetrahydrochinolin-1-ylcarbonyl]-octansäure-N-(2-carbamoyl-2-methyl-propyl)amid
gemäß Anspruch 1 oder jeweils ein Salz davon.

10. Verbindungen gemäss einem der Ansprüche 1 bis 9 zur Anwendung in einem Verfahren zur therapeutischen Behandlung des menschlichen oder tierischen Körpers.

11. Pharmazeutische Präparate, neben üblichen pharmazeutischen Hilfsstoffen als pharmazeutischen Wirkstoff enthaltend eine Verbindung gemäss einem der Ansprüche 1, 3, 9 und 10 in freier Form oder in pharmazeutisch verwendbarer Salstorm.

12. Pharmazeutische Präparate, neben üblichen pharmazeutischen Hilfsstoffen als pharmazeutischen Wirkstoff enthaltend eine Verbindung gemäss einem der Ansprüche 2 und 4 bis 8 in freier Form oder in pharmazeutisch verwendbarer Salzform.

13. Verfahren zur Herstellung von Verbindungen der Formel I 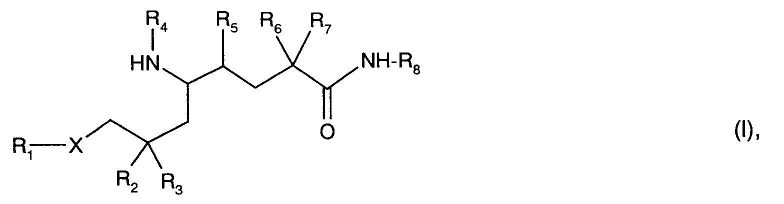
worin
R₁ Arylamino, N-Aryl-N-(niederalkoxyniederalkyl)-amino, N-Aryl-N-arylniederalkyl-amino oder über ein Ringstickstoffatom gebundenes Heterocyclyl bedeutet, X für eine Carbonyl- oder Methylengruppe steht,
R₂ und R₃ unabhängig voneinander Wasserstoff oder Niederalkyl bedeuten oder zusammen mit dem Kohlenstoffatom, mit dem sie verbunden sind, einen Cycloalkylidenrest darstellen,
R₄ Wasserstoff, Niederalkyl, Niederalkanoyl oder Niederalkoxycarbonyl ist,
R₅ Hydroxy, Niederalkanoyloxy oder Niederalkoxycarbonyloxy bedeutet,
R₆ Wasserstoff, Niederalkyl, Niederalkenyl, Niederalkinyl, Cycloalkyl, Cycloalkylniederalkyl, Arylniederalkyl oder Heteroarylniederalkyl mit 5 bis 7 Ringatomen im Heteroarylring und R₇ Wasserstoff oder Niederalkyl bedeutet oder
R₆ und R₇ zusammen mit dem Kohlenstoffatom, mit dem sie verbunden sind, einen Cycloalkylidenrest darstellen und
R₈ einen aliphatischen, cycloaliphatisch-aliphatischen oder heteroarylaliphatischen Rest bedeutet, nd ihrer Salze, dadurch gekennzeichnet, daß man
a) eine Verbindung der Formel II 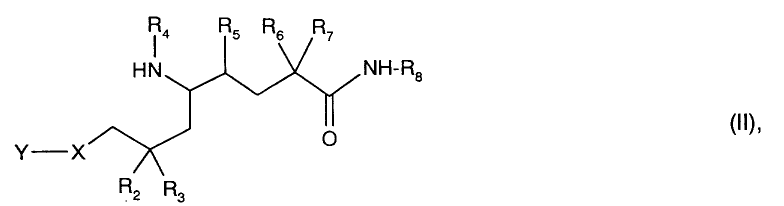 worin Y freies oder reaktionsfähiges funktionell abgewandeltes Hydroxy bedeutet und X, R₂, R₃, R₄, R₅, R₆, R₇ und R₈ die oben genannten Bedeutungen haben, oder ein Salz davon mit einer Verbindung der Formel R₁-H (III), worin R₁ die genannten Bedeutungen hat, unter Bildung einer Amin- bzw. Amidbildung kondensiert, wobei in den Reaktionskomponenten vorhandene freie funktionelle Gruppen mit Ausnahme der an der Reaktion teilnehmenden Gruppen in geschützter Form vorliegen, und vorhandene Schutzgruppen abspaltet oder
b) eine Carbonsäure der Formel IV 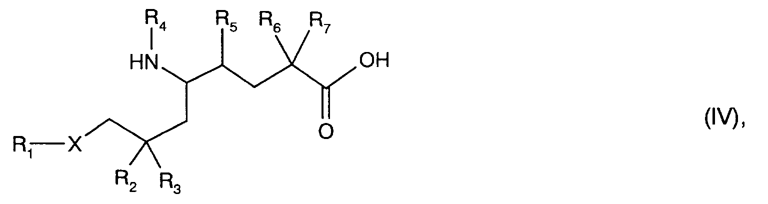 worin R₁, X, R₂, R₃, R₄, R₅, R₆ und R₇ die oben genannten Bedeutungen haben, oder ein Salz oder ein reaktionsfähiges Säurederivat davon mit einer Verbindung der Formel H-N(H)-R₈ (V) oder einem reaktionsfähigen Derivat davon mit einer reaktionsfähigen Aminogruppe, worin R₈ die genannten Bedeutungen hat unter Bildung einer Amidbildung kondensiert, wobei in den Reaktionskomponenten vorhandene freie funktionelle Gruppen mit Ausnahme der an der Reaktion teilnehmenden Gruppen in geschützter Form vorliegen, und vorhandene Schutzgruppen abspaltet oder
c) zur Herstellung einer Verbindung der Formel I, worin R₅ Hydroxy bedeutet und die übrigen Substituenten die genannten Bedeutungen haben, in einer Verbindung der Formel VI 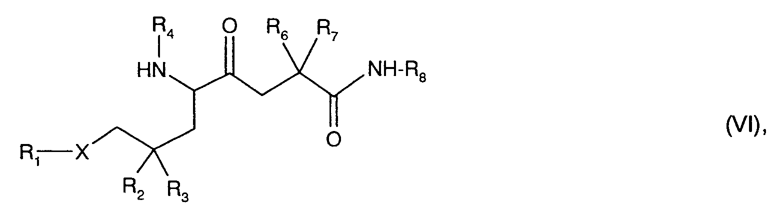 in der R₁, X, R₂, R₃, R₄, R₆, R₇ und R₈ die oben genannten Bedeutungen haben und freie funktionelle Gruppen mit Ausnahme der an der Reaktion teilnehmenden Ketogruppe in geschützter Form vorliegen, oder in einem Salz davon die Ketogruppe zu Hydroxymethylen reduziert und gegebenenfalls vorhandene Schutzgruppen abspaltet oder
d) zur Herstellung einer Verbindung der Formel I, worin R₆ Alkyl, Arylalkyl oder Heteroaralkyl, insbesondere Methyl, und R₇ Wasserstoff bedeutet und die übrigen Substituenten die genannten Bedeutungen haben, in einer Verbindung der Formel VII 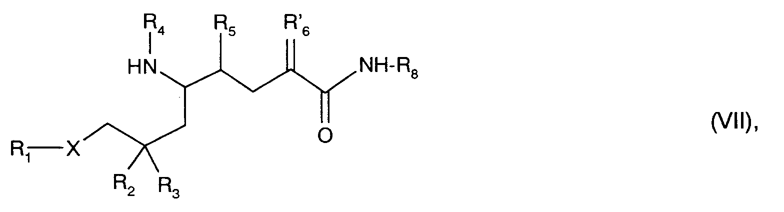 worin R'₆ Alkyliden, Arylalkyliden oder Heteroaralkyliden, insbesondere Methylen, bedeutet und R₁, X, R₂, R₃, R₄, R₅ und R₈ die genannten Bedeutungen haben, wobei freie funktionelle Gruppen gegebenenfalls in geschützter Form vorliegen, oder einem Salz davon die Gruppe R 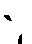 ₆ zur gewünschten Gruppe R₆ reduziert oder
e) zur Herstellung einer Verbindung der Formel I, in der R₄ Wasserstoff bedeutet und die übrigen Substituenten die genannten Bedeutungen haben, in einer Verbindung der Formel VIII 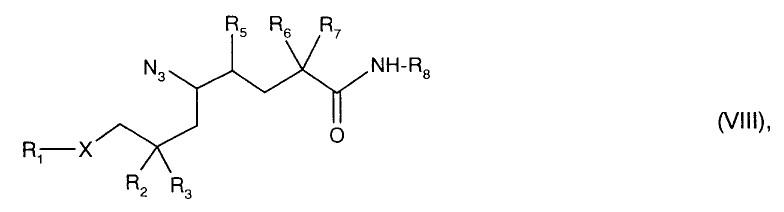 worin die Substituenten R₁, X, R₂, R₃, R₅, R₆, R₇ und R₈ die genannten Bedeutungen haben und freie funktionelle Gruppen gegebenenfalls in geschützter Form vorliegen, oder einem Salz davon die Azidogruppe zu Amino reduziert und vorhandene Schutzgruppen abspaltet und jeweils gewünschtenfalls eine nach einem der vorstehend genannten Verfahren a) bis e) erhältliche Verbindung der Formel I mit mindestens einer salzbildenden Gruppe in ihr Salz oder ein erhältliches Salz in die freie Verbindung oder in ein anderes Salz überführt und/oder gegebenenfalls erhältliche Isomerengemische auftrennt und/oder eine erfindungsgemäße Verbindung der Formel I in eine andere erfindungsgemäße Verbindung der Formel I umwandelt.
